(19) Europäisches Patentamt / European Patent Office / Office européen des brevets

(11) EP 3 137 501 B1

(12) **EUROPEAN PATENT SPECIFICATION**

(45) Date of publication and mention of the grant of the patent:
**29.09.2021 Bulletin 2021/39**

(21) Application number: **15720962.8**

(22) Date of filing: **02.05.2015**

(51) Int Cl.:
***C07K 16/28*** *(2006.01)*

(86) International application number:
**PCT/EP2015/059633**

(87) International publication number:
**WO 2015/166105 (05.11.2015 Gazette 2015/44)**

(54) **ION CHANNEL MODULATORS AND USES THEREOF**

IONENKANALMODULATOREN UND VERWENDUNGEN DAVON

MODULATEURS DES CANAUX IONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2014 US 201461987929 P**

(43) Date of publication of application:
**08.03.2017 Bulletin 2017/10**

(60) Divisional application:
**21191978.2**

(73) Proprietor: **Medimmune Limited**
**Cambridge, Cambridgeshire CB21 6GH (GB)**

(72) Inventors:
- **WILLIAMS, Wendy A**
  **Cambridge**
  **Cambridgeshire CB21 6GH (GB)**
- **JONES, Clare**
  **Cambridge**
  **Cambridgeshire CB21 6GH (GB)**
- **BUTTON, James**
  **Cambridge**
  **Cambridgeshire CB21 6GH (GB)**
- **LINLEY, John**
  **Cambridge**
  **Cambridgeshire CB21 6GH (GB)**
- **HUANG, Ling**
  **Cambridge**
  **Cambridgeshire CB21 6GH (GB)**
- **SNIJDER, Harm Jan**
  **431 83 Molndal (SE)**
- **SHIBATA, Yoko**
  **Cambridge**
  **Cambridgeshire CB21 6GH (GB)**
- **SRIDHARAN, Sudharsan**
  **Cambridge**
  **Cambridgeshire CB21 6GH (GB)**
- **GROVES, Maria**
  **Cambridge**
  **Cambridgeshire CB21 6GH (GB)**
- **DOBSON, Claire**
  **Cambridge**
  **Cambridgeshire CB21 6GH (GB)**

(74) Representative: **AstraZeneca Intellectual Property**
**Milstein Building**
**Granta Park**
**Cambridge CB21 6GH (GB)**

(56) References cited:

| | |
|---|---|
| **WO-A1-02/48395** | **WO-A1-02/48395** |
| **WO-A1-02/48395** | **US-A1- 2005 074 819** |
| **US-A1- 2005 074 819** | **US-A1- 2005 074 819** |
| **US-A1- 2008 287 467** | **US-A1- 2008 287 467** |
| **US-A1- 2008 287 467** | **US-B1- 6 242 216** |
| **US-B1- 6 242 216** | **US-B1- 6 242 216** |

- **M. T. YOUNG ET AL: "Molecular Shape, Architecture, and Size of P2X4 Receptors Determined Using Fluorescence Resonance Energy Transfer and Electron Microscopy", JOURNAL OF BIOLOGICAL CHEMISTRY, vol. 283, no. 38, 19 September 2008 (2008-09-19), pages 26241-26251, XP055205467, ISSN: 0021-9258, DOI: 10.1074/jbc.M804458200**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 137 501 B1

Printed by Jouve, 75001 PARIS (FR)

**(Cont. next page)**

- **TSUDA MAKOTO ET AL: "P2X4 receptors induced in spinal microglia gate tactile allodynia after nerve injury", NATURE, MACMILLAN JOURNALS LTD., ETC, GB, vol. 424, no. 6950, 14 August 2003 (2003-08-14), pages 778-783, XP002376086, ISSN: 0028-0836, DOI: 10.1038/NATURE01786**
- **E. TOULME ET AL: "P2X4 receptors in activated C8-B4 cells of cerebellar microglial origin", THE JOURNAL OF GENERAL PHYSIOLOGY, vol. 4, no. 1, 1 April 2010 (2010-04-01), pages 4.19-353, XP055142775, ISSN: 1934-2616, DOI: 10.1006/bbrc.1996.0380**
- **GARCIA-GUZMAN M ET AL: "CHARACTERIZATION OF RECOMBINANT HUMAN P2X4 RECEPTOR REVEALS PHARMACOLOGICAL DIFFERENCES TO THE RAT HOMOLOGUE", MOLECULAR PHARMACOLOGY, AMERICAN SOCIETY FOR PHARMACOLOGY AND EXPERIMENTAL THERAPEUTICS, US, vol. 51, 1 January 1997 (1997-01-01), pages 109-118, XP002059398, ISSN: 0026-895X**
- **MARIA VALENTE ET AL: "Expression, purification, electron microscopy, N-glycosylation mutagenesis and molecular modeling of human P2X4 andP2XA", BIOCHIMICA ET BIOPHYSICA ACTA (BBA) - BIOMEMBRANES, ELSEVIER, AMSTERDAM, NL, vol. 1808, no. 12, 19 August 2011 (2011-08-19), pages 2859-2866, XP028316556, ISSN: 0005-2736, DOI: 10.1016/J.BBAMEM.2011.08.025 [retrieved on 2011-08-26]**
- **REBECCA J GUM ET AL: "P2X receptor antagonists for pain management: examination of binding and physicochemical properties", PURINERGIC SIGNALLING, KLUWER ACADEMIC PUBLISHERS, DO, vol. 8, no. 1, 16 November 2011 (2011-11-16), pages 41-56, XP035006784, ISSN: 1573-9546, DOI: 10.1007/S11302-011-9272-5**
- **M. T. YOUNG ET AL: "Molecular Shape, Architecture, and Size of P2X4 Receptors Determined Using Fluorescence Resonance Energy Transfer and Electron Microscopy", JOURNAL OF BIOLOGICAL CHEMISTRY, vol. 283, no. 38, 19 September 2008 (2008-09-19), pages 26241-26251, XP055205467, ISSN: 0021-9258, DOI: 10.1074/jbc.M804458200**
- **TSUDA MAKOTO ET AL: "P2X4 receptors induced in spinal microglia gate tactile allodynia after nerve injury", NATURE, NATURE PUBLISHING GROUP, UNITED KINGDOM, vol. 424, no. 6950, 14 August 2003 (2003-08-14), pages 778-783, XP002376086, ISSN: 0028-0836, DOI: 10.1038/NATURE01786**
- **E. TOULME ET AL: "P2X4 receptors in activated C8-B4 cells of cerebellar microglial origin", THE JOURNAL OF GENERAL PHYSIOLOGY, vol. 4, no. 1, 1 April 2010 (2010-04-01), pages 4.19-353, XP055142775, ISSN: 1934-2616, DOI: 10.1006/bbrc.1996.0380**
- **GARCIA-GUZMAN M ET AL: "CHARACTERIZATION OF RECOMBINANT HUMAN P2X4 RECEPTOR REVEALS PHARMACOLOGICAL DIFFERENCES TO THE RAT HOMOLOGUE", MOLECULAR PHARMACOLOGY, AMERICAN SOCIETY FOR PHARMACOLOGY AND EXPERIMENTAL THERAPEUTICS, US, vol. 51, 1 January 1997 (1997-01-01), pages 109-118, XP002059398, ISSN: 0026-895X**
- **REBECCA J GUM ET AL: "P2X receptor antagonists for pain management: examination of binding and physicochemical properties", PURINERGIC SIGNALLING, KLUWER ACADEMIC PUBLISHERS, DO, vol. 8, no. 1, 16 November 2011 (2011-11-16), pages 41-56, XP035006784, ISSN: 1573-9546, DOI: 10.1007/S11302-011-9272-5**
- **MARIA VALENTE ET AL: "Expression, purification, electron microscopy, N-glycosylation mutagenesis and molecular modeling of human P2X4 andP2XA", BIOCHIMICA ET BIOPHYSICA ACTA (BBA) - BIOMEMBRANES, ELSEVIER, AMSTERDAM, NL, vol. 1808, no. 12, 19 August 2011 (2011-08-19), pages 2859-2866, XP028316556, ISSN: 0005-2736, DOI: 10.1016/J.BBAMEM.2011.08.025 [retrieved on 2011-08-26]**
- **M. T. YOUNG ET AL: "Molecular Shape, Architecture, and Size of P2X4 Receptors Determined Using Fluorescence Resonance Energy Transfer and Electron Microscopy", JOURNAL OF BIOLOGICAL CHEMISTRY, vol. 283, no. 38, 19 September 2008 (2008-09-19), pages 26241-26251, XP055205467, ISSN: 0021-9258, DOI: 10.1074/jbc.M804458200**
- **TSUDA MAKOTO ET AL: "P2X4 receptors induced in spinal microglia gate tactile allodynia after nerve injury", NATURE, MACMILLAN JOURNALS LTD., ETC, GB, vol. 424, no. 6950, 14 August 2003 (2003-08-14), pages 778-783, XP002376086, ISSN: 0028-0836, DOI: 10.1038/NATURE01786**
- **E. TOULME ET AL: "P2X4 receptors in activated C8-B4 cells of cerebellar microglial origin", THE JOURNAL OF GENERAL PHYSIOLOGY, vol. 4, no. 1, 1 April 2010 (2010-04-01), pages 4.19-353, XP055142775, ISSN: 1934-2616, DOI: 10.1006/bbrc.1996.0380**

- GARCIA-GUZMAN M ET AL: "CHARACTERIZATION OF RECOMBINANT HUMAN P2X4 RECEPTOR REVEALS PHARMACOLOGICAL DIFFERENCES TO THE RAT HOMOLOGUE", MOLECULAR PHARMACOLOGY, AMERICAN SOCIETY FOR PHARMACOLOGY AND EXPERIMENTAL THERAPEUTICS, US, vol. 51, 1 January 1997 (1997-01-01), pages 109-118, XP002059398, ISSN: 0026-895X
- MARIA VALENTE ET AL: "Expression, purification, electron microscopy, N-glycosylation mutagenesis and molecular modeling of human P2X4 andP2XA", BIOCHIMICA ET BIOPHYSICA ACTA (BBA) - BIOMEMBRANES, ELSEVIER, AMSTERDAM, NL, vol. 1808, no. 12 19 August 2011 (2011-08-19), pages 2859-2866, XP028316556, ISSN: 0005-2736, DOI: 10.1016/J.BBAMEM.2011.08.025 [retrieved on 2011-08-26]
- REBECCA J GUM ET AL: "P2X receptor antagonists for pain management: examination of binding and physicochemical properties", PURINERGIC SIGNALLING, KLUWER ACADEMIC PUBLISHERS, DO, vol. 8, no. 1, 16 November 2011 (2011-11-16), pages 41-56, XP035006784, ISSN: 1573-9546, DOI: 10.1007/S11302-011-9272-5
- MATHIEU DONDELINGER ET AL: "Understanding the Significance and Implications of Antibody Numbering and Antigen-Binding Surface/Residue Definition", FRONTIERS IN IMMUNOLOGY, vol. 9, 16 October 2018 (2018-10-16), pages 1-15, XP055572450, DOI: 10.3389/fimmu.2018.02278
- RUDIKOFF S ET AL: "Single amino acid substitution altering antigen-binding specificity", PROCEEDINGS OF THE NATIONAL ACADEMY OF SCIENCES, NATIONAL ACADEMY OF SCIENCES, vol. 79, 1 March 1982 (1982-03-01), pages 1979-1983, XP007901436, ISSN: 0027-8424, DOI: 10.1073/PNAS.79.6.1979

<110> Williams, Wendy
Jones, Clare
Button, James
Linley, John
Snijder, Arjan
Huang, Ling
Shibata, Yoko
Sridharan, Sudharsan
Dobson, Claire
Groves, Maria

<120> Ion Channel Modulators and Uses thereof

<140>
<141> 01-May-2015

<150> 61/987,929
<151> 02-May-2014

<160> 378

<170> Medimmune Ltd patent software March 2010 release. Output verified by USPTO Checker version 4.4.0.

<210> 1
<211> 387
<212> DNA
<213> Homo sapiens

<400> 1

```
gaggtgcagc tgttggagtc tgggggaggc ttggtacagc ctggggggtc cctgagactc      60
tcctgtgcag cctctggatt cacctttagc ggctatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgtgc gagagaagaa     300
cgagggagtt actttggttt tagtggttat tactacacat actactttga ctactggggc     360
cgagggacaa tggtcaccgt ctcgagt                                         387
```

<210> 2
<211> 129
<212> **PRT**
<213> Homo sapiens

<400> 2

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Gly Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
```

50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Glu Arg Gly Ser Tyr Phe Gly Phe Ser Gly Tyr Tyr Tyr
100 105 110

Thr Tyr Tyr Phe Asp Tyr Trp Gly Arg Gly Thr Met Val Thr Val Ser
115 120 125

Ser

<210> 3
<211> 5
<212> PRT
<213> Homo sapiens

<400> 3

Gly Tyr Ala Met Ser
5

<210> 4
<211> 17
<212> PRT
<213> Homo sapiens

<400> 4

Ala Ile Ser Gly Ser Gly Gly Ser Thr Tyr Tyr Ala Asp Ser Val Lys
5 10 15

Gly

<210> 5
<211> 20
<212> PRT
<213> Homo sapiens

<400> 5

Glu Glu Arg Gly Ser Tyr Phe Gly Phe Ser Gly Tyr Tyr Tyr Thr Tyr
5 10 15

Tyr Phe Asp Tyr
20

<210> 6
<211> 30
<212> PRT
<213> Homo sapiens

<400> 6

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser
20 25 30

<210> 7
<211> 14
<212> PRT
<213> Homo sapiens

<400> 7

Trp Val Arg Gln Ala Pro Gly Lys Gly Leu Glu Trp Val Ser
5 10

<210> 8
<211> 32
<212> PRT
<213> Homo sapiens

<400> 8

Arg Phe Thr Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ala Arg
20 25 30

<210> 9
<211> 11
<212> PRT
<213> Homo sapiens

<400> 9

Trp Gly Arg Gly Thr Met Val Thr Val Ser Ser
5 10

<210> 10
<211> 327
<212> DNA
<213> Homo sapiens

<400> 10

caggctgtgc tgactcagcc gtcctcagtg tctggggccc cagggcagag ggtcaccatc 60

tcctgcactg ggagcagctc caacatcggg gcaggttatg atgtacactg gtaccagcag 120

cttccaggaa cagcccccaa actcctcatc tacggtaaca acaatcggcc ctccggggtc 180

cctgaccgat tctctggctc caagtctggc acctcagcct ccctggccat cactgggctc 240

caggctgagg atgaagctga ttattactgc cagtcctatg acaccaacct gaaagttttt 300

ggcggaggga ccaagctgac cgtccta 327

<210> 11
<211> 109
<212> PRT
<213> Homo sapiens

<400> 11

```
Gln Ala Val Leu Thr Gln Pro Ser Ser Val Ser Gly Ala Pro Gly Gln
                5                   10                  15

Arg Val Thr Ile Ser Cys Thr Gly Ser Ser Ser Asn Ile Gly Ala Gly
            20                  25                  30

Tyr Asp Val His Trp Tyr Gln Gln Leu Pro Gly Thr Ala Pro Lys Leu
        35                  40                  45

Leu Ile Tyr Gly Asn Asn Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Thr Asn
                85                  90                  95

Leu Lys Val Phe Gly Gly Gly Thr Lys Leu Thr Val Leu
            100                 105
```

<210> 12
<211> 14
<212> PRT
<213> Homo sapiens

<400> 12

```
Thr Gly Ser Ser Ser Asn Ile Gly Ala Gly Tyr Asp Val His
                5                   10
```

<210> 13
<211> 7
<212> PRT
<213> Homo sapiens

<400> 13

```
Gly Asn Asn Asn Arg Pro Ser
                5
```

<210> 14
<211> 9
<212> PRT
<213> Homo sapiens

<400> 14

```
Gln Ser Tyr Asp Thr Asn Leu Lys Val
                5
```

<210> 15
<211> 22
<212> PRT
<213> Homo sapiens

<400> 15

```
Gln Ala Val Leu Thr Gln Pro Ser Ser Val Ser Gly Ala Pro Gly Gln
                5                   10                  15

Arg Val Thr Ile Ser Cys
            20
```

<210> 16
<211> 15
<212> PRT
<213> Homo sapiens

<400> 16

```
Trp Tyr Gln Gln Leu Pro Gly Thr Ala Pro Lys Leu Leu Ile Tyr
                5                   10                  15
```

<210> 17
<211> 32
<212> PRT
<213> Homo sapiens

<400> 17

```
Gly Val Pro Asp Arg Phe Ser Gly Ser Lys Ser Gly Thr Ser Ala Ser
                5                   10                  15

Leu Ala Ile Thr Gly Leu Gln Ala Glu Asp Glu Ala Asp Tyr Tyr Cys
            20                  25                  30
```

<210> 18
<211> 10
<212> PRT
<213> Homo sapiens

<400> 18

```
Phe Gly Gly Gly Thr Lys Leu Thr Val Leu
                5                   10
```

<210> 19
<211> 363
<212> DNA
<213> Homo sapiens

<400> 19

```
caggtgcagc tggtgcaatc tggagcagag gtgaaaaagc ccggggagtc tctgaagatc      60
tcctgtaagg gttctggata cagctttacc agctactgga tcggctgggt gcgccagatg     120
cccgggaaag gcctggagtg gatggggatc atctatcctg gtgactctga taccagatac     180
agcccgtcct tccaaggcca ggtcaccatc tcagccgaca agtccatcag caccgcctac     240
ctgcagtgga gcagcctgaa ggcctcggac accgccatgt attactgtgc gagccggact     300
ggggattact actactacgg tatggacgtc tggggcaaag ggacaatggt caccgtctcg     360
agt                                                                   363
```

<210> 20
<211> 121
<212> PRT
<213> Homo sapiens

<400> 20

```
Gln Val Gln Leu Val Gln Ser Gly Ala Glu Val Lys Lys Pro Gly Glu
                5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Ser Arg Thr Gly Asp Tyr Tyr Tyr Tyr Gly Met Asp Val Trp Gly
            100                 105                 110

Lys Gly Thr Met Val Thr Val Ser Ser
        115                 120
```

<210> 21
<211> 5
<212> PRT
<213> Homo sapiens

<400> 21

```
Ser Tyr Trp Ile Gly
                5
```

<210> 22
<211> 17
<212> PRT
<213> Homo sapiens

<400> 22

Ile Ile Tyr Pro Gly Asp Ser Asp Thr Arg Tyr Ser Pro Ser Phe Gln
5 10 15

Gly

<210> 23
<211> 12
<212> PRT
<213> Homo sapiens

<400> 23

Arg Thr Gly Asp Tyr Tyr Tyr Tyr Gly Met Asp Val
5 10

<210> 24
<211> 30
<212> PRT
<213> Homo sapiens

<400> 24

Gln Val Gln Leu Val Gln Ser Gly Ala Glu Val Lys Lys Pro Gly Glu

5 10 15

Ser Leu Lys Ile Ser Cys Lys Gly Ser Gly Tyr Ser Phe Thr
20 25 30

<210> 25
<211> 14
<212> PRT
<213> Homo sapiens

<400> 25

Trp Val Arg Gln Met Pro Gly Lys Gly Leu Glu Trp Met Gly
5 10

<210> 26
<211> 32
<212> PRT
<213> Homo sapiens

<400> 26

Gln Val Thr Ile Ser Ala Asp Lys Ser Ile Ser Thr Ala Tyr Leu Gln
5 10 15

Trp Ser Ser Leu Lys Ala Ser Asp Thr Ala Met Tyr Tyr Cys Ala Ser
20 25 30

<210> 27
<211> 11
<212> PRT

<213> Homo sapiens

<400> 27

```
Trp Gly Lys Gly Thr Met Val Thr Val Ser Ser
                5                   10
```

<210> 28
<211> 324
<212> DNA
<213> Homo sapiens

<400> 28

```
tcctatgtgc tgactcagcc accctcggtg tcagtgtccc taggacagac ggccaggatc      60
acctgctctg gagatgcatt gccaaagcaa tatgcttatt ggtaccagca gaagccaggc     120
caggcccctg tgctggtgat atataaagac agtgagaggc cctcagggat ccctgagcga     180
ttctctggct ccagctcagg gacaacagtc acgttgacca tcagtggagt ccaggcagaa     240
gacgaggctg actattactg tcaatcagca gacagcagtg gtacttatgt ggtattcggc     300
ggagggacca agctgaccgt ccta                                            324
```

<210> 29
<211> 108
<212> PRT
<213> Homo sapiens

<400> 29

```
Ser Tyr Val Leu Thr Gln Pro Pro Ser Val Ser Val Ser Leu Gly Gln
                5                   10                  15
Thr Ala Arg Ile Thr Cys Ser Gly Asp Ala Leu Pro Lys Gln Tyr Ala
            20                  25                  30
Tyr Trp Tyr Gln Gln Lys Pro Gly Gln Ala Pro Val Leu Val Ile Tyr
        35                  40                  45
Lys Asp Ser Glu Arg Pro Ser Gly Ile Pro Glu Arg Phe Ser Gly Ser
    50                  55                  60
Ser Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Val Val Phe Gly Gly Gly Thr Lys Leu Thr Val Leu
            100                 105
```

<210> 30
<211> 11
<212> PRT
<213> Homo sapiens

<400> 30

Ser Gly Asp Ala Leu Pro Lys Gln Tyr Ala Tyr
5 10

<210> 31
<211> 7
<212> PRT
<213> Homo sapiens

<400> 31

Lys Asp Ser Glu Arg Pro Ser
5

<210> 32
<211> 11
<212> PRT
<213> Homo sapiens

<400> 32

Gln Ser Ala Asp Ser Ser Gly Thr Tyr Val Val
5 10

<210> 33
<211> 22
<212> PRT
<213> Homo sapiens

<400> 33

Ser Tyr Val Leu Thr Gln Pro Pro Ser Val Ser Val Ser Leu Gly Gln
5 10 15

Thr Ala Arg Ile Thr Cys

20

<210> 34
<211> 15
<212> PRT
<213> Homo sapiens

<400> 34

Trp Tyr Gln Gln Lys Pro Gly Gln Ala Pro Val Leu Val Ile Tyr
5 10 15

<210> 35
<211> 32
<212> PRT
<213> Homo sapiens

<400> 35

```
Gly Ile Pro Glu Arg Phe Ser Gly Ser Ser Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 36
<211> 10
<212> PRT
<213> Homo sapiens

<400> 36

```
Phe Gly Gly Gly Thr Lys Leu Thr Val Leu
                5                   10
```

<210> 37
<211> 378
<212> DNA
<213> Homo sapiens

<400> 37

```
gaggtgcagc tggtgcagtc tgggggaggc ctggtacagc ctggcaggtc cctgagactc      60
tcctgtacag cctctggatt cacctttgat gattattcca tgcactgggt ccggcaagct     120
ccagggaagg gcctggagtg ggtctcaagt attagttgga gtagtggtag cataggctat     180
gcggactctg tgaagggccg attcaccatc tccagagaca acgccaagaa ctccttgtct     240
ctgcaaatga acagtctgag agttgaggac acggccttgt attactgtgt aaaggatcga     300
atgtattact atgatactgg tggatattat tctggttttg atatgtgggg ccaagggaca     360
atggtcaccg tctcgagt                                                   378
```

<210> 38
<211> 126
<212> PRT
<213> Homo sapiens

<400> 38

Glu Val Gln Leu Val Gln Ser Gly Gly Gly Leu Val Gln Pro Gly Arg
5 10 15

Ser Leu Arg Leu Ser Cys Thr Ala Ser Gly Phe Thr Phe Asp Asp Tyr
20 25 30

Ser Met His Trp Val Arg Gln Ala Pro Gly Lys Gly Leu Glu Trp Val
35 40 45

Ser Ser Ile Ser Trp Ser Ser Gly Ser Ile Gly Tyr Ala Asp Ser Val
50 55 60

Lys Gly Arg Phe Thr Ile Ser Arg Asp Asn Ala Lys Asn Ser Leu Ser
65 70 75 80

Leu Gln Met Asn Ser Leu Arg Val Glu Asp Thr Ala Leu Tyr Tyr Cys
85 90 95

Val Lys Asp Arg Met Tyr Tyr Tyr Asp Thr Gly Gly Tyr Tyr Ser Gly
100 105 110

Phe Asp Met Trp Gly Gln Gly Thr Met Val Thr Val Ser Ser
115 120 125

<210> 39
<211> 5
<212> PRT
<213> Homo sapiens

<400> 39

Asp Tyr Ser Met His
5

<210> 40
<211> 17
<212> PRT
<213> Homo sapiens

<400> 40

Ser Ile Ser Trp Ser Ser Gly Ser Ile Gly Tyr Ala Asp Ser Val Lys
5 10 15

Gly

<210> 41
<211> 17
<212> PRT
<213> Homo sapiens

<400> 41

Asp Arg Met Tyr Tyr Tyr Asp Thr Gly Gly Tyr Tyr Ser Gly Phe Asp
5 10 15

Met

<210> 42

<211> 30
<212> PRT
<213> Homo sapiens

<400> 42

```
Glu Val Gln Leu Val Gln Ser Gly Gly Gly Leu Val Gln Pro Gly Arg
                5                   10                  15

Ser Leu Arg Leu Ser Cys Thr Ala Ser Gly Phe Thr Phe Asp
            20                  25                  30
```

<210> 43
<211> 14
<212> PRT
<213> Homo sapiens

<400> 43

```
    Trp Val Arg Gln Ala Pro Gly Lys Gly Leu Glu Trp Val Ser
                    5                   10
```

<210> 44
<211> 32
<212> PRT
<213> Homo sapiens

<400> 44

```
Arg Phe Thr Ile Ser Arg Asp Asn Ala Lys Asn Ser Leu Ser Leu Gln
                5                   10                  15

Met Asn Ser Leu Arg Val Glu Asp Thr Ala Leu Tyr Tyr Cys Val Lys
            20                  25                  30
```

<210> 45
<211> 11
<212> PRT
<213> Homo sapiens

<400> 45

```
          Trp Gly Gln Gly Thr Met Val Thr Val Ser Ser
                          5                   10
```

<210> 46
<211> 318
<212> DNA
<213> Homo sapiens

<400> 46

```
tcctatgagc tgactcagcc accctcagtg tccgtgtccc caggacagac agccaccatc      60
acctgctctg gagataaatt ggatgataaa tatatatctt ggtatcaaag gaagccaggc     120
cagtcccctg tcctgctcat ctatcaagat atagagcggc cctcagggat ccctgaccga     180
ttctctggct ctaattctgg gaacacagcc actctgtcca tcagcgggac ccagtctatg     240
gatgaggctg agtattactg tcaggcgtgg gacaacggtg ctattgtatt cggcggaggg     300
accaagctga ccgtccta                                                    318
```

<210> 47
<211> 106
<212> PRT
<213> Homo sapiens

<400> 47

```
Ser Tyr Glu Leu Thr Gln Pro Pro Ser Val Ser Val Ser Pro Gly Gln
                5                   10                  15

Thr Ala Thr Ile Thr Cys Ser Gly Asp Lys Leu Asp Asp Lys Tyr Ile
            20                  25                  30

Ser Trp Tyr Gln Arg Lys Pro Gly Gln Ser Pro Val Leu Leu Ile Tyr
        35                  40                  45

Gln Asp Ile Glu Arg Pro Ser Gly Ile Pro Asp Arg Phe Ser Gly Ser
    50                  55                  60

Asn Ser Gly Asn Thr Ala Thr Leu Ser Ile Ser Gly Thr Gln Ser Met
65                  70                  75                  80

Asp Glu Ala Glu Tyr Tyr Cys Gln Ala Trp Asp Ser Gly Ala Ile Val
                85                  90                  95

Phe Gly Gly Gly Thr Lys Leu Thr Val Leu
            100                 105
```

<210> 48
<211> 11
<212> PRT
<213> Homo sapiens

<400> 48

```
Ser Gly Asp Lys Leu Asp Asp Lys Tyr Ile Ser
                5                   10
```

<210> 49
<211> 7
<212> PRT
<213> Homo sapiens

<400> 49

```
Gln Asp Ile Glu Arg Pro Ser
                5
```

<210> 50
<211> 9
<212> PRT
<213> Homo sapiens

<400> 50

Gln Ala Trp Asp Ser Gly Ala Ile Val
5

<210> 51
<211> 22
<212> PRT
<213> Homo sapiens

<400> 51

Ser Tyr Glu Leu Thr Gln Pro Pro Ser Val Ser Val Ser Pro Gly Gln
5 10 15

Thr Ala Thr Ile Thr Cys
20

<210> 52
<211> 15
<212> PRT
<213> Homo sapiens

<400> 52

Trp Tyr Gln Arg Lys Pro Gly Gln Ser Pro Val Leu Leu Ile Tyr
5 10 15

<210> 53
<211> 32
<212> PRT
<213> Homo sapiens

<400> 53

Gly Ile Pro Asp Arg Phe Ser Gly Ser Asn Ser Gly Asn Thr Ala Thr
5 10 15

Leu Ser Ile Ser Gly Thr Gln Ser Met Asp Glu Ala Glu Tyr Tyr Cys
20 25 30

<210> 54
<211> 10
<212> PRT
<213> Homo sapiens

<400> 54

Phe Gly Gly Gly Thr Lys Leu Thr Val Leu
5 10

<210> 55
<211> 375

<212> DNA
<213> Homo sapiens

<400> 55

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgtgc gagagatagg     300
gatttttgga gtacttattc gggtccaact gcttttgatc tctggggcag aggcaccctg     360
gtcaccgtct cgagt                                                      375
```

<210> 56
<211> 125
<212> PRT
<213> Homo sapiens

<400> 56

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 57
<211> 5
<212> PRT
<213> Homo sapiens

<400> 57

```
Ser Tyr Ala Met Ser
                5
```

<210> 58
<211> 17
<212> PRT
<213> Homo sapiens

<400> 58

```
Ala Ile Ser Gly Ser Gly Gly Ser Thr Tyr Tyr Ala Asp Ser Val Lys
                5                   10                  15

Gly
```

<210> 59
<211> 16
<212> PRT
<213> Homo sapiens

<400> 59

```
Asp Arg Asp Phe Trp Ser Thr Tyr Ser Gly Pro Thr Ala Phe Asp Leu
                5                   10                  15
```

<210> 60
<211> 30
<212> PRT
<213> Homo sapiens

<400> 60

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 61
<211> 14
<212> PRT
<213> Homo sapiens

<400> 61

```
    Trp Val Arg Gln Ala Pro Gly Lys Gly Leu Glu Trp Val Ser
                    5                   10
```

<210> 62
<211> 32
<212> PRT
<213> Homo sapiens

<400> 62

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 63
<211> 11
<212> PRT
<213> Homo sapiens

<400> 63

```
Trp Gly Arg Gly Thr Leu Val Thr Val Ser Ser
                1               5                   10
```

<210> 64
<211> 324
<212> DNA
<213> Homo sapiens

<400> 64

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 65
<211> 108
<212> PRT
<213> Homo sapiens

<400> 65

```
Ser Tyr Glu Leu Ala Gln Pro Pro Ser Val Ser Val Ser Pro Gly Gln
1               5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 66
<211> 11

<212> PRT
<213> Homo sapiens

<400> 66

Ser Gly Asp Ala Leu Pro Arg Gln Tyr Ala Tyr
5 10

<210> 67
<211> 7
<212> PRT
<213> Homo sapiens

<400> 67

Lys Asp Ser Glu Arg Pro Ser
5

<210> 68
<211> 11
<212> PRT
<213> Homo sapiens

<400> 68

Gln Ser Ala Asp Ser Ser Gly Thr Tyr Val Val
5 10

<210> 69
<211> 22
<212> PRT
<213> Homo sapiens

<400> 69

Ser Tyr Glu Leu Ala Gln Pro Pro Ser Val Ser Val Ser Pro Gly Gln
5 10 15

Thr Ala Arg Ile Thr Cys
20

<210> 70
<211> 15
<212> PRT
<213> Homo sapiens

<400> 70

Trp Tyr Gln Gln Lys Pro Gly Gln Ala Pro Leu Leu Val Ile Tyr
5 10 15

<210> 71
<211> 32
<212> PRT
<213> Homo sapiens

<400> 71

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 72
<211> 10
<212> PRT
<213> Homo sapiens

<400> 72

```
Phe Gly Gly Gly Thr Lys Val Thr Val Leu
                5                   10
```

<210> 73
<211> 363
<212> DNA
<213> Homo sapiens

<400> 73

```
gaggtgcagc tggtggagtc cgggggaggc ttggtccagc ctgggggtc cctgagactc      60
tcctgtgcag cctctggatt tacctttagt aggtattgga tgagctgggt ccgccaggct     120
ccagggaagg ggctggagtg ggtggccaac ataaaggaag atggaagtga gaaaaactat     180
gtggactctg tgaagggccg actcaccatc tccagagaca acgccaagaa ctcactgtat     240
ctgcaaatga acagcctgag agccgaggac acggccgtgt attactgtgc gaactactat     300
gatagtagtg gttattatgc ccttgatagc tggggccgag gcaccctggt cactgtctcc     360
tca                                                                   363
```

<210> 74
<211> 121
<212> PRT
<213> Homo sapiens

<400> 74

Glu Val Gln Leu Val Glu Ser Gly Gly Gly Leu Val Gln Pro Gly Gly
5 10 15

Ser Leu Arg Leu Ser Cys Ala Ala Ser Gly Phe Thr Phe Ser Arg Tyr
20 25 30

Trp Met Ser Trp Val Arg Gln Ala Pro Gly Lys Gly Leu Glu Trp Val
35 40 45

Ala Asn Ile Lys Glu Asp Gly Ser Glu Lys Asn Tyr Val Asp Ser Val
50 55 60

Lys Gly Arg Leu Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Ala Asn Tyr Tyr Asp Ser Ser Gly Tyr Tyr Ala Leu Asp Ser Trp Gly
100 105 110

Arg Gly Thr Leu Val Thr Val Ser Ser
115 120

<210> 75
<211> 5
<212> PRT
<213> Homo sapiens

<400> 75

Arg Tyr Trp Met Ser
5

<210> 76
<211> 17
<212> PRT
<213> Homo sapiens

<400> 76

Asn Ile Lys Glu Asp Gly Ser Glu Lys Asn Tyr Val Asp Ser Val Lys
5 10 15

Gly

<210> 77
<211> 12
<212> PRT
<213> Homo sapiens

<400> 77

Tyr Tyr Asp Ser Ser Gly Tyr Tyr Ala Leu Asp Ser
5 10

<210> 78
<211> 30
<212> PRT

<213> Homo sapiens

<400> 78

```
Glu Val Gln Leu Val Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 79
<211> 14
<212> PRT
<213> Homo sapiens

<400> 79

```
Trp Val Arg Gln Ala Pro Gly Lys Gly Leu Glu Trp Val Ala
                5                   10
```

<210> 80
<211> 32
<212> PRT
<213> Homo sapiens

<400> 80

```
Arg Leu Thr Ile Ser Arg Asp Asn Ala Lys Asn Ser Leu Tyr Leu Gln
                5                   10                  15

Met Asn Ser Leu Arg Ala Glu Asp Thr Ala Val Tyr Tyr Cys Ala Asn
            20                  25                  30
```

<210> 81
<211> 11
<212> PRT
<213> Homo sapiens

<400> 81

```
Trp Gly Arg Gly Thr Leu Val Thr Val Ser Ser
                5                   10
```

<210> 82
<211> 324
<212> DNA
<213> Homo sapiens

<400> 82

```
tcctatgagc tgactcagcc accctcagtg tccgtgtccc caggacagac agccaccatc      60

acctgctctg gagatgcatt gacaaaacaa tatgcttttt ggtaccaaca gaagccaggc     120

caggccccta tattggtgat ctttagagac tctgagaggc cctcagggat ccctgagcga     180

ttctctggct ccagctcagg gacaacagcg acgttgacca tcagtggggt ccaggcagga     240

gacgaggctg actattactg tcaatctaca gacaatactg cgacctccgt cgtcttcggc     300

ggagggacca aggtcaccgt ccta                                            324
```

<210> 83
<211> 108
<212> PRT
<213> Homo sapiens

<400> 83

```
Ser Tyr Glu Leu Thr Gln Pro Pro Ser Val Ser Val Ser Pro Gly Gln
                5                   10                  15

Thr Ala Thr Ile Thr Cys Ser Gly Asp Ala Leu Thr Lys Gln Tyr Ala
            20                  25                  30

Phe Trp Tyr Gln Gln Lys Pro Gly Gln Ala Pro Ile Leu Val Ile Phe
        35                  40                  45

Arg Asp Ser Glu Arg Pro Ser Gly Ile Pro Glu Arg Phe Ser Gly Ser
    50                  55                  60

Ser Ser Gly Thr Thr Ala Thr Leu Thr Ile Ser Gly Val Gln Ala Gly
65                  70                  75                  80

Asp Glu Ala Asp Tyr Tyr Cys Gln Ser Thr Asp Asn Thr Ala Thr Ser
                85                  90                  95

Val Val Phe Gly Gly Gly Thr Lys Val Thr Val Leu
            100                 105
```

<210> 84
<211> 11
<212> PRT
<213> Homo sapiens

<400> 84

```
Ser Gly Asp Ala Leu Thr Lys Gln Tyr Ala Phe
                5                   10
```

<210> 85
<211> 7
<212> PRT
<213> Homo sapiens

<400> 85

Arg Asp Ser Glu Arg Pro Ser
5

<210> 86
<211> 11
<212> PRT
<213> Homo sapiens

<400> 86

Gln Ser Thr Asp Asn Thr Ala Thr Ser Val Val
5 10

<210> 87
<211> 22
<212> PRT
<213> Homo sapiens

<400> 87

Ser Tyr Glu Leu Thr Gln Pro Pro Ser Val Ser Val Ser Pro Gly Gln
5 10 15

Thr Ala Thr Ile Thr Cys
20

<210> 88
<211> 15
<212> PRT
<213> Homo sapiens

<400> 88

Trp Tyr Gln Gln Lys Pro Gly Gln Ala Pro Ile Leu Val Ile Phe
5 10 15

<210> 89
<211> 32
<212> PRT
<213> Homo sapiens

<400> 89

Gly Ile Pro Glu Arg Phe Ser Gly Ser Ser Ser Gly Thr Thr Ala Thr
5 10 15

Leu Thr Ile Ser Gly Val Gln Ala Gly Asp Glu Ala Asp Tyr Tyr Cys
20 25 30

<210> 90
<211> 10
<212> PRT
<213> Homo sapiens

<400> 90

Phe Gly Gly Gly Thr Lys Val Thr Val Leu
5 10

<210> 91
<211> 375
<212> DNA
<213> Homo sapiens

<400> 91

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgtgc gagacccgag     300
ctccctgaga cagctatggt tagaaactgg cacttcgatc tctggggcca ggggacaatg     360

gtcaccgtct cgagt                                                      375
```

<210> 92
<211> 125
<212> PRT
<213> Homo sapiens

<400> 92

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Pro Glu Leu Pro Glu Thr Ala Met Val Arg Asn Trp His Phe
            100                 105                 110

Asp Leu Trp Gly Gln Gly Thr Met Val Thr Val Ser Ser
        115                 120                 125
```

<210> 93
<211> 5
<212> PRT
<213> Homo sapiens

<400> 93

Ser Tyr Ala Met Ser
5

<210> 94
<211> 17
<212> PRT
<213> Homo sapiens

<400> 94

Ala Ile Ser Gly Ser Gly Gly Ser Thr Tyr Tyr Ala Asp Ser Val Lys
5 10 15

Gly

<210> 95
<211> 16
<212> PRT
<213> Homo sapiens

<400> 95

Pro Glu Leu Pro Glu Thr Ala Met Val Arg Asn Trp His Phe Asp Leu
5 10 15

<210> 96
<211> 30
<212> PRT
<213> Homo sapiens

<400> 96

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 97
<211> 14
<212> PRT
<213> Homo sapiens

<400> 97

Trp Val Arg Gln Ala Pro Gly Lys Gly Leu Glu Trp Val Ser
5 10

<210> 98
<211> 32
<212> PRT
<213> Homo sapiens

<400> 98

Arg Phe Thr Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ala Arg
20 25 30

<210> 99
<211> 11
<212> PRT
<213> Homo sapiens

<400> 99

Trp Gly Gln Gly Thr Met Val Thr Val Ser Ser
5 10

<210> 100
<211> 324
<212> DNA
<213> Homo sapiens

<400> 100

tcctatgtgc tgactcagcc accctcggtg tcagtgtccc caggacagac ggccaggatc 60

acctgctctg gagatgcatt gccaaagcaa tatgcttatt ggtaccagca gaagccaggc 120

caggcccctg tgctggtgat atataaagac agtgagaggc cctcagggat ccctgagcga 180

ttctctggct ccagctcagg gacaacagtc acgttgacca tcagtggagt ccaggcagaa 240

gatgaggctg actattactg tcaatcagca gacagcagtg gtacttatgt ggtattcggc 300

ggagggacca agctgaccgt ccta 324

<210> 101
<211> 108
<212> PRT
<213> Homo sapiens

<400> 101

Ser Tyr Val Leu Thr Gln Pro Pro Ser Val Ser Val Ser Pro Gly Gln
5 10 15

Thr Ala Arg Ile Thr Cys Ser Gly Asp Ala Leu Pro Lys Gln Tyr Ala
20 25 30

Tyr Trp Tyr Gln Gln Lys Pro Gly Gln Ala Pro Val Leu Val Ile Tyr
35 40 45

Lys Asp Ser Glu Arg Pro Ser Gly Ile Pro Glu Arg Phe Ser Gly Ser
50 55 60

Ser Ser Gly Thr Thr Val Thr Leu Thr Ile Ser Gly Val Gln Ala Glu
65 70 75 80

Asp Glu Ala Asp Tyr Tyr Cys Gln Ser Ala Asp Ser Ser Gly Thr Tyr
85 90 95

Val Val Phe Gly Gly Gly Thr Lys Leu Thr Val Leu
100 105

<210> 102
<211> 11
<212> PRT
<213> Homo sapiens

<400> 102

Ser Gly Asp Ala Leu Pro Lys Gln Tyr Ala Tyr
5 10

<210> 103
<211> 7
<212> PRT
<213> Homo sapiens

<400> 103

Lys Asp Ser Glu Arg Pro Ser
5

<210> 104
<211> 11
<212> PRT
<213> Homo sapiens

<400> 104

Gln Ser Ala Asp Ser Ser Gly Thr Tyr Val Val
5 10

<210> 105
<211> 22
<212> PRT
<213> Homo sapiens

<400> 105

Ser Tyr Val Leu Thr Gln Pro Pro Ser Val Ser Val Ser Pro Gly Gln
5 10 15

Thr Ala Arg Ile Thr Cys
20

<210> 106
<211> 15
<212> PRT
<213> Homo sapiens

<400> 106

Trp Tyr Gln Gln Lys Pro Gly Gln Ala Pro Val Leu Val Ile Tyr
5 10 15

<210> 107
<211> 32
<212> PRT
<213> Homo sapiens

<400> 107

Gly Ile Pro Glu Arg Phe Ser Gly Ser Ser Ser Gly Thr Thr Val Thr
5 10 15

Leu Thr Ile Ser Gly Val Gln Ala Glu Asp Glu Ala Asp Tyr Tyr Cys
20 25 30

<210> 108
<211> 10
<212> PRT
<213> Homo sapiens

<400> 108

Phe Gly Gly Gly Thr Lys Leu Thr Val Leu
5 10

<210> 109
<211> 387
<212> DNA
<213> Homo sapiens

<400> 109

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgtgc gagaagcttt 300

```
cattattgta gtagtaccaa ctgctatgtg gagggtcggg aaaactttga ctactggggc     360

caagggacaa tggtcaccgt ctcgagt                                          387
```

<210> 110
<211> 129
<212> PRT
<213> Homo sapiens

<400> 110

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Phe His Tyr Cys Ser Ser Thr Asn Cys Tyr Val Glu Gly
            100                 105                 110

Arg Glu Asn Phe Asp Tyr Trp Gly Gln Gly Thr Met Val Thr Val Ser
        115                 120                 125

Ser
```

<210> 111
<211> 5
<212> PRT
<213> Homo sapiens

<400> 111

```
Ser Tyr Ala Met Ser
                5
```

<210> 112
<211> 17
<212> PRT
<213> Homo sapiens

<400> 112

```
Ala Ile Ser Gly Ser Gly Gly Ser Thr Tyr Tyr Ala Asp Ser Val Lys
                5                   10                  15

Gly
```

<210> 113
<211> 20
<212> PRT
<213> Homo sapiens

<400> 113

```
Ser Phe His Tyr Cys Ser Ser Thr Asn Cys Tyr Val Glu Gly Arg Glu
                5                   10                  15

Asn Phe Asp Tyr
            20
```

<210> 114
<211> 30
<212> PRT
<213> Homo sapiens

<400> 114

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 115
<211> 14
<212> PRT
<213> Homo sapiens

<400> 115

```
    Trp Val Arg Gln Ala Pro Gly Lys Gly Leu Glu Trp Val Ser
                    5                   10
```

<210> 116
<211> 32
<212> PRT
<213> Homo sapiens

<400> 116

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 117
<211> 11
<212> PRT
<213> Homo sapiens

<400> 117

```
          Trp Gly Gln Gly Thr Met Val Thr Val Ser Ser
                          5                   10
```

<210> 118
<211> 318
<212> DNA
<213> Homo sapiens

<400> 118

```
tcctatgagc tgactcagcc accctcagtg tccgtgtccc cccgacagac agccagcatc      60
acctgctctg gagataaatt ggggaataaa tatgcttcgt ggtatcaaca gaagccaggc     120
cactcccctg tactggtcat ctatcaagat tccaagcggc cctcagggat ccctgagcga     180
ttctctggct ccaactctgg gaacacagcc actctgacca tcagcgggac ccaggctatg     240
gatgaggctg tctattactg tcaggcgtgg gacagcacca tcgtggtctt cggcggaggg     300
accaagctga ccgtccta                                                   318
```

<210> 119
<211> 106
<212> PRT
<213> Homo sapiens

<400> 119

```
Ser Tyr Glu Leu Thr Gln Pro Pro Ser Val Ser Val Ser Pro Arg Gln
                5                   10                  15
Thr Ala Ser Ile Thr Cys Ser Gly Asp Lys Leu Gly Asn Lys Tyr Ala
            20                  25                  30
Ser Trp Tyr Gln Gln Lys Pro Gly His Ser Pro Val Leu Val Ile Tyr
        35                  40                  45
Gln Asp Ser Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Met
65                  70                  75                  80
Asp Glu Ala Val Tyr Tyr Cys Gln Ala Trp Asp Ser Thr Ile Val Val
                85                  90                  95
Phe Gly Gly Gly Thr Lys Leu Thr Val Leu
            100                 105
```

<210> 120
<211> 11
<212> PRT
<213> Homo sapiens

<400> 120

```
Ser Gly Asp Lys Leu Gly Asn Lys Tyr Ala Ser
                5                   10
```

<210> 121
<211> 7

<212> PRT
<213> Homo sapiens

<400> 121

Gln Asp Ser Lys Arg Pro Ser
5

<210> 122
<211> 9
<212> PRT
<213> Homo sapiens

<400> 122

Gln Ala Trp Asp Ser Thr Ile Val Val
5

<210> 123
<211> 22
<212> PRT
<213> Homo sapiens

<400> 123

Ser Tyr Glu Leu Thr Gln Pro Pro Ser Val Ser Val Ser Pro Arg Gln
5 10 15

Thr Ala Ser Ile Thr Cys
20

<210> 124
<211> 15
<212> PRT
<213> Homo sapiens

<400> 124

Trp Tyr Gln Gln Lys Pro Gly His Ser Pro Val Leu Val Ile Tyr
5 10 15

<210> 125
<211> 32
<212> PRT
<213> Homo sapiens

<400> 125

Gly Ile Pro Glu Arg Phe Ser Gly Ser Asn Ser Gly Asn Thr Ala Thr
5 10 15

Leu Thr Ile Ser Gly Thr Gln Ala Met Asp Glu Ala Val Tyr Tyr Cys
20 25 30

<210> 126
<211> 10
<212> PRT
<213> Homo sapiens

<400> 126

Phe Gly Gly Gly Thr Lys Leu Thr Val Leu
5 10

<210> 127
<211> 351
<212> DNA
<213> Homo sapiens

<400> 127

```
gagatacagc tgcaggagtc aggacctggc cttgtgaaac cttcacagtc actctccctc      60

acctgttctg tcactggtta caccattacc agtggttatg attggagctg gatccggaag     120

ttcccaggaa ataaaatgga gtggatggga tacataagct acagtggtag cactaactac     180

aacccatcgc tcaaaagtcg aatctccatt accagagaca catccaagaa tcagttcttc     240

ctgcagttga actctgtaac tactgaggat acagccacat attactgtgc aagagggatg     300

atggtactta ttcctaactg gggccaggga gtcatggtca cagtctcctc a              351
```

<210> 128
<211> 117
<212> PRT
<213> Homo sapiens

<400> 128

```
Glu Ile Gln Leu Gln Glu Ser Gly Pro Gly Leu Val Lys Pro Ser Gln
                5                   10                  15

Ser Leu Ser Leu Thr Cys Ser Val Thr Gly Tyr Thr Ile Thr Ser Gly
            20                  25                  30

Tyr Asp Trp Ser Trp Ile Arg Lys Phe Pro Gly Asn Lys Met Glu Trp
        35                  40                  45

Met Gly Tyr Ile Ser Tyr Ser Gly Ser Thr Asn Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Gly Met Met Val Leu Ile Pro Asn Trp Gly Gln Gly Val Met
            100                 105                 110

Val Thr Val Ser Ser
        115
```

<210> 129
<211> 4
<212> PRT

<213> Homo sapiens

<400> 129
Ser Gly Tyr Asp

<210> 130
<211> 18
<212> PRT
<213> Homo sapiens

<400> 130

Met Gly Tyr Ile Ser Tyr Ser Gly Ser Thr Asn Tyr Asn Pro Ser Leu
5 10 15

Lys Ser

<210> 131
<211> 8
<212> PRT
<213> Homo sapiens

<400> 131

Gly Met Met Val Leu Ile Pro Asn
5

<210> 132
<211> 30
<212> PRT
<213> Homo sapiens

<400> 132

Glu Ile Gln Leu Gln Glu Ser Gly Pro Gly Leu Val Lys Pro Ser Gln
5 10 15

Ser Leu Ser Leu Thr Cys Ser Val Thr Gly Tyr Thr Ile Thr
20 25 30

<210> 133
<211> 14
<212> PRT
<213> Homo sapiens

<400> 133

Trp Ser Trp Ile Arg Lys Phe Pro Gly Asn Lys Met Glu Trp
5 10

<210> 134
<211> 32
<212> PRT
<213> Homo sapiens

<400> 134

Arg Ile Ser Ile Thr Arg Asp Thr Ser Lys Asn Gln Phe Phe Leu Gln
5 10 15

Leu Asn Ser Val Thr Thr Glu Asp Thr Ala Thr Tyr Tyr Cys Ala Arg
20 25 30

<210> 135
<211> 11
<212> PRT
<213> Homo sapiens

<400> 135

Trp Gly Gln Gly Val Met Val Thr Val Ser Ser
5 10

<210> 136
<211> 321
<212> DNA
<213> Homo sapiens

<400> 136

gatgtccaga tgacccagtc tccgtcttat cttactgcgt ctcctggaga aagtgtttcc 60

atcagttgca aggcaagtaa gagcattact aattatttag cctggtatca tcagaaacct 120

ggggaaccat ataaccttct tatctactct gggtcaactt tgcaatctgg aactccatca 180

aggttcagtg gcagtagatc tggtacagat ttcattctca ccatcagaag cctggagcct 240

gaagattttg gactctatta ctgtcaacag tattatgaaa aaccgtacac gtttggagct 300

gggaccaagc tggaactgaa a 321

<210> 137
<211> 107
<212> PRT
<213> Homo sapiens

<400> 137

Asp Val Gln Met Thr Gln Ser Pro Ser Tyr Leu Thr Ala Ser Pro Gly
5 10 15

Glu Ser Val Ser Ile Ser Cys Lys Ala Ser Lys Ser Ile Thr Asn Tyr
20 25 30

Leu Ala Trp Tyr His Gln Lys Pro Gly Glu Pro Tyr Asn Leu Leu Ile
35 40 45

Tyr Ser Gly Ser Thr Leu Gln Ser Gly Thr Pro Ser Arg Phe Ser Gly
50 55 60

Ser Arg Ser Gly Thr Asp Phe Ile Leu Thr Ile Arg Ser Leu Glu Pro
65 70 75 80

Glu Asp Phe Gly Leu Tyr Tyr Cys Gln Gln Tyr Tyr Glu Lys Pro Tyr
85 90 95

Thr Phe Gly Ala Gly Thr Lys Leu Glu Leu Lys
100 105

<210> 138
<211> 11
<212> PRT
<213> Homo sapiens

<400> 138

Lys Ala Ser Lys Ser Ile Thr Asn Tyr Leu Ala
5 10

<210> 139
<211> 7
<212> PRT
<213> Homo sapiens

<400> 139

Ser Gly Ser Thr Leu Gln Ser
5

<210> 140
<211> 9
<212> PRT
<213> Homo sapiens

<400> 140

Gln Gln Tyr Tyr Glu Lys Pro Tyr Thr
5

<210> 141
<211> 23
<212> PRT
<213> Homo sapiens

<400> 141

Asp Val Gln Met Thr Gln Ser Pro Ser Tyr Leu Thr Ala Ser Pro Gly
5 10 15

Glu Ser Val Ser Ile Ser Cys
20

<210> 142
<211> 15
<212> PRT
<213> Homo sapiens

<400> 142

Trp Tyr His Gln Lys Pro Gly Glu Pro Tyr Asn Leu Leu Ile Tyr
5 10 15

<210> 143
<211> 32
<212> PRT
<213> Homo sapiens

<400> 143

Gly Thr Pro Ser Arg Phe Ser Gly Ser Arg Ser Gly Thr Asp Phe Ile
5 10 15

Leu Thr Ile Arg Ser Leu Glu Pro Glu Asp Phe Gly Leu Tyr Tyr Cys
20 25 30

<210> 144
<211> 10
<212> PRT
<213> Homo sapiens

<400> 144

Phe Gly Ala Gly Thr Lys Leu Glu Leu Lys
5 10

<210> 163
<211> 363
<212> DNA
<213> Homo sapiens

<400> 163

```
caggtcaacc tactgcagtc tggggctgca ctggtgaagc ctggggcctc tgtgaagttg      60
tcttgcaaag cctctggtta tacattcact gactactata tacactgggt gaagcagagt     120
catggaatga gccttgagtg gattgggctt attaatcctg acagtggtta tcctaactac     180
aatgaaaatt tcaagggcaa ggccacattg actgttgaca aatccaccaa tacagcctat     240
atggagcttc gcagattgac atctgaggac tctgcaacct attactgtac aagatcgagg     300
atttactatg atggttcggt ttttgattac tggggccaag gagtcatggt cacagtctcc     360
tca                                                                   363
```

<210> 164
<211> 121
<212> PRT
<213> Homo sapiens

<400> 164

```
Gln Val Asn Leu Leu Gln Ser Gly Ala Ala Leu Val Lys Pro Gly Ala
                5                   10                  15

Ser Val Lys Leu Ser Cys Lys Ala Ser Gly Tyr Thr Phe Thr Asp Tyr
            20                  25                  30

Tyr Ile His Trp Val Lys Gln Ser His Gly Met Ser Leu Glu Trp Ile
        35                  40                  45

Gly Leu Ile Asn Pro Asp Ser Gly Tyr Pro Asn Tyr Asn Glu Asn Phe
    50                  55                  60

Lys Gly Lys Ala Thr Leu Thr Val Asp Lys Ser Thr Asn Thr Ala Tyr
65                  70                  75                  80

Met Glu Leu Arg Arg Leu Thr Ser Glu Asp Ser Ala Thr Tyr Tyr Cys
                85                  90                  95

Thr Arg Ser Arg Ile Tyr Tyr Asp Gly Ser Val Phe Asp Tyr Trp Gly
            100                 105                 110

Gln Gly Val Met Val Thr Val Ser Ser
        115                 120
```

<210> 165
<211> 5
<212> PRT
<213> Homo sapiens

<400> 165

```
Asp Tyr Tyr Ile His
                5
```

<210> 166
<211> 17
<212> PRT
<213> Homo sapiens

<400> 166

```
Leu Ile Asn Pro Asp Ser Gly Tyr Pro Asn Tyr Asn Glu Asn Phe Lys
                5                   10                  15

Gly
```

<210> 167
<211> 12
<212> PRT
<213> Homo sapiens

<400> 167

```
Ser Arg Ile Tyr Tyr Asp Gly Ser Val Phe Asp Tyr
                5                   10
```

<210> 168
<211> 30
<212> PRT
<213> Homo sapiens

<400> 168

```
Gln Val Asn Leu Leu Gln Ser Gly Ala Ala Leu Val Lys Pro Gly Ala
                5                   10                  15

Ser Val Lys Leu Ser Cys Lys Ala Ser Gly Tyr Thr Phe Thr
            20                  25                  30
```

<210> 169
<211> 14
<212> PRT
<213> Homo sapiens

<400> 169

```
Trp Val Lys Gln Ser His Gly Met Ser Leu Glu Trp Ile Gly
                5                   10
```

<210> 170
<211> 32
<212> PRT
<213> Homo sapiens

<400> 170

```
Lys Ala Thr Leu Thr Val Asp Lys Ser Thr Asn Thr Ala Tyr Met Glu
                5                   10                  15

Leu Arg Arg Leu Thr Ser Glu Asp Ser Ala Thr Tyr Tyr Cys Thr Arg
            20                  25                  30
```

<210> 171
<211> 11
<212> PRT
<213> Homo sapiens

<400> 171

Trp Gly Gln Gly Val Met Val Thr Val Ser Ser
5 10

<210> 172
<211> 318
<212> DNA
<213> Homo sapiens

<400> 172

```
gaaattgtgc taacccagtc tccaacaacc atggctgtat ctccggggga gaaggtcacc      60
atcacctgcc gtgccaggtc cagtgtaagc tacatgtact ggtaccagca gaagtcaggc     120
gcctccccta aaccctggat ttatgaaaca tccaaactgg cttctggagt cccagatcgc     180
ttcagtggca gtgggtctgg gacctcttat tcgttcacaa tcagctccat ggagactgaa     240
gatgctgcca cttattattg tcaccagtgg agtaggaccc cacccacgtt tggaggtggg     300
accaagctgg aaatgaga                                                   318
```

<210> 173
<211> 106
<212> PRT
<213> Homo sapiens

<400> 173

```
Glu Ile Val Leu Thr Gln Ser Pro Thr Thr Met Ala Val Ser Pro Gly
                5                   10                  15
Glu Lys Val Thr Ile Thr Cys Arg Ala Arg Ser Ser Val Ser Tyr Met
            20                  25                  30
Tyr Trp Tyr Gln Gln Lys Ser Gly Ala Ser Pro Lys Pro Trp Ile Tyr
        35                  40                  45
Glu Thr Ser Lys Leu Ala Ser Gly Val Pro Asp Arg Phe Ser Gly Ser
    50                  55                  60
Gly Ser Gly Thr Ser Tyr Ser Phe Thr Ile Ser Ser Met Glu Thr Glu
65                  70                  75                  80
Asp Ala Ala Thr Tyr Tyr Cys His Gln Trp Ser Arg Thr Pro Pro Thr
                85                  90                  95
Phe Gly Gly Gly Thr Lys Leu Glu Met Arg
            100                 105
```

<210> 174
<211> 10
<212> PRT
<213> Homo sapiens

<400> 174

Arg Ala Arg Ser Ser Val Ser Tyr Met Tyr
5 10

<210> 175
<211> 7
<212> PRT
<213> Homo sapiens

<400> 175

Glu Thr Ser Lys Leu Ala Ser
5

<210> 176
<211> 9
<212> PRT
<213> Homo sapiens

<400> 176

His Gln Trp Ser Arg Thr Pro Pro Thr
5

<210> 177
<211> 23
<212> PRT
<213> Homo sapiens

<400> 177

Glu Ile Val Leu Thr Gln Ser Pro Thr Thr Met Ala Val Ser Pro Gly
5 10 15

Glu Lys Val Thr Ile Thr Cys
20

<210> 178
<211> 15
<212> PRT
<213> Homo sapiens

<400> 178

Trp Tyr Gln Gln Lys Ser Gly Ala Ser Pro Lys Pro Trp Ile Tyr
5 10 15

<210> 179
<211> 32
<212> PRT
<213> Homo sapiens

<400> 179

Gly Val Pro Asp Arg Phe Ser Gly Ser Gly Ser Gly Thr Ser Tyr Ser
5 10 15

Phe Thr Ile Ser Ser Met Glu Thr Glu Asp Ala Ala Thr Tyr Tyr Cys
20 25 30

<210> 180
<211> 10
<212> PRT
<213> Homo sapiens

<400> 180

```
Phe Gly Gly Gly Thr Lys Leu Glu Met Arg
                5                   10
```

<210> 181
<211> 351
<212> DNA
<213> Homo sapiens

<400> 181

```
gagatacagc tgcaggagtc aggacctggc cttgtgaaac cttcacagtc actctccctc      60
acctgttctg tcactggtta caccattacc agcggttatg attggagctg gatccggaag     120
ttcccaggaa ataaaatgga gtggatggga tacataagct acagtggtaa cactaactac     180
aacccatcgc tcaaaagtcg aatctccatt accagagaca catccaagaa tcagttcttc     240
ctgcagttga actctgttac tactgaggat atagccacat attactgtgg aagagggatg     300
gtggtacttg ttagtacctg ggccaagga gtcatggtca cagtctcctc a              351
```

<210> 182
<211> 117
<212> PRT
<213> Homo sapiens

<400> 182

```
Glu Ile Gln Leu Gln Glu Ser Gly Pro Gly Leu Val Lys Pro Ser Gln
                5                   10                  15
Ser Leu Ser Leu Thr Cys Ser Val Thr Gly Tyr Thr Ile Thr Ser Gly
            20                  25                  30
Tyr Asp Trp Ser Trp Ile Arg Lys Phe Pro Gly Asn Lys Met Glu Trp
        35                  40                  45
Met Gly Tyr Ile Ser Tyr Ser Gly Asn Thr Asn Tyr Asn Pro Ser Leu
    50                  55                  60
Lys Ser Arg Ile Ser Ile Thr Arg Asp Thr Ser Lys Asn Gln Phe Phe
65                  70                  75                  80
Leu Gln Leu Asn Ser Val Thr Thr Glu Asp Ile Ala Thr Tyr Tyr Cys
                85                  90                  95
Gly Arg Gly Met Val Val Leu Val Ser Thr Trp Gly Gln Gly Val Met
            100                 105                 110
Val Thr Val Ser Ser
        115
```

<210> 183
<211> 4
<212> PRT
<213> Homo sapiens

<400> 183
Ser Gly Tyr Asp

<210> 184
<211> 18
<212> PRT
<213> Homo sapiens

<400> 184

Met Gly Tyr Ile Ser Tyr Ser Gly Asn Thr Asn Tyr Asn Pro Ser Leu
5 10 15

Lys Ser

<210> 185
<211> 8
<212> PRT
<213> Homo sapiens

<400> 185

Gly Met Val Val Leu Val Ser Thr
5

<210> 186
<211> 30
<212> PRT
<213> Homo sapiens

<400> 186

Glu Ile Gln Leu Gln Glu Ser Gly Pro Gly Leu Val Lys Pro Ser Gln
5 10 15

Ser Leu Ser Leu Thr Cys Ser Val Thr Gly Tyr Thr Ile Thr
20 25 30

<210> 187
<211> 14
<212> PRT
<213> Homo sapiens

<400> 187

Trp Ser Trp Ile Arg Lys Phe Pro Gly Asn Lys Met Glu Trp
5 10

<210> 188
<211> 32
<212> PRT
<213> Homo sapiens

<400> 188

```
Arg Ile Ser Ile Thr Arg Asp Thr Ser Lys Asn Gln Phe Phe Leu Gln
                5                   10                  15

Leu Asn Ser Val Thr Thr Glu Asp Ile Ala Thr Tyr Tyr Cys Gly Arg
            20                  25                  30
```

<210> 189
<211> 11
<212> PRT
<213> Homo sapiens

<400> 189

```
Trp Gly Gln Gly Val Met Val Thr Val Ser Ser
                5                   10
```

<210> 190
<211> 321
<212> DNA
<213> Homo sapiens

<400> 190

```
gatgtccaga tgacccagtc tccgtcttat cttgctgcgt ctcctggaga aagtgtttcc      60

atcagttgca agacaagtaa gagcattacc cattatttag cctggtatca acagaagcct     120

ggggaagcat ttaaacttct tatctattct gggtcaactt tgcaatctgg aactccatca     180

aggttcattg gcagtggagc tgttacagat ttcactctca ccatcagaag cctggagcct     240

gaagattttg gactctatta ctgtcaacag tattatgaaa acccgtacac gtttggagct     300

gggaccaagc tggaactgaa a                                               321
```

<210> 191
<211> 107
<212> PRT
<213> Homo sapiens

<400> 191

Asp Val Gln Met Thr Gln Ser Pro Ser Tyr Leu Ala Ala Ser Pro Gly
1               5                   10                  15

Glu Ser Val Ser Ile Ser Cys Lys Thr Ser Lys Ser Ile Thr His Tyr
            20                  25                  30

Leu Ala Trp Tyr Gln Gln Lys Pro Gly Glu Ala Phe Lys Leu Leu Ile
        35                  40                  45

Tyr Ser Gly Ser Thr Leu Gln Ser Gly Thr Pro Ser Arg Phe Ile Gly
    50                  55                  60

Ser Gly Ala Val Thr Asp Phe Thr Leu Thr Ile Arg Ser Leu Glu Pro
65                  70                  75                  80

Glu Asp Phe Gly Leu Tyr Tyr Cys Gln Gln Tyr Tyr Glu Asn Pro Tyr
                85                  90                  95

Thr Phe Gly Ala Gly Thr Lys Leu Glu Leu Lys
            100                 105

<210> 192
<211> 11
<212> PRT
<213> Homo sapiens

<400> 192

Lys Thr Ser Lys Ser Ile Thr His Tyr Leu Ala
1               5                   10

<210> 193
<211> 7
<212> PRT
<213> Homo sapiens

<400> 193

Ser Gly Ser Thr Leu Gln Ser
1               5

<210> 194
<211> 9
<212> PRT
<213> Homo sapiens

<400> 194

Gln Gln Tyr Tyr Glu Asn Pro Tyr Thr
1               5

<210> 195
<211> 23
<212> PRT
<213> Homo sapiens

<400> 195

```
Asp Val Gln Met Thr Gln Ser Pro Ser Tyr Leu Ala Ala Ser Pro Gly
                5                   10                  15

Glu Ser Val Ser Ile Ser Cys
            20
```

<210> 196
<211> 15
<212> PRT
<213> Homo sapiens

<400> 196

```
Trp Tyr Gln Gln Lys Pro Gly Glu Ala Phe Lys Leu Leu Ile Tyr
                5                   10                  15
```

<210> 197
<211> 32
<212> PRT
<213> Homo sapiens

<400> 197

```
Gly Thr Pro Ser Arg Phe Ile Gly Ser Gly Ala Val Thr Asp Phe Thr
                5                   10                  15

Leu Thr Ile Arg Ser Leu Glu Pro Glu Asp Phe Gly Leu Tyr Tyr Cys
            20                  25                  30
```

<210> 198
<211> 10
<212> PRT
<213> Homo sapiens

<400> 198

```
Phe Gly Ala Gly Thr Lys Leu Glu Leu Lys
                5                   10
```

<210> 217
<211> 372
<212> DNA
<213> Homo sapiens

<400> 217

```
caggttactc tgaaagagtc tggccctggg atattgcagc cttcccagac cctcagtctg      60
acttgctctt tctctgggtt ttcactgagc acttttggta tatgtgtgag ctggattcgt     120
cagccttcag ggaagggtct ggagtggctg gcaattattt gttgggagga tagtaagggc     180
tacaaccctt ctctgaagaa ccggctcaca atctccaagg acacctccaa caaccaagca     240
ttcctcaaga tctccagtgt ggacactgca gataccgcca tatactactg tgctcggagg     300
tccgttatgt atactacggc cccgtactac tttgattact ggggccaagg agtcatggtc     360
acagtctcct ca                                                         372
```

<210> 218
<211> 124
<212> PRT
<213> Homo sapiens

<400> 218

```
Gln Val Thr Leu Lys Glu Ser Gly Pro Gly Ile Leu Gln Pro Ser Gln
                5                   10                  15

Thr Leu Ser Leu Thr Cys Ser Phe Ser Gly Phe Ser Leu Ser Thr Phe
            20                  25                  30

Gly Ile Cys Val Ser Trp Ile Arg Gln Pro Ser Gly Lys Gly Leu Glu
        35                  40                  45

Trp Leu Ala Ile Ile Cys Trp Glu Asp Ser Lys Gly Tyr Asn Pro Ser
    50                  55                  60

Leu Lys Asn Arg Leu Thr Ile Ser Lys Asp Thr Ser Asn Asn Gln Ala
65                  70                  75                  80

Phe Leu Lys Ile Ser Ser Val Asp Thr Ala Asp Thr Ala Ile Tyr Tyr
                85                  90                  95

Cys Ala Arg Arg Ser Val Met Tyr Thr Thr Ala Pro Tyr Tyr Phe Asp
            100                 105                 110

Tyr Trp Gly Gln Gly Val Met Val Thr Val Ser Ser
        115                 120
```

<210> 219
<211> 7
<212> PRT
<213> Homo sapiens

<400> 219

```
Thr Phe Gly Ile Cys Val Ser
                5
```

<210> 220
<211> 16
<212> PRT
<213> Homo sapiens

<400> 220

```
Ile Ile Cys Trp Glu Asp Ser Lys Gly Tyr Asn Pro Ser Leu Lys Asn
                5                   10                  15
```

<210> 221
<211> 14
<212> PRT
<213> Homo sapiens

<400> 221

```
Arg Ser Val Met Tyr Thr Thr Ala Pro Tyr Tyr Phe Asp Tyr
                5                   10
```

<210> 222
<211> 30
<212> PRT
<213> Homo sapiens

<400> 222

```
Gln Val Thr Leu Lys Glu Ser Gly Pro Gly Ile Leu Gln Pro Ser Gln
                5                   10                  15

Thr Leu Ser Leu Thr Cys Ser Phe Ser Gly Phe Ser Leu Ser
            20                  25                  30
```

<210> 223
<211> 14
<212> PRT
<213> Homo sapiens

<400> 223

```
Trp Ile Arg Gln Pro Ser Gly Lys Gly Leu Glu Trp Leu Ala
                5                   10
```

<210> 224
<211> 32
<212> PRT
<213> Homo sapiens

<400> 224

```
Arg Leu Thr Ile Ser Lys Asp Thr Ser Asn Asn Gln Ala Phe Leu Lys
                5                   10                  15

Ile Ser Ser Val Asp Thr Ala Asp Thr Ala Ile Tyr Tyr Cys Ala Arg
            20                  25                  30
```

<210> 225
<211> 11
<212> PRT
<213> Homo sapiens

<400> 225

```
Trp Gly Gln Gly Val Met Val Thr Val Ser Ser
                5                   10
```

<210> 226
<211> 321
<212> DNA
<213> Homo sapiens

<400> 226

```
gacatccaga tgacacagtc tcctgcctcc ctgtctgcat ctctggaaga aattgtcacc      60
atcacatgcc aggcaagcca agacattggt aattggttgg catggtttca gcagaaaccg     120
gggaaatctc ctcagctcct gatttatgat gcaaccacct tggcagatgg ggtcccatca     180
cggttcagcg gcagtagatc tggcacacag ttttctctta agatcaacag attacaggtt     240
gaagatattg gaagctatta ctgtcaacag gctcataata atccgaacac gtttggacgg     300
gggaccaagc tggaaatgaa a                                               321
```

<210> 227
<211> 107
<212> PRT
<213> Homo sapiens

<400> 227

```
Asp Ile Gln Met Thr Gln Ser Pro Ala Ser Leu Ser Ala Ser Leu Glu
                5                   10                  15
Glu Ile Val Thr Ile Thr Cys Gln Ala Ser Gln Asp Ile Gly Asn Trp
            20                  25                  30
Leu Ala Trp Phe Gln Gln Lys Pro Gly Lys Ser Pro Gln Leu Leu Ile
        35                  40                  45
Tyr Asp Ala Thr Thr Leu Ala Asp Gly Val Pro Ser Arg Phe Ser Gly
    50                  55                  60
Ser Arg Ser Gly Thr Gln Phe Ser Leu Lys Ile Asn Arg Leu Gln Val
65                  70                  75                  80
Glu Asp Ile Gly Ser Tyr Tyr Cys Gln Gln Ala His Asn Asn Pro Asn
                85                  90                  95
Thr Phe Gly Arg Gly Thr Lys Leu Glu Met Lys
            100                 105
```

<210> 228
<211> 11
<212> PRT
<213> Homo sapiens

<400> 228

```
Gln Ala Ser Gln Asp Ile Gly Asn Trp Leu Ala
                5                   10
```

<210> 229
<211> 7
<212> PRT
<213> Homo sapiens

<400> 229

Asp Ala Thr Thr Leu Ala Asp
5

<210> 230
<211> 9
<212> PRT
<213> Homo sapiens

<400> 230

Gln Gln Ala His Asn Asn Pro Asn Thr
5

<210> 231
<211> 23
<212> PRT
<213> Homo sapiens

<400> 231

Asp Ile Gln Met Thr Gln Ser Pro Ala Ser Leu Ser Ala Ser Leu Glu
5 10 15

Glu Ile Val Thr Ile Thr Cys
20

<210> 232
<211> 15
<212> PRT
<213> Homo sapiens

<400> 232

Trp Phe Gln Gln Lys Pro Gly Lys Ser Pro Gln Leu Leu Ile Tyr
5 10 15

<210> 233
<211> 32
<212> PRT
<213> Homo sapiens

<400> 233

Gly Val Pro Ser Arg Phe Ser Gly Ser Arg Ser Gly Thr Gln Phe Ser
5 10 15

Leu Lys Ile Asn Arg Leu Gln Val Glu Asp Ile Gly Ser Tyr Tyr Cys
20 25 30

<210> 234
<211> 10

<212> PRT
<213> Homo sapiens

<400> 234

```
Phe Gly Arg Gly Thr Lys Leu Glu Met Lys
                5                   10
```

<210> 244
<211> 321
<212> DNA
<213> Homo sapiens

<400> 244

```
gacatccaga tgacacagtc tcctgcctcc ctgtctgcat ctctggaaga aattgtcacc      60

atcacatgcc aggcaagcca agacattggt aattggttgg catggtatca gcagaaaccg     120

gggaaatctc ctcagctcct gctttatgat gcaaccagct tggcagatgg ggtcccatca     180

cggttcagcg gcagtagatc tggcacacag tattctctta agatcagcag actacaggtt     240

gaagatattg gaagctatta ctgtcaacag gctcatagta atccttggac gttcggtgga     300

ggcaccaagc tggaattgaa a                                               321
```

<210> 245
<211> 107
<212> PRT
<213> Homo sapiens

<400> 245

```
Asp Ile Gln Met Thr Gln Ser Pro Ala Ser Leu Ser Ala Ser Leu Glu
                5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Leu
        35                  40                  45

Tyr Asp Ala Thr Ser Leu Ala Asp Gly Val Pro Ser Arg Phe Ser Gly
    50                  55                  60

Ser Arg Ser Gly Thr Gln Tyr Ser Leu Lys Ile Ser Arg Leu Gln Val
65                  70                  75                  80

Glu Asp Ile Gly Ser Tyr Tyr Cys Gln Gln Ala His Ser Asn Pro Trp
                85                  90                  95

Thr Phe Gly Gly Gly Thr Lys Leu Glu Leu Lys
            100                 105
```

<210> 246
<211> 11
<212> PRT
<213> Homo sapiens

<400> 246

Gln Ala Ser Gln Asp Ile Gly Asn Trp Leu Ala
5 10

<210> 247
<211> 7
<212> PRT
<213> Homo sapiens

<400> 247

Asp Ala Thr Ser Leu Ala Asp

5

<210> 248
<211> 9
<212> PRT
<213> Homo sapiens

<400> 248

Gln Gln Ala His Ser Asn Pro Trp Thr
5

<210> 249
<211> 23
<212> PRT
<213> Homo sapiens

<400> 249

Asp Ile Gln Met Thr Gln Ser Pro Ala Ser Leu Ser Ala Ser Leu Glu
5 10 15

Glu Ile Val Thr Ile Thr Cys
20

<210> 250
<211> 15
<212> PRT
<213> Homo sapiens

<400> 250

Trp Tyr Gln Gln Lys Pro Gly Lys Ser Pro Gln Leu Leu Leu Tyr
5 10 15

<210> 251
<211> 32
<212> PRT
<213> Homo sapiens

<400> 251

Gly Val Pro Ser Arg Phe Ser Gly Ser Arg Ser Gly Thr Gln Tyr Ser
5 10 15

Leu Lys Ile Ser Arg Leu Gln Val Glu Asp Ile Gly Ser Tyr Tyr Cys
20 25 30

<210> 252
<211> 10
<212> PRT
<213> Homo sapiens

<400> 252

Phe Gly Gly Gly Thr Lys Leu Glu Leu Lys
5 10

<210> 253
<211> 351
<212> DNA
<213> Homo sapiens

<400> 253

gagatacagc tgcaggagtc aggacctggc cttgtgaaac cttcacagtc actctccctc 60

acctgttctg tcactggtta caccattacc agtggttatg attggagctg gatccggaag 120

ttcccaggaa ataaaatgga gtggatggga tacataagct acagtggtag cactaactac 180

aacccatcgc tcaaaagtcg aatctccatt accagagaca catccaagaa tcagttcttc 240

ctgcagttga actctgttac tactgaggat atagccacat attactgtgc aagagggatg 300

atggtactta ttagtaactg gggccaagga gtcatggtca cagtctcctc a 351

<210> 254
<211> 117
<212> PRT
<213> Homo sapiens

<400> 254

Glu Ile Gln Leu Gln Glu Ser Gly Pro Gly Leu Val Lys Pro Ser Gln
5 10 15

Ser Leu Ser Leu Thr Cys Ser Val Thr Gly Tyr Thr Ile Thr Ser Gly
20 25 30

Tyr Asp Trp Ser Trp Ile Arg Lys Phe Pro Gly Asn Lys Met Glu Trp
35 40 45

Met Gly Tyr Ile Ser Tyr Ser Gly Ser Thr Asn Tyr Asn Pro Ser Leu
50 55 60

Lys Ser Arg Ile Ser Ile Thr Arg Asp Thr Ser Lys Asn Gln Phe Phe
65 70 75 80

Leu Gln Leu Asn Ser Val Thr Thr Glu Asp Ile Ala Thr Tyr Tyr Cys
85 90 95

Ala Arg Gly Met Met Val Leu Ile Ser Asn Trp Gly Gln Gly Val Met
100 105 110

Val Thr Val Ser Ser
115

<210> 255
<211> 4
<212> PRT
<213> Homo sapiens

<400> 255
Ser Gly Tyr Asp

<210> 256
<211> 18
<212> PRT
<213> Homo sapiens

<400> 256

Met Gly Tyr Ile Ser Tyr Ser Gly Ser Thr Asn Tyr Asn Pro Ser Leu
5 10 15

Lys Ser

<210> 257
<211> 8
<212> PRT
<213> Homo sapiens

<400> 257

Gly Met Met Val Leu Ile Ser Asn
5

<210> 258
<211> 30

<212> PRT
<213> Homo sapiens

<400> 258

```
Glu Ile Gln Leu Gln Glu Ser Gly Pro Gly Leu Val Lys Pro Ser Gln
                5                   10                  15

Ser Leu Ser Leu Thr Cys Ser Val Thr Gly Tyr Thr Ile Thr
            20                  25                  30
```

<210> 259
<211> 14
<212> PRT
<213> Homo sapiens

<400> 259

```
Trp Ser Trp Ile Arg Lys Phe Pro Gly Asn Lys Met Glu Trp
                5                   10
```

<210> 260
<211> 32
<212> PRT
<213> Homo sapiens

<400> 260

```
Arg Ile Ser Ile Thr Arg Asp Thr Ser Lys Asn Gln Phe Phe Leu Gln
                5                   10                  15

Leu Asn Ser Val Thr Thr Glu Asp Ile Ala Thr Tyr Tyr Cys Ala Arg
            20                  25                  30
```

<210> 261
<211> 11
<212> PRT
<213> Homo sapiens

<400> 261

```
Trp Gly Gln Gly Val Met Val Thr Val Ser Ser
                5                   10
```

<210> 262
<211> 321
<212> DNA
<213> Homo sapiens

<400> 262

```
gatgtccaga tgacccagtc tccgtcttat cttgctgcgt ctcctggaga aagtgtttcc      60

atcagttgca aggcaagtaa gagcattact cattatttag cctggtatca acagaagcct     120

ggggaagcat ataaacttct tatctactct gggtcaactt tgcaatctgg aactccatca     180

aggttcattg gcactggagc tgttacagat ttcactctca ccatcagaag cctggagcct     240

gaagattttg gactctatta ctgtcaacag tattatgaaa aaccgtacac gtttggagct     300

gggaccaagc tggaactgaa a                                               321
```

<210> 263
<211> 107
<212> PRT
<213> Homo sapiens

<400> 263

```
Asp Val Gln Met Thr Gln Ser Pro Ser Tyr Leu Ala Ala Ser Pro Gly
                5                   10                  15

Glu Ser Val Ser Ile Ser Cys Lys Ala Ser Lys Ser Ile Thr His Tyr
            20                  25                  30

Leu Ala Trp Tyr Gln Gln Lys Pro Gly Glu Ala Tyr Lys Leu Leu Ile
        35                  40                  45

Tyr Ser Gly Ser Thr Leu Gln Ser Gly Thr Pro Ser Arg Phe Ile Gly
    50                  55                  60

Thr Gly Ala Val Thr Asp Phe Thr Leu Thr Ile Arg Ser Leu Glu Pro
65                  70                  75                  80

Glu Asp Phe Gly Leu Tyr Tyr Cys Gln Gln Tyr Tyr Glu Lys Pro Tyr
                85                  90                  95

Thr Phe Gly Ala Gly Thr Lys Leu Glu Leu Lys
            100                 105
```

<210> 264
<211> 11
<212> PRT
<213> Homo sapiens

<400> 264

```
Lys Ala Ser Lys Ser Ile Thr His Tyr Leu Ala
                5                   10
```

<210> 265
<211> 7
<212> PRT
<213> Homo sapiens

<400> 265

```
Ser Gly Ser Thr Leu Gln Ser
```

5

<210> 266
<211> 9
<212> PRT
<213> Homo sapiens

<400> 266

Gln Gln Tyr Tyr Glu Lys Pro Tyr Thr
5

<210> 267
<211> 23
<212> PRT
<213> Homo sapiens

<400> 267

Asp Val Gln Met Thr Gln Ser Pro Ser Tyr Leu Ala Ala Ser Pro Gly
5 10 15

Glu Ser Val Ser Ile Ser Cys
20

<210> 268
<211> 15
<212> PRT
<213> Homo sapiens

<400> 268

Trp Tyr Gln Gln Lys Pro Gly Glu Ala Tyr Lys Leu Leu Ile Tyr
5 10 15

<210> 269
<211> 32
<212> PRT
<213> Homo sapiens

<400> 269

Gly Thr Pro Ser Arg Phe Ile Gly Thr Gly Ala Val Thr Asp Phe Thr
5 10 15

Leu Thr Ile Arg Ser Leu Glu Pro Glu Asp Phe Gly Leu Tyr Tyr Cys
20 25 30

<210> 270
<211> 10
<212> PRT
<213> Homo sapiens

<400> 270

Phe Gly Ala Gly Thr Lys Leu Glu Leu Lys
5 10

<210> 271
<211> 357
<212> DNA
<213> Homo sapiens

<400> 271

caggtgcagc tgaaggagtc aggacctggt ctggtgcagc cctcacagac cctgtccctc 60

acctgcactg tctctgggtt ctcactaacc cgctatgatg tgcactgggt tcgccagcct 120

ccaggaaagg gtctggagtg gatgggagga atatggggtg atggaagcac agattataat 180

tcagctctca aatcccgact gagcatcagc agggacacct ccaagagtca agtgttctta 240

aaaatgaaca gtctgcaaac tgaagacaca gccatttact tctgtaccag atctctggac 300

tacagtggtg acgggtttgg ttattggggc caaggcactc tggtcactgt ctcttca 357

<210> 272
<211> 119
<212> PRT
<213> Homo sapiens

<400> 272

Gln Val Gln Leu Lys Glu Ser Gly Pro Gly Leu Val Gln Pro Ser Gln
5 10 15

Thr Leu Ser Leu Thr Cys Thr Val Ser Gly Phe Ser Leu Thr Arg Tyr
20 25 30

Asp Val His Trp Val Arg Gln Pro Pro Gly Lys Gly Leu Glu Trp Met
35 40 45

Gly Gly Ile Trp Gly Asp Gly Ser Thr Asp Tyr Asn Ser Ala Leu Lys
50 55 60

Ser Arg Leu Ser Ile Ser Arg Asp Thr Ser Lys Ser Gln Val Phe Leu
65 70 75 80

Lys Met Asn Ser Leu Gln Thr Glu Asp Thr Ala Ile Tyr Phe Cys Thr
85 90 95

Arg Ser Leu Asp Tyr Ser Gly Asp Gly Phe Gly Tyr Trp Gly Gln Gly
100 105 110

Thr Leu Val Thr Val Ser Ser
115

<210> 273
<211> 5
<212> PRT
<213> Homo sapiens

<400> 273

Arg Tyr Asp Val His
5

<210> 274
<211> 16
<212> PRT
<213> Homo sapiens

<400> 274

Gly Ile Trp Gly Asp Gly Ser Thr Asp Tyr Asn Ser Ala Leu Lys Ser

5 10 15

<210> 275
<211> 11
<212> PRT
<213> Homo sapiens

<400> 275

Ser Leu Asp Tyr Ser Gly Asp Gly Phe Gly Tyr
5 10

<210> 276
<211> 30
<212> PRT
<213> Homo sapiens

<400> 276

Gln Val Gln Leu Lys Glu Ser Gly Pro Gly Leu Val Gln Pro Ser Gln
5 10 15

Thr Leu Ser Leu Thr Cys Thr Val Ser Gly Phe Ser Leu Thr
20 25 30

<210> 277
<211> 14
<212> PRT
<213> Homo sapiens

<400> 277

Trp Val Arg Gln Pro Pro Gly Lys Gly Leu Glu Trp Met Gly
5 10

<210> 278
<211> 32
<212> PRT
<213> Homo sapiens

<400> 278

```
Arg Leu Ser Ile Ser Arg Asp Thr Ser Lys Ser Gln Val Phe Leu Lys
                5                   10                  15

Met Asn Ser Leu Gln Thr Glu Asp Thr Ala Ile Tyr Phe Cys Thr Arg
            20                  25                  30
```

<210> 279
<211> 11
<212> PRT
<213> Homo sapiens

<400> 279

```
Trp Gly Gln Gly Thr Leu Val Thr Val Ser Ser
                5                   10
```

<210> 280
<211> 321
<212> DNA
<213> Homo sapiens

<400> 280

```
gacatccaga tgacacagtc tcctccctcc ctgtctgcat ctctgggaga caaagtcacc      60

atcacttgcc aggcaagtca aaacattaac aagtatatag cttggtatca gcaaaagcct     120

ggaaaagctc ctaggcagct catacgttac acatctgcac tagtgtcagg cacctcatcg     180

aggttcagtg gcagtggatc tgggagagat tattcattca gcatcagcaa cgtggagtct     240

gaagatattg caagttatta ctgtctacag tacgataacc ttccgtacac atttggagct     300

gggaccaagc tggaactgaa a                                               321
```

<210> 281
<211> 107
<212> PRT
<213> Homo sapiens

<400> 281

Asp Ile Gln Met Thr Gln Ser Pro Pro Ser Leu Ser Ala Ser Leu Gly
1               5                   10                  15

Asp Lys Val Thr Ile Thr Cys Gln Ala Ser Gln Asn Ile Asn Lys Tyr
            20                  25                  30

Ile Ala Trp Tyr Gln Gln Lys Pro Gly Lys Ala Pro Arg Gln Leu Ile
        35                  40                  45

Arg Tyr Thr Ser Ala Leu Val Ser Gly Thr Ser Ser Arg Phe Ser Gly
    50                  55                  60

Ser Gly Ser Gly Arg Asp Tyr Ser Phe Ser Ile Ser Asn Val Glu Ser
65                  70                  75                  80

Glu Asp Ile Ala Ser Tyr Tyr Cys Leu Gln Tyr Asp Asn Leu Pro Tyr
                85                  90                  95

Thr Phe Gly Ala Gly Thr Lys Leu Glu Leu Lys
            100                 105

<210> 282
<211> 11
<212> PRT
<213> Homo sapiens

<400> 282

Gln Ala Ser Gln Asn Ile Asn Lys Tyr Ile Ala
                5                   10

<210> 283
<211> 7
<212> PRT
<213> Homo sapiens

<400> 283

Tyr Thr Ser Ala Leu Val Ser
                5

<210> 284
<211> 9
<212> PRT
<213> Homo sapiens

<400> 284

Leu Gln Tyr Asp Asn Leu Pro Tyr Thr
                5

<210> 285
<211> 23
<212> PRT
<213> Homo sapiens

<400> 285

Asp Ile Gln Met Thr Gln Ser Pro Pro Ser Leu Ser Ala Ser Leu Gly
1               5                   10                  15

Asp Lys Val Thr Ile Thr Cys
            20

<210> 286
<211> 15
<212> PRT
<213> Homo sapiens

<400> 286

Trp Tyr Gln Gln Lys Pro Gly Lys Ala Pro Arg Gln Leu Ile Arg
                5                   10                  15

<210> 287
<211> 32
<212> PRT
<213> Homo sapiens

<400> 287

Gly Thr Ser Ser Arg Phe Ser Gly Ser Gly Ser Gly Arg Asp Tyr Ser
                5                   10                  15

Phe Ser Ile Ser Asn Val Glu Ser Glu Asp Ile Ala Ser Tyr Tyr Cys
            20                  25                  30

<210> 288
<211> 10
<212> PRT
<213> Homo sapiens

<400> 288

Phe Gly Ala Gly Thr Lys Leu Glu Leu Lys
                5                   10

<210> 289
<211> 360
<212> DNA
<213> Homo sapiens

<400> 289

caggttactc tgaaagagtc tggccctggg atattgcagc cttcccagac cctcagtctg 60

acttgctctt tctctgggtt ttcactgaac acttatggta tatgtgtgag ctggattcgt 120

cagccttcag ggaagggtct ggagtggctg gcaactattt gttgggagga tagtaaggtc 180

tacaaccctt ctctgaagaa ccggctcaca atctccaagg acacctccaa caaccaagca 240

ttcctcaaga tcaccagtgt ggacactgca gataccgcca tatactactg tgctcggagg 300

agggtttggt catactactt tgattactgg ggccaaggag tcatggtcac agtctcctca 360

<210> 290
<211> 120
<212> PRT
<213> Homo sapiens

<400> 290

```
Gln Val Thr Leu Lys Glu Ser Gly Pro Gly Ile Leu Gln Pro Ser Gln
                5                   10                  15

Thr Leu Ser Leu Thr Cys Ser Phe Ser Gly Phe Ser Leu Asn Thr Tyr
            20                  25                  30

Gly Ile Cys Val Ser Trp Ile Arg Gln Pro Ser Gly Lys Gly Leu Glu
        35                  40                  45

Trp Leu Ala Thr Ile Cys Trp Glu Asp Ser Lys Val Tyr Asn Pro Ser
    50                  55                  60

Leu Lys Asn Arg Leu Thr Ile Ser Lys Asp Thr Ser Asn Asn Gln Ala
65                  70                  75                  80

Phe Leu Lys Ile Thr Ser Val Asp Thr Ala Asp Thr Ala Ile Tyr Tyr
                85                  90                  95

Cys Ala Arg Arg Arg Val Trp Ser Tyr Tyr Phe Asp Tyr Trp Gly Gln
            100                 105                 110

Gly Val Met Val Thr Val Ser Ser
        115                 120
```

<210> 291
<211> 7
<212> PRT
<213> Homo sapiens

<400> 291

```
Thr Tyr Gly Ile Cys Val Ser
                5
```

<210> 292
<211> 16
<212> PRT
<213> Homo sapiens

<400> 292

```
Thr Ile Cys Trp Glu Asp Ser Lys Val Tyr Asn Pro Ser Leu Lys Asn
                5                   10                  15
```

<210> 293
<211> 10
<212> PRT
<213> Homo sapiens

<400> 293

Arg Arg Val Trp Ser Tyr Tyr Phe Asp Tyr
5 10

<210> 294
<211> 30
<212> PRT
<213> Homo sapiens

<400> 294

Gln Val Thr Leu Lys Glu Ser Gly Pro Gly Ile Leu Gln Pro Ser Gln
5 10 15

Thr Leu Ser Leu Thr Cys Ser Phe Ser Gly Phe Ser Leu Asn
20 25 30

<210> 295
<211> 14
<212> PRT
<213> Homo sapiens

<400> 295

Trp Ile Arg Gln Pro Ser Gly Lys Gly Leu Glu Trp Leu Ala
5 10

<210> 296
<211> 32
<212> PRT
<213> Homo sapiens

<400> 296

Arg Leu Thr Ile Ser Lys Asp Thr Ser Asn Asn Gln Ala Phe Leu Lys
5 10 15

Ile Thr Ser Val Asp Thr Ala Asp Thr Ala Ile Tyr Tyr Cys Ala Arg
20 25 30

<210> 297
<211> 11
<212> PRT
<213> Homo sapiens

<400> 297

Trp Gly Gln Gly Val Met Val Thr Val Ser Ser
5 10

<210> 298
<211> 321
<212> DNA
<213> Homo sapiens

<400> 298

gacatccaga tgacacagtc tcctgcctcc ctgtctgcat ctctggaaga aattgtcacc 60

atcacatgcc aggcaagcca agacattggt aattggttgg catggtatca gcagaaaccg 120

gggaaatctc ctcagctcct gatttatgat gcaaccagct tggcagatgg ggtcccatca 180

cggttcagcg gcagtagatc tggcacacag tattctctta agatcagcag actacaggtt 240

gaagatattg gaagctatta ctgtcaacag gctcatacta atccgctcac gttcggttct 300

gggaccaagc tggagatcaa a 321

<210> 299
<211> 107
<212> PRT
<213> Homo sapiens

<400> 299

```
Asp Ile Gln Met Thr Gln Ser Pro Ala Ser Leu Ser Ala Ser Leu Glu
                5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Ile
        35                  40                  45

Tyr Asp Ala Thr Ser Leu Ala Asp Gly Val Pro Ser Arg Phe Ser Gly
    50                  55                  60

Ser Arg Ser Gly Thr Gln Tyr Ser Leu Lys Ile Ser Arg Leu Gln Val
65                  70                  75                  80

Glu Asp Ile Gly Ser Tyr Tyr Cys Gln Gln Ala His Thr Asn Pro Leu
                85                  90                  95

Thr Phe Gly Ser Gly Thr Lys Leu Glu Ile Lys
            100                 105
```

<210> 300
<211> 11
<212> PRT
<213> Homo sapiens

<400> 300

```
Gln Ala Ser Gln Asp Ile Gly Asn Trp Leu Ala
                5                   10
```

<210> 301
<211> 7
<212> PRT
<213> Homo sapiens

<400> 301

```
Asp Ala Thr Ser Leu Ala Asp
                5
```

<210> 302

<211> 9
<212> PRT
<213> Homo sapiens

<400> 302

Gln Gln Ala His Thr Asn Pro Leu Thr
1 5

<210> 303
<211> 23
<212> PRT
<213> Homo sapiens

<400> 303

Asp Ile Gln Met Thr Gln Ser Pro Ala Ser Leu Ser Ala Ser Leu Glu
5 10 15

Glu Ile Val Thr Ile Thr Cys
20

<210> 304
<211> 15
<212> PRT
<213> Homo sapiens

<400> 304

Trp Tyr Gln Gln Lys Pro Gly Lys Ser Pro Gln Leu Leu Ile Tyr
5 10 15

<210> 305
<211> 32
<212> PRT
<213> Homo sapiens

<400> 305

Gly Val Pro Ser Arg Phe Ser Gly Ser Arg Ser Gly Thr Gln Tyr Ser
5 10 15

Leu Lys Ile Ser Arg Leu Gln Val Glu Asp Ile Gly Ser Tyr Tyr Cys
20 25 30

<210> 306
<211> 10
<212> PRT
<213> Homo sapiens

<400> 306

Phe Gly Ser Gly Thr Lys Leu Glu Ile Lys
5 10

<210> 307
<211> 351
<212> DNA

<213> Homo sapiens

<400> 307

```
gagatacagc tgcaggagtc aggacctggc cttgtgaaac cttcacagtc actctccctc      60
acctgttctg tcactggtta caccattacc agtggttttg attggagctg gttccggaag     120
ttcccaggaa ataaaatgga gtggatggga tacataagct acagtggtag cactaactac     180
aacccatcgc tcaaaagtcg aatctccatt accagagaca catccaagaa tcagttcttc     240
ctgcagttga actctgtaac tactgaagat acagccacat attactgtgc aagaggggtc     300
tcctccctgt ctgcttactg gggccaaggc actctggtca ctgtctcttc a              351
```

<210> 308
<211> 117
<212> PRT
<213> Homo sapiens

<400> 308

```
Glu Ile Gln Leu Gln Glu Ser Gly Pro Gly Leu Val Lys Pro Ser Gln
                5                   10                  15

Ser Leu Ser Leu Thr Cys Ser Val Thr Gly Tyr Thr Ile Thr Ser Gly
            20                  25                  30

Phe Asp Trp Ser Trp Phe Arg Lys Phe Pro Gly Asn Lys Met Glu Trp
        35                  40                  45

Met Gly Tyr Ile Ser Tyr Ser Gly Ser Thr Asn Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Gly Val Ser Ser Leu Ser Ala Tyr Trp Gly Gln Gly Thr Leu
            100                 105                 110

Val Thr Val Ser Ser
        115
```

<210> 309
<211> 4
<212> PRT
<213> Homo sapiens

<400> 309
Ser Gly Phe Asp

<210> 310
<211> 18
<212> PRT

<213> Homo sapiens

<400> 310

Met Gly Tyr Ile Ser Tyr Ser Gly Ser Thr Asn Tyr Asn Pro Ser Leu
1               5                   10                  15

Lys Ser

<210> 311
<211> 8
<212> PRT
<213> Homo sapiens

<400> 311

Gly Val Ser Ser Leu Ser Ala Tyr
1               5

<210> 312
<211> 30
<212> PRT
<213> Homo sapiens

<400> 312

Glu Ile Gln Leu Gln Glu Ser Gly Pro Gly Leu Val Lys Pro Ser Gln
                5                   10                  15

Ser Leu Ser Leu Thr Cys Ser Val Thr Gly Tyr Thr Ile Thr
            20                  25                  30

<210> 313
<211> 14
<212> PRT
<213> Homo sapiens

<400> 313

Trp Ser Trp Phe Arg Lys Phe Pro Gly Asn Lys Met Glu Trp
                5                   10

<210> 314
<211> 32
<212> PRT
<213> Homo sapiens

<400> 314

Arg Ile Ser Ile Thr Arg Asp Thr Ser Lys Asn Gln Phe Phe Leu Gln
                5                   10                  15

Leu Asn Ser Val Thr Thr Glu Asp Thr Ala Thr Tyr Tyr Cys Ala Arg
            20                  25                  30

<210> 315
<211> 11
<212> PRT

<213> Homo sapiens

<400> 315

Trp Gly Gln Gly Thr Leu Val Thr Val Ser Ser
5 10

<210> 316
<211> 321
<212> DNA
<213> Homo sapiens

<400> 316

gatgtccaga tgacccagtc tccgtcttat cttgctgcgt ctcctggaga gagtgtttcc 60

atcagttgca aggcaagtaa gctcattact aattatttag cctggtatca acagaaacct 120

ggggaaccat ataaccttct tatctactct gggtcaactt tgcaatctgg cactccatca 180

aggttcagtg gcagtagatc tggtacagat ttcactctca ccatcagaag cctgcagcct 240

gaagattttg gactctatta ctgtcaacag tattatgaaa aaccgtacac gtttggaggt 300

gggaccaagc tggaactgaa a 321

<210> 317
<211> 107
<212> PRT
<213> Homo sapiens

<400> 317

Asp Val Gln Met Thr Gln Ser Pro Ser Tyr Leu Ala Ala Ser Pro Gly
5 10 15

Glu Ser Val Ser Ile Ser Cys Lys Ala Ser Lys Leu Ile Thr Asn Tyr
20 25 30

Leu Ala Trp Tyr Gln Gln Lys Pro Gly Glu Pro Tyr Asn Leu Leu Ile
35 40 45

Tyr Ser Gly Ser Thr Leu Gln Ser Gly Thr Pro Ser Arg Phe Ser Gly
50 55 60

Ser Arg Ser Gly Thr Asp Phe Thr Leu Thr Ile Arg Ser Leu Gln Pro
65 70 75 80

Glu Asp Phe Gly Leu Tyr Tyr Cys Gln Gln Tyr Tyr Glu Lys Pro Tyr
85 90 95

Thr Phe Gly Gly Gly Thr Lys Leu Glu Leu Lys
100 105

<210> 318
<211> 11
<212> PRT
<213> Homo sapiens

<400> 318

Lys Ala Ser Lys Leu Ile Thr Asn Tyr Leu Ala
5 10

<210> 319
<211> 7
<212> PRT
<213> Homo sapiens

<400> 319

Ser Gly Ser Thr Leu Gln Ser
5

<210> 320
<211> 9
<212> PRT
<213> Homo sapiens

<400> 320

Gln Gln Tyr Tyr Glu Lys Pro Tyr Thr
5

<210> 321
<211> 23
<212> PRT
<213> Homo sapiens

<400> 321

Asp Val Gln Met Thr Gln Ser Pro Ser Tyr Leu Ala Ala Ser Pro Gly
5 10 15

Glu Ser Val Ser Ile Ser Cys
20

<210> 322
<211> 15
<212> PRT
<213> Homo sapiens

<400> 322

Trp Tyr Gln Gln Lys Pro Gly Glu Pro Tyr Asn Leu Leu Ile Tyr
5 10 15

<210> 323
<211> 32
<212> PRT
<213> Homo sapiens

<400> 323

```
Gly Thr Pro Ser Arg Phe Ser Gly Ser Arg Ser Gly Thr Asp Phe Thr
                5                   10                  15

Leu Thr Ile Arg Ser Leu Gln Pro Glu Asp Phe Gly Leu Tyr Tyr Cys
            20                  25                  30
```

<210> 324
<211> 10
<212> PRT
<213> Homo sapiens

<400> 324

```
Phe Gly Gly Gly Thr Lys Leu Glu Leu Lys
                5                   10
```

<210> 325
<211> 366
<212> DNA
<213> Homo sapiens

<400> 325

```
caggtgcagc tgaaggagtc aggacctggc ctggtgaagc cctcagagac cctgtctctc      60
acctgcactg tctctgggtt ctcattaacc agctatcatg taagctgggt tcgacagcct     120
ccaggaaagg gtctggagtg gatgggaata atatggggtg atggaagcac agcatataat     180
tcagctctca aatcccgact gagcatcagc agggacacct cgaagagcca agttttctta     240
aaaatgaaca gtctgcaaac tgaagacaca gccacttact actgtgtcag agccggacat     300
tattctgatg gtagttatta ctacggggct tactggggcc aaggcactct ggtcactgtc     360
tcttca                                                                366
```

<210> 326
<211> 122
<212> PRT
<213> Homo sapiens

<400> 326

Gln Val Gln Leu Lys Glu Ser Gly Pro Gly Leu Val Lys Pro Ser Glu
5 10 15

Thr Leu Ser Leu Thr Cys Thr Val Ser Gly Phe Ser Leu Thr Ser Tyr
20 25 30

His Val Ser Trp Val Arg Gln Pro Pro Gly Lys Gly Leu Glu Trp Met
35 40 45

Gly Ile Ile Trp Gly Asp Gly Ser Thr Ala Tyr Asn Ser Ala Leu Lys
50 55 60

Ser Arg Leu Ser Ile Ser Arg Asp Thr Ser Lys Ser Gln Val Phe Leu
65 70 75 80

Lys Met Asn Ser Leu Gln Thr Glu Asp Thr Ala Thr Tyr Tyr Cys Val
85 90 95

Arg Ala Gly His Tyr Ser Asp Gly Ser Tyr Tyr Tyr Gly Ala Tyr Trp
100 105 110

Gly Gln Gly Thr Leu Val Thr Val Ser Ser
115 120

<210> 327
<211> 5
<212> PRT
<213> Homo sapiens

<400> 327

Ser Tyr His Val Ser
5

<210> 328
<211> 16
<212> PRT
<213> Homo sapiens

<400> 328

Ile Ile Trp Gly Asp Gly Ser Thr Ala Tyr Asn Ser Ala Leu Lys Ser
5 10 15

<210> 329
<211> 14
<212> PRT
<213> Homo sapiens

<400> 329

Ala Gly His Tyr Ser Asp Gly Ser Tyr Tyr Tyr Gly Ala Tyr
5 10

<210> 330
<211> 30
<212> PRT
<213> Homo sapiens

<400> 330

```
Gln Val Gln Leu Lys Glu Ser Gly Pro Gly Leu Val Lys Pro Ser Glu
                5                   10                  15

Thr Leu Ser Leu Thr Cys Thr Val Ser Gly Phe Ser Leu Thr
            20                  25                  30
```

<210> 331
<211> 14
<212> PRT
<213> Homo sapiens

<400> 331

```
Trp Val Arg Gln Pro Pro Gly Lys Gly Leu Glu Trp Met Gly
                5                   10
```

<210> 332
<211> 32
<212> PRT
<213> Homo sapiens

<400> 332

```
Arg Leu Ser Ile Ser Arg Asp Thr Ser Lys Ser Gln Val Phe Leu Lys
                5                   10                  15

Met Asn Ser Leu Gln Thr Glu Asp Thr Ala Thr Tyr Tyr Cys Val Arg
            20                  25                  30
```

<210> 333
<211> 11
<212> PRT
<213> Homo sapiens

<400> 333

```
Trp Gly Gln Gly Thr Leu Val Thr Val Ser Ser
                5                   10
```

<210> 334
<211> 321
<212> DNA
<213> Homo sapiens

<400> 334

```
gacatccaga tgacacagtc tcctgcctcc ctgtctgcat ctctggaaga gattgtcacc      60
```

```
atcacatgcc aggcaagcca agacattggt aattggttgg catggtatca gcagaagccg     120
gggaaatctc ctcagctcct gatttatgat gcaaccacct tggcagatgg ggtcccatca     180
cggttcagcg gcagtagatc tggcacacag tattctctta agatcagcag actacaggtt     240
gaagatattg gaagctatta ctgtcaacag actcatagtc atcctcggac gttcggtgga     300
ggcaccaacc tggaattgaa a                                               321
```

<210> 335
<211> 107
<212> PRT
<213> Homo sapiens

<400> 335

```
Asp Ile Gln Met Thr Gln Ser Pro Ala Ser Leu Ser Ala Ser Leu Glu
                5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Ile
        35                  40                  45

Tyr Asp Ala Thr Thr Leu Ala Asp Gly Val Pro Ser Arg Phe Ser Gly
    50                  55                  60

Ser Arg Ser Gly Thr Gln Tyr Ser Leu Lys Ile Ser Arg Leu Gln Val
65                  70                  75                  80

Glu Asp Ile Gly Ser Tyr Tyr Cys Gln Gln Thr His Ser His Pro Arg
                85                  90                  95

Thr Phe Gly Gly Gly Thr Asn Leu Glu Leu Lys
            100                 105
```

<210> 336
<211> 11
<212> PRT
<213> Homo sapiens

<400> 336

```
Gln Ala Ser Gln Asp Ile Gly Asn Trp Leu Ala
                5                   10
```

<210> 337
<211> 7
<212> PRT
<213> Homo sapiens

<400> 337

```
Asp Ala Thr Thr Leu Ala Asp
                5
```

<210> 338

<211> 9
<212> PRT
<213> Homo sapiens

<400> 338

```
Gln Gln Thr His Ser His Pro Arg Thr
                5
```

<210> 339
<211> 23
<212> PRT
<213> Homo sapiens

<400> 339

```
Asp Ile Gln Met Thr Gln Ser Pro Ala Ser Leu Ser Ala Ser Leu Glu
                5                   10                  15

Glu Ile Val Thr Ile Thr Cys
            20
```

<210> 340
<211> 15
<212> PRT
<213> Homo sapiens

<400> 340

```
Trp Tyr Gln Gln Lys Pro Gly Lys Ser Pro Gln Leu Leu Ile Tyr
                5                   10                  15
```

<210> 341
<211> 32
<212> PRT
<213> Homo sapiens

<400> 341

```
Gly Val Pro Ser Arg Phe Ser Gly Ser Arg Ser Gly Thr Gln Tyr Ser
                5                   10                  15

Leu Lys Ile Ser Arg Leu Gln Val Glu Asp Ile Gly Ser Tyr Tyr Cys
            20                  25                  30
```

<210> 342
<211> 10
<212> PRT
<213> Homo sapiens

<400> 342

```
Phe Gly Gly Gly Thr Asn Leu Glu Leu Lys
                5                   10
```

<210> 343
<211> 351
<212> DNA

<213> Homo sapiens

<400> 343

```
gagatacagc tgcaggagtc aggacctggc cttgtgaaac cttcacagtc actctccctc      60
acctgttctg tcactggtta caccattacc agtggttttg attggagctg gttccggaag     120
ttcccaggaa ataaaatgga gtggatggga tacattagct acagtggtat cactaactac     180
aacccatcgc tcaaaagtcg aatctccatt accagagaca catccaagaa tcagttcttc     240
ctgcagttga actctgtaac tactgaagat acagccacat attactgtgc aagaggggtc     300
tcctccctgt ctgcttactg gggccaaggc actctggtca ctgtctcttc a              351
```

<210> 344
<211> 117
<212> PRT
<213> Homo sapiens

<400> 344

```
Glu Ile Gln Leu Gln Glu Ser Gly Pro Gly Leu Val Lys Pro Ser Gln
                5                   10                  15

Ser Leu Ser Leu Thr Cys Ser Val Thr Gly Tyr Thr Ile Thr Ser Gly
            20                  25                  30

Phe Asp Trp Ser Trp Phe Arg Lys Phe Pro Gly Asn Lys Met Glu Trp
        35                  40                  45

Met Gly Tyr Ile Ser Tyr Ser Gly Ile Thr Asn Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Gly Val Ser Ser Leu Ser Ala Tyr Trp Gly Gln Gly Thr Leu
            100                 105                 110

Val Thr Val Ser Ser
        115
```

<210> 345
<211> 4
<212> PRT
<213> Homo sapiens

<400> 345
Ser Gly Phe Asp

<210> 346
<211> 18
<212> PRT
<213> Homo sapiens

<400> 346

Met Gly Tyr Ile Ser Tyr Ser Gly Ile Thr Asn Tyr Asn Pro Ser Leu
5 10 15

Lys Ser

<210> 347
<211> 8
<212> PRT
<213> Homo sapiens

<400> 347

Gly Val Ser Ser Leu Ser Ala Tyr
5

<210> 348
<211> 30
<212> PRT
<213> Homo sapiens

<400> 348

Glu Ile Gln Leu Gln Glu Ser Gly Pro Gly Leu Val Lys Pro Ser Gln
5 10 15

Ser Leu Ser Leu Thr Cys Ser Val Thr Gly Tyr Thr Ile Thr
20 25 30

<210> 349
<211> 14
<212> PRT
<213> Homo sapiens

<400> 349

Trp Ser Trp Phe Arg Lys Phe Pro Gly Asn Lys Met Glu Trp
5 10

<210> 350
<211> 32
<212> PRT
<213> Homo sapiens

<400> 350

Arg Ile Ser Ile Thr Arg Asp Thr Ser Lys Asn Gln Phe Phe Leu Gln
5 10 15

Leu Asn Ser Val Thr Thr Glu Asp Thr Ala Thr Tyr Tyr Cys Ala Arg
20 25 30

<210> 351
<211> 11
<212> PRT
<213> Homo sapiens

<400> 351

```
Trp Gly Gln Gly Thr Leu Val Thr Val Ser Ser
                5                   10
```

<210> 352
<211> 321
<212> DNA
<213> Homo sapiens

<400> 352

```
gatgtccaga tgacccagtc tccgtcttat cttgctgcgt ctcctggaga aagtgtttcc      60

atcagttgca aggcaagtaa gatcattact aattatttag cctggtatca acagaaacct     120

ggggaaccat ataaccttct tatccactct gggtcaactt tgcaatctgg cactccatca     180

aggttcagtg gcagtagatc tggtacagat ttcactctca ccatcagaag cctgcagcct     240

gaagattttg gactctatta ctgccaacag tattatgaaa acccgtacac gtttggagct     300

gggaccaagc tggaactgaa a                                               321
```

<210> 353
<211> 107
<212> PRT
<213> Homo sapiens

<400> 353

```
Asp Val Gln Met Thr Gln Ser Pro Ser Tyr Leu Ala Ala Ser Pro Gly
                5                   10                  15

Glu Ser Val Ser Ile Ser Cys Lys Ala Ser Lys Ile Ile Thr Asn Tyr
            20                  25                  30

Leu Ala Trp Tyr Gln Gln Lys Pro Gly Glu Pro Tyr Asn Leu Leu Ile
        35                  40                  45

His Ser Gly Ser Thr Leu Gln Ser Gly Thr Pro Ser Arg Phe Ser Gly
    50                  55                  60

Ser Arg Ser Gly Thr Asp Phe Thr Leu Thr Ile Arg Ser Leu Gln Pro
65                  70                  75                  80

Glu Asp Phe Gly Leu Tyr Tyr Cys Gln Gln Tyr Tyr Glu Asn Pro Tyr
                85                  90                  95

Thr Phe Gly Ala Gly Thr Lys Leu Glu Leu Lys
            100                 105
```

<210> 354
<211> 11
<212> PRT
<213> Homo sapiens

<400> 354

Lys Ala Ser Lys Ile Ile Thr Asn Tyr Leu Ala
5 10

<210> 355
<211> 7
<212> PRT
<213> Homo sapiens

<400> 355

Ser Gly Ser Thr Leu Gln Ser
5

<210> 356
<211> 9
<212> PRT
<213> Homo sapiens

<400> 356

Gln Gln Tyr Tyr Glu Asn Pro Tyr Thr
5

<210> 357
<211> 23
<212> PRT
<213> Homo sapiens

<400> 357

Asp Val Gln Met Thr Gln Ser Pro Ser Tyr Leu Ala Ala Ser Pro Gly
5 10 15

Glu Ser Val Ser Ile Ser Cys
20

<210> 358
<211> 15
<212> PRT
<213> Homo sapiens

<400> 358

Trp Tyr Gln Gln Lys Pro Gly Glu Pro Tyr Asn Leu Leu Ile His
5 10 15

<210> 359
<211> 32
<212> PRT
<213> Homo sapiens

<400> 359

Gly Thr Pro Ser Arg Phe Ser Gly Ser Arg Ser Gly Thr Asp Phe Thr
5 10 15

Leu Thr Ile Arg Ser Leu Gln Pro Glu Asp Phe Gly Leu Tyr Tyr Cys
20 25 30

<210> 360
<211> 10
<212> PRT
<213> Homo sapiens

<400> 360

```
Phe Gly Ala Gly Thr Lys Leu Glu Leu Lys
                5                   10
```

<210> 361
<211> 366
<212> DNA
<213> Homo sapiens

<400> 361

```
caggtgcagc tgaaggagtc aggacctggc ctggtgaagc cctcagagac cctgtctctc      60
acctgcactg tctctgggtt ctcattaacc agctatcatg tgagctgggt tcgacagcct     120
ccaggaaagg gtctggaatg gatgggagta atatggggtg atggaagcac agcatttaat     180
tcagctctca aatcccgact gagcatcagc agggacacct cgaagagcca agttttctta     240
aaaatgaaca gtctgcaaac tgaagacaca gccacttact actgtgccag agccggggtt     300
tactatgatg gtagttatta ctactttgct tactggggcc aaggcactct ggtcactgtc     360
tcttca                                                                366
```

<210> 362
<211> 122
<212> PRT
<213> Homo sapiens

<400> 362

Gln Val Gln Leu Lys Glu Ser Gly Pro Gly Leu Val Lys Pro Ser Glu
5 10 15

Thr Leu Ser Leu Thr Cys Thr Val Ser Gly Phe Ser Leu Thr Ser Tyr
20 25 30

His Val Ser Trp Val Arg Gln Pro Pro Gly Lys Gly Leu Glu Trp Met
35 40 45

Gly Val Ile Trp Gly Asp Gly Ser Thr Ala Phe Asn Ser Ala Leu Lys
50 55 60

Ser Arg Leu Ser Ile Ser Arg Asp Thr Ser Lys Ser Gln Val Phe Leu
65 70 75 80

Lys Met Asn Ser Leu Gln Thr Glu Asp Thr Ala Thr Tyr Tyr Cys Ala
85 90 95

Arg Ala Gly Val Tyr Tyr Asp Gly Ser Tyr Tyr Tyr Phe Ala Tyr Trp
100 105 110

Gly Gln Gly Thr Leu Val Thr Val Ser Ser
115 120

<210> 363
<211> 5
<212> PRT
<213> Homo sapiens

<400> 363

Ser Tyr His Val Ser
5

<210> 364
<211> 16
<212> PRT
<213> Homo sapiens

<400> 364

Val Ile Trp Gly Asp Gly Ser Thr Ala Phe Asn Ser Ala Leu Lys Ser
5 10 15

<210> 365
<211> 14
<212> PRT
<213> Homo sapiens

<400> 365

Ala Gly Val Tyr Tyr Asp Gly Ser Tyr Tyr Tyr Phe Ala Tyr
5 10

<210> 366
<211> 30
<212> PRT
<213> Homo sapiens

<400> 366

```
Gln Val Gln Leu Lys Glu Ser Gly Pro Gly Leu Val Lys Pro Ser Glu
                5                   10                  15

Thr Leu Ser Leu Thr Cys Thr Val Ser Gly Phe Ser Leu Thr
            20                  25                  30
```

<210> 367
<211> 14
<212> PRT
<213> Homo sapiens

<400> 367

```
Trp Val Arg Gln Pro Pro Gly Lys Gly Leu Glu Trp Met Gly
                5                   10
```

<210> 368
<211> 32
<212> PRT
<213> Homo sapiens

<400> 368

```
Arg Leu Ser Ile Ser Arg Asp Thr Ser Lys Ser Gln Val Phe Leu Lys
                5                   10                  15

Met Asn Ser Leu Gln Thr Glu Asp Thr Ala Thr Tyr Tyr Cys Ala Arg
            20                  25                  30
```

<210> 369
<211> 11
<212> PRT
<213> Homo sapiens

<400> 369

```
Trp Gly Gln Gly Thr Leu Val Thr Val Ser Ser
                5                   10
```

<210> 370
<211> 321
<212> DNA
<213> Homo sapiens

<400> 370

```
gacatccaga tgacacagtc tcctgcctcc ctgtctgcat ctctgggaga aattgtcacc      60

atcacatgcc aggcaagcca agacattggt aattggttgg catggtatca gcagaaaccg     120
```

gggaaatctc ctcacctcct gatttatgat gcaaccacct tggcagatgg ggtcccatca 180

cggttcagcg gcagtagatc tggcacacag tattctctta agatcagcag actacaagtt 240

gaagatattg gaagctatta ctgtcaaaag gctcatagta atccgtggac gttcggtgga 300

ggcaccaagc tggaattgaa a 321

<210> 371
<211> 107
<212> PRT
<213> Homo sapiens

<400> 371

Asp Ile Gln Met Thr Gln Ser Pro Ala Ser Leu Ser Ala Ser Leu Gly
1 5 10 15

Glu Ile Val Thr Ile Thr Cys Gln Ala Ser Gln Asp Ile Gly Asn Trp
20 25 30

Leu Ala Trp Tyr Gln Gln Lys Pro Gly Lys Ser Pro His Leu Leu Ile
35 40 45

Tyr Asp Ala Thr Thr Leu Ala Asp Gly Val Pro Ser Arg Phe Ser Gly
50 55 60

Ser Arg Ser Gly Thr Gln Tyr Ser Leu Lys Ile Ser Arg Leu Gln Val
65 70 75 80

Glu Asp Ile Gly Ser Tyr Tyr Cys Gln Lys Ala His Ser Asn Pro Trp
85 90 95

Thr Phe Gly Gly Gly Thr Lys Leu Glu Leu Lys
100 105

<210> 372
<211> 11
<212> PRT
<213> Homo sapiens

<400> 372

Gln Ala Ser Gln Asp Ile Gly Asn Trp Leu Ala
1 5 10

<210> 373
<211> 7
<212> PRT
<213> Homo sapiens

<400> 373

Asp Ala Thr Thr Leu Ala Asp
1 5

<210> 374
<211> 9
<212> PRT

<213> Homo sapiens

<400> 374

```
Gln Lys Ala His Ser Asn Pro Trp Thr
                5
```

<210> 375
<211> 23
<212> PRT
<213> Homo sapiens

<400> 375

```
Asp Ile Gln Met Thr Gln Ser Pro Ala Ser Leu Ser Ala Ser Leu Gly
                5                   10                  15

Glu Ile Val Thr Ile Thr Cys
            20
```

<210> 376
<211> 15
<212> PRT
<213> Homo sapiens

<400> 376

```
Trp Tyr Gln Gln Lys Pro Gly Lys Ser Pro His Leu Leu Ile Tyr
                5                   10                  15
```

<210> 377
<211> 32
<212> PRT
<213> Homo sapiens

<400> 377

```
Gly Val Pro Ser Arg Phe Ser Gly Ser Arg Ser Gly Thr Gln Tyr Ser
                5                   10                  15

Leu Lys Ile Ser Arg Leu Gln Val Glu Asp Ile Gly Ser Tyr Tyr Cys
            20                  25                  30
```

<210> 378
<211> 10
<212> PRT
<213> Homo sapiens

<400> 378

```
Phe Gly Gly Gly Thr Lys Leu Glu Leu Lys
                5                   10
```

<210> 379
<211> 375
<212> DNA
<213> Homo sapiens

<400> 379

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgtgc gcggcactgg     300
gactggtgga gtacttattc gggtccaact gcttttgatc tctggggcag aggcaccctg     360
gtcaccgtct cgagt                                                      375
```

<210> 380
<211> 125
<212> PRT
<213> Homo sapiens

<400> 380

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Trp Asp Trp Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 381
<211> 5
<212> PRT
<213> Homo sapiens

<400> 381

```
Ser Tyr Ala Met Ser
                5
```

<210> 382
<211> 17

<212> PRT
<213> Homo sapiens

<400> 382

Ala Ile Ser Gly Ser Gly Gly Ser Thr Tyr Tyr Ala Asp Ser Val Lys
1               5                   10                  15

Gly

<210> 383
<211> 16
<212> PRT
<213> Homo sapiens

<400> 383

His Trp Asp Trp Trp Ser Thr Tyr Ser Gly Pro Thr Ala Phe Asp Leu
                5                   10                  15

<210> 384
<211> 30
<212> PRT
<213> Homo sapiens

<400> 384

Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30

<210> 385
<211> 14
<212> PRT
<213> Homo sapiens

<400> 385

Trp Val Arg Gln Ala Pro Gly Lys Gly Leu Glu Trp Val Ser
                5                   10

<210> 386
<211> 32
<212> PRT
<213> Homo sapiens

<400> 386

Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30

<210> 387
<211> 11

<212> PRT
<213> Homo sapiens

<400> 387

```
Trp Gly Arg Gly Thr Leu Val Thr Val Ser Ser
                5                   10
```

<210> 388
<211> 324
<212> DNA
<213> Homo sapiens

<400> 388

```
tcctatgagc tggctcagcc accctcggtg tcagtgtccc caggacagac ggccaggatc      60

acctgctctg gagatgcatt gccaaggcaa tatgcttact ggtaccagca gaagccaggc     120

caggcccctc tactggtgat atataaagac agtgagaggc cctcagggat ccctgagcga     180

ttctctggct ccggctcagg gacaacagtc acgttgacca tcagtggagt ccaggcagaa     240

gacgaggctg actattactg tcaatcagca gacagcagtg gtacctatgt ggtattcggc     300

ggagggacca aggtcaccgt ccta                                            324
```

<210> 389
<211> 108
<212> PRT
<213> Homo sapiens

<400> 389

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 390
<211> 11
<212> PRT

<213> Homo sapiens

<400> 390

Ser Gly Asp Ala Leu Pro Arg Gln Tyr Ala Tyr
1               5                   10

<210> 391
<211> 7
<212> PRT
<213> Homo sapiens

<400> 391

Lys Asp Ser Glu Arg Pro Ser
1               5

<210> 392
<211> 11
<212> PRT
<213> Homo sapiens

<400> 392

Gln Ser Ala Asp Ser Ser Gly Thr Tyr Val Val
1               5                   10

<210> 393
<211> 22
<212> PRT
<213> Homo sapiens

<400> 393

Ser Tyr Glu Leu Ala Gln Pro Pro Ser Val Ser Val Ser Pro Gly Gln
1               5                   10                  15

Thr Ala Arg Ile Thr Cys
            20

<210> 394
<211> 15
<212> PRT
<213> Homo sapiens

<400> 394

Trp Tyr Gln Gln Lys Pro Gly Gln Ala Pro Leu Leu Val Ile Tyr
1               5                   10                  15

<210> 395
<211> 32
<212> PRT
<213> Homo sapiens

<400> 395

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 396
<211> 10
<212> PRT
<213> Homo sapiens

<400> 396

Phe Gly Gly Gly Thr Lys Val Thr Val Leu
5 10

<210> 397
<211> 375
<212> DNA
<213> Homo sapiens

<400> 397

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgtgc cagggacatg 300

gactactgga gtacttattc gggtccaact gcttttgatc tctggggcag aggcaccctg 360

gtcaccgtct cgagt 375

<210> 398
<211> 125
<212> PRT
<213> Homo sapiens

<400> 398

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Asp Met Asp Tyr Trp Ser Thr Tyr Ser Gly Pro Thr Ala Phe
100 105 110

Asp Leu Trp Gly Arg Gly Thr Leu Val Thr Val Ser Ser
115 120 125

<210> 399
<211> 5
<212> PRT
<213> Homo sapiens

<400> 399

Ser Tyr Ala Met Ser
5

<210> 400
<211> 17
<212> PRT
<213> Homo sapiens

<400> 400

Ala Ile Ser Gly Ser Gly Gly Ser Thr Tyr Tyr Ala Asp Ser Val Lys
5 10 15

Gly

<210> 401
<211> 16
<212> PRT
<213> Homo sapiens

<400> 401

Asp Met Asp Tyr Trp Ser Thr Tyr Ser Gly Pro Thr Ala Phe Asp Leu
5 10 15

<210> 402
<211> 30
<212> PRT

<213> Homo sapiens

<400> 402

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 403
<211> 14
<212> PRT
<213> Homo sapiens

<400> 403

```
Trp Val Arg Gln Ala Pro Gly Lys Gly Leu Glu Trp Val Ser
                5                   10
```

<210> 404
<211> 32
<212> PRT
<213> Homo sapiens

<400> 404

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 405
<211> 11
<212> PRT
<213> Homo sapiens

<400> 405

```
Trp Gly Arg Gly Thr Leu Val Thr Val Ser Ser
                5                   10
```

<210> 406
<211> 324
<212> DNA
<213> Homo sapiens

<400> 406

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 407
<211> 108
<212> PRT
<213> Homo sapiens

<400> 407

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 408
<211> 11
<212> PRT
<213> Homo sapiens

<400> 408

```
Ser Gly Asp Ala Leu Pro Arg Gln Tyr Ala Tyr
                5                   10
```

<210> 409
<211> 7
<212> PRT
<213> Homo sapiens

<400> 409

```
Lys Asp Ser Glu Arg Pro Ser
                5
```

<210> 410
<211> 11
<212> PRT
<213> Homo sapiens

<400> 410

```
Gln Ser Ala Asp Ser Ser Gly Thr Tyr Val Val
                1                   5                   10
```

<210> 411
<211> 22
<212> PRT
<213> Homo sapiens

<400> 411

```
Ser Tyr Glu Leu Ala Gln Pro Pro Ser Val Ser Val Ser Pro Gly Gln
1               5                   10                  15

Thr Ala Arg Ile Thr Cys
            20
```

<210> 412
<211> 15
<212> PRT
<213> Homo sapiens

<400> 412

```
Trp Tyr Gln Gln Lys Pro Gly Gln Ala Pro Leu Leu Val Ile Tyr
1               5                   10                  15
```

<210> 413
<211> 32
<212> PRT
<213> Homo sapiens

<400> 413

```
Gly Ile Pro Glu Arg Phe Ser Gly Ser Gly Ser Gly Thr Thr Val Thr
1               5                   10                  15

Leu Thr Ile Ser Gly Val Gln Ala Glu Asp Glu Ala Asp Tyr Tyr Cys
            20                  25                  30
```

<210> 414
<211> 10
<212> PRT
<213> Homo sapiens

<400> 414

```
Phe Gly Gly Gly Thr Lys Val Thr Val Leu
1               5                   10
```

<210> 415
<211> 375

<212> DNA
<213> Homo sapiens

<400> 415

```
gaggtgcagc tgttggagtc tgggggaggc ttggtacagc ctggggggtc cctgagactc      60

tcctgtgcag cctctggatt cacctttagc agctatgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcaaatga acagcctgag agccgaggac acggccgtgt attactgtgc ccgggaccac     300

gactactgga gtacttattc gggtccaact gcttttgatc tctggggcag aggcaccctg     360

gtcaccgtct cgagt                                                      375
```

<210> 416
<211> 125
<212> PRT
<213> Homo sapiens

<400> 416

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp His Asp Tyr Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 417
<211> 5
<212> PRT
<213> Homo sapiens

<400> 417

```
Ser Tyr Ala Met Ser
                5
```

<210> 418
<211> 17
<212> PRT
<213> Homo sapiens

<400> 418

```
Ala Ile Ser Gly Ser Gly Gly Ser Thr Tyr Tyr Ala Asp Ser Val Lys
                5                   10                  15

Gly
```

<210> 419
<211> 16
<212> PRT
<213> Homo sapiens

<400> 419

```
Asp His Asp Tyr Trp Ser Thr Tyr Ser Gly Pro Thr Ala Phe Asp Leu
                5                   10                  15
```

<210> 420
<211> 30
<212> PRT
<213> Homo sapiens

<400> 420

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 421
<211> 14
<212> PRT
<213> Homo sapiens

<400> 421

```
    Trp Val Arg Gln Ala Pro Gly Lys Gly Leu Glu Trp Val Ser
                    5                   10
```

<210> 422
<211> 32
<212> PRT
<213> Homo sapiens

<400> 422

Arg Phe Thr Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ala Arg
20 25 30

<210> 423
<211> 11
<212> PRT
<213> Homo sapiens

<400> 423

Trp Gly Arg Gly Thr Leu Val Thr Val Ser Ser
5 10

<210> 424
<211> 324
<212> DNA
<213> Homo sapiens

<400> 424

tcctatgagc tggctcagcc accctcggtg tcagtgtccc caggacagac ggccaggatc 60

acctgctctg gagatgcatt gccaaggcaa tatgcttact ggtaccagca gaagccaggc 120

caggcccctc tactggtgat atataaagac agtgagaggc cctcagggat ccctgagcga 180

ttctctggct ccggctcagg gacaacagtc acgttgacca tcagtggagt ccaggcagaa 240

gacgaggctg actattactg tcaatcagca gacagcagtg gtacctatgt ggtattcggc 300

ggagggacca aggtcaccgt ccta 324

<210> 425
<211> 108
<212> PRT
<213> Homo sapiens

<400> 425

Ser Tyr Glu Leu Ala Gln Pro Pro Ser Val Ser Val Ser Pro Gly Gln
5 10 15

Thr Ala Arg Ile Thr Cys Ser Gly Asp Ala Leu Pro Arg Gln Tyr Ala
20 25 30

Tyr Trp Tyr Gln Gln Lys Pro Gly Gln Ala Pro Leu Leu Val Ile Tyr
35 40 45

Lys Asp Ser Glu Arg Pro Ser Gly Ile Pro Glu Arg Phe Ser Gly Ser
50 55 60

Gly Ser Gly Thr Thr Val Thr Leu Thr Ile Ser Gly Val Gln Ala Glu
65 70 75 80

Asp Glu Ala Asp Tyr Tyr Cys Gln Ser Ala Asp Ser Ser Gly Thr Tyr
85 90 95

Val Val Phe Gly Gly Gly Thr Lys Val Thr Val Leu
100 105

<210> 426
<211> 11
<212> PRT
<213> Homo sapiens

<400> 426

Ser Gly Asp Ala Leu Pro Arg Gln Tyr Ala Tyr
5 10

<210> 427
<211> 7
<212> PRT
<213> Homo sapiens

<400> 427

Lys Asp Ser Glu Arg Pro Ser
5

<210> 428
<211> 11
<212> PRT
<213> Homo sapiens

<400> 428

Gln Ser Ala Asp Ser Ser Gly Thr Tyr Val Val
5 10

<210> 429
<211> 22
<212> PRT
<213> Homo sapiens

<400> 429

Ser Tyr Glu Leu Ala Gln Pro Pro Ser Val Ser Val Ser Pro Gly Gln
5 10 15

Thr Ala Arg Ile Thr Cys
20

<210> 430
<211> 15
<212> PRT
<213> Homo sapiens

<400> 430

Trp Tyr Gln Gln Lys Pro Gly Gln Ala Pro Leu Leu Val Ile Tyr
5 10 15

<210> 431
<211> 32
<212> PRT
<213> Homo sapiens

<400> 431

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 432
<211> 10
<212> PRT
<213> Homo sapiens

<400> 432

Phe Gly Gly Gly Thr Lys Val Thr Val Leu
5 10

<210> 433
<211> 375
<212> DNA
<213> Homo sapiens

<400> 433

```
gaggtgcagc tgttggagtc tgggggaggc ttggtacagc ctggggggtc cctgagactc      60

tcctgtgcag cctctggatt cacctttagc agctatgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcaaatga acagcctgag agccgaggac acggccgtgt attactgttc gcggcagttc     300

gactactgga gtacttattc gggtccaact gcttttgatc tctggggcag aggcaccctg     360

gtcaccgtct cgagt                                                      375
```

<210> 434
<211> 125
<212> PRT
<213> Homo sapiens

<400> 434

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ser Arg Gln Phe Asp Tyr Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 435
<211> 5
<212> PRT
<213> Homo sapiens

<400> 435

```
Ser Tyr Ala Met Ser
                5
```

<210> 436
<211> 17
<212> PRT
<213> Homo sapiens

<400> 436

Ala Ile Ser Gly Ser Gly Gly Ser Thr Tyr Tyr Ala Asp Ser Val Lys
5 10 15

Gly

<210> 437
<211> 16
<212> PRT
<213> Homo sapiens

<400> 437

Gln Phe Asp Tyr Trp Ser Thr Tyr Ser Gly Pro Thr Ala Phe Asp Leu
5 10 15

<210> 438
<211> 30
<212> PRT
<213> Homo sapiens

<400> 438

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 439
<211> 14
<212> PRT
<213> Homo sapiens

<400> 439

Trp Val Arg Gln Ala Pro Gly Lys Gly Leu Glu Trp Val Ser
5 10

<210> 440
<211> 32
<212> PRT
<213> Homo sapiens

<400> 440

Arg Phe Thr Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ser Arg
20 25 30

<210> 441
<211> 11

<212> PRT
<213> Homo sapiens

<400> 441

```
Trp Gly Arg Gly Thr Leu Val Thr Val Ser Ser
                5                   10
```

<210> 442
<211> 324
<212> DNA
<213> Homo sapiens

<400> 442

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 443
<211> 108
<212> PRT
<213> Homo sapiens

<400> 443

```
Ser Tyr Glu Leu Ala Gln Pro Pro Ser Val Ser Val Ser Pro Gly Gln
                5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Glu Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Val Val Phe Gly Gly Gly Thr Lys Val Thr Val Leu
            100                 105
```

<210> 444
<211> 11
<212> PRT
<213> Homo sapiens

<400> 444

Ser Gly Asp Ala Leu Pro Arg Gln Tyr Ala Tyr
5 10

<210> 445
<211> 7
<212> PRT
<213> Homo sapiens

<400> 445

Lys Asp Ser Glu Arg Pro Ser

5

<210> 446
<211> 11
<212> PRT
<213> Homo sapiens

<400> 446

Gln Ser Ala Asp Ser Ser Gly Thr Tyr Val Val
5 10

<210> 447
<211> 22
<212> PRT
<213> Homo sapiens

<400> 447

Ser Tyr Glu Leu Ala Gln Pro Pro Ser Val Ser Val Ser Pro Gly Gln
5 10 15

Thr Ala Arg Ile Thr Cys
20

<210> 448
<211> 15
<212> PRT
<213> Homo sapiens

<400> 448

Trp Tyr Gln Gln Lys Pro Gly Gln Ala Pro Leu Leu Val Ile Tyr
5 10 15

<210> 449
<211> 32
<212> PRT
<213> Homo sapiens

<400> 449

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 450
<211> 10
<212> PRT
<213> Homo sapiens

<400> 450

Phe Gly Gly Gly Thr Lys Val Thr Val Leu
5 10

<210> 451
<211> 375
<212> DNA
<213> Homo sapiens

<400> 451

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgtgc gagagatagg 300

gatttttgga gtaaccggcc gtcccccacc gcttttgatc tctggggcag aggcaccctg 360

gtcaccgtct cgagt 375

<210> 452
<211> 125
<212> PRT
<213> Homo sapiens

<400> 452

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Asp Arg Asp Phe Trp Ser Asn Arg Pro Ser Pro Thr Ala Phe
100 105 110

Asp Leu Trp Gly Arg Gly Thr Leu Val Thr Val Ser Ser
115 120 125

<210> 453
<211> 5
<212> PRT
<213> Homo sapiens

<400> 453

Ser Tyr Ala Met Ser
5

<210> 454
<211> 17
<212> PRT
<213> Homo sapiens

<400> 454

Ala Ile Ser Gly Ser Gly Gly Ser Thr Tyr Tyr Ala Asp Ser Val Lys
5 10 15

Gly

<210> 455
<211> 16
<212> PRT
<213> Homo sapiens

<400> 455

Asp Arg Asp Phe Trp Ser Asn Arg Pro Ser Pro Thr Ala Phe Asp Leu
5 10 15

<210> 456
<211> 30
<212> PRT

<213> Homo sapiens

<400> 456

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 457
<211> 14
<212> PRT
<213> Homo sapiens

<400> 457

```
Trp Val Arg Gln Ala Pro Gly Lys Gly Leu Glu Trp Val Ser
                5                   10
```

<210> 458
<211> 32
<212> PRT
<213> Homo sapiens

<400> 458

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 459
<211> 11
<212> PRT
<213> Homo sapiens

<400> 459

```
Trp Gly Arg Gly Thr Leu Val Thr Val Ser Ser
                5                   10
```

<210> 460
<211> 324
<212> DNA
<213> Homo sapiens

<400> 460

```
tcctatgagc tggctcagcc accctcggtg tcagtgtccc caggacagac ggccaggatc      60

acctgctctg gagatgcatt gccaaggcaa tatgcttact ggtaccagca gaagccaggc     120

caggcccctc tactggtgat atataaagac agtgagaggc cctcagggat ccctgagcga     180

ttctctggct ccggctcagg gacaacagtc acgttgacca tcagtggagt ccaggcagaa     240

gacgaggctg actattactg tcaatcagca gacagcagtg gtacctatgt ggtattcggc     300

ggagggacca aggtcaccgt ccta                                            324
```

<210> 461
<211> 108
<212> PRT
<213> Homo sapiens

<400> 461

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 462
<211> 11
<212> PRT
<213> Homo sapiens

<400> 462

```
Ser Gly Asp Ala Leu Pro Arg Gln Tyr Ala Tyr
                5                   10
```

<210> 463
<211> 7
<212> PRT
<213> Homo sapiens

<400> 463

```
Lys Asp Ser Glu Arg Pro Ser
                5
```

<210> 464
<211> 11
<212> PRT
<213> Homo sapiens

<400> 464

```
Gln Ser Ala Asp Ser Ser Gly Thr Tyr Val Val
                5                   10
```

<210> 465
<211> 22
<212> PRT
<213> Homo sapiens

<400> 465

```
Ser Tyr Glu Leu Ala Gln Pro Pro Ser Val Ser Val Ser Pro Gly Gln
                5                   10                  15

Thr Ala Arg Ile Thr Cys
            20
```

<210> 466
<211> 15
<212> PRT
<213> Homo sapiens

<400> 466

```
Trp Tyr Gln Gln Lys Pro Gly Gln Ala Pro Leu Leu Val Ile Tyr
                5                   10                  15
```

<210> 467
<211> 32
<212> PRT
<213> Homo sapiens

<400> 467

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 468
<211> 10
<212> PRT
<213> Homo sapiens

<400> 468

```
Phe Gly Gly Gly Thr Lys Val Thr Val Leu
                5                   10
```

<210> 469
<211> 375

<212> DNA
<213> Homo sapiens

<400> 469

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgtgc gagagatagg     300
gatttttgga gtggcgcgaa ccggatgacc gcttttgatc tctggggcag aggcaccctg     360
gtcaccgtct cgagt                                                      375
```

<210> 470
<211> 125
<212> PRT
<213> Homo sapiens

<400> 470

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Gly Ala Asn Arg Met Thr Ala Phe
            100                 105                 110

Asp Leu Trp Gly Arg Gly Thr Leu Val Thr Val Ser Ser
        115                 120                 125
```

<210> 471
<211> 5
<212> PRT
<213> Homo sapiens

<400> 471

```
Ser Tyr Ala Met Ser
                5
```

<210> 472
<211> 17
<212> PRT
<213> Homo sapiens

<400> 472

```
Ala Ile Ser Gly Ser Gly Gly Ser Thr Tyr Tyr Ala Asp Ser Val Lys
                5                   10                  15

Gly
```

<210> 473
<211> 16
<212> PRT
<213> Homo sapiens

<400> 473

```
Asp Arg Asp Phe Trp Ser Gly Ala Asn Arg Met Thr Ala Phe Asp Leu
                5                   10                  15
```

<210> 474
<211> 30
<212> PRT
<213> Homo sapiens

<400> 474

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 475
<211> 14
<212> PRT
<213> Homo sapiens

<400> 475

```
    Trp Val Arg Gln Ala Pro Gly Lys Gly Leu Glu Trp Val Ser
                    5                   10
```

<210> 476
<211> 32
<212> PRT
<213> Homo sapiens

<400> 476

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 477
<211> 11
<212> PRT
<213> Homo sapiens

<400> 477

```
Trp Gly Arg Gly Thr Leu Val Thr Val Ser Ser

                5                   10
```

<210> 478
<211> 324
<212> DNA
<213> Homo sapiens

<400> 478

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 479
<211> 108
<212> PRT
<213> Homo sapiens

<400> 479

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 480
<211> 11
<212> PRT
<213> Homo sapiens

<400> 480

Ser Gly Asp Ala Leu Pro Arg Gln Tyr Ala Tyr
1               5                   10

<210> 481
<211> 7
<212> PRT
<213> Homo sapiens

<400> 481

Lys Asp Ser Glu Arg Pro Ser
1               5

<210> 482
<211> 11
<212> PRT
<213> Homo sapiens

<400> 482

Gln Ser Ala Asp Ser Ser Gly Thr Tyr Val Val
1               5                   10

<210> 483
<211> 22
<212> PRT
<213> Homo sapiens

<400> 483

Ser Tyr Glu Leu Ala Gln Pro Pro Ser Val Ser Val Ser Pro Gly Gln
1               5                   10                  15

Thr Ala Arg Ile Thr Cys
            20

<210> 484
<211> 15
<212> PRT
<213> Homo sapiens

<400> 484

Trp Tyr Gln Gln Lys Pro Gly Gln Ala Pro Leu Leu Val Ile Tyr
1               5                   10                  15

<210> 485
<211> 32
<212> PRT
<213> Homo sapiens

<400> 485

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 486
<211> 10
<212> PRT
<213> Homo sapiens

<400> 486

```
Phe Gly Gly Gly Thr Lys Val Thr Val Leu
                5                   10
```

<210> 487
<211> 375
<212> DNA
<213> Homo sapiens

<400> 487

```
gaggtgcagc tgttggagtc tgggggaggc ttggtacagc ctggggggtc cctgagactc      60

tcctgtgcag cctctggatt cacctttagc agctatgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcaaatga acagcctgag agccgaggac acggccgtgt attactgtgc gagagatagg     300

gatttttgga gtggctccag caaggccacg gcttttgatc tctggggcag aggcaccctg     360

gtcaccgtct cgagt                                                      375
```

<210> 488
<211> 125
<212> PRT
<213> Homo sapiens

<400> 488

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Gly Ser Ser Lys Ala Thr Ala Phe
            100                 105                 110

Asp Leu Trp Gly Arg Gly Thr Leu Val Thr Val Ser Ser
        115                 120                 125
```

<210> 489
<211> 5
<212> PRT
<213> Homo sapiens

<400> 489

```
Ser Tyr Ala Met Ser
                5
```

<210> 490
<211> 17
<212> PRT
<213> Homo sapiens

<400> 490

```
Ala Ile Ser Gly Ser Gly Gly Ser Thr Tyr Tyr Ala Asp Ser Val Lys
                5                   10                  15

Gly
```

<210> 491
<211> 16
<212> PRT
<213> Homo sapiens

<400> 491

```
Asp Arg Asp Phe Trp Ser Gly Ser Ser Lys Ala Thr Ala Phe Asp Leu
                5                   10                  15
```

<210> 492
<211> 30
<212> PRT

<213> Homo sapiens

<400> 492

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 493
<211> 14
<212> PRT
<213> Homo sapiens

<400> 493

```
Trp Val Arg Gln Ala Pro Gly Lys Gly Leu Glu Trp Val Ser
                5                   10
```

<210> 494
<211> 32
<212> PRT
<213> Homo sapiens

<400> 494

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 495
<211> 11
<212> PRT
<213> Homo sapiens

<400> 495

```
Trp Gly Arg Gly Thr Leu Val Thr Val Ser Ser
                5                   10
```

<210> 496
<211> 324
<212> DNA
<213> Homo sapiens

<400> 496

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 497
<211> 108
<212> PRT
<213> Homo sapiens

<400> 497

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 498
<211> 11
<212> PRT
<213> Homo sapiens

<400> 498

```
Ser Gly Asp Ala Leu Pro Arg Gln Tyr Ala Tyr
                5                   10
```

<210> 499
<211> 7
<212> PRT
<213> Homo sapiens

<400> 499

```
Lys Asp Ser Glu Arg Pro Ser
                5
```

<210> 500
<211> 11
<212> PRT
<213> Homo sapiens

<400> 500

```
Gln Ser Ala Asp Ser Ser Gly Thr Tyr Val Val
                1                   5                   10
```

<210> 501
<211> 22
<212> PRT
<213> Homo sapiens

<400> 501

```
Ser Tyr Glu Leu Ala Gln Pro Pro Ser Val Ser Val Ser Pro Gly Gln
                5                   10                  15

Thr Ala Arg Ile Thr Cys
            20
```

<210> 502
<211> 15
<212> PRT
<213> Homo sapiens

<400> 502

```
Trp Tyr Gln Gln Lys Pro Gly Gln Ala Pro Leu Leu Val Ile Tyr
                5                   10                  15
```

<210> 503
<211> 32
<212> PRT
<213> Homo sapiens

<400> 503

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 504
<211> 10
<212> PRT
<213> Homo sapiens

<400> 504

```
Phe Gly Gly Gly Thr Lys Val Thr Val Leu
                5                   10
```

<210> 505
<211> 375

<212> DNA
<213> Homo sapiens

<400> 505

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgtgc gagagatagg     300
gatttttgga gtcccgggac cggcctcacc gcttttgatc tctggggcag aggcaccctg     360
gtcaccgtct cgagt                                                      375
```

<210> 506
<211> 125
<212> PRT
<213> Homo sapiens

<400> 506

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Pro Gly Thr Gly Leu Thr Ala Phe
            100                 105                 110

Asp Leu Trp Gly Arg Gly Thr Leu Val Thr Val Ser Ser
        115                 120                 125
```

<210> 507
<211> 5
<212> PRT
<213> Homo sapiens

<400> 507

```
Ser Tyr Ala Met Ser
                5
```

<210> 508
<211> 17
<212> PRT
<213> Homo sapiens

<400> 508

```
Ala Ile Ser Gly Ser Gly Gly Ser Thr Tyr Tyr Ala Asp Ser Val Lys
                5                   10                  15

Gly
```

<210> 509
<211> 16
<212> PRT
<213> Homo sapiens

<400> 509

```
Asp Arg Asp Phe Trp Ser Pro Gly Thr Gly Leu Thr Ala Phe Asp Leu
                5                   10                  15
```

<210> 510
<211> 30
<212> PRT
<213> Homo sapiens

<400> 510

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 511
<211> 14
<212> PRT
<213> Homo sapiens

<400> 511

```
    Trp Val Arg Gln Ala Pro Gly Lys Gly Leu Glu Trp Val Ser
                    5                   10
```

<210> 512
<211> 32
<212> PRT
<213> Homo sapiens

<400> 512

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 513
<211> 11
<212> PRT
<213> Homo sapiens

<400> 513

```
Trp Gly Arg Gly Thr Leu Val Thr Val Ser Ser
                5                   10
```

<210> 514
<211> 324
<212> DNA
<213> Homo sapiens

<400> 514

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 515
<211> 108
<212> PRT
<213> Homo sapiens

<400> 515

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 516
<211> 11

<212> PRT
<213> Homo sapiens

<400> 516

Ser Gly Asp Ala Leu Pro Arg Gln Tyr Ala Tyr
1 5 10

<210> 517
<211> 7
<212> PRT
<213> Homo sapiens

<400> 517

Lys Asp Ser Glu Arg Pro Ser
5

<210> 518
<211> 11
<212> PRT
<213> Homo sapiens

<400> 518

Gln Ser Ala Asp Ser Ser Gly Thr Tyr Val Val
5 10

<210> 519
<211> 22
<212> PRT
<213> Homo sapiens

<400> 519

Ser Tyr Glu Leu Ala Gln Pro Pro Ser Val Ser Val Ser Pro Gly Gln
5 10 15

Thr Ala Arg Ile Thr Cys
20

<210> 520
<211> 15
<212> PRT
<213> Homo sapiens

<400> 520

Trp Tyr Gln Gln Lys Pro Gly Gln Ala Pro Leu Leu Val Ile Tyr
5 10 15

<210> 521
<211> 32
<212> PRT
<213> Homo sapiens

<400> 521

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 522
<211> 10
<212> PRT
<213> Homo sapiens

<400> 522

Phe Gly Gly Gly Thr Lys Val Thr Val Leu
5 10

<210> 523
<211> 375
<212> DNA
<213> Homo sapiens

<400> 523

gaggtgcagc tgctggagtc tgggggaggc ttggtacagc ctggggggtc cctgagactc 60

tcctgtgcag cctctggatt cacctttagc agctttgcca tgagctgggt ccgccaggct 120

cctgggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgtgc gagagatagg 300

gatttttgga gtacttattc gggtccaact gcttttgatc tctggggcag aggcaccctg 360

gtcaccgtct cctca 375

<210> 524
<211> 125
<212> PRT
<213> Homo sapiens

<400> 524

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Phe
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Asp Arg Asp Phe Trp Ser Thr Tyr Ser Gly Pro Thr Ala Phe
100 105 110

Asp Leu Trp Gly Arg Gly Thr Leu Val Thr Val Ser Ser
115 120 125

<210> 525
<211> 5
<212> PRT
<213> Homo sapiens

<400> 525

Ser Phe Ala Met Ser

5

<210> 526
<211> 17
<212> PRT
<213> Homo sapiens

<400> 526

Ala Ile Ser Gly Ser Gly Gly Ser Thr Tyr Tyr Ala Asp Ser Val Lys
5 10 15

Gly

<210> 527
<211> 16
<212> PRT
<213> Homo sapiens

<400> 527

Asp Arg Asp Phe Trp Ser Thr Tyr Ser Gly Pro Thr Ala Phe Asp Leu
5 10 15

<210> 528
<211> 30
<212> PRT
<213> Homo sapiens

<400> 528

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 529
<211> 14
<212> PRT
<213> Homo sapiens

<400> 529

```
Trp Val Arg Gln Ala Pro Gly Lys Gly Leu Glu Trp Val Ser
                5                   10
```

<210> 530
<211> 32
<212> PRT
<213> Homo sapiens

<400> 530

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 531
<211> 11
<212> PRT
<213> Homo sapiens

<400> 531

```
Trp Gly Arg Gly Thr Leu Val Thr Val Ser Ser
                5                   10
```

<210> 532
<211> 324
<212> DNA
<213> Homo sapiens

<400> 532

```
tcctatgagc tggctcagcc accctcggtg tcagtgtccc caggacagac ggccaggatc      60

acctgctctg gagatgcatt gccaaggcaa tatgcttact ggtaccagca gaagccaggc     120

caggcccctc tactggtgat atataaagac agtgagaggc cctcagggat ccctgagcga     180

ttctctggct ccggctcagg gacaacagtc acgttgacca tcagtggagt ccaggcagaa     240

gacgaggctg actattactg tcaatcagca gacagcagtg gtacctatgt ggtattcggc     300

ggagggacca aggtcaccgt ccta                                            324
```

<210> 533
<211> 108
<212> PRT
<213> Homo sapiens

<400> 533

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 534
<211> 11
<212> PRT
<213> Homo sapiens

<400> 534

```
Ser Gly Asp Ala Leu Pro Arg Gln Tyr Ala Tyr

                5                   10
```

<210> 535
<211> 7
<212> PRT
<213> Homo sapiens

<400> 535

Lys Asp Ser Glu Arg Pro Ser
5

<210> 536
<211> 11
<212> PRT
<213> Homo sapiens

<400> 536

Gln Ser Ala Asp Ser Ser Gly Thr Tyr Val Val
5 10

<210> 537
<211> 22
<212> PRT
<213> Homo sapiens

<400> 537

Ser Tyr Glu Leu Ala Gln Pro Pro Ser Val Ser Val Ser Pro Gly Gln
5 10 15

Thr Ala Arg Ile Thr Cys
20

<210> 538
<211> 15
<212> PRT
<213> Homo sapiens

<400> 538

Trp Tyr Gln Gln Lys Pro Gly Gln Ala Pro Leu Leu Val Ile Tyr
5 10 15

<210> 539
<211> 32
<212> PRT
<213> Homo sapiens

<400> 539

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 540
<211> 10
<212> PRT
<213> Homo sapiens

<400> 540

Phe Gly Gly Gly Thr Lys Val Thr Val Leu
5 10

<210> 541
<211> 375
<212> DNA
<213> Homo sapiens

<400> 541

```
gaggtgcagc tgttggagtc tgggggaggc ttggtacagc ctggggggtc cctgagactc      60
tcctgtgcag cctctggatt cacctttagc agctttgcca tgagctgggt ccgccaggct     120
ccagggaagg ggctggagtg ggtctcagct attagtggta gtggtggtag cacatactac     180
gcagactccg tgaagggccg gctcaccatc tccagagaca attccaagaa cacgctgtat     240
ctgcaaatga acagcctgag agccgaggac acggccgtgt attactgtgc gagagatagg     300
gatttttgga gtacttattc gggtccaact gcttttgatc tctggggcag aggcaccctg     360
gtcaccgtct cgagt                                                      375
```

<210> 542
<211> 125
<212> PRT
<213> Homo sapiens

<400> 542

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Phe
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Leu Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 543
<211> 5
<212> PRT
<213> Homo sapiens

<400> 543

Ser Phe Ala Met Ser
5

<210> 544
<211> 17
<212> PRT
<213> Homo sapiens

<400> 544

Ala Ile Ser Gly Ser Gly Gly Ser Thr Tyr Tyr Ala Asp Ser Val Lys
1 5 10 15

Gly

<210> 545
<211> 16
<212> PRT
<213> Homo sapiens

<400> 545

Asp Arg Asp Phe Trp Ser Thr Tyr Ser Gly Pro Thr Ala Phe Asp Leu
5 10 15

<210> 546
<211> 30
<212> PRT
<213> Homo sapiens

<400> 546

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 547
<211> 14
<212> PRT
<213> Homo sapiens

<400> 547

Trp Val Arg Gln Ala Pro Gly Lys Gly Leu Glu Trp Val Ser
5 10

<210> 548
<211> 32
<212> PRT
<213> Homo sapiens

<400> 548

```
Arg Leu Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 549
<211> 11
<212> PRT
<213> Homo sapiens

<400> 549

```
Trp Gly Arg Gly Thr Leu Val Thr Val Ser Ser
                5                   10
```

<210> 550
<211> 324
<212> DNA
<213> Homo sapiens

<400> 550

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 551
<211> 108
<212> PRT
<213> Homo sapiens

<400> 551

Ser Tyr Glu Leu Ala Gln Pro Pro Ser Val Ser Val Ser Pro Gly Gln
1               5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105

<210> 552
<211> 11
<212> PRT
<213> Homo sapiens

<400> 552

Ser Gly Asp Ala Leu Pro Arg Gln Tyr Ala Tyr
                5                   10

<210> 553
<211> 7
<212> PRT
<213> Homo sapiens

<400> 553

Lys Asp Ser Glu Arg Pro Ser
                5

<210> 554
<211> 11
<212> PRT
<213> Homo sapiens

<400> 554

Gln Ser Ala Asp Ser Ser Gly Thr Tyr Val Val
                5                   10

<210> 555
<211> 22
<212> PRT
<213> Homo sapiens

<400> 555

Ser Tyr Glu Leu Ala Gln Pro Pro Ser Val Ser Val Ser Pro Gly Gln
5 10 15

Thr Ala Arg Ile Thr Cys
20

<210> 556
<211> 15
<212> PRT
<213> Homo sapiens

<400> 556

Trp Tyr Gln Gln Lys Pro Gly Gln Ala Pro Leu Leu Val Ile Tyr
5 10 15

<210> 557
<211> 32
<212> PRT
<213> Homo sapiens

<400> 557

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 558
<211> 10
<212> PRT
<213> Homo sapiens

<400> 558

Phe Gly Gly Gly Thr Lys Val Thr Val Leu
5 10

<210> 559
<211> 375
<212> DNA
<213> Homo sapiens

<400> 559

```
gaggtgcggc tgttggagtc tgggggaggc ttggtacagc ctgggggtc cctgagactc      60
tcctgtgcag cctctggatt cacctttagc agctttgcca tgagctgggt ccgccaggct     120
ccagggaagg ggctggagtg ggtctcagct attagtggta gtggtggtag cacatactac     180
gcagactccg tgaagggccg gttcaccatc tctagagaca attccaagaa cacgctgtat     240
ctgcaaatga acagcctgag agccgaggac acggccgtgt attactgtgc gagagatagg     300
gatttttgga gtacttattc gggtccaact gcttttgatc tctggggcag aggcaccctg     360
gtcaccgtct cgagt                                                      375
```

<210> 560
<211> 125
<212> PRT
<213> Homo sapiens

<400> 560

```
Glu Val Arg Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Phe
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 561
<211> 5
<212> PRT
<213> Homo sapiens

<400> 561

```
Ser Phe Ala Met Ser
                5
```

<210> 562
<211> 17
<212> PRT
<213> Homo sapiens

<400> 562

```
Ala Ile Ser Gly Ser Gly Gly Ser Thr Tyr Tyr Ala Asp Ser Val Lys
                5                   10                  15

Gly
```

<210> 563
<211> 16
<212> PRT
<213> Homo sapiens

<400> 563

```
Asp Arg Asp Phe Trp Ser Thr Tyr Ser Gly Pro Thr Ala Phe Asp Leu
                5                   10                  15
```

<210> 564
<211> 30
<212> PRT
<213> Homo sapiens

<400> 564

```
Glu Val Arg Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 565
<211> 14
<212> PRT
<213> Homo sapiens

<400> 565

```
    Trp Val Arg Gln Ala Pro Gly Lys Gly Leu Glu Trp Val Ser
                    5                   10
```

<210> 566
<211> 32
<212> PRT
<213> Homo sapiens

<400> 566

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg


          20                  25                  30
```

<210> 567
<211> 11
<212> PRT

<213> Homo sapiens

<400> 567

```
Trp Gly Arg Gly Thr Leu Val Thr Val Ser Ser
                5                   10
```

<210> 568
<211> 324
<212> DNA
<213> Homo sapiens

<400> 568

```
tcctatgagc tggctcagcc accctcggtg tcagtgtccc caggacagac ggccaggatc      60
acctgctctg gtgatgcatt gccaaggcaa tacgcttact ggtaccagca gaagccaggc     120
caggcccctc tactggtgat atataaagac agtgagaggc cctcagggat cccagagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 569
<211> 108
<212> PRT
<213> Homo sapiens

<400> 569

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 570
<211> 11
<212> PRT
<213> Homo sapiens

<400> 570

Ser Gly Asp Ala Leu Pro Arg Gln Tyr Ala Tyr
5 10

<210> 571
<211> 7
<212> PRT
<213> Homo sapiens

<400> 571

Lys Asp Ser Glu Arg Pro Ser
5

<210> 572
<211> 11
<212> PRT
<213> Homo sapiens

<400> 572

Gln Ser Ala Asp Ser Ser Gly Thr Tyr Val Val
5 10

<210> 573
<211> 22
<212> PRT
<213> Homo sapiens

<400> 573

Ser Tyr Glu Leu Ala Gln Pro Pro Ser Val Ser Val Ser Pro Gly Gln
5 10 15

Thr Ala Arg Ile Thr Cys
20

<210> 574
<211> 15
<212> PRT
<213> Homo sapiens

<400> 574

Trp Tyr Gln Gln Lys Pro Gly Gln Ala Pro Leu Leu Val Ile Tyr
5 10 15

<210> 575
<211> 32
<212> PRT
<213> Homo sapiens

<400> 575

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 576
<211> 10
<212> PRT
<213> Homo sapiens

<400> 576

Phe Gly Gly Gly Thr Lys Val Thr Val Leu
5 10

<210> 577
<211> 375
<212> DNA
<213> Homo sapiens

<400> 577

gaggtgcagc tgatggagtc tgggggaggc ttggtacagc ctggggggtc cctgaggctc 60

tcctgtgcag cctctggatt cacctttagc agctttgcca tgagctgggt ccgccaggct 120

ccagggaagg ggctggagtg ggtctcagct attagtggta gtggtggtag cacatactac 180

gcagactccg cgaagggccg gttcaccatc tccagagaca attccaagaa cacgctgtat 240

ctgcaaatga acagcctgag agccgaggac acggccgtgt attactgtgc gagagatagg 300

gatttttgga gtacttattc gggtccaact gcttttgatc tctggggcag aggcaccctg 360

gtcaccgtct cctca 375

<210> 578
<211> 125
<212> PRT
<213> Homo sapiens

<400> 578

```
Glu Val Gln Leu Met Glu Ser Gly Gly Gly Leu Val Gln Pro Gly Gly
                5                   10                  15

Ser Leu Arg Leu Ser Cys Ala Ala Ser Gly Phe Thr Phe Ser Ser Phe
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Ala
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 579
<211> 5
<212> PRT
<213> Homo sapiens

<400> 579

```
Ser Phe Ala Met Ser
                5
```

<210> 580
<211> 17
<212> PRT
<213> Homo sapiens

<400> 580

```
Ala Ile Ser Gly Ser Gly Gly Ser Thr Tyr Tyr Ala Asp Ser Ala Lys
                5                   10                  15

Gly
```

<210> 581
<211> 16
<212> PRT
<213> Homo sapiens

<400> 581

```
Asp Arg Asp Phe Trp Ser Thr Tyr Ser Gly Pro Thr Ala Phe Asp Leu
                5                   10                  15
```

<210> 582
<211> 30
<212> PRT

<213> Homo sapiens

<400> 582

```
Glu Val Gln Leu Met Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 583
<211> 14
<212> PRT
<213> Homo sapiens

<400> 583

```
Trp Val Arg Gln Ala Pro Gly Lys Gly Leu Glu Trp Val Ser
                5                   10
```

<210> 584
<211> 32
<212> PRT
<213> Homo sapiens

<400> 584

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 585
<211> 11
<212> PRT
<213> Homo sapiens

<400> 585

```
Trp Gly Arg Gly Thr Leu Val Thr Val Ser Ser
                5                   10
```

<210> 586
<211> 324
<212> DNA
<213> Homo sapiens

<400> 586

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc taccggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 587
<211> 108
<212> PRT
<213> Homo sapiens

<400> 587

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Pro Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 588
<211> 11
<212> PRT
<213> Homo sapiens

<400> 588

```
Ser Gly Asp Ala Leu Pro Arg Gln Tyr Ala Tyr
                5                   10
```

<210> 589
<211> 7
<212> PRT
<213> Homo sapiens

<400> 589

```
Lys Asp Ser Glu Arg Pro Ser
                5
```

<210> 590
<211> 11
<212> PRT
<213> Homo sapiens

<400> 590

```
Gln Ser Ala Asp Ser Ser Gly Thr Tyr Val Val
                1                   5                   10
```

<210> 591
<211> 22
<212> PRT
<213> Homo sapiens

<400> 591

```
Ser Tyr Glu Leu Ala Gln Pro Pro Ser Val Ser Val Ser Pro Gly Gln
                5                   10                  15

Thr Ala Arg Ile Thr Cys
            20
```

<210> 592
<211> 15
<212> PRT
<213> Homo sapiens

<400> 592

```
Trp Tyr Gln Gln Lys Pro Gly Gln Ala Pro Leu Pro Val Ile Tyr
                5                   10                  15
```

<210> 593
<211> 32
<212> PRT
<213> Homo sapiens

<400> 593

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 594
<211> 10
<212> PRT
<213> Homo sapiens

<400> 594

```
Phe Gly Gly Gly Thr Lys Val Thr Val Leu
                5                   10
```

<210> 595
<211> 375

<212> DNA
<213> Homo sapiens

<400> 595

```
gaggtgcagc tgttggagtc tgggggaggc ttggtacagc ctggggggcc cctgagactc      60
tcctgtgcag cctctggatt cacctttagc agctatgcca tgagctgggt ccgccaggct     120
ccagggaagg ggctggagtg ggtctcggct attagtggta gtggtggtag cacatactac     180
gcagactccg tgaagggccg gttcaccatc tccagagaca attccaagaa cacgctgtat     240
ctgcaaatga acagcctgag agccgaggac acggccgcgt attactgtgc gagagattgg     300
gatttttgga gtacttattc gggtccaact gcttttgatc tctggggcag aggcaccctg     360
gtcaccgtct cgagt                                                      375
```

<210> 596
<211> 125
<212> PRT
<213> Homo sapiens

<400> 596

```
Glu Val Gln Leu Leu Glu Ser Gly Gly Gly Leu Val Gln Pro Gly Gly
                5                   10                  15

Pro Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ala Tyr Tyr Cys
                85                  90                  95

Ala Arg Asp Trp Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 597
<211> 5
<212> PRT
<213> Homo sapiens

<400> 597

```
Ser Tyr Ala Met Ser
                5
```

<210> 598
<211> 17
<212> PRT
<213> Homo sapiens

<400> 598

Ala Ile Ser Gly Ser Gly Gly Ser Thr Tyr Tyr Ala Asp Ser Val Lys
1               5                   10                  15

Gly

<210> 599
<211> 16
<212> PRT
<213> Homo sapiens

<400> 599

Asp Trp Asp Phe Trp Ser Thr Tyr Ser Gly Pro Thr Ala Phe Asp Leu
                5                   10                  15

<210> 600
<211> 30
<212> PRT
<213> Homo sapiens

<400> 600

Glu Val Gln Leu Leu Glu Ser Gly Gly Gly Leu Val Gln Pro Gly Gly
                5                   10                  15

Pro Leu Arg Leu Ser Cys Ala Ala Ser Gly Phe Thr Phe Ser
            20                  25                  30

<210> 601
<211> 14
<212> PRT
<213> Homo sapiens

<400> 601

Trp Val Arg Gln Ala Pro Gly Lys Gly Leu Glu Trp Val Ser
                5                   10

<210> 602
<211> 32
<212> PRT
<213> Homo sapiens

<400> 602

Arg Phe Thr Ile Ser Arg Asp Asn Ser Lys Asn Thr Leu Tyr Leu Gln
                5                   10                  15

Met Asn Ser Leu Arg Ala Glu Asp Thr Ala Ala Tyr Tyr Cys Ala Arg
            20                  25                  30

<210> 603
<211> 11
<212> PRT
<213> Homo sapiens

<400> 603

Trp Gly Arg Gly Thr Leu Val Thr Val Ser Ser
1 5 10

<210> 604
<211> 324
<212> DNA
<213> Homo sapiens

<400> 604

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccccc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 605
<211> 108
<212> PRT
<213> Homo sapiens

<400> 605

```
Ser Tyr Glu Leu Ala Gln Pro Pro Ser Val Ser Val Ser Pro Gly Gln
1               5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Glu Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Val Val Phe Gly Gly Gly Thr Lys Val Thr Val Leu
            100                 105
```

<210> 606
<211> 11

<212> PRT
<213> Homo sapiens

<400> 606

Ser Gly Asp Ala Leu Pro Arg Gln Tyr Ala Tyr
1               5                   10

<210> 607
<211> 7
<212> PRT
<213> Homo sapiens

<400> 607

Lys Asp Ser Glu Arg Pro Ser
                5

<210> 608
<211> 11
<212> PRT
<213> Homo sapiens

<400> 608

Gln Ser Ala Asp Ser Ser Gly Thr Tyr Val Val
                5                   10

<210> 609
<211> 22
<212> PRT
<213> Homo sapiens

<400> 609

Ser Tyr Glu Leu Ala Gln Pro Pro Ser Val Ser Val Ser Pro Gly Gln
                5                   10                  15

Thr Ala Arg Ile Thr Cys
            20

<210> 610
<211> 15
<212> PRT
<213> Homo sapiens

<400> 610

Trp Tyr Gln Gln Lys Pro Gly Gln Ala Pro Leu Leu Val Ile Tyr
                5                   10                  15

<210> 611
<211> 32
<212> PRT
<213> Homo sapiens

<400> 611

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 612
<211> 10
<212> PRT
<213> Homo sapiens

<400> 612

```
Phe Gly Gly Gly Thr Lys Val Thr Val Leu
                5                   10
```

<210> 613
<211> 375
<212> DNA
<213> Homo sapiens

<400> 613

```
gaggtgcagc tgttggagtc tgggggaggc ttggtacagc ctggggggtc cctgagactc      60

tcctgtgcag cctctggatt cacctttagc agctttgcca tgagctgggt ccgccaggct     120

ccagggaagg ggctggagtg ggtctcagct attagtggta gtggtagtag cacatactac     180

gcagactccg tgaagggccg gttcgccatc tccagagaca attccaagaa cacgctgtat     240

ctgcaaatga acagcctgag agccgaggac acggccgtgt attactgtgc gagagatagg     300

gatttttgga gtacttattc gggtccaact gcttttgatc tctggggcag aggcaccctg     360

gtcaccgtct cgagt                                                      375
```

<210> 614
<211> 125
<212> PRT
<213> Homo sapiens

<400> 614

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Phe
            20                  25                  30

Ala Met Ser Trp Val Arg Gln Ala Pro Gly Lys Gly Leu Glu Trp Val
        35                  40                  45

Ser Ala Ile Ser Gly Ser Gly Ser Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser


          115                 120                 125
```

<210> 615
<211> 5
<212> PRT
<213> Homo sapiens

<400> 615

```
                      Ser Phe Ala Met Ser
                                      5
```

<210> 616
<211> 17
<212> PRT
<213> Homo sapiens

<400> 616

```
Ala Ile Ser Gly Ser Gly Ser Ser Thr Tyr Tyr Ala Asp Ser Val Lys
                5                   10                  15

Gly
```

<210> 617
<211> 16
<212> PRT
<213> Homo sapiens

<400> 617

```
Asp Arg Asp Phe Trp Ser Thr Tyr Ser Gly Pro Thr Ala Phe Asp Leu
                5                   10                  15
```

<210> 618

<211> 30
<212> PRT
<213> Homo sapiens

<400> 618

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 619
<211> 14
<212> PRT
<213> Homo sapiens

<400> 619

Trp Val Arg Gln Ala Pro Gly Lys Gly Leu Glu Trp Val Ser
5 10

<210> 620
<211> 32
<212> PRT
<213> Homo sapiens

<400> 620

Arg Phe Ala Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ala Arg
20 25 30

<210> 621
<211> 11
<212> PRT
<213> Homo sapiens

<400> 621

Trp Gly Arg Gly Thr Leu Val Thr Val Ser Ser
5 10

<210> 622
<211> 324
<212> DNA
<213> Homo sapiens

<400> 622

```
tcctatgagc tggcccagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagcg gacagcagtg gtacctatgc ggtattcggc     300
ggagggacca aggtcaccgt ccta                                            324
```

<210> 623
<211> 108
<212> PRT
<213> Homo sapiens

<400> 623

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Ala Val Phe Gly Gly Gly Thr Lys Val Thr Val Leu


                    100                 105
```

<210> 624
<211> 11
<212> PRT
<213> Homo sapiens

<400> 624

```
Ser Gly Asp Ala Leu Pro Arg Gln Tyr Ala Tyr
                5                   10
```

<210> 625
<211> 7
<212> PRT
<213> Homo sapiens

<400> 625

Lys Asp Ser Glu Arg Pro Ser
5

<210> 626
<211> 11
<212> PRT
<213> Homo sapiens

<400> 626

Gln Ser Ala Asp Ser Ser Gly Thr Tyr Ala Val
5 10

<210> 627
<211> 22
<212> PRT
<213> Homo sapiens

<400> 627

Ser Tyr Glu Leu Ala Gln Pro Pro Ser Val Ser Val Ser Pro Gly Gln
5 10 15

Thr Ala Arg Ile Thr Cys
20

<210> 628
<211> 15
<212> PRT
<213> Homo sapiens

<400> 628

Trp Tyr Gln Gln Lys Pro Gly Gln Ala Pro Leu Leu Val Ile Tyr
5 10 15

<210> 629
<211> 32
<212> PRT
<213> Homo sapiens

<400> 629

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 630
<211> 10
<212> PRT
<213> Homo sapiens

<400> 630

```
Phe Gly Gly Gly Thr Lys Val Thr Val Leu
                5                   10
```

<210> 631
<211> 375
<212> DNA
<213> Homo sapiens

<400> 631

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcgccatc tccagagaca attccaagaa cacgctgtat     240
ctgcaaatga acagcctgag agccgaggac acggccgtgt attactgttc gcggcagttc     300
gactactgga gtacttattc gggtccaact gcttttgatc tctggggcag aggcaccctg     360
gtcaccgtct cgagt                                                      375
```

<210> 632
<211> 125
<212> PRT
<213> Homo sapiens

<400> 632

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ser Arg Gln Phe Asp Tyr Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 633
<211> 5
<212> PRT
<213> Homo sapiens

<400> 633

Ser Tyr Ala Met Ser
5

<210> 634
<211> 17
<212> PRT
<213> Homo sapiens

<400> 634

Ala Ile Ser Gly Ser Gly Gly Ser Thr Tyr Tyr Ala Asp Ser Val Lys
5 10 15

Gly

<210> 635
<211> 16
<212> PRT
<213> Homo sapiens

<400> 635

Gln Phe Asp Tyr Trp Ser Thr Tyr Ser Gly Pro Thr Ala Phe Asp Leu
5 10 15

<210> 636
<211> 30
<212> PRT
<213> Homo sapiens

<400> 636

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 637
<211> 14
<212> PRT
<213> Homo sapiens

<400> 637

Trp Val Arg Gln Ala Pro Gly Lys Gly Leu Glu Trp Val Ser
5 10

<210> 638
<211> 32
<212> PRT
<213> Homo sapiens

<400> 638

Arg Phe Ala Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ser Arg
20 25 30

<210> 639
<211> 11
<212> PRT
<213> Homo sapiens

<400> 639

Trp Gly Arg Gly Thr Leu Val Thr Val Ser Ser
5 10

<210> 640
<211> 324
<212> DNA
<213> Homo sapiens

<400> 640

tcctatgagc tggctcagcc accctcggtg tcagtgtccc caggacagac ggccaggatc 60

acctgctctg gagatgcatt gccaaggcaa tatgcttact ggtaccagca gaagccaggc 120

caggcccctc tactggtgat atataaagac agtttcaggc cctcagggat ccctgagcga 180

ttctctggct ccggctcagg gacaacagtc acgttgacca tcagtggagt ccaggcagaa 240

gacgaggctg actattactg tcaatcagca gacagcagtg gtacctatgc ggtattcggc 300

ggagggacca aggtcaccgt ccta 324

<210> 641
<211> 108
<212> PRT
<213> Homo sapiens

<400> 641

Ser Tyr Glu Leu Ala Gln Pro Pro Ser Val Ser Val Ser Pro Gly Gln
5 10 15

Thr Ala Arg Ile Thr Cys Ser Gly Asp Ala Leu Pro Arg Gln Tyr Ala
20 25 30

Tyr Trp Tyr Gln Gln Lys Pro Gly Gln Ala Pro Leu Leu Val Ile Tyr
35 40 45

Lys Asp Ser Phe Arg Pro Ser Gly Ile Pro Glu Arg Phe Ser Gly Ser
50 55 60

Gly Ser Gly Thr Thr Val Thr Leu Thr Ile Ser Gly Val Gln Ala Glu
65 70 75 80

Asp Glu Ala Asp Tyr Tyr Cys Gln Ser Ala Asp Ser Ser Gly Thr Tyr
85 90 95

Ala Val Phe Gly Gly Gly Thr Lys Val Thr Val Leu
100 105

<210> 642
<211> 11
<212> PRT
<213> Homo sapiens

<400> 642

Ser Gly Asp Ala Leu Pro Arg Gln Tyr Ala Tyr
5 10

<210> 643
<211> 7
<212> PRT
<213> Homo sapiens

<400> 643

Lys Asp Ser Phe Arg Pro Ser
5

<210> 644
<211> 11
<212> PRT
<213> Homo sapiens

<400> 644

Gln Ser Ala Asp Ser Ser Gly Thr Tyr Ala Val
5 10

<210> 645
<211> 22
<212> PRT
<213> Homo sapiens

<400> 645

Ser Tyr Glu Leu Ala Gln Pro Pro Ser Val Ser Val Ser Pro Gly Gln
5 10 15

Thr Ala Arg Ile Thr Cys
20

<210> 646
<211> 15
<212> PRT
<213> Homo sapiens

<400> 646

Trp Tyr Gln Gln Lys Pro Gly Gln Ala Pro Leu Leu Val Ile Tyr
5 10 15

<210> 647
<211> 32

<212> PRT
<213> Homo sapiens

<400> 647

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 648
<211> 10
<212> PRT
<213> Homo sapiens

<400> 648

```
Phe Gly Gly Gly Thr Lys Val Thr Val Leu
                5                   10
```

<210> 649
<211> 375
<212> DNA
<213> Homo sapiens

<400> 649

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcgccatc tccagagaca attccaagaa cacgctgtat     240
ctgcaaatga acagcctgag agccgaggac acggccgtgt attactgttc gcggcagttc     300
gactactgga gtacttattc gggtccaact gcttttgatc tctggggcag aggcaccctg     360
gtcaccgtct cgagt                                                      375
```

<210> 650
<211> 125
<212> PRT
<213> Homo sapiens

<400> 650

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Ala Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ser Arg Gln Phe Asp Tyr Trp Ser Thr Tyr Ser Gly Pro Thr Ala Phe
100 105 110

Asp Leu Trp Gly Arg Gly Thr Leu Val Thr Val Ser Ser
115 120 125

<210> 651
<211> 5
<212> PRT
<213> Homo sapiens

<400> 651
Ser Tyr Ala Met Ser 5

<210> 652
<211> 17
<212> PRT
<213> Homo sapiens

<400> 652

Ala Ile Ser Gly Ser Gly Gly Ser Thr Tyr Tyr Ala Asp Ser Val Lys
5 10 15

Gly

<210> 653
<211> 16
<212> PRT
<213> Homo sapiens

<400> 653

Gln Phe Asp Tyr Trp Ser Thr Tyr Ser Gly Pro Thr Ala Phe Asp Leu
5 10 15

<210> 654
<211> 30
<212> PRT
<213> Homo sapiens

<400> 654

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 655
<211> 14
<212> PRT
<213> Homo sapiens

<400> 655

```
    Trp Val Arg Gln Ala Pro Gly Lys Gly Leu Glu Trp Val Ser
                    5                   10
```

<210> 656
<211> 32
<212> PRT
<213> Homo sapiens

<400> 656

```
Arg Phe Ala Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30
```

<210> 657
<211> 11
<212> PRT
<213> Homo sapiens

<400> 657

```
          Trp Gly Arg Gly Thr Leu Val Thr Val Ser Ser
                          5                   10
```

<210> 658
<211> 324
<212> DNA
<213> Homo sapiens

<400> 658

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 659
<211> 108
<212> PRT
<213> Homo sapiens

<400> 659

```
Ser Tyr Glu Leu Ala Gln Pro Pro Ser Val Ser Val Ser Pro Gly Gln
                5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Glu Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Val Val Phe Gly Gly Gly Thr Lys Val Thr Val Leu
            100                 105
```

<210> 660
<211> 11
<212> PRT
<213> Homo sapiens

<400> 660

```
Ser Gly Asp Ala Leu Pro Arg Gln Tyr Ala Tyr
                5                   10
```

<210> 661
<211> 7
<212> PRT
<213> Homo sapiens

<400> 661

Lys Asp Ser Glu Arg Pro Ser
5

<210> 662
<211> 11
<212> PRT
<213> Homo sapiens

<400> 662

Gln Ser Ala Asp Ser Ser Gly Thr Tyr Val Val
5 10

<210> 663
<211> 22
<212> PRT
<213> Homo sapiens

<400> 663

Ser Tyr Glu Leu Ala Gln Pro Pro Ser Val Ser Val Ser Pro Gly Gln
5 10 15

Thr Ala Arg Ile Thr Cys
20

<210> 664
<211> 15
<212> PRT
<213> Homo sapiens

<400> 664

Trp Tyr Gln Gln Lys Pro Gly Gln Ala Pro Leu Leu Val Ile Tyr
5 10 15

<210> 665
<211> 32
<212> PRT
<213> Homo sapiens

<400> 665

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 666
<211> 10
<212> PRT
<213> Homo sapiens

<400> 666

Phe Gly Gly Gly Thr Lys Val Thr Val Leu
5 10

<210> 667
<211> 375
<212> DNA
<213> Homo sapiens

<400> 667

```
gaggtgcagc tgttggagtc tgggggaggc ttggtacagc ctgggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcgccatc tccagagaca attccaagaa cacgctgtat     240
ctgcaaatga acagcctgag agccgaggac acggccgtgt attactgttc gcggcagttc     300
gactactgga gtacttattc gggtccaact gcttttgatc tctggggcag aggcaccctg     360
gtcaccgtct cgagt                                                      375
```

<210> 668
<211> 125
<212> PRT
<213> Homo sapiens

<400> 668

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys


                85                  90                  95

Ser Arg Gln Phe Asp Tyr Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 669
<211> 5
<212> PRT
<213> Homo sapiens

<400> 669

Ser Tyr Ala Met Ser
5

<210> 670
<211> 17
<212> PRT
<213> Homo sapiens

<400> 670

Ala Ile Ser Gly Ser Gly Gly Ser Thr Tyr Tyr Ala Asp Ser Val Lys
5 10 15

Gly

<210> 671
<211> 16
<212> PRT
<213> Homo sapiens

<400> 671

Gln Phe Asp Tyr Trp Ser Thr Tyr Ser Gly Pro Thr Ala Phe Asp Leu
5 10 15

<210> 672
<211> 30
<212> PRT
<213> Homo sapiens

<400> 672

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 673
<211> 14
<212> PRT
<213> Homo sapiens

<400> 673

Trp Val Arg Gln Ala Pro Gly Lys Gly Leu Glu Trp Val Ser
5 10

<210> 674
<211> 32
<212> PRT
<213> Homo sapiens

<400> 674

Arg Phe Ala Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ser Arg
20 25 30

<210> 675
<211> 11
<212> PRT
<213> Homo sapiens

<400> 675

Trp Gly Arg Gly Thr Leu Val Thr Val Ser Ser
5 10

<210> 676
<211> 324
<212> DNA
<213> Homo sapiens

<400> 676

tcctatgagc tggctcagcc accctcggtg tcagtgtccc caggacagac ggccaggatc 60

acctgctctg gagatgcatt gccaaggcaa tatgcttact ggtaccagca gaagccaggc 120

caggcccctc tactggtgat atataaagac agtgagaggc cctcagggat ccctgagcga 180

ttctctggct ccggctcagg gacaacagtc acgttgacca tcagtggagt ccaggcagaa 240

gacgaggctg actattactg tcaatcagca gacagcagtg gtacctatgc ggtattcggc 300

ggagggacca aggtcaccgt ccta 324

<210> 677
<211> 108
<212> PRT
<213> Homo sapiens

<400> 677

Ser Tyr Glu Leu Ala Gln Pro Pro Ser Val Ser Val Ser Pro Gly Gln
5 10 15

Thr Ala Arg Ile Thr Cys Ser Gly Asp Ala Leu Pro Arg Gln Tyr Ala
20 25 30

Tyr Trp Tyr Gln Gln Lys Pro Gly Gln Ala Pro Leu Leu Val Ile Tyr
35 40 45

Lys Asp Ser Glu Arg Pro Ser Gly Ile Pro Glu Arg Phe Ser Gly Ser
50 55 60

Gly Ser Gly Thr Thr Val Thr Leu Thr Ile Ser Gly Val Gln Ala Glu

65 70 75 80

Asp Glu Ala Asp Tyr Tyr Cys Gln Ser Ala Asp Ser Ser Gly Thr Tyr
85 90 95

Ala Val Phe Gly Gly Gly Thr Lys Val Thr Val Leu
100 105

<210> 678
<211> 11
<212> PRT
<213> Homo sapiens

<400> 678

Ser Gly Asp Ala Leu Pro Arg Gln Tyr Ala Tyr
5 10

<210> 679
<211> 7
<212> PRT
<213> Homo sapiens

<400> 679

Lys Asp Ser Glu Arg Pro Ser
5

<210> 680
<211> 11
<212> PRT
<213> Homo sapiens

<400> 680

Gln Ser Ala Asp Ser Ser Gly Thr Tyr Ala Val
5 10

<210> 681
<211> 22
<212> PRT
<213> Homo sapiens

<400> 681

Ser Tyr Glu Leu Ala Gln Pro Pro Ser Val Ser Val Ser Pro Gly Gln
5 10 15

Thr Ala Arg Ile Thr Cys
20

<210> 682
<211> 15
<212> PRT
<213> Homo sapiens

<400> 682

Trp Tyr Gln Gln Lys Pro Gly Gln Ala Pro Leu Leu Val Ile Tyr
5 10 15

<210> 683
<211> 32
<212> PRT
<213> Homo sapiens

<400> 683

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 684
<211> 10
<212> PRT
<213> Homo sapiens

<400> 684

Phe Gly Gly Gly Thr Lys Val Thr Val Leu
5 10

<210> 685
<211> 375
<212> DNA
<213> Homo sapiens

<400> 685

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtagtag cacatactac 180

gcagactccg tgaagggccg gttcaccatc tccagagaca attccaagaa cacgctgtat 240

ctgcaaatga acagcctgag agccgaggac acggccgtgt attactgtgc gagagatagg 300

gatttttgga gtacttattc gggtccaact gcttttgatc tctggggcag aggcaccctg 360

gtcaccgtct cgagt 375

<210> 686
<211> 125
<212> PRT
<213> Homo sapiens

<400> 686

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Ser Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Asp Arg Asp Phe Trp Ser Thr Tyr Ser Gly Pro Thr Ala Phe
100 105 110

Asp Leu Trp Gly Arg Gly Thr Leu Val Thr Val Ser Ser
115 120 125

<210> 687
<211> 5
<212> PRT
<213> Homo sapiens

<400> 687

Ser Tyr Ala Met Ser
5

<210> 688
<211> 17
<212> PRT
<213> Homo sapiens

<400> 688

Ala Ile Ser Gly Ser Gly Ser Ser Thr Tyr Tyr Ala Asp Ser Val Lys
5 10 15

Gly

<210> 689
<211> 16
<212> PRT
<213> Homo sapiens

<400> 689

Asp Arg Asp Phe Trp Ser Thr Tyr Ser Gly Pro Thr Ala Phe Asp Leu
5 10 15

<210> 690
<211> 30

<212> PRT
<213> Homo sapiens

<400> 690

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 691
<211> 14
<212> PRT
<213> Homo sapiens

<400> 691

```
Trp Val Arg Gln Ala Pro Gly Lys Gly Leu Glu Trp Val Ser
                5                   10
```

<210> 692
<211> 32
<212> PRT
<213> Homo sapiens

<400> 692

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 693
<211> 11
<212> PRT
<213> Homo sapiens

<400> 693

```
Trp Gly Arg Gly Thr Leu Val Thr Val Ser Ser
                5                   10
```

<210> 694
<211> 324
<212> DNA
<213> Homo sapiens

<400> 694

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 695
<211> 108
<212> PRT
<213> Homo sapiens

<400> 695

```
Ser Tyr Glu Leu Ala Gln Pro Pro Ser Val Ser Val Ser Pro Gly Gln
                5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Glu Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Val Val Phe Gly Gly Gly Thr Lys Val Thr Val Leu
            100                 105
```

<210> 696
<211> 11
<212> PRT
<213> Homo sapiens

<400> 696

```
Ser Gly Asp Ala Leu Pro Arg Gln Tyr Ala Tyr
                5                   10
```

<210> 697
<211> 7
<212> PRT
<213> Homo sapiens

<400> 697

```
Lys Asp Ser Glu Arg Pro Ser
                    5
```

<210> 698
<211> 11
<212> PRT
<213> Homo sapiens

<400> 698

```
Gln Ser Ala Asp Ser Ser Gly Thr Tyr Val Val
                5                   10
```

<210> 699
<211> 22
<212> PRT
<213> Homo sapiens

<400> 699

```
Ser Tyr Glu Leu Ala Gln Pro Pro Ser Val Ser Val Ser Pro Gly Gln
                5                   10                  15

Thr Ala Arg Ile Thr Cys
            20
```

<210> 700
<211> 15
<212> PRT
<213> Homo sapiens

<400> 700

```
Trp Tyr Gln Gln Lys Pro Gly Gln Ala Pro Leu Leu Val Ile Tyr
                5                   10                  15
```

<210> 701
<211> 32
<212> PRT
<213> Homo sapiens

<400> 701

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 702
<211> 10
<212> PRT
<213> Homo sapiens

<400> 702

```
Phe Gly Gly Gly Thr Lys Val Thr Val Leu
                5                   10
```

<210> 703
<211> 375
<212> DNA
<213> Homo sapiens

<400> 703

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcgccatc tccagagaca attccaagaa cacgctgtat     240
ctgcaaatga acagcctgag agccgaggac acggccgtgt attactgtgc gagagatagg     300
gatttttgga gtacttattc gggtccaact gcttttgatc tctggggcag aggcaccctg     360
gtcaccgtct cgagt                                                      375
```

<210> 704
<211> 125
<212> PRT
<213> Homo sapiens

<400> 704

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 705
<211> 5
<212> PRT
<213> Homo sapiens

<400> 705

Ser Tyr Ala Met Ser
5

<210> 706
<211> 17
<212> PRT
<213> Homo sapiens

<400> 706

Ala Ile Ser Gly Ser Gly Gly Ser Thr Tyr Tyr Ala Asp Ser Val Lys
5 10 15

Gly

<210> 707
<211> 16
<212> PRT
<213> Homo sapiens

<400> 707

Asp Arg Asp Phe Trp Ser Thr Tyr Ser Gly Pro Thr Ala Phe Asp Leu
5 10 15

<210> 708
<211> 30
<212> PRT
<213> Homo sapiens

<400> 708

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 709
<211> 14
<212> PRT
<213> Homo sapiens

<400> 709

Trp Val Arg Gln Ala Pro Gly Lys Gly Leu Glu Trp Val Ser
5 10

<210> 710
<211> 32
<212> PRT
<213> Homo sapiens

<400> 710

Arg Phe Ala Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ala Arg
20 25 30

<210> 711
<211> 11
<212> PRT
<213> Homo sapiens

<400> 711

Trp Gly Arg Gly Thr Leu Val Thr Val Ser Ser
5 10

<210> 712
<211> 324
<212> DNA
<213> Homo sapiens

<400> 712

tcctatgagc tggctcagcc accctcggtg tcagtgtccc caggacagac ggccaggatc 60

acctgctctg gagatgcatt gccaaggcaa tatgcttact ggtaccagca gaagccaggc 120

caggcccctc tactggtgat atataaagac agtgagaggc cctcagggat ccctgagcga 180

ttctctggct ccggctcagg gacaacagtc acgttgacca tcagtggagt ccaggcagaa 240

gacgaggctg actattactg tcaatcagca gacagcagtg gtacctatgt ggtattcggc 300

ggagggacca aggtcaccgt ccta 324

<210> 713
<211> 108
<212> PRT
<213> Homo sapiens

<400> 713

Ser Tyr Glu Leu Ala Gln Pro Pro Ser Val Ser Val Ser Pro Gly Gln
5 10 15

Thr Ala Arg Ile Thr Cys Ser Gly Asp Ala Leu Pro Arg Gln Tyr Ala
20 25 30

Tyr Trp Tyr Gln Gln Lys Pro Gly Gln Ala Pro Leu Leu Val Ile Tyr
35 40 45

```
Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 714
<211> 11
<212> PRT
<213> Homo sapiens

<400> 714

```
Ser Gly Asp Ala Leu Pro Arg Gln Tyr Ala Tyr
                5                   10
```

<210> 715
<211> 7
<212> PRT
<213> Homo sapiens

<400> 715

```
Lys Asp Ser Glu Arg Pro Ser
                5
```

<210> 716
<211> 11
<212> PRT
<213> Homo sapiens

<400> 716

```
Gln Ser Ala Asp Ser Ser Gly Thr Tyr Val Val
                5                   10
```

<210> 717
<211> 22
<212> PRT
<213> Homo sapiens

<400> 717

```
Ser Tyr Glu Leu Ala Gln Pro Pro Ser Val Ser Val Ser Pro Gly Gln
                5                   10                  15

Thr Ala Arg Ile Thr Cys
            20
```

<210> 718
<211> 15
<212> PRT

<213> Homo sapiens

<400> 718

Trp Tyr Gln Gln Lys Pro Gly Gln Ala Pro Leu Leu Val Ile Tyr
5 10 15

<210> 719
<211> 32
<212> PRT
<213> Homo sapiens

<400> 719

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 720
<211> 10
<212> PRT
<213> Homo sapiens

<400> 720

Phe Gly Gly Gly Thr Lys Val Thr Val Leu
5 10

<210> 721
<211> 375
<212> DNA
<213> Homo sapiens

<400> 721

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgtgc gagagatagg 300

gatttttgga gtacttattc gggtccaact gcttttgatc tctggggcag aggcaccctg 360

gtcaccgtct cgagt 375

<210> 722
<211> 125
<212> PRT
<213> Homo sapiens

<400> 722

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125

<210> 723
<211> 5
<212> PRT
<213> Homo sapiens

<400> 723

Ser Tyr Ala Met Ser
                5

<210> 724
<211> 17
<212> PRT
<213> Homo sapiens

<400> 724

Ala Ile Ser Gly Ser Gly Gly Ser Thr Tyr Tyr Ala Asp Ser Val Lys
                5                   10                  15

Gly

<210> 725
<211> 16
<212> PRT
<213> Homo sapiens

<400> 725

Asp Arg Asp Phe Trp Ser Thr Tyr Ser Gly Pro Thr Ala Phe Asp Leu
                5                   10                  15

<210> 726
<211> 30
<212> PRT
<213> Homo sapiens

<400> 726

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 727
<211> 14
<212> PRT
<213> Homo sapiens

<400> 727

```
Trp Val Arg Gln Ala Pro Gly Lys Gly Leu Glu Trp Val Ser
                5                   10
```

<210> 728
<211> 32
<212> PRT
<213> Homo sapiens

<400> 728

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 729
<211> 11
<212> PRT
<213> Homo sapiens

<400> 729

```
Trp Gly Arg Gly Thr Leu Val Thr Val Ser Ser
                5                   10
```

<210> 730
<211> 324
<212> DNA
<213> Homo sapiens

<400> 730

tcctatgagc tggctcagcc accctcggtg tcagtgtccc caggacagac ggccaggatc 60

acctgctctg gagatgcatt gccaaggcaa tatgcttact ggtaccagca gaagccaggc 120

caggcccctc tactggtgat atataaagac agtgagaggc cctcagggat ccctgagcga 180

ttctctggct ccggctcagg gacaacagtc acgttgacca tcagtggagt ccaggcagaa 240

gacgaggctg actattactg tcaatcagca gacagcagtg gtacctatgc ggtattcggc 300

ggagggacca aggtcaccgt ccta 324

<210> 731
<211> 108
<212> PRT
<213> Homo sapiens

<400> 731

Ser Tyr Glu Leu Ala Gln Pro Pro Ser Val Ser Val Ser Pro Gly Gln
5 10 15

Thr Ala Arg Ile Thr Cys Ser Gly Asp Ala Leu Pro Arg Gln Tyr Ala
20 25 30

Tyr Trp Tyr Gln Gln Lys Pro Gly Gln Ala Pro Leu Leu Val Ile Tyr

35 40 45

Lys Asp Ser Glu Arg Pro Ser Gly Ile Pro Glu Arg Phe Ser Gly Ser
50 55 60

Gly Ser Gly Thr Thr Val Thr Leu Thr Ile Ser Gly Val Gln Ala Glu
65 70 75 80

Asp Glu Ala Asp Tyr Tyr Cys Gln Ser Ala Asp Ser Ser Gly Thr Tyr
85 90 95

Ala Val Phe Gly Gly Gly Thr Lys Val Thr Val Leu
100 105

<210> 732
<211> 11
<212> PRT
<213> Homo sapiens

<400> 732

Ser Gly Asp Ala Leu Pro Arg Gln Tyr Ala Tyr
5 10

<210> 733
<211> 7
<212> PRT
<213> Homo sapiens

<400> 733

Lys Asp Ser Glu Arg Pro Ser
5

<210> 734
<211> 11
<212> PRT
<213> Homo sapiens

<400> 734

Gln Ser Ala Asp Ser Ser Gly Thr Tyr Ala Val
5 10

<210> 735
<211> 22
<212> PRT
<213> Homo sapiens

<400> 735

Ser Tyr Glu Leu Ala Gln Pro Pro Ser Val Ser Val Ser Pro Gly Gln
5 10 15

Thr Ala Arg Ile Thr Cys
20

<210> 736
<211> 15
<212> PRT
<213> Homo sapiens

<400> 736

Trp Tyr Gln Gln Lys Pro Gly Gln Ala Pro Leu Leu Val Ile Tyr
5 10 15

<210> 737
<211> 32
<212> PRT
<213> Homo sapiens

<400> 737

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 738
<211> 10
<212> PRT
<213> Homo sapiens

<400> 738

Phe Gly Gly Gly Thr Lys Val Thr Val Leu
5 10

<210> 739
<211> 375
<212> DNA
<213> Homo sapiens

<400> 739

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcgccatc tccagagaca attccaagaa cacgctgtat     240
ctgcaaatga acagcctgag agccgaggac acggccgtgt attactgttc gcggcagttc     300
gactactgga gtacttattc gggtccaact gcttttgatc tctggggcag aggcaccctg     360
gtcaccgtct cgagt                                                      375
```

<210> 740
<211> 125
<212> PRT
<213> Homo sapiens

<400> 740

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ser Arg Gln Phe Asp Tyr Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 741
<211> 5
<212> PRT
<213> Homo sapiens

<400> 741

Ser Tyr Ala Met Ser
5

<210> 742
<211> 17
<212> PRT
<213> Homo sapiens

<400> 742

Ala Ile Ser Gly Ser Gly Gly Ser Thr Tyr Tyr Ala Asp Ser Val Lys
5 10 15

Gly

<210> 743
<211> 16
<212> PRT
<213> Homo sapiens

<400> 743

Gln Phe Asp Tyr Trp Ser Thr Tyr Ser Gly Pro Thr Ala Phe Asp Leu
5 10 15

<210> 744
<211> 30
<212> PRT
<213> Homo sapiens

<400> 744

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 745
<211> 14
<212> PRT
<213> Homo sapiens

<400> 745

Trp Val Arg Gln Ala Pro Gly Lys Gly Leu Glu Trp Val Ser
5 10

<210> 746
<211> 32
<212> PRT
<213> Homo sapiens

<400> 746

```
Arg Phe Ala Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30
```

<210> 747
<211> 11
<212> PRT
<213> Homo sapiens

<400> 747

```
Trp Gly Arg Gly Thr Leu Val Thr Val Ser Ser
                5                   10
```

<210> 748
<211> 324
<212> DNA
<213> Homo sapiens

<400> 748

```
tcctatgagc tggctcagcc accctcggtg tcagtgtccc caggacagac ggccaggatc     60
acctgctctg gagatgcatt gccaaggcaa tatgcttact ggtaccagca gaagccaggc    120
caggcccctc tactggtgat atataaagac agtttcaggc cctcagggat ccctgagcga    180
ttctctggct ccggctcagg gacaacagtc acgttgacca tcagtggagt ccaggcagaa    240
gacgaggctg actattactg tcaatcagca gacagcagtg gtacctatgt ggtattcggc    300
ggagggacca aggtcaccgt ccta                                           324
```

<210> 749
<211> 108
<212> PRT
<213> Homo sapiens

<400> 749

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30
```

Tyr Trp Tyr Gln Gln Lys Pro Gly Gln Ala Pro Leu Leu Val Ile Tyr
35 40 45

Lys Asp Ser Phe Arg Pro Ser Gly Ile Pro Glu Arg Phe Ser Gly Ser
50 55 60

Gly Ser Gly Thr Thr Val Thr Leu Thr Ile Ser Gly Val Gln Ala Glu
65 70 75 80

Asp Glu Ala Asp Tyr Tyr Cys Gln Ser Ala Asp Ser Ser Gly Thr Tyr
85 90 95

Val Val Phe Gly Gly Gly Thr Lys Val Thr Val Leu
100 105

<210> 750
<211> 11
<212> PRT
<213> Homo sapiens

<400> 750

Ser Gly Asp Ala Leu Pro Arg Gln Tyr Ala Tyr
5 10

<210> 751
<211> 7
<212> PRT
<213> Homo sapiens

<400> 751

Lys Asp Ser Phe Arg Pro Ser
5

<210> 752
<211> 11
<212> PRT
<213> Homo sapiens

<400> 752

Gln Ser Ala Asp Ser Ser Gly Thr Tyr Val Val
5 10

<210> 753
<211> 22
<212> PRT
<213> Homo sapiens

<400> 753

Ser Tyr Glu Leu Ala Gln Pro Pro Ser Val Ser Val Ser Pro Gly Gln
5 10 15

Thr Ala Arg Ile Thr Cys
20

<210> 754
<211> 15
<212> PRT
<213> Homo sapiens

<400> 754

```
Trp Tyr Gln Gln Lys Pro Gly Gln Ala Pro Leu Leu Val Ile Tyr
                5                   10                  15
```

<210> 755
<211> 32
<212> PRT
<213> Homo sapiens

<400> 755

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 756
<211> 10
<212> PRT
<213> Homo sapiens

<400> 756

```
Phe Gly Gly Gly Thr Lys Val Thr Val Leu
                5                   10
```

<210> 757
<211> 375
<212> DNA
<213> Homo sapiens

<400> 757

```
gaggtgcagc tgttggagtc tgggggaggc ttggtacagc ctggggggtc cctgagactc      60

tcctgtgcag cctctggatt cacctttagc agctatgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcaaatga acagcctgag agccgaggac acggccgtgt attactgttc gcggcagttc     300

gactactgga gtacttattc gggtccaact gcttttgatc tctggggcag aggcaccctg     360

gtcaccgtct cgagt                                                      375
```

<210> 758
<211> 125
<212> PRT
<213> Homo sapiens

<400> 758

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ser Arg Gln Phe Asp Tyr Trp Ser Thr Tyr Ser Gly Pro Thr Ala Phe
            100                 105                 110

Asp Leu Trp Gly Arg Gly Thr Leu Val Thr Val Ser Ser
        115                 120                 125

<210> 759
<211> 5
<212> PRT
<213> Homo sapiens

<400> 759

Ser Tyr Ala Met Ser
                5

<210> 760
<211> 17
<212> PRT
<213> Homo sapiens

<400> 760

Ala Ile Ser Gly Ser Gly Gly Ser Thr Tyr Tyr Ala Asp Ser Val Lys
                5                   10                  15

Gly

<210> 761
<211> 16
<212> PRT
<213> Homo sapiens

<400> 761

Gln Phe Asp Tyr Trp Ser Thr Tyr Ser Gly Pro Thr Ala Phe Asp Leu
                5                   10                  15

<210> 762

<211> 30
<212> PRT
<213> Homo sapiens

<400> 762

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210> 763
<211> 14
<212> PRT
<213> Homo sapiens

<400> 763

Trp Val Arg Gln Ala Pro Gly Lys Gly Leu Glu Trp Val Ser
                5                   10

<210> 764
<211> 32
<212> PRT
<213> Homo sapiens

<400> 764

Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30

<210> 765
<211> 11
<212> PRT
<213> Homo sapiens

<400> 765

Trp Gly Arg Gly Thr Leu Val Thr Val Ser Ser
                5                   10

<210> 766
<211> 324
<212> DNA
<213> Homo sapiens

<400> 766

tcctatgagc tggctcagcc accctcggtg tcagtgtccc caggacagac ggccaggatc 60

acctgctctg gagatgcatt gccaaggcaa tatgcttact ggtaccagca gaagccaggc 120

caggcccctc tactggtgat atataaagac agtgagaggc cctcagggat ccctgagcga 180

ttctctggct ccggctcagg gacaacagtc acgttgacca tcagtggagt ccaggcagaa 240

gacgaggctg actattactg tcaatcagca gacagcagtg gtacctatgc ggtattcggc 300

ggagggacca aggtcaccgt ccta 324

<210> 767
<211> 108
<212> PRT
<213> Homo sapiens

<400> 767

Ser Tyr Glu Leu Ala Gln Pro Pro Ser Val Ser Val Ser Pro Gly Gln
5 10 15

Thr Ala Arg Ile Thr Cys Ser Gly Asp Ala Leu Pro Arg Gln Tyr Ala
20 25 30

Tyr Trp Tyr Gln Gln Lys Pro Gly Gln Ala Pro Leu Leu Val Ile Tyr
35 40 45

Lys Asp Ser Glu Arg Pro Ser Gly Ile Pro Glu Arg Phe Ser Gly Ser
50 55 60

Gly Ser Gly Thr Thr Val Thr Leu Thr Ile Ser Gly Val Gln Ala Glu
65 70 75 80

Asp Glu Ala Asp Tyr Tyr Cys Gln Ser Ala Asp Ser Ser Gly Thr Tyr
85 90 95

Ala Val Phe Gly Gly Gly Thr Lys Val Thr Val Leu
100 105

<210> 768
<211> 11
<212> PRT
<213> Homo sapiens

<400> 768

Ser Gly Asp Ala Leu Pro Arg Gln Tyr Ala Tyr
5 10

<210> 769
<211> 7
<212> PRT
<213> Homo sapiens

<400> 769

Lys Asp Ser Glu Arg Pro Ser
5

<210> 770
<211> 11
<212> PRT
<213> Homo sapiens

<400> 770

```
Gln Ser Ala Asp Ser Ser Gly Thr Tyr Ala Val
                1               5                   10
```

<210> 771
<211> 22
<212> PRT
<213> Homo sapiens

<400> 771

```
Ser Tyr Glu Leu Ala Gln Pro Pro Ser Val Ser Val Ser Pro Gly Gln
1               5                   10                  15

Thr Ala Arg Ile Thr Cys
            20
```

<210> 772
<211> 15
<212> PRT
<213> Homo sapiens

<400> 772

```
Trp Tyr Gln Gln Lys Pro Gly Gln Ala Pro Leu Leu Val Ile Tyr
1               5                   10                  15
```

<210> 773
<211> 32
<212> PRT
<213> Homo sapiens

<400> 773

```
Gly Ile Pro Glu Arg Phe Ser Gly Ser Gly Ser Gly Thr Thr Val Thr
1               5                   10                  15

Leu Thr Ile Ser Gly Val Gln Ala Glu Asp Glu Ala Asp Tyr Tyr Cys
            20                  25                  30
```

<210> 774
<211> 10
<212> PRT
<213> Homo sapiens

<400> 774

```
Phe Gly Gly Gly Thr Lys Val Thr Val Leu
1               5                   10
```

<210> 775
<211> 375

<212> DNA
<213> Homo sapiens

<400> 775

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtggtag cacatactac     180
gcagactccg tgaagggccg gttcgccatc tccagagaca attccaagaa cacgctgtat     240
ctgcaaatga acagcctgag agccgaggac acggccgtgt attactgtgc gagagatagg     300
gatttttgga gtacttattc gggtccaact gcttttgatc tctggggcag aggcaccctg     360
gtcaccgtct cgagt                                                      375
```

<210> 776
<211> 125
<212> PRT
<213> Homo sapiens

<400> 776

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Tyr

            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 777
<211> 5
<212> PRT
<213> Homo sapiens

<400> 777

Ser Tyr Ala Met Ser
5

<210> 778
<211> 17
<212> PRT
<213> Homo sapiens

<400> 778

Ala Ile Ser Gly Ser Gly Gly Ser Thr Tyr Tyr Ala Asp Ser Val Lys
5 10 15

Gly

<210> 779
<211> 16
<212> PRT
<213> Homo sapiens

<400> 779

Asp Arg Asp Phe Trp Ser Thr Tyr Ser Gly Pro Thr Ala Phe Asp Leu
5 10 15

<210> 780
<211> 30
<212> PRT
<213> Homo sapiens

<400> 780

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 781
<211> 14
<212> PRT
<213> Homo sapiens

<400> 781

Trp Val Arg Gln Ala Pro Gly Lys Gly Leu Glu Trp Val Ser
5 10

<210> 782
<211> 32
<212> PRT
<213> Homo sapiens

<400> 782

```
Arg Phe Ala Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ala Arg
            20                  25                  30
```

<210> 783
<211> 11
<212> PRT
<213> Homo sapiens

<400> 783

```
Trp Gly Arg Gly Thr Leu Val Thr Val Ser Ser
                5                   10
```

<210> 784
<211> 324
<212> DNA
<213> Homo sapiens

<400> 784

```
tcctatgagc tggctcagcc accctcggtg tcagtgtccc caggacagac ggccaggatc      60

acctgctctg gagatgcatt gccaaggcaa tatgcttact ggtaccagca gaagccaggc     120

caggcccctc tactggtgat atataaagac agtgagaggc cctcagggat ccctgagcga     180

ttctctggct ccggctcagg gacaacagtc acgttgacca tcagtggagt ccaggcagaa     240

gacgaggctg actattactg tcaatcagca gacagcagtg gtacctatgc ggtattcggc     300

ggagggacca aggtcaccgt ccta                                            324
```

<210> 785
<211> 108
<212> PRT
<213> Homo sapiens

<400> 785

```
Ser Tyr Glu Leu Ala Gln Pro Pro Ser Val Ser Val Ser Pro Gly Gln
```

```
                    5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Glu Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Ala Val Phe Gly Gly Gly Thr Lys Val Thr Val Leu
            100                 105
```

<210> 786
<211> 11
<212> PRT
<213> Homo sapiens

<400> 786

```
Ser Gly Asp Ala Leu Pro Arg Gln Tyr Ala Tyr
                5                   10
```

<210> 787
<211> 7
<212> PRT
<213> Homo sapiens

<400> 787

```
Lys Asp Ser Glu Arg Pro Ser
                5
```

<210> 788
<211> 11
<212> PRT
<213> Homo sapiens

<400> 788

```
Gln Ser Ala Asp Ser Ser Gly Thr Tyr Ala Val
                5                   10
```

<210> 789
<211> 22
<212> PRT
<213> Homo sapiens

<400> 789

```
Ser Tyr Glu Leu Ala Gln Pro Pro Ser Val Ser Val Ser Pro Gly Gln
                5                   10                  15

Thr Ala Arg Ile Thr Cys
            20
```

<210> 790
<211> 15
<212> PRT
<213> Homo sapiens

<400> 790

```
Trp Tyr Gln Gln Lys Pro Gly Gln Ala Pro Leu Leu Val Ile Tyr
                5                   10                  15
```

<210> 791
<211> 32
<212> PRT
<213> Homo sapiens

<400> 791

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 792
<211> 10
<212> PRT
<213> Homo sapiens

<400> 792

```
Phe Gly Gly Gly Thr Lys Val Thr Val Leu
                5                   10
```

<210> 793
<211> 375
<212> DNA
<213> Homo sapiens

<400> 793

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgttc gcggcagttc 300

gactactgga gtacttattc gggtccaact gcttttgatc tctggggcag aggcaccctg 360

gtcaccgtct cgagt 375

<210> 794
<211> 125
<212> PRT
<213> Homo sapiens

<400> 794

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ser Arg Gln Phe Asp Tyr Trp Ser Thr Tyr Ser Gly Pro Thr Ala Phe
100 105 110

Asp Leu Trp Gly Arg Gly Thr Leu Val Thr Val Ser Ser
115 120 125

<210> 795
<211> 5
<212> PRT
<213> Homo sapiens

<400> 795

Ser Tyr Ala Met Ser
5

<210> 796
<211> 17
<212> PRT
<213> Homo sapiens

<400> 796

```
Ala Ile Ser Gly Ser Gly Gly Ser Thr Tyr Tyr Ala Asp Ser Val Lys
                5                   10                  15

Gly
```

<210> 797
<211> 16
<212> PRT
<213> Homo sapiens

<400> 797

```
Gln Phe Asp Tyr Trp Ser Thr Tyr Ser Gly Pro Thr Ala Phe Asp Leu
                5                   10                  15
```

<210> 798
<211> 30
<212> PRT
<213> Homo sapiens

<400> 798

```
Glu Val Gln Leu Leu Glu Ser Gly Gly Gly Leu Val Gln Pro Gly Gly


                   5                   10                  15

   Ser Leu Arg Leu Ser Cys Ala Ala Ser Gly Phe Thr Phe Ser
               20                  25                  30
```

<210> 799
<211> 14
<212> PRT
<213> Homo sapiens

<400> 799

```
     Trp Val Arg Gln Ala Pro Gly Lys Gly Leu Glu Trp Val Ser
                     5                   10
```

<210> 800
<211> 32
<212> PRT
<213> Homo sapiens

<400> 800

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30
```

<210> 801
<211> 11
<212> PRT

<213> Homo sapiens

<400> 801

```
Trp Gly Arg Gly Thr Leu Val Thr Val Ser Ser
                5                   10
```

<210> 802
<211> 324
<212> DNA
<213> Homo sapiens

<400> 802

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtttcaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 803
<211> 108
<212> PRT
<213> Homo sapiens

<400> 803

```
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Phe Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 804
<211> 11
<212> PRT
<213> Homo sapiens

<400> 804

Ser Gly Asp Ala Leu Pro Arg Gln Tyr Ala Tyr
1 5 10

<210> 805
<211> 7
<212> PRT
<213> Homo sapiens

<400> 805

Lys Asp Ser Phe Arg Pro Ser
5

<210> 806
<211> 11
<212> PRT
<213> Homo sapiens

<400> 806

Gln Ser Ala Asp Ser Ser Gly Thr Tyr Val Val
5 10

<210> 807
<211> 22
<212> PRT
<213> Homo sapiens

<400> 807

Ser Tyr Glu Leu Ala Gln Pro Pro Ser Val Ser Val Ser Pro Gly Gln
5 10 15

Thr Ala Arg Ile Thr Cys

20

<210> 808
<211> 15
<212> PRT
<213> Homo sapiens

<400> 808

Trp Tyr Gln Gln Lys Pro Gly Gln Ala Pro Leu Leu Val Ile Tyr
5 10 15

<210> 809
<211> 32
<212> PRT
<213> Homo sapiens

<400> 809

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 810
<211> 10
<212> PRT
<213> Homo sapiens

<400> 810

```
Phe Gly Gly Gly Thr Lys Val Thr Val Leu
                5                   10
```

<210> 811
<211> 375
<212> DNA
<213> Homo sapiens

<400> 811

```
gaggtgcagc tgttggagtc tgggggaggc ttggtacagc ctggggggtc cctgagactc      60

tcctgtgcag cctctggatt cacctttagc agctttgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcaaatga acagcctgag agccgaggac acggccgtgt attactgttc gcggcagttc     300

gactactgga gtacttattc gggtccaact gcttttgatc tctggggcag aggcaccctg     360

gtcaccgtct cgagt                                                      375
```

<210> 812
<211> 125
<212> PRT
<213> Homo sapiens

<400> 812

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Phe
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ser Arg Gln Phe Asp Tyr Trp Ser Thr Tyr Ser Gly Pro Thr Ala Phe
100 105 110

Asp Leu Trp Gly Arg Gly Thr Leu Val Thr Val Ser Ser
115 120 125

<210> 813
<211> 5
<212> PRT
<213> Homo sapiens

<400> 813

Ser Phe Ala Met Ser
5

<210> 814
<211> 17
<212> PRT
<213> Homo sapiens

<400> 814

Ala Ile Ser Gly Ser Gly Gly Ser Thr Tyr Tyr Ala Asp Ser Val Lys
5 10 15

Gly

<210> 815
<211> 16
<212> PRT
<213> Homo sapiens

<400> 815

Gln Phe Asp Tyr Trp Ser Thr Tyr Ser Gly Pro Thr Ala Phe Asp Leu
5 10 15

<210> 816
<211> 30

<212> PRT
<213> Homo sapiens

<400> 816

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 817
<211> 14
<212> PRT
<213> Homo sapiens

<400> 817

```
Trp Val Arg Gln Ala Pro Gly Lys Gly Leu Glu Trp Val Ser
                5                   10
```

<210> 818
<211> 32
<212> PRT
<213> Homo sapiens

<400> 818

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30
```

<210> 819
<211> 11
<212> PRT
<213> Homo sapiens

<400> 819

```
Trp Gly Arg Gly Thr Leu Val Thr Val Ser Ser
                5                   10
```

<210> 820
<211> 324
<212> DNA
<213> Homo sapiens

<400> 820

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 821
<211> 108
<212> PRT
<213> Homo sapiens

```
<400>   821
Ser Tyr Glu Leu Ala Gln Pro Pro Ser Val Ser Val Ser Pro Gly Gln
                5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Glu Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Val Val Phe Gly Gly Gly Thr Lys Val Thr Val Leu
            100                 105
```

<210> 822
<211> 11
<212> PRT
<213> Homo sapiens

<400> 822

```
Ser Gly Asp Ala Leu Pro Arg Gln Tyr Ala Tyr
                1   5               10
```

<210> 823
<211> 7
<212> PRT
<213> Homo sapiens

<400> 823

```
Lys Asp Ser Glu Arg Pro Ser
                5
```

<210> 824
<211> 11
<212> PRT
<213> Homo sapiens

<400> 824

Gln Ser Ala Asp Ser Ser Gly Thr Tyr Val Val
1               5                   10

<210> 825
<211> 22
<212> PRT
<213> Homo sapiens

<400> 825

Ser Tyr Glu Leu Ala Gln Pro Pro Ser Val Ser Val Ser Pro Gly Gln
1               5                   10                  15

Thr Ala Arg Ile Thr Cys
            20

<210> 826
<211> 15
<212> PRT
<213> Homo sapiens

<400> 826

Trp Tyr Gln Gln Lys Pro Gly Gln Ala Pro Leu Leu Val Ile Tyr
1               5                   10                  15

<210> 827
<211> 32
<212> PRT
<213> Homo sapiens

<400> 827

Gly Ile Pro Glu Arg Phe Ser Gly Ser Gly Ser Gly Thr Thr Val Thr
1               5                   10                  15

Leu Thr Ile Ser Gly Val Gln Ala Glu Asp Glu Ala Asp Tyr Tyr Cys
            20                  25                  30

<210> 828
<211> 10
<212> PRT
<213> Homo sapiens

<400> 828

Phe Gly Gly Gly Thr Lys Val Thr Val Leu
1               5                   10

<210> 829
<211> 375

<212> DNA
<213> Homo sapiens

<400> 829

```
gaggtgcagc tgttggagtc tgggggaggc ttggtacagc ctggggggtc cctgagactc      60
tcctgtgcag cctctggatt cacctttagc agctttgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcaaatga acagcctgag agccgaggac acggccgtgt attactgttc gcggcagttc     300
gactactgga gtacttattc gggtccaact gcttttgatc tctggggcag aggcaccctg     360
gtcaccgtct cgagt                                                      375
```

<210> 830
<211> 125
<212> PRT
<213> Homo sapiens

<400> 830

```
Glu Val Gln Leu Leu Glu Ser Gly Gly Gly Leu Val Gln Pro Gly Gly
                5                   10                  15
Ser Leu Arg Leu Ser Cys Ala Ala Ser Gly Phe Thr Phe Ser Ser Phe
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ser Arg Gln Phe Asp Tyr Trp Ser Thr Tyr Ser Gly Pro Thr Ala Phe
            100                 105                 110
Asp Leu Trp Gly Arg Gly Thr Leu Val Thr Val Ser Ser
        115                 120                 125
```

<210> 831
<211> 5
<212> PRT
<213> Homo sapiens

<400> 831

```
Ser Phe Ala Met Ser
                5
```

<210> 832
<211> 17
<212> PRT
<213> Homo sapiens

<400> 832

```
Ala Ile Ser Gly Ser Gly Gly Ser Thr Tyr Tyr Ala Asp Ser Val Lys
                5                   10                  15

Gly
```

<210> 833
<211> 16
<212> PRT
<213> Homo sapiens

<400> 833

```
Gln Phe Asp Tyr Trp Ser Thr Tyr Ser Gly Pro Thr Ala Phe Asp Leu
                5                   10                  15
```

<210> 834
<211> 30
<212> PRT
<213> Homo sapiens

<400> 834

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 835
<211> 14
<212> PRT
<213> Homo sapiens

<400> 835

```
    Trp Val Arg Gln Ala Pro Gly Lys Gly Leu Glu Trp Val Ser
                    5                   10
```

<210> 836
<211> 32
<212> PRT
<213> Homo sapiens

<400> 836

```
Arg Phe Thr Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30
```

<210> 837
<211> 11
<212> PRT
<213> Homo sapiens

<400> 837

```
Trp Gly Arg Gly Thr Leu Val Thr Val Ser Ser
                5                   10
```

<210> 838
<211> 324
<212> DNA
<213> Homo sapiens

<400> 838

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtttcaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgt ggtattcggc     300
ggagggacca aggtcaccgt ccta                                            324
```

<210> 839
<211> 108
<212> PRT
<213> Homo sapiens

<400> 839

```
Ser Tyr Glu Leu Ala Gln Pro Pro Ser Val Ser Val Ser Pro Gly Gln
                5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Phe Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Val Val Phe Gly Gly Gly Thr Lys Val Thr Val Leu
            100                 105
```

<210> 840
<211> 11

<212> PRT
<213> Homo sapiens

<400> 840

Ser Gly Asp Ala Leu Pro Arg Gln Tyr Ala Tyr
1               5                   10

<210> 841
<211> 7
<212> PRT
<213> Homo sapiens

<400> 841

Lys Asp Ser Phe Arg Pro Ser
                5

<210> 842
<211> 11
<212> PRT
<213> Homo sapiens

<400> 842

Gln Ser Ala Asp Ser Ser Gly Thr Tyr Val Val
                5                   10

<210> 843
<211> 22
<212> PRT
<213> Homo sapiens

<400> 843

Ser Tyr Glu Leu Ala Gln Pro Pro Ser Val Ser Val Ser Pro Gly Gln
                5                   10                  15

Thr Ala Arg Ile Thr Cys
            20

<210> 844
<211> 15
<212> PRT
<213> Homo sapiens

<400> 844

Trp Tyr Gln Gln Lys Pro Gly Gln Ala Pro Leu Leu Val Ile Tyr
                5                   10                  15

<210> 845
<211> 32
<212> PRT
<213> Homo sapiens

<400> 845

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 846
<211> 10
<212> PRT
<213> Homo sapiens

<400> 846

```
Phe Gly Gly Gly Thr Lys Val Thr Val Leu
                5                   10
```

<210> 847
<211> 375
<212> DNA
<213> Homo sapiens

<400> 847

```
gaggtgcagc tgttggagtc tgggggaggc ttggtacagc ctggggggtc cctgagactc      60
tcctgtgcag cctctggatt cacctttagc agctatgcca tgagctgggt ccgccaggct     120
ccagggaagg ggctggagtg ggtctcagct attagtggta gtggtagtag cacatactac     180
gcagactccg tgaagggccg gttcgccatc tccagagaca attccaagaa cacgctgtat     240
ctgcaaatga acagcctgag agccgaggac acggccgtgt attactgttc gcggcagttc     300
gactactgga gtacttattc gggtccaact gcttttgatc tctggggcag aggcaccctg     360
gtcaccgtct cgagt                                                      375
```

<210> 848
<211> 125
<212> PRT
<213> Homo sapiens

<400> 848

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Ser Ser Thr Tyr Tyr Ala Asp Ser Val
50 55 60

Lys Gly Arg Phe Ala Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ser Arg Gln Phe Asp Tyr Trp Ser Thr Tyr Ser Gly Pro Thr Ala Phe
100 105 110

Asp Leu Trp Gly Arg Gly Thr Leu Val Thr Val Ser Ser
115 120 125

<210> 849
<211> 5
<212> PRT
<213> Homo sapiens

<400> 849

Ser Tyr Ala Met Ser
5

<210> 850
<211> 17
<212> PRT
<213> Homo sapiens

<400> 850

Ala Ile Ser Gly Ser Gly Ser Ser Thr Tyr Tyr Ala Asp Ser Val Lys
5 10 15

Gly

<210> 851
<211> 16
<212> PRT
<213> Homo sapiens

<400> 851

Gln Phe Asp Tyr Trp Ser Thr Tyr Ser Gly Pro Thr Ala Phe Asp Leu
5 10 15

<210> 852
<211> 30
<212> PRT

<213> Homo sapiens

<400> 852

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser
            20                  25                  30
```

<210> 853
<211> 14
<212> PRT
<213> Homo sapiens

<400> 853

```
Trp Val Arg Gln Ala Pro Gly Lys Gly Leu Glu Trp Val Ser
                5                   10
```

<210> 854
<211> 32
<212> PRT
<213> Homo sapiens

<400> 854

```
Arg Phe Ala Ile Ser Arg Asp Asn Ser Lys Asn Thr Leu Tyr Leu Gln
                5                   10                  15

Met Asn Ser Leu Arg Ala Glu Asp Thr Ala Val Tyr Tyr Cys Ser Arg
            20                  25                  30
```

<210> 855
<211> 11
<212> PRT
<213> Homo sapiens

<400> 855

```
Trp Gly Arg Gly Thr Leu Val Thr Val Ser Ser
                5                   10
```

<210> 856
<211> 324
<212> DNA
<213> Homo sapiens

<400> 856

```
tcctatgagc tggctcagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtgagaggc cctcagggat ccctgagcga     180
ttctctggct ccggctcagg gacaacagtc acgttgacca tcagtggagt ccaggcagaa     240
gacgaggctg actattactg tcaatcagca gacagcagtg gtacctatgc ggtattcggc     300
ggagggacca aggtcaccgt ccta                                            324
```

<210> 857
<211> 108
<212> PRT
<213> Homo sapiens

<400> 857

```
Ser Tyr Glu Leu Ala Gln Pro Pro Ser Val Ser Val Ser Pro Gly Gln
                5                   10                  15
Thr Ala Arg Ile Thr Cys Ser Gly Asp Ala Leu Pro Arg Gln Tyr Ala
            20                  25                  30
Tyr Trp Tyr Gln Gln Lys Pro Gly Gln Ala Pro Leu Leu Val Ile Tyr
        35                  40                  45
Lys Asp Ser Glu Arg Pro Ser Gly Ile Pro Glu Arg Phe Ser Gly Ser
    50                  55                  60
Gly Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95
Ala Val Phe Gly Gly Gly Thr Lys Val Thr Val Leu
            100                 105
```

<210> 858
<211> 11
<212> PRT
<213> Homo sapiens

<400> 858

```
Ser Gly Asp Ala Leu Pro Arg Gln Tyr Ala Tyr
                5                   10
```

<210> 859
<211> 7
<212> PRT
<213> Homo sapiens

<400> 859

```
Lys Asp Ser Glu Arg Pro Ser
                5
```

<210> 860
<211> 11
<212> PRT
<213> Homo sapiens

<400> 860

```
Gln Ser Ala Asp Ser Ser Gly Thr Tyr Ala Val
                5                   10
```

<210> 861
<211> 22
<212> PRT
<213> Homo sapiens

<400> 861

```
Ser Tyr Glu Leu Ala Gln Pro Pro Ser Val Ser Val Ser Pro Gly Gln
                5                   10                  15

Thr Ala Arg Ile Thr Cys
            20
```

<210> 862
<211> 15
<212> PRT
<213> Homo sapiens

<400> 862

```
Trp Tyr Gln Gln Lys Pro Gly Gln Ala Pro Leu Leu Val Ile Tyr
                5                   10                  15
```

<210> 863
<211> 32
<212> PRT
<213> Homo sapiens

<400> 863

```
Gly Ile Pro Glu Arg Phe Ser Gly Ser Gly Ser Gly Thr Thr Val Thr
                5                   10                  15

Leu Thr Ile Ser Gly Val Gln Ala Glu Asp Glu Ala Asp Tyr Tyr Cys
            20                  25                  30
```

<210> 864
<211> 10
<212> PRT
<213> Homo sapiens

<400> 864

```
Phe Gly Gly Gly Thr Lys Val Thr Val Leu
                5                   10
```

<210> 865
<211> 375

<212> DNA
<213> Homo sapiens

<400> 865

```
gaggtgcagc tgttggagtc tgggggaggc ttggtacagc ctggggggtc cctgagactc      60
tcctgtgcag cctctggatt cacctttagc agctttgcca tgagctgggt ccgccaggct     120
ccagggaagg ggctggagtg ggtctcagct attagtggta gtggtagtag cacatactac     180
gcagactccg tgaagggccg gttcgccatc tccagagaca attccaagaa cacgctgtat     240
ctgcaaatga acagcctgag agccgaggac acggccgtgt attactgtgc gagagatagg     300
gatttttgga gtacttattc gggtccaact gcttttgatc tctggggcag aggcaccctg     360
gtcaccgtct cgagt                                                      375
```

<210> 866
<211> 125
<212> PRT
<213> Homo sapiens

<400> 866

```
Glu Val Gln Leu Leu Glu Ser Gly Gly Gly Leu Val Gln Pro Gly Gly
                5                   10                  15

Ser Leu Arg Leu Ser Cys Ala Ala Ser Gly Phe Thr Phe Ser Ser Phe
            20                  25                  30

Ala Met Ser Trp Val Arg Gln Ala Pro Gly Lys Gly Leu Glu Trp Val
        35                  40                  45

Ser Ala Ile Ser Gly Ser Gly Ser Ser Thr Tyr Tyr Ala Asp Ser Val
    50                  55                  60

Lys Gly Arg Phe Ala Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Phe Trp Ser Thr Tyr Ser Gly Pro Thr Ala Phe
            100                 105                 110

Asp Leu Trp Gly Arg Gly Thr Leu Val Thr Val Ser Ser
        115                 120                 125
```

<210> 867
<211> 5
<212> PRT
<213> Homo sapiens

<400> 867

```
Ser Phe Ala Met Ser
                5
```

<210> 868
<211> 17
<212> PRT
<213> Homo sapiens

<400> 868

Ala Ile Ser Gly Ser Gly Ser Ser Thr Tyr Tyr Ala Asp Ser Val Lys
5 10 15

Gly

<210> 869
<211> 16
<212> PRT
<213> Homo sapiens

<400> 869

Asp Arg Asp Phe Trp Ser Thr Tyr Ser Gly Pro Thr Ala Phe Asp Leu
5 10 15

<210> 870
<211> 30
<212> PRT
<213> Homo sapiens

<400> 870

Glu Val Gln Leu Leu Glu Ser Gly Gly Gly Leu Val Gln Pro Gly Gly
5 10 15

Ser Leu Arg Leu Ser Cys Ala Ala Ser Gly Phe Thr Phe Ser
20 25 30

<210> 871
<211> 14
<212> PRT
<213> Homo sapiens

<400> 871

Trp Val Arg Gln Ala Pro Gly Lys Gly Leu Glu Trp Val Ser
5 10

<210> 872
<211> 32
<212> PRT
<213> Homo sapiens

<400> 872

Arg Phe Ala Ile Ser Arg Asp Asn Ser Lys Asn Thr Leu Tyr Leu Gln
5 10 15

Met Asn Ser Leu Arg Ala Glu Asp Thr Ala Val Tyr Tyr Cys Ala Arg
20 25 30

<210> 873
<211> 11
<212> PRT
<213> Homo sapiens

<400> 873

```
Trp Gly Arg Gly Thr Leu Val Thr Val Ser Ser
                1               5                   10
```

<210> 874
<211> 324
<212> DNA
<213> Homo sapiens

<400> 874

```
tcctatgagc tggcccagcc accctcggtg tcagtgtccc caggacagac ggccaggatc      60
acctgctctg gagatgcatt gccaaggcaa tatgcttact ggtaccagca gaagccaggc     120
caggcccctc tactggtgat atataaagac agtttcaggc cctcagggat ccctgagcga     180
ttctctggct ccggctcagg gacaacagtc acgttgacca tcagtggagt ccaggcagaa     240
gacgaggctg actattactg tcaatcagcg gacagcagtg gtacctatgc ggtattcggc     300
ggagggacca aggtcaccgt ccta                                            324
```

<210> 875
<211> 108
<212> PRT
<213> Homo sapiens

<400> 875

```
Ser Tyr Glu Leu Ala Gln Pro Pro Ser Val Ser Val Ser Pro Gly Gln
1               5                   10                  15

Thr Ala Arg Ile Thr Cys Ser Gly Asp Ala Leu Pro Arg Gln Tyr Ala
            20                  25                  30

Tyr Trp Tyr Gln Gln Lys Pro Gly Gln Ala Pro Leu Leu Val Ile Tyr
        35                  40                  45

Lys Asp Ser Phe Arg Pro Ser Gly Ile Pro Glu Arg Phe Ser Gly Ser
    50                  55                  60

Gly Ser Gly Thr Thr Val Thr Leu Thr Ile Ser Gly Val Gln Ala Glu
65                  70                  75                  80

Asp Glu Ala Asp Tyr Tyr Cys Gln Ser Ala Asp Ser Ser Gly Thr Tyr
                85                  90                  95

Ala Val Phe Gly Gly Gly Thr Lys Val Thr Val Leu
            100                 105
```

<210> 876

<211> 11
<212> PRT
<213> Homo sapiens

<400> 876

Ser Gly Asp Ala Leu Pro Arg Gln Tyr Ala Tyr
5 10

<210> 877
<211> 7
<212> PRT
<213> Homo sapiens

<400> 877

Lys Asp Ser Phe Arg Pro Ser
5

<210> 878
<211> 11
<212> PRT
<213> Homo sapiens

<400> 878

Gln Ser Ala Asp Ser Ser Gly Thr Tyr Ala Val
5 10

<210> 879
<211> 22
<212> PRT
<213> Homo sapiens

<400> 879

Ser Tyr Glu Leu Ala Gln Pro Pro Ser Val Ser Val Ser Pro Gly Gln
5 10 15

Thr Ala Arg Ile Thr Cys
20

<210> 880
<211> 15
<212> PRT
<213> Homo sapiens

<400> 880

Trp Tyr Gln Gln Lys Pro Gly Gln Ala Pro Leu Leu Val Ile Tyr
5 10 15

<210> 881
<211> 32
<212> PRT
<213> Homo sapiens

<400> 881

Gly Ile Pro Glu Arg Phe Ser Gly Ser Gly Ser Gly Thr Thr Val Thr
5 10 15

Leu Thr Ile Ser Gly Val Gln Ala Glu Asp Glu Ala Asp Tyr Tyr Cys
20 25 30

<210> 882
<211> 10
<212> PRT
<213> Homo sapiens

<400> 882

Phe Gly Gly Gly Thr Lys Val Thr Val Leu
5 10

<210> 883
<211> 366
<212> DNA
<213> Rattus rattus

<400> 883

caggtgcagc tgaaggagtc aggacctggc ctggtgaagc cctcagagac cctgtctctc 60

acctgcactg tctctgggtt ctcattaacc aactatcatg taaactgggt tcgacagcct 120

ccaggaaagg gtctggagtg gatgggagta atatggggtg atggaagcac agcatataat 180

tcagctctca aatcccgact gagcatcagc agggacacct cgaagagcca agttttctta 240

aaaatgaaca gtctgcaaac tgaagacaca gccacttact actgtgccag aggcggggat 300

tactatgatg gtagttatta ctacgagggc tactggggcc aaggagtcat ggtcaccgtc 360

tcctca 366

<210> 884
<211> 122
<212> PRT
<213> Rattus rattus

<400> 884

```
Gln Val Gln Leu Lys Glu Ser Gly Pro Gly Leu Val Lys Pro Ser Glu
                5                   10                  15

Thr Leu Ser Leu Thr Cys Thr Val Ser Gly Phe Ser Leu Thr Asn Tyr
            20                  25                  30

His Val Asn Trp Val Arg Gln Pro Pro Gly Lys Gly Leu Glu Trp Met
        35                  40                  45

Gly Val Ile Trp Gly Asp Gly Ser Thr Ala Tyr Asn Ser Ala Leu Lys
    50                  55                  60

Ser Arg Leu Ser Ile Ser Arg Asp Thr Ser Lys Ser Gln Val Phe Leu
65                  70                  75                  80

Lys Met Asn Ser Leu Gln Thr Glu Asp Thr Ala Thr Tyr Tyr Cys Ala
                85                  90                  95

Arg Gly Gly Asp Tyr Tyr Asp Gly Ser Tyr Tyr Tyr Glu Gly Tyr Trp
            100                 105                 110

Gly Gln Gly Val Met Val Thr Val Ser Ser
        115                 120
```

<210> 885
<211> 5
<212> PRT
<213> Rattus rattus

<400> 885

```
Asn Tyr His Val Asn
                5
```

<210> 886
<211> 16
<212> PRT
<213> Rattus rattus

<400> 886

```
Val Ile Trp Gly Asp Gly Ser Thr Ala Tyr Asn Ser Ala Leu Lys Ser
                5                   10                  15
```

<210> 887
<211> 14
<212> PRT
<213> Rattus rattus

<400> 887

```
Gly Gly Asp Tyr Tyr Asp Gly Ser Tyr Tyr Tyr Glu Gly Tyr
                5                   10
```

<210> 888
<211> 30
<212> PRT
<213> Rattus rattus

<400> 888

Gln Val Gln Leu Lys Glu Ser Gly Pro Gly Leu Val Lys Pro Ser Glu
1               5                   10                  15

Thr Leu Ser Leu Thr Cys Thr Val Ser Gly Phe Ser Leu Thr
            20                  25                  30

<210> 889
<211> 14
<212> PRT
<213> Rattus rattus

<400> 889

Trp Val Arg Gln Pro Pro Gly Lys Gly Leu Glu Trp Met Gly
1               5                   10

<210> 890
<211> 32
<212> PRT
<213> Rattus rattus

<400> 890

Arg Leu Ser Ile Ser Arg Asp Thr Ser Lys Ser Gln Val Phe Leu Lys
                5                   10                  15

Met Asn Ser Leu Gln Thr Glu Asp Thr Ala Thr Tyr Tyr Cys Ala Arg
            20                  25                  30

<210> 891
<211> 11
<212> PRT
<213> Rattus rattus

<400> 891

Trp Gly Gln Gly Val Met Val Thr Val Ser Ser
                5                   10

<210> 892
<211> 321
<212> DNA
<213> Rattus rattus

<400> 892

```
gacatccaga tgacacagtc tcctgcctcc ctgtctgcat ctctggaaga aattgtcacc      60
atcacatgcc aggcaagcca ggacattggt aattggttat catggtatca gcagaaacca     120
gggaaatctc ctcagctcct gatctatggt gcaaccagct tggcagatgg ggtcccatca     180
aggttcagcg gcagtagatc tggcacacag ttttctctta agatcagcag actacaggtt     240
gaagatattg gaatctatta ctgtctacag gcttatagtg ctccgtggac gttcggtgga     300
ggcaccaagc tggaattgaa a                                               321
```

<210> 893
<211> 107
<212> PRT
<213> Rattus rattus

<400> 893

```
Asp Ile Gln Met Thr Gln Ser Pro Ala Ser Leu Ser Ala Ser Leu Glu
                5                   10                  15

Glu Ile Val Thr Ile Thr Cys Gln Ala Ser Gln Asp Ile Gly Asn Trp
            20                  25                  30

Leu Ser Trp Tyr Gln Gln Lys Pro Gly Lys Ser Pro Gln Leu Leu Ile
        35                  40                  45

Tyr Gly Ala Thr Ser Leu Ala Asp Gly Val Pro Ser Arg Phe Ser Gly
    50                  55                  60

Ser Arg Ser Gly Thr Gln Phe Ser Leu Lys Ile Ser Arg Leu Gln Val
65                  70                  75                  80

Glu Asp Ile Gly Ile Tyr Tyr Cys Leu Gln Ala Tyr Ser Ala Pro Trp
                85                  90                  95

Thr Phe Gly Gly Gly Thr Lys Leu Glu Leu Lys
            100                 105
```

<210> 894
<211> 11
<212> PRT
<213> Rattus rattus

<400> 894

```
Gln Ala Ser Gln Asp Ile Gly Asn Trp Leu Ser
                5                   10
```

<210> 895
<211> 7
<212> PRT
<213> Rattus rattus

<400> 895

```
Gly Ala Thr Ser Leu Ala Asp
                5
```

<210> 896
<211> 9
<212> PRT
<213> Rattus rattus

<400> 896

```
Leu Gln Ala Tyr Ser Ala Pro Trp Thr
                5
```

<210> 897
<211> 23
<212> PRT
<213> Rattus rattus

<400> 897

```
Asp Ile Gln Met Thr Gln Ser Pro Ala Ser Leu Ser Ala Ser Leu Glu
                5                   10                  15

Glu Ile Val Thr Ile Thr Cys
            20
```

<210> 898
<211> 15
<212> PRT
<213> Rattus rattus

<400> 898

```
Trp Tyr Gln Gln Lys Pro Gly Lys Ser Pro Gln Leu Leu Ile Tyr
                5                   10                  15
```

<210> 899
<211> 32
<212> PRT
<213> Rattus rattus

<400> 899

```
Gly Val Pro Ser Arg Phe Ser Gly Ser Arg Ser Gly Thr Gln Phe Ser
                5                   10                  15

Leu Lys Ile Ser Arg Leu Gln Val Glu Asp Ile Gly Ile Tyr Tyr Cys
            20                  25                  30
```

<210> 900
<211> 10
<212> PRT
<213> Rattus rattus

<400> 900

```
Phe Gly Gly Gly Thr Lys Leu Glu Leu Lys
                5                   10
```

<210> 901
<211> 366

<212> DNA
<213> Rattus rattus

<400> 901

```
caggtgcagc tgaaggagtc aggacctggc ctggtgaagc cctcagagac cctgtctctc      60
acctgcactg tctctgggtt ctcattaacc aactatcatg taaactgggt tcgacagcct     120
ccaggaaagg gtctggagtg gatgggagta atatggggtg atggaagcac agcatataat     180
tcagctctca aatcccgact gagcatcagc agggacacct cgaagagcca agttttctta     240
aaaatgaaca gtctgcaaac tgaagacaca gccacttact actgtgccag aggcggggat     300
tactatgatg gtagttatta ctacgagggc tactggggcc aaggagtcat ggtcaccgtc     360
tcctca                                                                366
```

<210> 902
<211> 122
<212> PRT
<213> Rattus rattus

<400> 902

```
Gln Val Gln Leu Lys Glu Ser Gly Pro Gly Leu Val Lys Pro Ser Glu
                5                   10                  15

Thr Leu Ser Leu Thr Cys Thr Val Ser Gly Phe Ser Leu Thr Asn Tyr
            20                  25                  30

His Val Asn Trp Val Arg Gln Pro Pro Gly Lys Gly Leu Glu Trp Met
        35                  40                  45

Gly Val Ile Trp Gly Asp Gly Ser Thr Ala Tyr Asn Ser Ala Leu Lys
    50                  55                  60

Ser Arg Leu Ser Ile Ser Arg Asp Thr Ser Lys Ser Gln Val Phe Leu
65                  70                  75                  80

Lys Met Asn Ser Leu Gln Thr Glu Asp Thr Ala Thr Tyr Tyr Cys Ala
                85                  90                  95

Arg Gly Gly Asp Tyr Tyr Asp Gly Ser Tyr Tyr Tyr Glu Gly Tyr Trp
            100                 105                 110

Gly Gln Gly Val Met Val Thr Val Ser Ser
        115                 120
```

<210> 903
<211> 5
<212> PRT
<213> Rattus rattus

<400> 903

```
Asn Tyr His Val Asn
                5
```

<210> 904
<211> 16
<212> PRT
<213> Rattus rattus

<400> 904

```
Val Ile Trp Gly Asp Gly Ser Thr Ala Tyr Asn Ser Ala Leu Lys Ser
                5                   10                  15
```

<210> 905
<211> 14
<212> PRT
<213> Rattus rattus

<400> 905

```
Gly Gly Asp Tyr Tyr Asp Gly Ser Tyr Tyr Tyr Glu Gly Tyr
                5                   10
```

<210> 906
<211> 30
<212> PRT
<213> Rattus rattus

<400> 906

```
Gln Val Gln Leu Lys Glu Ser Gly Pro Gly Leu Val Lys Pro Ser Glu
                5                   10                  15

Thr Leu Ser Leu Thr Cys Thr Val Ser Gly Phe Ser Leu Thr
            20                  25                  30
```

<210> 907
<211> 14
<212> PRT
<213> Rattus rattus

<400> 907

```
Trp Val Arg Gln Pro Pro Gly Lys Gly Leu Glu Trp Met Gly
                5                   10
```

<210> 908
<211> 32
<212> PRT
<213> Rattus rattus

<400> 908

```
Arg Leu Ser Ile Ser Arg Asp Thr Ser Lys Ser Gln Val Phe Leu Lys
                5                   10                  15

Met Asn Ser Leu Gln Thr Glu Asp Thr Ala Thr Tyr Tyr Cys Ala Arg
            20                  25                  30
```

<210> 909
<211> 11

<212> PRT
<213> Rattus rattus

<400> 909

Trp Gly Gln Gly Val Met Val Thr Val Ser Ser
                5                   10

<210> 910
<211> 321
<212> DNA
<213> Rattus rattus

<400> 910

gacatccaga tgacacagtc tcctgcctcc ctgtctgcat ctctggaaga aattgtcacc 60

atcacatgcc aggcaagcca agacattggt aattggttgg catggtatca gcagaaaccg 120

gggaaatctc ctcagctcct gatttatgat gcaaccagct tggcagatgg ggtcccatca 180

cggttcagcg gcagtagatc tggcacacag tattctctta agatcagcag actacaggtt 240

gaagatattg gaagctatta ctgtcaccag gctcatagta atcctcggac gttcggtgga 300

ggcaccaagc tggaattgaa a 321

<210> 911
<211> 107
<212> PRT
<213> Rattus rattus

<400> 911

Asp Ile Gln Met Thr Gln Ser Pro Ala Ser Leu Ser Ala Ser Leu Glu
                5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Ile
        35                  40                  45

Tyr Asp Ala Thr Ser Leu Ala Asp Gly Val Pro Ser Arg Phe Ser Gly
    50                  55                  60

Ser Arg Ser Gly Thr Gln Tyr Ser Leu Lys Ile Ser Arg Leu Gln Val
65                  70                  75                  80

Glu Asp Ile Gly Ser Tyr Tyr Cys His Gln Ala His Ser Asn Pro Arg
                85                  90                  95

Thr Phe Gly Gly Gly Thr Lys Leu Glu Leu Lys
            100                 105

<210> 912
<211> 11
<212> PRT
<213> Rattus rattus

<400> 912

Gln Ala Ser Gln Asp Ile Gly Asn Trp Leu Ala
5 10

<210> 913
<211> 7
<212> PRT
<213> Rattus rattus

<400> 913

Asp Ala Thr Ser Leu Ala Asp
5

<210> 914
<211> 9
<212> PRT
<213> Rattus rattus

<400> 914

His Gln Ala His Ser Asn Pro Arg Thr
5

<210> 915
<211> 23
<212> PRT
<213> Rattus rattus

<400> 915

Asp Ile Gln Met Thr Gln Ser Pro Ala Ser Leu Ser Ala Ser Leu Glu
5 10 15

Glu Ile Val Thr Ile Thr Cys
20

<210> 916
<211> 15
<212> PRT
<213> Rattus rattus

<400> 916

Trp Tyr Gln Gln Lys Pro Gly Lys Ser Pro Gln Leu Leu Ile Tyr
5 10 15

<210> 917
<211> 32
<212> PRT
<213> Rattus rattus

<400> 917

```
Gly Val Pro Ser Arg Phe Ser Gly Ser Arg Ser Gly Thr Gln Tyr Ser
                5                   10                  15

Leu Lys Ile Ser Arg Leu Gln Val Glu Asp Ile Gly Ser Tyr Tyr Cys
            20                  25                  30
```

<210> 918
<211> 10
<212> PRT
<213> Rattus rattus

<400> 918

```
Phe Gly Gly Gly Thr Lys Leu Glu Leu Lys
                5                   10
```

<210> 919
<211> 366
<212> DNA
<213> Rattus rattus

<400> 919

```
caggtgcagc tgaaggagtc aggacctggc ctggtgaagc cctcagagac cctgtctctc      60
acctgcactg tctctgggtt ctcattaacc aactatcatg taaactgggt tcgacagcct     120
ccaggaaagg gtctggagtg gatgggagta atatggggtg atggaagcac agcatataat     180
tcagctctca aatcccgact gagcatcagc agggacacct cgaagagcca agttttctta     240
aaaatgaaca gtctgcaaac tgaagacaca gccacttact actgtgccag aggcggggat     300
tactatgatg gtagttatta ctacgagggc tactggggcc aaggagtcat ggtcaccgtc     360
tcctca                                                                366
```

<210> 920
<211> 122
<212> PRT
<213> Rattus rattus

<400> 920

Gln Val Gln Leu Lys Glu Ser Gly Pro Gly Leu Val Lys Pro Ser Glu
5 10 15

Thr Leu Ser Leu Thr Cys Thr Val Ser Gly Phe Ser Leu Thr Asn Tyr
20 25 30

His Val Asn Trp Val Arg Gln Pro Pro Gly Lys Gly Leu Glu Trp Met
35 40 45

Gly Val Ile Trp Gly Asp Gly Ser Thr Ala Tyr Asn Ser Ala Leu Lys
50 55 60

Ser Arg Leu Ser Ile Ser Arg Asp Thr Ser Lys Ser Gln Val Phe Leu
65 70 75 80

Lys Met Asn Ser Leu Gln Thr Glu Asp Thr Ala Thr Tyr Tyr Cys Ala
85 90 95

Arg Gly Gly Asp Tyr Tyr Asp Gly Ser Tyr Tyr Tyr Glu Gly Tyr Trp
100 105 110

Gly Gln Gly Val Met Val Thr Val Ser Ser
115 120

<210> 921
<211> 5
<212> PRT
<213> Rattus rattus

<400> 921

Asn Tyr His Val Asn
5

<210> 922
<211> 16
<212> PRT
<213> Rattus rattus

<400> 922

Val Ile Trp Gly Asp Gly Ser Thr Ala Tyr Asn Ser Ala Leu Lys Ser
5 10 15

<210> 923
<211> 14
<212> PRT
<213> Rattus rattus

<400> 923

Gly Gly Asp Tyr Tyr Asp Gly Ser Tyr Tyr Tyr Glu Gly Tyr
5 10

<210> 924
<211> 30
<212> PRT
<213> Rattus rattus

<400> 924

```
Gln Val Gln Leu Lys Glu Ser Gly Pro Gly Leu Val Lys Pro Ser Glu
                5                   10                  15

Thr Leu Ser Leu Thr Cys Thr Val Ser Gly Phe Ser Leu Thr
            20                  25                  30
```

<210> 925
<211> 14
<212> PRT
<213> Rattus rattus

<400> 925

```
Trp Val Arg Gln Pro Pro Gly Lys Gly Leu Glu Trp Met Gly
                5                   10
```

<210> 926
<211> 32
<212> PRT
<213> Rattus rattus

<400> 926

```
Arg Leu Ser Ile Ser Arg Asp Thr Ser Lys Ser Gln Val Phe Leu Lys
                5                   10                  15

Met Asn Ser Leu Gln Thr Glu Asp Thr Ala Thr Tyr Tyr Cys Ala Arg
            20                  25                  30
```

<210> 927
<211> 11
<212> PRT
<213> Rattus rattus

<400> 927

```
Trp Gly Gln Gly Val Met Val Thr Val Ser Ser
                5                   10
```

<210> 928
<211> 321
<212> DNA
<213> Rattus rattus

<400> 928

```
gacatccaga tgacacagtc tcctgcctcc ctgtctgcat ctctggaaga aattgtcacc      60
atcacatgcc aggcaagcca agacattggt aattggttgg catggtatca gcagaaaccg     120
gggaaatctc ctcaactcct gatttatgat gcaaccagct tggcagatgg ggtcccatca     180
cggttcagcg gcagtagatc tggcacacag tattctctta agatcagcag actacaggtt     240
gaagatattg gaatctatta ctgtctacag gcttatagtg ctccgtggac gttcggtgga     300
ggcaccaagc tggaattgaa a                                               321
```

<210> 929
<211> 107
<212> PRT
<213> Rattus rattus

<400> 929

```
Asp Ile Gln Met Thr Gln Ser Pro Ala Ser Leu Ser Ala Ser Leu Glu
                5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Ile
        35                  40                  45

Tyr Asp Ala Thr Ser Leu Ala Asp Gly Val Pro Ser Arg Phe Ser Gly
    50                  55                  60

Ser Arg Ser Gly Thr Gln Tyr Ser Leu Lys Ile Ser Arg Leu Gln Val
65                  70                  75                  80

Glu Asp Ile Gly Ile Tyr Tyr Cys Leu Gln Ala Tyr Ser Ala Pro Trp
                85                  90                  95

Thr Phe Gly Gly Gly Thr Lys Leu Glu Leu Lys
            100                 105
```

<210> 930
<211> 11
<212> PRT
<213> Rattus rattus

<400> 930

```
Gln Ala Ser Gln Asp Ile Gly Asn Trp Leu Ala
                5                   10
```

<210> 931
<211> 7
<212> PRT
<213> Rattus rattus

<400> 931

```
Asp Ala Thr Ser Leu Ala Asp
                5
```

<210> 932
<211> 9
<212> PRT
<213> Rattus rattus

<400> 932

Leu Gln Ala Tyr Ser Ala Pro Trp Thr
5

<210> 933
<211> 23
<212> PRT
<213> Rattus rattus

<400> 933

Asp Ile Gln Met Thr Gln Ser Pro Ala Ser Leu Ser Ala Ser Leu Glu
5 10 15

Glu Ile Val Thr Ile Thr Cys
20

<210> 934
<211> 15
<212> PRT
<213> Rattus rattus

<400> 934

Trp Tyr Gln Gln Lys Pro Gly Lys Ser Pro Gln Leu Leu Ile Tyr
5 10 15

<210> 935
<211> 32
<212> PRT
<213> Rattus rattus

<400> 935

Gly Val Pro Ser Arg Phe Ser Gly Ser Arg Ser Gly Thr Gln Tyr Ser
5 10 15

Leu Lys Ile Ser Arg Leu Gln Val Glu Asp Ile Gly Ile Tyr Tyr Cys
20 25 30

<210> 936
<211> 10
<212> PRT
<213> Rattus rattus

<400> 936

Phe Gly Gly Gly Thr Lys Leu Glu Leu Lys
5 10

<210> 937
<211> 360

<212> DNA
<213> Mus musculus

<400> 937

```
caggtgcagc tgaaggagtc tggacctggc ctggtggcgc cctcacagag cctgtccatc      60
acttgcactg tctctgggtt ttcattaacc acctatggta tacactgggt tcgccagcct     120
ccaggaaagg gtctggagtg gctgggagta atatggactc gtggaagcac aaattataat     180
tcggctctca tgtccagact gagcatcagc aaagacaact ccaagagcca agttttctta     240
aaaatgaaca gtctgcaaac tgatgacaca gccatgtact tctgtgccag agatggttac     300
tacggttatt actatgcttt ggactactgg ggtcaaggaa cctcagtcac cgtctcctca     360
```

<210> 938
<211> 120
<212> PRT
<213> Mus musculus

<400> 938

```
Gln Val Gln Leu Lys Glu Ser Gly Pro Gly Leu Val Ala Pro Ser Gln
                5                   10                  15
Ser Leu Ser Ile Thr Cys Thr Val Ser Gly Phe Ser Leu Thr Thr Tyr
            20                  25                  30
Gly Ile His Trp Val Arg Gln Pro Pro Gly Lys Gly Leu Glu Trp Leu
        35                  40                  45
Gly Val Ile Trp Thr Arg Gly Ser Thr Asn Tyr Asn Ser Ala Leu Met
    50                  55                  60
Ser Arg Leu Ser Ile Ser Lys Asp Asn Ser Lys Ser Gln Val Phe Leu
65                  70                  75                  80
Lys Met Asn Ser Leu Gln Thr Asp Asp Thr Ala Met Tyr Phe Cys Ala
                85                  90                  95
Arg Asp Gly Tyr Tyr Gly Tyr Tyr Tyr Ala Leu Asp Tyr Trp Gly Gln
            100                 105                 110
Gly Thr Ser Val Thr Val Ser Ser
        115                 120
```

<210> 939
<211> 5
<212> PRT
<213> Mus musculus

<400> 939

```
Thr Tyr Gly Ile His
                5
```

<210> 940

<211> 16
<212> PRT
<213> Mus musculus

<400> 940

Val Ile Trp Thr Arg Gly Ser Thr Asn Tyr Asn Ser Ala Leu Met Ser
1 5 10 15

<210> 941
<211> 12
<212> PRT
<213> Mus musculus

<400> 941

Asp Gly Tyr Tyr Gly Tyr Tyr Tyr Ala Leu Asp Tyr
1 5 10

<210> 942
<211> 30
<212> PRT
<213> Mus musculus

<400> 942

Gln Val Gln Leu Lys Glu Ser Gly Pro Gly Leu Val Ala Pro Ser Gln
1 5 10 15

Ser Leu Ser Ile Thr Cys Thr Val Ser Gly Phe Ser Leu Thr
20 25 30

<210> 943
<211> 14
<212> PRT
<213> Mus musculus

<400> 943

Trp Val Arg Gln Pro Pro Gly Lys Gly Leu Glu Trp Leu Gly
1 5 10

<210> 944
<211> 32
<212> PRT
<213> Mus musculus

<400> 944

Arg Leu Ser Ile Ser Lys Asp Asn Ser Lys Ser Gln Val Phe Leu Lys
1 5 10 15

Met Asn Ser Leu Gln Thr Asp Asp Thr Ala Met Tyr Phe Cys Ala Arg
20 25 30

<210> 945
<211> 11
<212> PRT

<213> Mus musculus

<400> 945

Trp Gly Gln Gly Thr Ser Val Thr Val Ser Ser
5 10

<210> 946
<211> 321
<212> DNA
<213> Mus musculus

<400> 946

caaattgttc tcaaacagtc tccagcaatc atgtctgcat ctccagggga gaaggtcacc 60

atgacctgca gtgccagctc aagtgtaagt tacatgtact ggtaccagca gaagccagga 120

tcctccccca gactcctgat ttatgacaca tccaacctgg cttctggagt ccctgttcgc 180

ttcagttaca gtgggtctgg gacctcttac tctctcacaa tcagccgaat ggaggctgaa 240

gatgctgcca cttattactg ccagcagtgg agtagttacc cacccatgac gttcggtgga 300

ggcaccaagc tggaaatcaa a 321

<210> 947
<211> 107
<212> PRT
<213> Mus musculus

<400> 947

Gln Ile Val Leu Lys Gln Ser Pro Ala Ile Met Ser Ala Ser Pro Gly
5 10 15

Glu Lys Val Thr Met Thr Cys Ser Ala Ser Ser Ser Val Ser Tyr Met
20 25 30

Tyr Trp Tyr Gln Gln Lys Pro Gly Ser Ser Pro Arg Leu Leu Ile Tyr
35 40 45

Asp Thr Ser Asn Leu Ala Ser Gly Val Pro Val Arg Phe Ser Tyr Ser
50 55 60

Gly Ser Gly Thr Ser Tyr Ser Leu Thr Ile Ser Arg Met Glu Ala Glu
65 70 75 80

Asp Ala Ala Thr Tyr Tyr Cys Gln Gln Trp Ser Ser Tyr Pro Pro Met
85 90 95

Thr Phe Gly Gly Gly Thr Lys Leu Glu Ile Lys
100 105

<210> 948
<211> 10
<212> PRT
<213> Mus musculus

<400> 948

Ser Ala Ser Ser Ser Val Ser Tyr Met Tyr
5 10

<210> 949
<211> 7
<212> PRT
<213> Mus musculus

<400> 949

Asp Thr Ser Asn Leu Ala Ser
5

<210> 950
<211> 10
<212> PRT
<213> Mus musculus

<400> 950

Gln Gln Trp Ser Ser Tyr Pro Pro Met Thr
5 10

<210> 951
<211> 23
<212> PRT
<213> Mus musculus

<400> 951

Gln Ile Val Leu Lys Gln Ser Pro Ala Ile Met Ser Ala Ser Pro Gly
5 10 15

Glu Lys Val Thr Met Thr Cys
20

<210> 952
<211> 15
<212> PRT
<213> Mus musculus

<400> 952

Trp Tyr Gln Gln Lys Pro Gly Ser Ser Pro Arg Leu Leu Ile Tyr
5 10 15

<210> 953
<211> 32
<212> PRT
<213> Mus musculus

<400> 953

Gly Val Pro Val Arg Phe Ser Tyr Ser Gly Ser Gly Thr Ser Tyr Ser
5 10 15

Leu Thr Ile Ser Arg Met Glu Ala Glu Asp Ala Ala Thr Tyr Tyr Cys
20 25 30

<210> 954
<211> 10
<212> PRT
<213> Mus musculus

<400> 954

Phe Gly Gly Gly Thr Lys Leu Glu Ile Lys
5 10

<210> 955
<211> 369
<212> DNA
<213> Mus musculus

<400> 955

caggtccagc ttcagcagtc tggggctgaa ctggcaaaac ccggggcctc agtgaggatg 60

tcctgcaaga cttctggcta cacctttagt acctactgga tgcactgggt aaaacagagg 120

cctggacagg gtctggaatg gattggatac attaatcctc ccactggtta tactgagtat 180

aatcagaagt tcaaggacaa ggccacattg actgcagaca aatcctccag cacagcctac 240

atgcaactga tcagcctgac atctgaagac tctgcagtct attactgtgt gcatgagggg 300

ggtatgatta cgaccgactt tcatgctttg gactactggg gtcaaggaac ctcagtcacc 360

gtctcctca 369

<210> 956
<211> 123
<212> PRT
<213> Mus musculus

<400> 956

Gln Val Gln Leu Gln Gln Ser Gly Ala Glu Leu Ala Lys Pro Gly Ala
5 10 15

Ser Val Arg Met Ser Cys Lys Thr Ser Gly Tyr Thr Phe Ser Thr Tyr
20 25 30

Trp Met His Trp Val Lys Gln Arg Pro Gly Gln Gly Leu Glu Trp Ile
35 40 45

Gly Tyr Ile Asn Pro Pro Thr Gly Tyr Thr Glu Tyr Asn Gln Lys Phe
50 55 60

Lys Asp Lys Ala Thr Leu Thr Ala Asp Lys Ser Ser Ser Thr Ala Tyr
65 70 75 80

Met Gln Leu Ile Ser Leu Thr Ser Glu Asp Ser Ala Val Tyr Tyr Cys
85 90 95

Val His Glu Gly Gly Met Ile Thr Thr Asp Phe His Ala Leu Asp Tyr
100 105 110

Trp Gly Gln Gly Thr Ser Val Thr Val Ser Ser
115 120

<210> 957
<211> 5
<212> PRT
<213> Mus musculus

<400> 957

Thr Tyr Trp Met His
5

<210> 958
<211> 17
<212> PRT
<213> Mus musculus

<400> 958

Tyr Ile Asn Pro Pro Thr Gly Tyr Thr Glu Tyr Asn Gln Lys Phe Lys
5 10 15

Asp

<210> 959
<211> 14
<212> PRT
<213> Mus musculus

<400> 959

Glu Gly Gly Met Ile Thr Thr Asp Phe His Ala Leu Asp Tyr
5 10

<210> 960
<211> 30
<212> PRT

<213> Mus musculus

<400> 960

```
Gln Val Gln Leu Gln Gln Ser Gly Ala Glu Leu Ala Lys Pro Gly Ala
                5                   10                  15

Ser Val Arg Met Ser Cys Lys Thr Ser Gly Tyr Thr Phe Ser
            20                  25                  30
```

<210> 961
<211> 14
<212> PRT
<213> Mus musculus

<400> 961

```
Trp Val Lys Gln Arg Pro Gly Gln Gly Leu Glu Trp Ile Gly
                5                   10
```

<210> 962
<211> 32
<212> PRT
<213> Mus musculus

<400> 962

```
Lys Ala Thr Leu Thr Ala Asp Lys Ser Ser Ser Thr Ala Tyr Met Gln
                5                   10                  15

Leu Ile Ser Leu Thr Ser Glu Asp Ser Ala Val Tyr Tyr Cys Val His
            20                  25                  30
```

<210> 963
<211> 11
<212> PRT
<213> Mus musculus

<400> 963

```
Trp Gly Gln Gly Thr Ser Val Thr Val Ser Ser
                5                   10
```

<210> 964
<211> 336
<212> DNA
<213> Mus musculus

<400> 964

gacattgtga tgtcacagtc tccatcctcc ctggctgtgt cagcaggaga gaaggtcact 60

atgagctgca aatccagtca gagtctgctc aacagtagaa cccgaaagaa ctacttggct 120

tggtaccagc agaaaccagg gcagtctcct aaactgctga tctactgggc atccactagg 180

gaatctgggg tccctgatcg cttcacaggc agtggatctg ggacagattt cactctcacc 240

atcagcagtg tgcaggctga agacctggca gtttattact gcaagcaatc ttataatctg 300

tacacgttcg gaggggggac caagctggaa ataaaa 336

<210> 965
<211> 112
<212> PRT
<213> Mus musculus

<400> 965

Asp Ile Val Met Ser Gln Ser Pro Ser Ser Leu Ala Val Ser Ala Gly
1 5 10 15

Glu Lys Val Thr Met Ser Cys Lys Ser Ser Gln Ser Leu Leu Asn Ser
20 25 30

Arg Thr Arg Lys Asn Tyr Leu Ala Trp Tyr Gln Gln Lys Pro Gly Gln
35 40 45

Ser Pro Lys Leu Leu Ile Tyr Trp Ala Ser Thr Arg Glu Ser Gly Val
50 55 60

Pro Asp Arg Phe Thr Gly Ser Gly Ser Gly Thr Asp Phe Thr Leu Thr
65 70 75 80

Ile Ser Ser Val Gln Ala Glu Asp Leu Ala Val Tyr Tyr Cys Lys Gln
85 90 95

Ser Tyr Asn Leu Tyr Thr Phe Gly Gly Gly Thr Lys Leu Glu Ile Lys
100 105 110

<210> 966
<211> 17
<212> PRT
<213> Mus musculus

<400> 966

Lys Ser Ser Gln Ser Leu Leu Asn Ser Arg Thr Arg Lys Asn Tyr Leu
1 5 10 15

Ala

<210> 967
<211> 7
<212> PRT
<213> Mus musculus

<400> 967

Trp Ala Ser Thr Arg Glu Ser
5

<210> 968
<211> 8
<212> PRT
<213> Mus musculus

<400> 968

Lys Gln Ser Tyr Asn Leu Tyr Thr
5

<210> 969
<211> 23
<212> PRT
<213> Mus musculus

<400> 969

Asp Ile Val Met Ser Gln Ser Pro Ser Ser Leu Ala Val Ser Ala Gly
5 10 15

Glu Lys Val Thr Met Ser Cys
20

<210> 970
<211> 15
<212> PRT
<213> Mus musculus

<400> 970

Trp Tyr Gln Gln Lys Pro Gly Gln Ser Pro Lys Leu Leu Ile Tyr
5 10 15

<210> 971
<211> 32
<212> PRT
<213> Mus musculus

<400> 971

Gly Val Pro Asp Arg Phe Thr Gly Ser Gly Ser Gly Thr Asp Phe Thr
5 10 15

Leu Thr Ile Ser Ser Val Gln Ala Glu Asp Leu Ala Val Tyr Tyr Cys
20 25 30

<210> 972
<211> 10
<212> PRT
<213> Mus musculus

<400> 972

Phe Gly Gly Gly Thr Lys Leu Glu Ile Lys
5 10

<210> 973
<211> 360
<212> DNA
<213> Mus musculus

<400> 973

```
caggtgcagc tgaagcagtc aggacctggc ctagtgcagc cctcacagag cctgtccatc      60
acctgcacag tctctggttt ctcattaact acctatggtg tacactgggt tcgccagtct     120
ccaggaaagg gtctggagtg gctgggagtg atatggagtg gtggatccac agactataat     180
gcagctttca tatccagact gagcatcacc aaggacaatt ccaagagcca agttttcttt     240
aaaatgaaca gtctgcaagc taatgacaca gccatatatt actgtgccag ggatggttac     300
tacgcccttt actatgctat ggactactgg ggtcaaggaa cctcagtcac cgtctcctca     360
```

<210> 974
<211> 120
<212> PRT
<213> Mus musculus

<400> 974

```
Gln Val Gln Leu Lys Gln Ser Gly Pro Gly Leu Val Gln Pro Ser Gln
                5                   10                  15
Ser Leu Ser Ile Thr Cys Thr Val Ser Gly Phe Ser Leu Thr Thr Tyr
            20                  25                  30
Gly Val His Trp Val Arg Gln Ser Pro Gly Lys Gly Leu Glu Trp Leu
        35                  40                  45
Gly Val Ile Trp Ser Gly Gly Ser Thr Asp Tyr Asn Ala Ala Phe Ile
    50                  55                  60
Ser Arg Leu Ser Ile Thr Lys Asp Asn Ser Lys Ser Gln Val Phe Phe
65                  70                  75                  80
Lys Met Asn Ser Leu Gln Ala Asn Asp Thr Ala Ile Tyr Tyr Cys Ala
                85                  90                  95
Arg Asp Gly Tyr Tyr Ala Leu Tyr Tyr Ala Met Asp Tyr Trp Gly Gln
            100                 105                 110
Gly Thr Ser Val Thr Val Ser Ser
        115                 120
```

<210> 975
<211> 5
<212> PRT
<213> Mus musculus

<400> 975

```
Thr Tyr Gly Val His
                5
```

<210> 976
<211> 16
<212> PRT
<213> Mus musculus

<400> 976

```
Val Ile Trp Ser Gly Gly Ser Thr Asp Tyr Asn Ala Ala Phe Ile Ser
                5                   10                  15
```

<210> 977
<211> 12
<212> PRT
<213> Mus musculus

<400> 977

```
Asp Gly Tyr Tyr Ala Leu Tyr Tyr Ala Met Asp Tyr
                5                   10
```

<210> 978
<211> 30
<212> PRT
<213> Mus musculus

<400> 978

```
Gln Val Gln Leu Lys Gln Ser Gly Pro Gly Leu Val Gln Pro Ser Gln
                5                   10                  15

Ser Leu Ser Ile Thr Cys Thr Val Ser Gly Phe Ser Leu Thr
            20                  25                  30
```

<210> 979
<211> 14
<212> PRT
<213> Mus musculus

<400> 979

```
Trp Val Arg Gln Ser Pro Gly Lys Gly Leu Glu Trp Leu Gly
                5                   10
```

<210> 980
<211> 32
<212> PRT
<213> Mus musculus

<400> 980

```
Arg Leu Ser Ile Thr Lys Asp Asn Ser Lys Ser Gln Val Phe Phe Lys
                5                   10                  15

Met Asn Ser Leu Gln Ala Asn Asp Thr Ala Ile Tyr Tyr Cys Ala Arg
            20                  25                  30
```

<210> 981
<211> 11

<212> PRT
<213> Mus musculus

<400> 981

Trp Gly Gln Gly Thr Ser Val Thr Val Ser Ser
5 10

<210> 982
<211> 321
<212> DNA
<213> Mus musculus

<400> 982

gacattgtga tgacccagtc tcacaaattc atgtccacat cagtaggaga cagggtcagc 60

atcacctgca aggccagtca ggatgtgagt actgctgtag cctggtatca acagaaacca 120

ggacaatctc ctaaactact gatttactcg gcatcctacc ggtacactgg agtccctgat 180

cgcttcactg gcagtggatc tgggacggat ttcactttca ccatcagcag tgtgcaggct 240

gaagacctgg cagtttatta ctgtcagcaa cattatagta ctccgtggac gttcggtgga 300

ggcaccaagc tggaaatcaa a 321

<210> 983
<211> 107
<212> PRT
<213> Mus musculus

<400> 983

Asp Ile Val Met Thr Gln Ser His Lys Phe Met Ser Thr Ser Val Gly
5 10 15

Asp Arg Val Ser Ile Thr Cys Lys Ala Ser Gln Asp Val Ser Thr Ala
20 25 30

Val Ala Trp Tyr Gln Gln Lys Pro Gly Gln Ser Pro Lys Leu Leu Ile
35 40 45

Tyr Ser Ala Ser Tyr Arg Tyr Thr Gly Val Pro Asp Arg Phe Thr Gly
50 55 60

Ser Gly Ser Gly Thr Asp Phe Thr Phe Thr Ile Ser Ser Val Gln Ala
65 70 75 80

Glu Asp Leu Ala Val Tyr Tyr Cys Gln Gln His Tyr Ser Thr Pro Trp
85 90 95

Thr Phe Gly Gly Gly Thr Lys Leu Glu Ile Lys
100 105

<210> 984
<211> 11
<212> PRT
<213> Mus musculus

<400> 984

Lys Ala Ser Gln Asp Val Ser Thr Ala Val Ala
5 10

<210> 985
<211> 7
<212> PRT
<213> Mus musculus

<400> 985

Ser Ala Ser Tyr Arg Tyr Thr
5

<210> 986
<211> 9
<212> PRT
<213> Mus musculus

<400> 986

Gln Gln His Tyr Ser Thr Pro Trp Thr
5

<210> 987
<211> 23
<212> PRT
<213> Mus musculus

<400> 987

Asp Ile Val Met Thr Gln Ser His Lys Phe Met Ser Thr Ser Val Gly
5 10 15

Asp Arg Val Ser Ile Thr Cys
20

<210> 988
<211> 15
<212> PRT
<213> Mus musculus

<400> 988

Trp Tyr Gln Gln Lys Pro Gly Gln Ser Pro Lys Leu Leu Ile Tyr
5 10 15

<210> 989
<211> 32
<212> PRT
<213> Mus musculus

<400> 989

Gly Val Pro Asp Arg Phe Thr Gly Ser Gly Ser Gly Thr Asp Phe Thr
5 10 15

Phe Thr Ile Ser Ser Val Gln Ala Glu Asp Leu Ala Val Tyr Tyr Cys
20 25 30

<210> 990
<211> 10
<212> PRT
<213> Mus musculus

<400> 990

Phe Gly Gly Gly Thr Lys Leu Glu Ile Lys
5 10

<210> 991
<211> 354
<212> DNA
<213> Rattus rattus

<400> 991

caggtgcagc tgaaggagtc aggacctggc ctggtgaagc cctcagagac cctgtctctc 60

acctgcactg tgtctgggtt ctcattaacc aactatccta taagctgggt tcgacagcct 120

ccaggaaagg gtctggagtg gatgggagta atatggggtg atggaagcac atcatataat 180

ttagctctca aatcccgact gagcatcagc agggacacct cgaagagcca agttttatta 240

aaaatgaaca gtctggaaac tgaagacaca gccacttact actgtgccag agtaggagta 300

tactacggat tattaggtta ctggggccaa ggagtcatgg tcaccgtctc ctca 354

<210> 992
<211> 118
<212> PRT
<213> Rattus rattus

<400> 992

Gln Val Gln Leu Lys Glu Ser Gly Pro Gly Leu Val Lys Pro Ser Glu
5 10 15

Thr Leu Ser Leu Thr Cys Thr Val Ser Gly Phe Ser Leu Thr Asn Tyr
20 25 30

Pro Ile Ser Trp Val Arg Gln Pro Pro Gly Lys Gly Leu Glu Trp Met
35 40 45

Gly Val Ile Trp Gly Asp Gly Ser Thr Ser Tyr Asn Leu Ala Leu Lys
50 55 60

Ser Arg Leu Ser Ile Ser Arg Asp Thr Ser Lys Ser Gln Val Leu Leu
65 70 75 80

Lys Met Asn Ser Leu Glu Thr Glu Asp Thr Ala Thr Tyr Tyr Cys Ala
85 90 95

Arg Val Gly Val Tyr Tyr Gly Leu Leu Gly Tyr Trp Gly Gln Gly Val
100 105 110

Met Val Thr Val Ser Ser
115

<210> 993
<211> 5
<212> PRT
<213> Rattus rattus

<400> 993

Asn Tyr Pro Ile Ser
5

<210> 994
<211> 16
<212> PRT
<213> Rattus rattus

<400> 994

Val Ile Trp Gly Asp Gly Ser Thr Ser Tyr Asn Leu Ala Leu Lys Ser
5 10 15

<210> 995
<211> 10
<212> PRT
<213> Rattus rattus

<400> 995

Val Gly Val Tyr Tyr Gly Leu Leu Gly Tyr
5 10

<210> 996
<211> 30
<212> PRT
<213> Rattus rattus

<400> 996

Gln Val Gln Leu Lys Glu Ser Gly Pro Gly Leu Val Lys Pro Ser Glu
1               5                   10                  15

Thr Leu Ser Leu Thr Cys Thr Val Ser Gly Phe Ser Leu Thr
            20                  25                  30

<210> 997
<211> 14
<212> PRT
<213> Rattus rattus

<400> 997

Trp Val Arg Gln Pro Pro Gly Lys Gly Leu Glu Trp Met Gly
1               5                   10

<210> 998
<211> 32
<212> PRT
<213> Rattus rattus

<400> 998

Arg Leu Ser Ile Ser Arg Asp Thr Ser Lys Ser Gln Val Leu Leu Lys
1               5                   10                  15

Met Asn Ser Leu Glu Thr Glu Asp Thr Ala Thr Tyr Tyr Cys Ala Arg
            20                  25                  30

<210> 999
<211> 11
<212> PRT
<213> Rattus rattus

<400> 999

Trp Gly Gln Gly Val Met Val Thr Val Ser Ser
1               5                   10

<210> 1000
<211> 321
<212> DNA
<213> Rattus rattus

<400> 1000

```
aacactgtga tgactcagtc tcccacatcc atgttcagat cagtaggaga cagggtcacc      60
atgaactgca aggccagtca gaatgtgggt actaatgtag actggtacca acagaaaaca     120
gggcagtctc ctaaactgct tatctatggg gcatccaacc ggtacactgg agtccctgat     180
cgcttcacag gcagtggatc tggaacagat ttcactctca ccatcagcaa catgcaggct     240
gaagacttgg ctgtttatta ctgtttacag tataactaca atccgtacac gtttggagct     300
gggaccaagc tggaactgaa a                                               321
```

<210> 1001
<211> 107
<212> PRT
<213> Rattus rattus

<400> 1001

```
Asn Thr Val Met Thr Gln Ser Pro Thr Ser Met Phe Arg Ser Val Gly
                5                   10                  15

Asp Arg Val Thr Met Asn Cys Lys Ala Ser Gln Asn Val Gly Thr Asn
            20                  25                  30

Val Asp Trp Tyr Gln Gln Lys Thr Gly Gln Ser Pro Lys Leu Leu Ile
        35                  40                  45

Tyr Gly Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ala
65                  70                  75                  80

Glu Asp Leu Ala Val Tyr Tyr Cys Leu Gln Tyr Asn Tyr Asn Pro Tyr
                85                  90                  95

Thr Phe Gly Ala Gly Thr Lys Leu Glu Leu Lys
            100                 105
```

<210> 1002
<211> 11
<212> PRT
<213> Rattus rattus

<400> 1002

```
Lys Ala Ser Gln Asn Val Gly Thr Asn Val Asp
                5                   10
```

<210> 1003
<211> 7
<212> PRT
<213> Rattus rattus

<400> 1003

```
Gly Ala Ser Asn Arg Tyr Thr
                5
```

<210> 1004
<211> 9
<212> PRT
<213> Rattus rattus

<400> 1004

```
Leu Gln Tyr Asn Tyr Asn Pro Tyr Thr
                5
```

<210> 1005
<211> 23
<212> PRT
<213> Rattus rattus

<400> 1005

```
Asn Thr Val Met Thr Gln Ser Pro Thr Ser Met Phe Arg Ser Val Gly
                5                   10                  15

Asp Arg Val Thr Met Asn Cys
            20
```

<210> 1006
<211> 15
<212> PRT
<213> Rattus rattus

<400> 16

```
Trp Tyr Gln Gln Lys Thr Gly Gln Ser Pro Lys Leu Leu Ile Tyr
                5                   10                  15
```

<210> 1007
<211> 32
<212> PRT
<213> Rattus rattus

<400> 1007

```
Gly Val Pro Asp Arg Phe Thr Gly Ser Gly Ser Gly Thr Asp Phe Thr
                5                   10                  15

Leu Thr Ile Ser Asn Met Gln Ala Glu Asp Leu Ala Val Tyr Tyr Cys
            20                  25                  30
```

<210> 1008
<211> 10
<212> PRT
<213> Rattus rattus

<400> 1008

```
Phe Gly Ala Gly Thr Lys Leu Glu Leu Lys
                5                   10
```

## Description

### BACKGROUND OF THE INVENTION

Chronic pain serves no beneficial purpose, but arises from pathological alterations in nociceptive neural networks. Neuropathic pain is a form of chronic pain that arises after nerve injury caused by trauma, infection, or pathology. Neuropathic pain persists long after the initiating event has healed. While neurons are involved in neuropathic pain, they are unlikely to be the sole cell type mediating this condition. There is a growing body of evidence that supports a role for glia-neuron interactions in establishing and maintaining neuropathic pain. Microglia, in particular, have emerged as key players in neuropathic pain. The microglial P2X4 receptor appears to be important in the development and maintenance of neuropathic pain.

The ion channel P2X4 is one of seven members of a family of purinergic, cation permeable channels. Each P2X4 subunit has two transmembrane domains, separated by a large ~280 amino acid extracellular domain. Functional channels are formed of a trimeric arrangement of subunits with a central pore. The P2X4 channel is activated by binding of the ligand adenosine 5'-triphosphate (ATP) to residues contained within its extracellular domain. Activation of these receptors instigates a series of conformational changes that allow cations, such as $Ca^{2+}$ and $Na^{+}$, entry into the cell through a cation selective channel. P2X4 activation and upregulation is thought to be a key driver of neuropathic pain. Downstream of P2X4 activation, microglia release brain derived neurotrophic factor (BDNF), which acts on spinal lamina I neurons to reduce expression of a neuronal chloride transporter KCC2, thereby altering the electrochemical gradient for chloride and rendering one of the main inhibitory neurotransmitters GABA excitatory. Therefore, P2X4-mediated BDNF release in spinal cord is thought to be a key driver of neuropathic pain.

Neuropathic pain fails to respond to currently available analgesics, and is considered to be one of the most debilitating chronic pain conditions. Accordingly, improved methods for treating neuropathic pain, particularly pain mediated by P2X4 are urgently required.

Young et al (2008 JBC 283:26241-51) relates to the molecular shape, architecture, and size of P2X4 receptors determined using fluorescence resonance energy transfer and electron microscopy. WO0248395 relates to P2Y receptor expression for identifying preneoplastic and neoplastic states. US2005074819 relates to a screening method for a compound useful for treatment of neuropathic pain. US20080287467 relates to a therapeutic agent for respiratory diseases, which comprises a compound antagonistic to P2X receptor. Gum et al (2012 Purinergic Signal 8:41-56 discusses P2X receptor antagonists for pain management. Valente et al (2011 Biochim Biophys Acta 1808:2859-66) relates to the expression, purification, electron microscopy, N-glycosylation mutagenesis and molecular modeling of human P2X4 andP2XA.

### SUMMARY OF THE INVENTION

As described below, the present disclosure provides antibodies that specifically bind a P2X4 polypeptide and modulate P2X4 channel activity, recombinant P2X4 polypeptides and methods for generating such polypeptides, as well as compositions and methods for generating anti-P2X4 antibodies, and methods of using P2X4 antibodies for the treatment of neuropathic pain and other indications.

In a first aspect, the invention provides an antibody or antigen binding fragment thereof that specifically binds a human P2X4 polypeptide and modulates channel activity, wherein the antibody or fragment thereof comprises a VH comprising: a heavy chain variable region CDR1 comprising the sequence: SFAMS; a heavy chain variable region CDR2 comprising the sequence: AISGSGGSTYYADSVKG; a heavy chain variable region CDR3 comprising the sequence: QFDYWSTYSGPTAFDL; in combination with a VL comprising: a light chain variable region CDR1 comprising the sequence SGDALPRQYAY; a light chain variable region CDR2 comprising the sequence KDSERPS; a light chain variable region CDR3 comprising the sequence QSADSSGTYVV

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

By "P2X purinoceptor 4 (P2RX4 or P2X4) polypeptide" is meant a purinergic receptor protein or fragment thereof having at least about 85% or greater amino acid identity to the amino acid sequence provided at NCBI Accession No. Q99571 and having P2X4 biological activity. P2X4 biological activity includes $Ca^{2+}/Na^{+}$ conducting activity in response to ATP binding and/or P2X4 antibody binding. An exemplary human P2X4 sequence is provided below:

```
magccaalaa flfeydtpri vlirsrkvgl mnravqllil ayvigwvfvw ekgyqetdsv 61
vssvttkvkg vavtntsklg friwdvadyv ipaqeenslf vmtnviltmn qtqglcpeip 121
dattvcksda sctagsagth sngvstgrcv afngsvktce vaawcpvedd thvpqpaflk 181
aaenftllvk nniwypkfnf skrnilpnit ttylksciyd aktdpfcpif rlgkivenag 241
hsfqdmaveg gimgiqvnwd cnldraaslc lprysfrrld trdvehnvsp gynfrfakyy 301
rdlagneqrt likaygirfd iivfgkagkf diiptminig sglallgmat vlcdiivlyc 361
mkkrlyyrek kykyvedyeq glaseldq
```

In embodiments of the invention, a human P2X4 polypeptide has at least about 65%, 70%, 80%, 85%, 90%, 95%, or even 100% identity to NCBI Accession No. Q99571. In other embodiments, the invention provides P2X4 polypeptides comprising one or more amino acid substitutions relative to the Q99571 reference sequence, including for example: E95Q, V105M, G114D, A122V, S131N, A151P, G154R, L303P, and N306K.

An exemplary murine P2X purinoceptor 4 is provided at NCBI Accession No. Q9JJX6, which has the following sequence:

```
magccsvlgs flfeydtpri vlirsrkvgl mnrvvqllil ayvigwvfvw ekgyqetdsv 61
vssvttkakg vavtntsqlg friwdvadyv vpaqeenslf imtnmivtvn qtqgtcpeip 121
dktsicdsda nctlgssdth ssgigtgrcv pfnasvktce vaawcpvend agvptpaflk 181
aaenftllvk nniwypkfnf skrnilpnit tsylksciyn artdpfcpif rlgqivadag 241
hsfqemaveg gimgiqikwd cnldraashc lprysfrrld trdlehnvsp gynfrfakyy 301
rdlagneqrt ltkaygirfd iivfgkagkf diiptminvg sglallgvat vlcdvivlyc 361
mkkryyyrdk kykyvedyeq glsgemnq
```

An exemplary rat P2X purinoceptor 4 sequence is provided at NCBI Accession No. P51577, which has the following sequence:

```
magccsvlgs flfeydtpri vlirsrkvgl mnravqllil ayvigwvfvw ekgyqetdsv 61
vssvttkakg vavtntsqlg friwdvadyv ipaqeenslf imtnmivtvn qtqstcpeip 121
dktsicnsda dctpgsvdth ssgvatgrcv pfnesvktce vaawcpvend vgvptpaflk 181
aaenftllvk nniwypkfnf skrnilpnit tsylksciyn aqtdpfcpif rlgtivedag 241
hsfqemaveg gimgiqikwd cnldraaslc lprysfrrld trdlehnvsp gynfrfakyy 301
rdlagkeqrt ltkaygirfd iivfgkagkf diiptminvg sglallgvat vlcdvivlyc 361
mkkkyyyrdk kykyvedyeq glsgemnq
```

An exemplary cynomologus monkey (e.g. macaque) P2X purinoceptor 4 sequence, which has the following sequence:

```
magccaalaa flfeydtpri vlirsrkvgl mnravqllil ayvigwvfvw ekgyqetdsv 61
vssvttkvkg vavtntsklg friwdvadyv ipaqqenslf vmtnmiltmn qtqdlcpeip 121
dvttvcksda nctagsagth sngvstgrcv pfnrsvktce vaawcpvedd thvpqpaflk 181
aaenftllvk nniwypkfnf skrnilpnit ttylksciyd aktdpfcpif rlgkivenag 241
hsfqdmaveg gimgiqvnwd cnldraaslc lprysfrrld trdvehnvsp gynfrfakyy 301
rdpagkeqrt likaygirfd iivfgkagkf diiptminig sglallgmat vlcdiivlyc 361
mkkrlyyrek kykyvedyeq glaseldp
```

By "P2X4 nucleic acid molecule" is meant a polynucleotide encoding a P2X4 polypeptide or fragment thereof. An exemplary human P2X4 polynucleotide sequence is provided at NCBI Accession No. NM_002560, the sequence of which follows:

```
   1 aagtgctggg atgacaggtg tgagccaccg cccccggccc ctcgcccgcc ttttgaagga
  61 gcctttcgtc ctcaagggcg aggccactcc ccccccgcga gttccatgcc ccctagaggg
 121 tcatcgttcc cgacggggag gtggcgccct cccccgggcc ccgggccccg accgcccgtg
 181 ctgcctcctt ccgggccctc ctccgcgatg acggcgccgc cagcaggcca ggcggactgg
 241 gcggggctcc gagcggggac tgggacccag accgactagg ggactgggag cgggcggcgc
 301 ggccatggcg ggctgctgcg ccgcgctggc ggccttcctg ttcgagtacg acacgccgcg
 361 catcgtgctc atccgcagcc gcaaagtggg gctcatgaac cgcgccgtgc aactgctcat
 421 cctggcctac gtcatcgggt gggtgtttgt gtgggaaaag ggctaccagg aaactgactc
 481 cgtggtcagc tccgttacga ccaaggtcaa gggcgtggct gtgaccaaca cttctaaact
 541 tggattccgg atctgggatg tggcggatta tgtgatacca gctcaggagg aaaactccct
 601 cttcgtcatg accaacgtga tcctcaccat gaaccagaca cagggcctgt gccccgagat
 661 tccagatgcg accactgtgt gtaaatcaga tgccagctgt actgccggct ctgccggcac
 721 ccacagcaac ggagtctcaa caggcaggtg cgtagctttc aacgggtctg tcaagacgtg
 781 tgaggtggcg gcctggtgcc cggtggagga tgacacacac gtgccacaac ctgctttttt
 841 aaaggctgca gaaaacttca ctcttttggt taagaacaac atctggtatc ccaaatttaa
 901 tttcagcaag aggaatatcc ttcccaacat caccactact tacctcaagt cgtgcattta
 961 tgatgctaaa acagatccct tctgccccat attccgtctt ggcaaaatag tggagaacgc
1021 aggacacagt ttccaggaca tggccgtgga gggaggcatc atgggcatcc aggtcaactg
1081 ggactgcaac ctggacagag ccgcctccct ctgcttgccc aggtactcct tccgccgcct
1141 cgatacacgg gacgttgagc acaacgtatc tcctggctac aatttcaggt ttgccaagta
1201 ctacagagac ctggctggca acgagcagcg cacgctcatc aaggcctatg gcatccgctt
1261 cgacatcatt gtgtttggga aggcagggaa atttgacatc atccccacta tgatcaacat
1321 cggctctggc ctggcactgc taggcatggc gaccgtgctg tgtgacatca tagtcctcta
1381 ctgcatgaag aaaagactct actatcggga gaagaaatat aaatatgtgg aagattacga
1441 gcagggtctt gctagtgagc tggaccagtg aggcctaccc cacacctggg ctctccacag
1501 ccccatcaaa gaacagagag gaggaggagg gagaaatggc caccacatca ccccagagaa
1561 atttctggaa tctgattgag tctccactcc acaagcactc agggttcccc agcagctcct

1621 gtgtgttgtg tgcaggatct gtttgcccac tcggcccagg aggtcagcag tctgttcttg
1681 gctgggtcaa ctctgctttt cccgcaacct ggggttgtcg gggagcgct ggcccgacgc
1741 agtggcactg ctgtggcttt cagggctgga gctggctttg ctcagaagcc tcctgtctcc
1801 agctctctcc aggacaggcc cagtcctctg aggcacggcg gctctgttca agcactttat
1861 gcggcagggg aggccgcctg gctgcagtca ctagacttgt agcaggcctg ggctgcaggc
1921 ttccccccga ccattccctg cagccatgcg gcagagctgg catttctcct cagagaagcg
1981 ctgtgctaag gtgatcgagg accagacatt aaagcgtgat tttcttaaaa aaaaaaaaaa
2041 aaa
```

By "P2X4 biological activity" is meant ion channel conducting activity or ion channel mediated changes in cytosolic calcium levels.

By "ameliorate" is meant decrease, suppress, attenuate, diminish, arrest, or stabilize the development or progression of a disease.

The term "antibody," as used in this disclosure, refers to an immunoglobulin or a fragment or a derivative thereof, and encompasses any polypeptide comprising an antigen-binding site, regardless of whether it is produced *in vitro* or *in vivo.* The term includes, but is not limited to, polyclonal, monoclonal, monospecific, polyspecific, non-specific, humanized, single-chain, chimeric, synthetic, recombinant, hybrid, mutated, and grafted antibodies. Unless otherwise modified by the term "intact," as in "intact antibodies," for the purposes of this disclosure, the term "antibody" also includes antibody fragments such as Fab, F(ab')2, Fv, scFv, Fd, dAb, and other antibody fragments that retain antigen-binding function, i.e., the ability to bind a P2X4 polypeptide specifically. Typically, such fragments would comprise an antigen-binding domain.

The terms "antigen-binding domain," "antigen-binding fragment," and "binding fragment" refer to a part of an antibody molecule that comprises amino acids responsible for the specific binding between the antibody and the antigen. In instances, where an antigen is large, the antigen-binding domain may only bind to a part of the antigen. A portion of the antigen molecule that is responsible for specific interactions with the antigen-binding domain is referred to as "epitope" or "antigenic determinant." In particular embodiments, an antigen-binding domain comprises an antibody light chain variable region ($V_L$) and an antibody heavy chain variable region ($V_H$), however, it does not necessarily have to comprise both. For example, a so-called Fd antibody fragment consists only of a $V_H$ domain, but still retains some antigen-binding function of the intact antibody.

Binding fragments of an antibody are produced by recombinant DNA techniques, or by enzymatic or chemical cleavage of intact antibodies. Binding fragments include Fab, Fab', F(ab')2, Fv, and single-chain antibodies. An antibody other than a "bispecific" or "bifunctional" antibody is understood to have each of its binding sites identical. Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a F(ab')2 fragment in which the two arms of the antibody molecule remain linked and comprise two-antigen binding sites. The F(ab')2 fragment has the ability to crosslink antigen. "Fv" when used herein refers to the minimum fragment of an antibody that retains both antigen-recognition and antigen-binding sites. "Fab" when used herein refers to a fragment of an antibody that comprises the constant domain of the light chain and the CHI domain of the heavy chain.

The term "mAb" refers to monoclonal antibody. Antibodies of the invention comprise without limitation whole native antibodies, bispecific antibodies; chimeric antibodies; Fab, Fab', single chain V region fragments (scFv), fusion polypeptides, and unconventional antibodies.

In this disclosure, "comprises," "comprising," "containing" and "having" and the like can have the meaning ascribed to them in U.S. Patent law and can mean " includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

"Detect" refers to identifying the presence, absence or amount of the analyte to be detected. In one embodiment, an antibody of the invention or fragment thereof is used to detect the presence or level of a P2X4 polypeptide.

By "detectable label" is meant a composition that when linked to a molecule of interest renders the latter detectable, via spectroscopic, photochemical, biochemical, immunochemical, or chemical means. For example, useful labels include radioactive isotopes, magnetic beads, metallic beads, colloidal particles, fluorescent dyes, electron-dense reagents, enzymes (for example, as commonly used in an ELISA), biotin, digoxigenin, or haptens.

By "disease" is meant any condition or disorder that damages or interferes with the normal function of a cell, tissue, or organ. Examples of diseases include neuropathic pain, particularly pain associated with P2X4 channel activity or the activity of a pathway responsive to P2X4.

The term "effective amount" refers to a dosage or amount of an agent that is sufficient to reduce the activity of a P2X4 polypeptide to result in amelioration of symptoms in a patient or to achieve a desired biological outcome. Desired biological outcomes include, for example, the amelioration of chronic pain or a symptom thereof, modulation of P2X4 biological activity, or the modulation of a pathway responsive to P2X4 activity.

The term "isolated" refers to a molecule that is substantially free of other elements present in its natural environment. For instance, an isolated protein is substantially free of cellular material or other proteins from the cell or tissue source from which it is derived. The term "isolated" also refers to preparations where the isolated protein is sufficiently pure to be administered as a pharmaceutical composition, or at least 70-80% (w/w) pure, more preferably, at least 80-90% (w/w) pure, even more preferably, 90-95% pure; and, most preferably, at least 95%, 96%, 97%, 98%, 99%, or 100% (w/w) pure.

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. In a particular embodiment, a fragment of a P2X4 polypeptide may contain 5, 10, 20, 30, 40, 50, 60, 70, 80, 90, 100, 200, or 300 amino acids.

By "reference" is meant a standard of comparison.

A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or therebetween.

The term "repertoire" refers to a genetically diverse collection of nucleotides derived wholly or partially from sequences that encode expressed immunoglobulins. The sequences are generated by *in vivo* rearrangement of, e.g., V, D, and J segments for H chains and, e.g., V and J segment for L chains. Alternatively, the sequences may be generated from a cell line by *in vitro* stimulation, in response to which the rearrangement occurs. Alternatively, part or all of the sequences may be obtained by combining, e.g., unrearranged V segments with D and J segments, by nucleotide synthesis, randomised mutagenesis, and other methods, e.g., as disclosed in U.S. Pat. No. 5,565,332.

By "specifically binds" is meant an agent (*e.g.,* antibody) that recognizes and binds a molecule (*e.g.,* polypeptide), but which does not substantially recognize and bind other molecules in a sample, for example, a biological sample. For example, two molecules that specifically bind form a complex that is relatively stable under physiologic conditions. Specific binding is characterized by a high affinity and a low to moderate capacity as distinguished from nonspecific binding which usually has a low affinity with a moderate to high capacity. Typically, binding is considered specific when the affinity constant $K_A$ is higher than $10^7$ $M^{-1}$, or more preferably higher than $10^8$ $M^{-1}$.

The strength of the binding between P2X4 and an antibody can be measured using, for example, an enzyme-linked immunoadsorption assay (ELISA), radio-immunoassay (RIA), or surface plasmon resonance-based technology (e.g., Biacore), all of which are techniques well known in the art. If necessary, non-specific binding can be reduced without substantially affecting specific binding by varying the binding conditions. The appropriate binding conditions such as concentration of antibodies, ionic strength of the solution, temperature, time allowed for binding, concentration of a blocking agent (e.g., serum albumin, milk casein), etc., may be optimized by a skilled artisan using routine techniques.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

## BRIEF DESCRIPTION OF THE DRAWINGS

Figure 1A-1D shows an analysis of Antibody Nos. 1, 11, 29, and 33 binding to human P2X4 variants.

Figure 2 provides the VH & VL sequences of Antibody Nos. 1-34, which were identified using phage display technology.

Figure 3 provides a summary of P2X4 orthologue binding properties of antibodies identified using phage display selection (antibodies 1 to 34) and a summary of their functional effects measured by electrophysiology assays and reported as fraction of control current. Key: + indicates that binding was observed in FMAT assay;

- indicates that no binding was observed in FMAT assay; NT indicates that the antibody was not tested in the assay.

Figure 4 shows results of the antibodies 5, 8, 11, 18, 29, and 33 screened in electrophysiology assays. Each of these antibodies was identified as a P2X4 antagonist. Peak agonist induced inward current in response to 3 $\mu$M ATP in the presence of antibody is indicated as a fraction of control current in the absence of antibody at human or cynomolgus (cyno) P2X4.

Figure 5 provides a summary of P2X4 orthologue binding properties of antibodies isolated from hybridomas (Antibody Nos. 35-48) and a summary of their functional effects measured by electrophysiology assays and reported as fraction of control current.

Figure 6 provides the VH & VL sequences of Antibody Nos. 35-48.

Figure 7 provides a summary of results of binding assays for 252 antibodies (Antibody Nos. 35-286) isolated using hybridoma technology and a summary of their functional effects measured by electrophysiology assays and reported as fraction of control current.

Figure 8A-8F shows whole cell current traces obtained using QPatch 16X showing the agonist response (indicated temporally by the grey bar) before (black trace) and after (grey trace) addition of antibodies. Traces are superimposed for comparison. Dotted line indicates zero current. P2X4 species investigated is indicated where appropriate by (m, mouse) (hu, human).

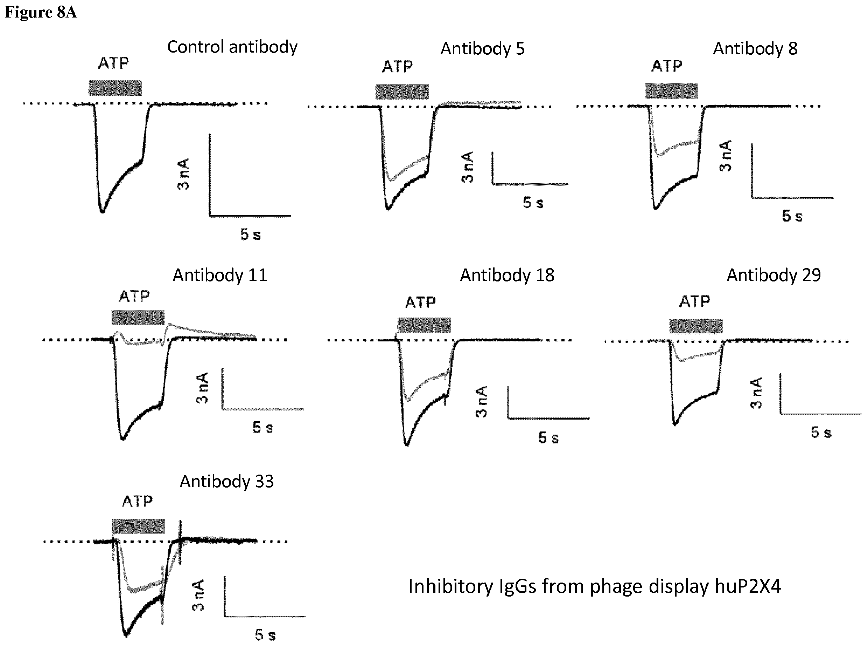

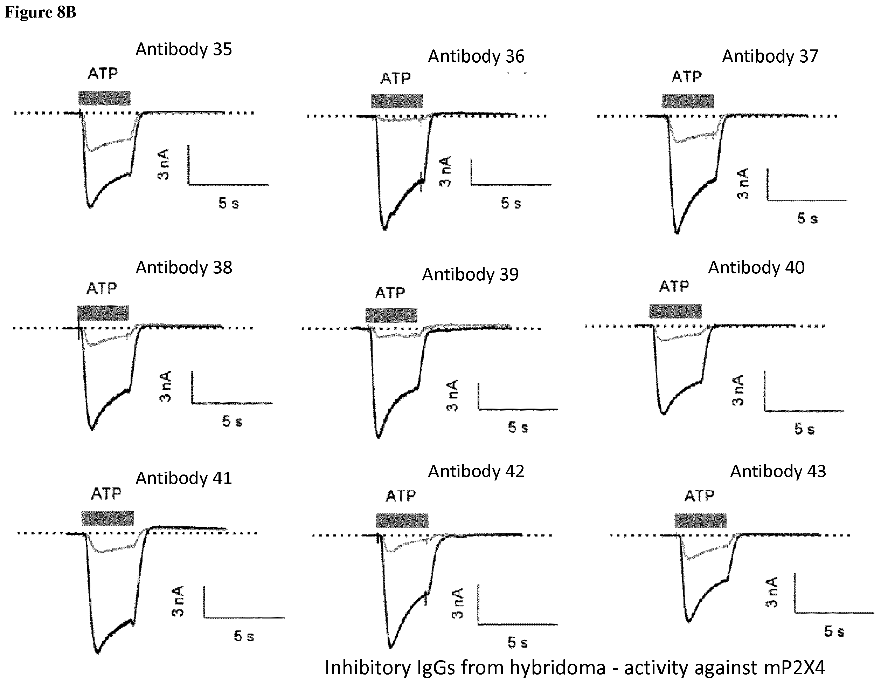

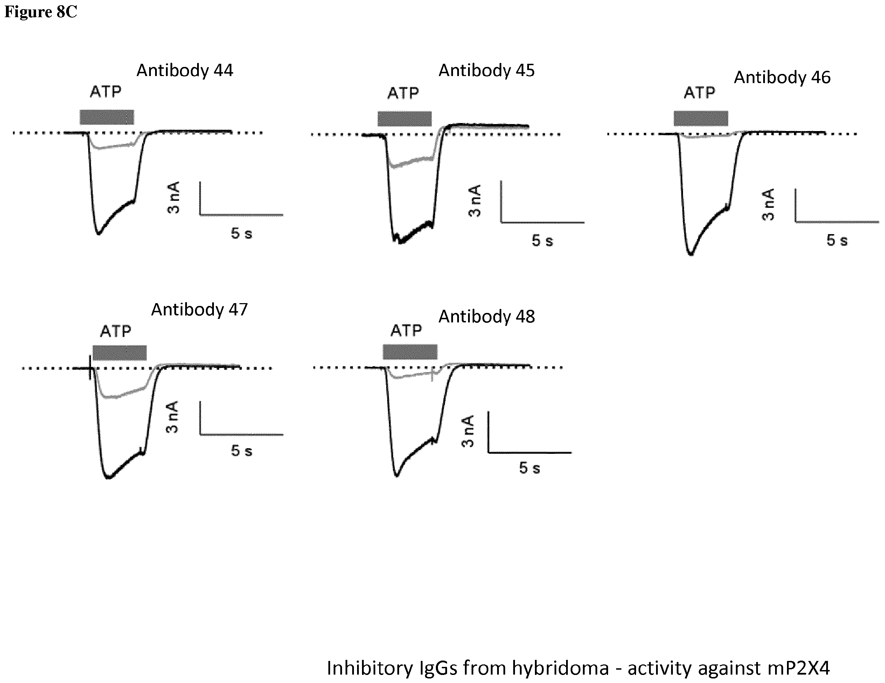

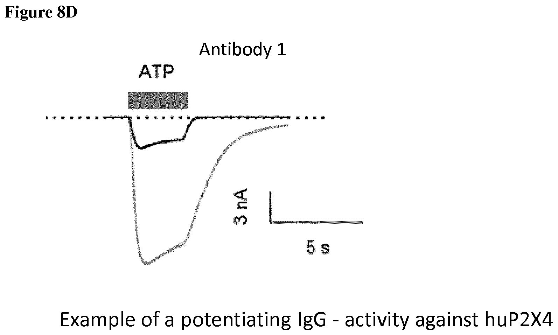

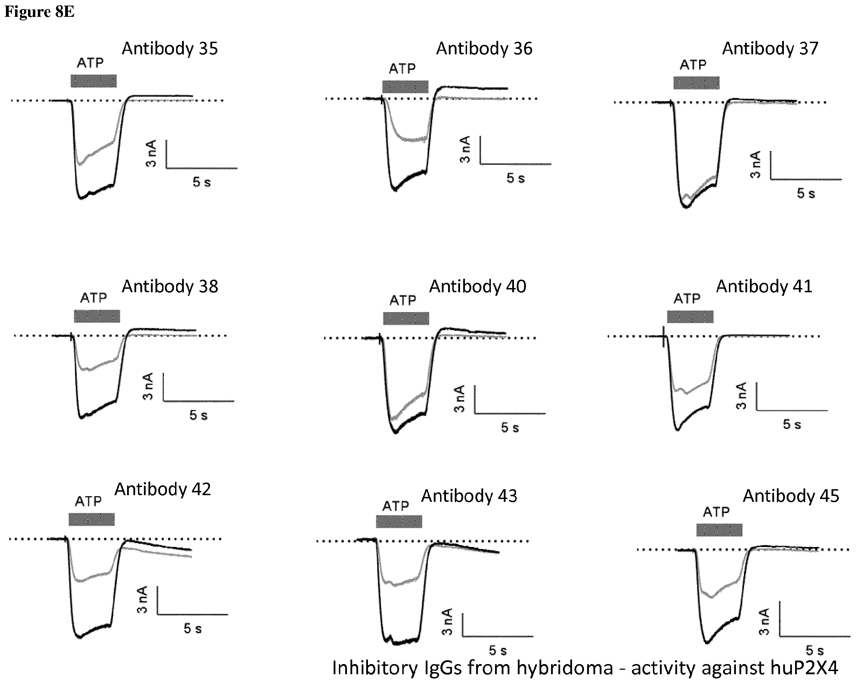

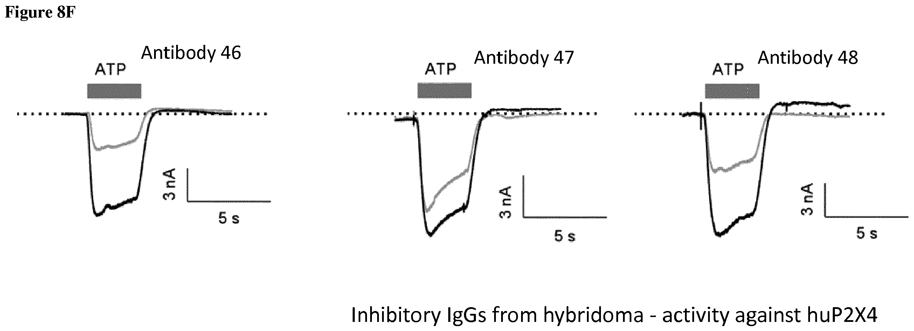

Figure 8A, 8D, 8E, 8F show activity against huP2X4. Figures 8B and 8C show activity against mP2X4.

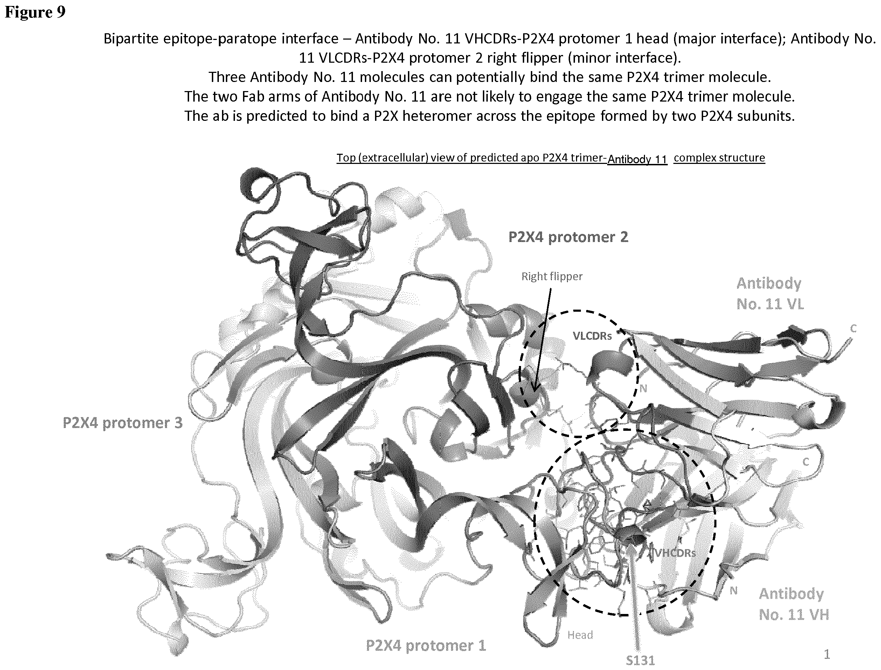

Figure 9 shows schematically an extracellular view of predicted P2X4 trimer-Antibody No. 11 Fv complex structure. In particular, Figure 9 illustrates the bipartite epitope-paratope interface - Antibody No. 11 VHCDRs-P2X4 protomer 1 head (major interface - large dotted circle); Antibody No. 11 VLCDRs-P2X4 protomer 2 right flipper (minor interface - small dotted circle). Three Antibody No. 11 molecules can potentially bind the same P2X4 trimer molecule. The two Fab arms of Antibody No. 11 are not likely to engage the same P2X4 trimer molecule. Antibody No. 11 is predicted to bind a P2X heteromer across the epitope formed by two P2X4 subunits.

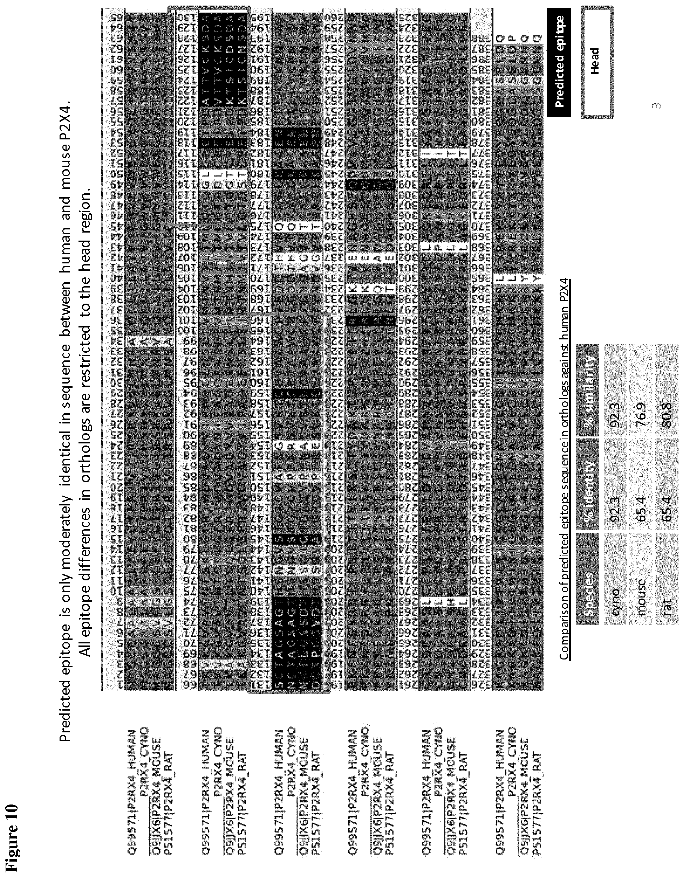

Figure 10 provides alignments of human, cyno, mouse and rat P2X4 sequences. The head region of the protein is indicated. Amino acids within the predicted epitope are also indicated.

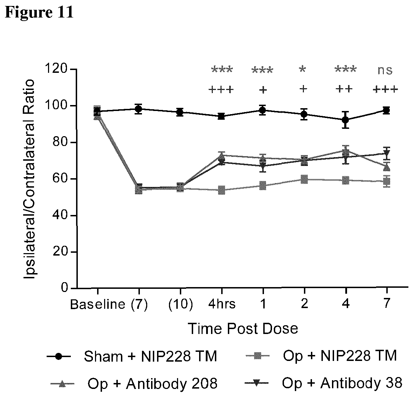

Figure 11 shows the effect of P2X4 antibody Nos. 38 and 208 and an isotype control antibody (NIP228 TM) dosed intra-thecally (5 μg per mouse) on reversal of peripheral nerve ligation (PNL)-induced mechanical hyperalgesia as measured by the ipsilateral/contralateral ratio of paw withdrawal threshold in response to paw pressure ($n$ = 9-10 per group). Data analysed using 2 way ANOVA with time and treatment as dependant factors. Subsequent statistical significance obtained using Tukey's Post Hoc test. * P<0.05; *** P<0.001 - Op + NIP228 TM vs Op + Antibody 208: + P<0.05; ++ P<0.01; +++ P<0.001 - Op + NIP228 TM vs Op + Antibody 38

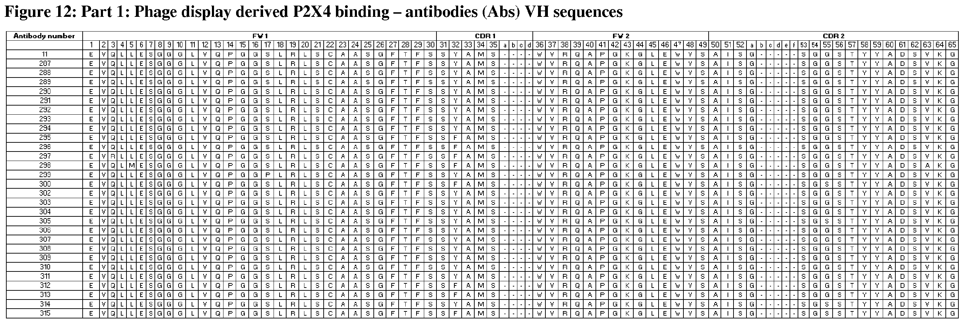

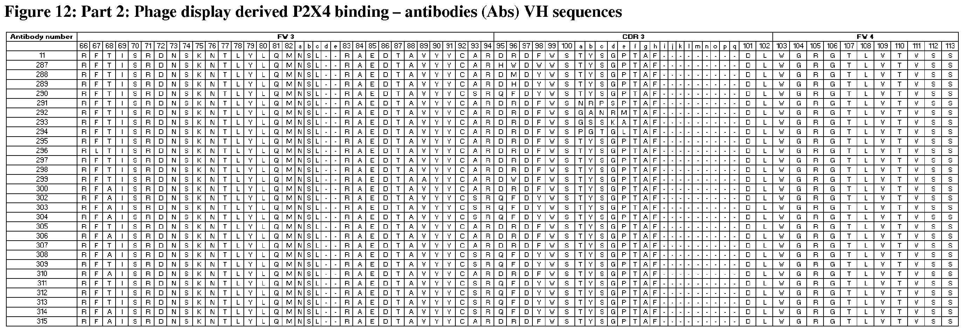

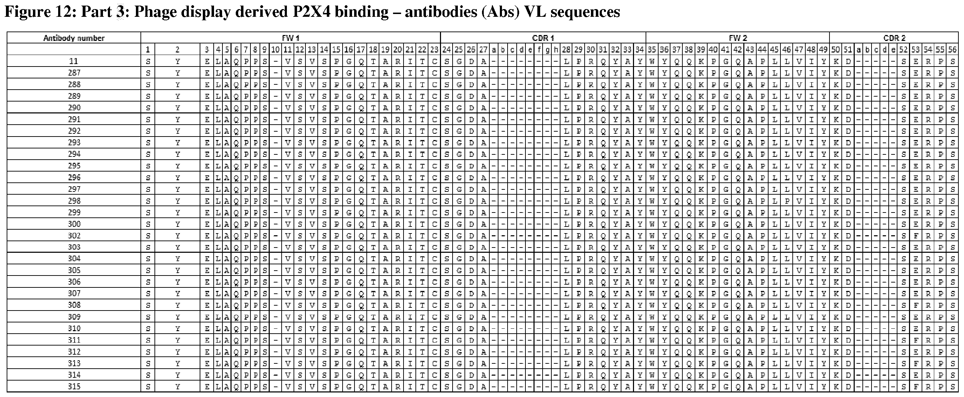

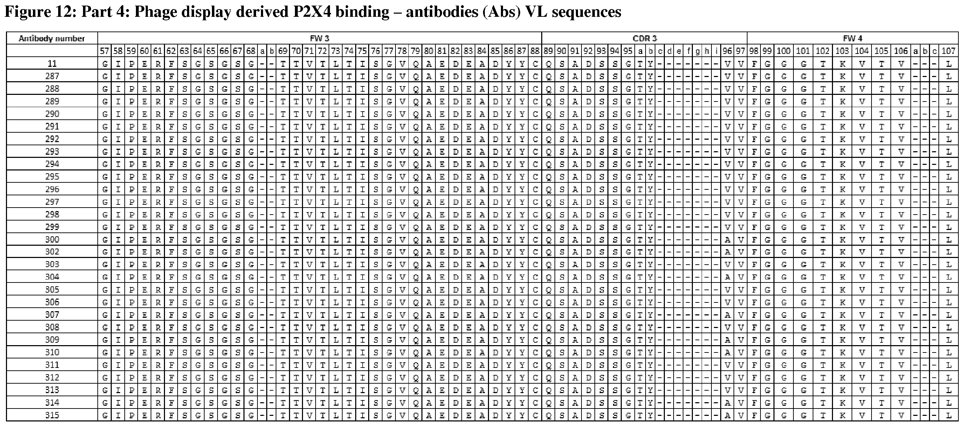

Figure 12 provides an alignment of the VH & VL sequences of Antibody Nos. 287 to 315, which are derived from Antibody 11.

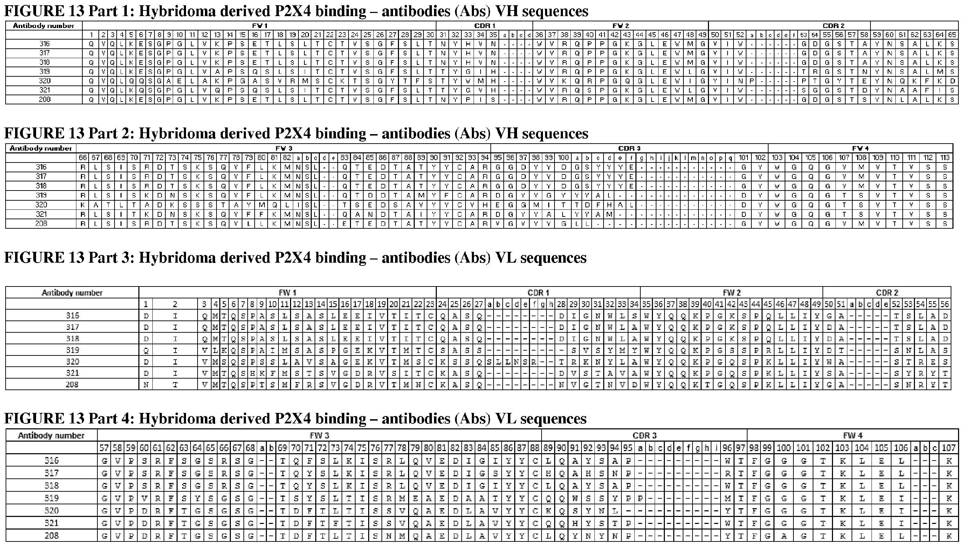

Figure 13 provides the VH & VL sequences of Antibody Nos. 208 and 316 to 321

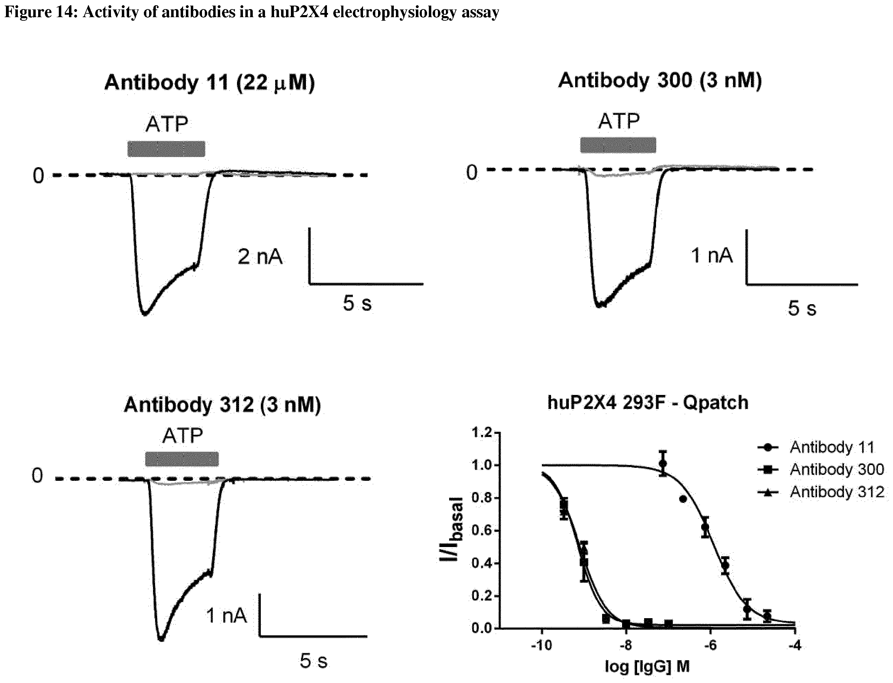

Figure 14 shows the effect of antibody Nos. 11, 300 and 312 on huP2X4 currents recorded on Qpatch 16X. The black trace indicates the ATP response prior to IgG addition whereas the grey trace indicates the ATP response after incubation with IgG. The dashed line indicates the zero current level and the grey bar indicates the time at which ATP was added to the cell bathing solution. Traces are overlayed for comparison. Dose response curves are plotted with normalised current values as described in Example 14 and represent mean +/- SEM, n = 4.

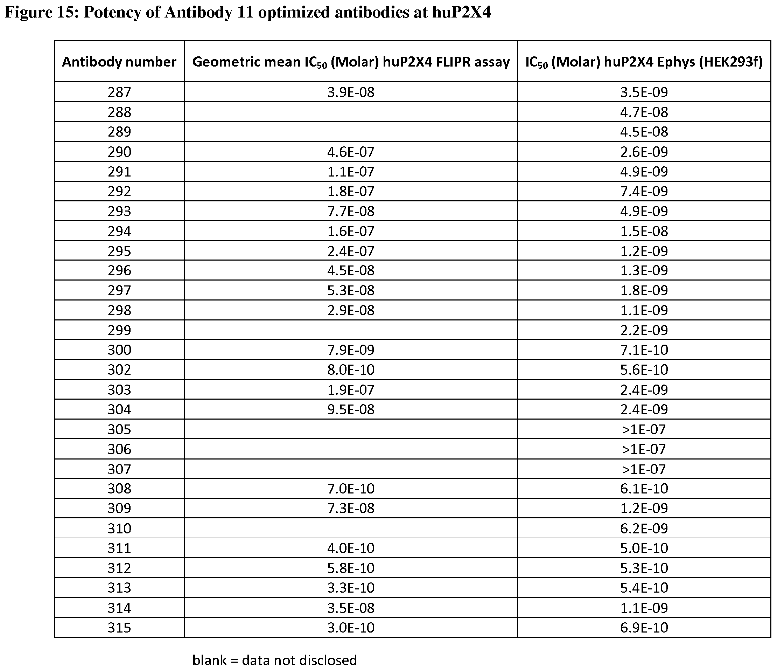

Figure 15 summarises $IC_{50}$ values for optimised versions of Antibody 11 at huP2X4 using either FLIPR or Qpatch 16X. Values are geometric means.

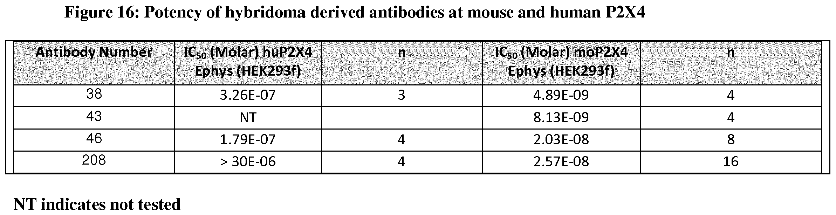

Figure 16 summarises $IC_{50}$ values for antibodies 38, 43, 46 & 208 at murineP2X4 and/or huP2X4 expressed in HEK 293F cells obtained on Qpatch 16X.

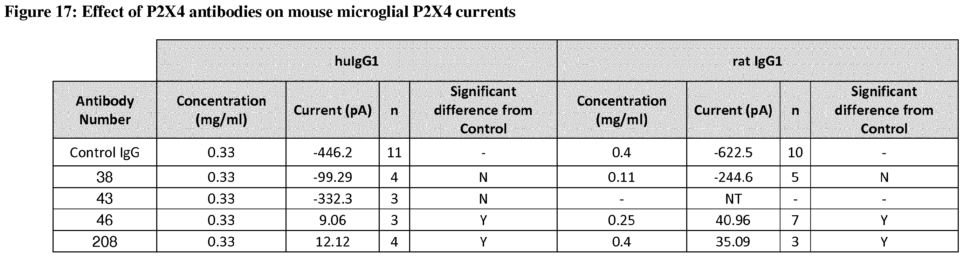

Figure 17 summarises the effect of antibodies 38, 43, 46 & 208 on mouse microglial P2X4 currents measured on Qpatch 16X.

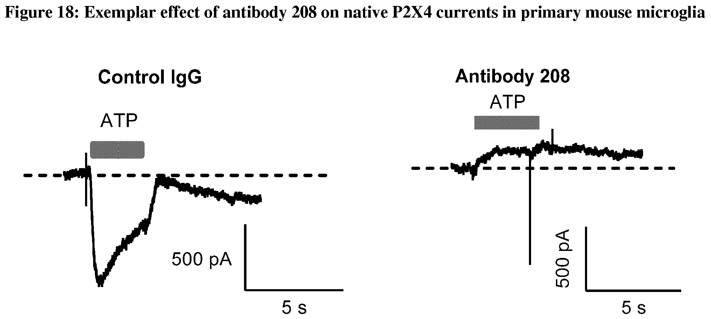

Figure 18 shows exemplary electrophysiology current traces from mouse microglia pretreated with either the control antibody NIP 228 or antibody 208 (0.33 mg/ml). The grey bar indicates the time at which ATP (30 μM) was added to the cells and the dotted line indicates the zero current level.

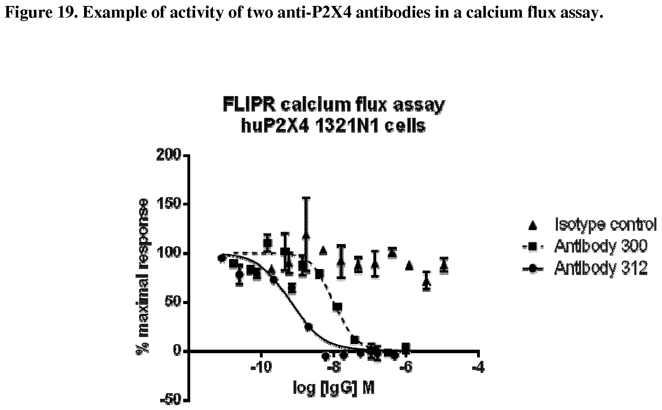

Figure 19 shows example $IC_{50}$ curves obtained with two of the anti-P2X4 antibodies in the ATP stimulated calcium response assay.

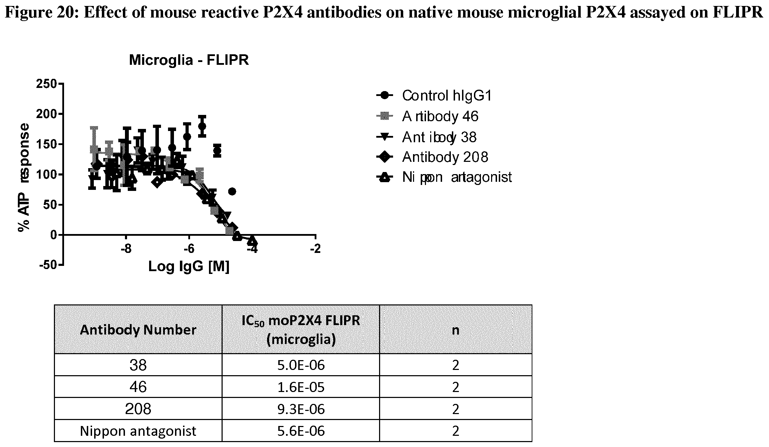

Figure 20 shows $IC_{50}$ curves (mean +/- SEM) for antibodies 46, 38 and 208 obtained from mouse microglia assayed on FLIPR. Mean $IC_{50}$ values are presented below.

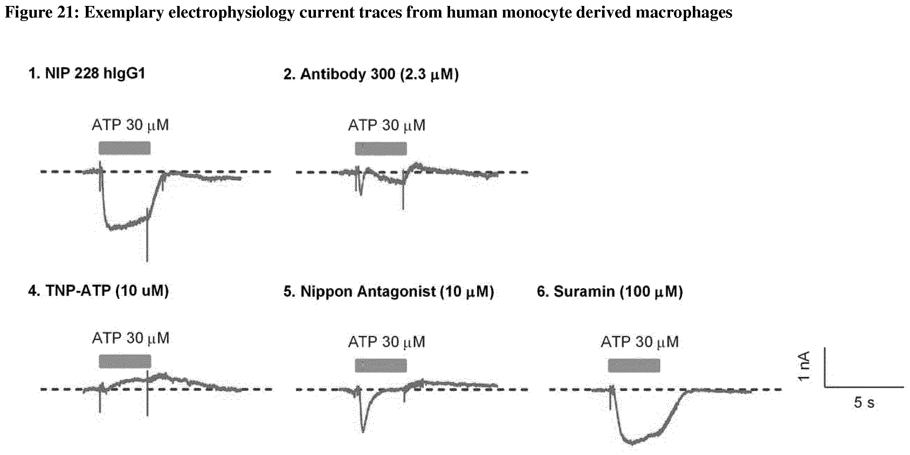

Figure 21 shows exemplar electrophysiology current traces from human monocyte derived macrophages. Time at which ATP was added to the cells is indicated by the open box. Dotted line indicates zero current.

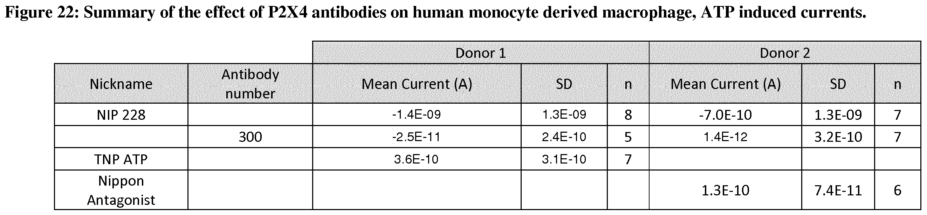

Figure 22 summarises the steady state inward current from human monocyte derived macrophages in response to ATP (30 μM).

Figure 23 summarises the effect of antibodies 319-321 on huP2X4 currents.

## DETAILED DESCRIPTION

As described below, the present disclosure provides antibodies that specifically bind a P2X4 polypeptide and modulate P2X4 channel activity, recombinant P2X4 polypeptides and methods for generating such polypeptides, as well as compositions and methods for generating anti-P2X4 antibodies, and methods of using P2X4 antibodies for the treatment of neuropathic pain and other indications.

### Recombinant Expression of P2X4

The present disclosure provides purified isolated recombinant P2X4 polypeptides that form stable trimeric complexes. The disclosure further provides methods for the large scale production of purified and isolated human and murine P2X4 polypeptides, which is sufficient to produce milligram quantities of P2X4 protein, where the isolated and purified recombinant proteins are predominantly present as stable trimers. The total quantities of P2X4 that were produced for the selection and screening experiments described herein included 6.2 mg hP2X4 and 3.2 mg mP2X4. The production level of purified protein was 0.2 mg/L insect cell culture. As assayed by fluorescent size exclusion chromatography the protein preparation contains 50-75% trimer.

Expression and purification of human-P2X4 with a C-terminal deca Histidine tag in HEK293 cells has been described (Young et al., J. Biol. Chem. 283 (2008) 26241-26251). The purification involved solubilization using dodecylmaltoside detergent and Ni-immobilized metal affinity chromatography. A polyacrylamide gel electrophoretic purification step was required to isolate the trimeric form. Although a fully trimeric preparation of hP2X4 was claimed to have been isolated, the described yield was only 40 $\mu$g per $2.5 \times 10^8$ cells.

Another example of small scale expression and purification of trimer rat P2X channels (subtypes 2, 4 and 7) has been performed (Antonio et al., Br. J. Pharmacol. 163 (2011) 1069-1077). Rat P2X4 receptors having a C-terminal Hemaglutinin tag were expressed transiently in tsA 201 cells (a sub-clone of HEK293 cells stably expressing the SV40 large T-antigen). Receptors were solubilized in CHAPS detergent and affinity purified. Compared to expression of P2X2 and P2X7, expression of P2X4 was relatively low. The purified receptors were used in AFM imaging, which showed trimeric arrangement of the receptors and also double trimers (dimers of trimers).

In another report Sf9 insect cell system was evaluated for expression of human P2X4 and *Dictyostelium discoideum* P2XA (Valente et al., Biochim. Biophys. Acta 1808 (2011) 2859-2866). While the *D. discoideum* P2XA could be obtained in a stable, purified and detergent soluble form, the human P2X4 was reported not to be amenable to be produced in a trimeric form.

The methods present in the prior art uniformly failed to isolate substantial quantities of recombinant P2X4 polypeptides. Moreover, the prior art failed to isolate human P2X4 complexes in milligram quantities where the majority of the isolated proteins were present in trimeric form. In contrast, the methods of the invention, which are suitable for scale up, have allowed milligram scale production of purified recombinant P2X4 polypeptide. The yield of purified P2X4 obtained was 0.2 mg/L insect cell culture medium, corresponding to approximately 8 $\mu$g per $1 \times 10^8$ cells.

For the large scale production of purified P2X4, the human P2X4 and mouse P2X4 receptors were expressed in *Sf9* insect cells using a baculovirus expression system. Expression and protein production are not limited to Sf9 insect cell lines, other insect cell and cell lines that support protein production include *Spodoptera frugiperda* Sf21 cells or *Trichoplusia ni* derived cell lines Tn-368 and High-Five™ BTI-TN-5B1-4. To increase protein production, P2X4 expression levels were monitored at the time of harvest, and the quality and homogeneity of the receptors was assessed using a modified size-exclusion chromatography while detecting fluorescence (FSEC) method as described by Backmark et al., (Protein Sci. 22 (2013) 1124-1132). This method is similar to the basic FSEC concept as described by Kawate and Gouaux (Structure 14 (2006) 673-681),, but applied a fluorescent probe that specifically interacts with a Histidine tag on the protein. To achieve the surprising yields reported herein, cells were innoculated at a density of $1.0 \times 10e^6$/mL in SF900II medium. Cells were infected with a multiplicity of infection of 2 at a cell density of $2 \times 10e^6$ cells/ml. Protein expression was performed at 27°C and cells were harvested 72 hours post infection. These conditions permitted an optimal quantity of the trimeric form of P2X4 to be produced. The homogeneity of protein was unexpected. While the total amount of expressed P2X4 protein increased with longer post infection times, the quality of the expressed protein as assayed by FSEC did not increase beyond 72 hours.

## Anti-P2X4 Antibodies

The disclosure provides anti-P2X4 antibodies that comprise novel antigen-binding fragments
In general, antibodies can be made, for example, using traditional hybridoma techniques (Kohler and Milstein (1975) Nature, 256: 495-499), recombinant DNA methods (U.S. Pat. No. 4,816,567), or phage display performed with antibody libraries (Clackson, T. and Lowman, H.B. Phage Display - A Practical Approach, 2004. Oxford University Press; (2) Thompson, J. et al. J Mol Biol. 256(1):77-88, 1996; (3) Osbourn, J.K. et al. Immunotechnology, 2(3):181-96, 1996). Exemplary antibodies 35-48 were obtained using hybridoma techniques as described herein. Exemplary antibodies 1-34 were obtained using phage display as described herein. For other antibody production techniques, see also Antibodies: A Laboratory Manual, eds. Harlow et al., Cold Spring Harbor Laboratory, 1988. The invention is not limited to any particular source, species of origin, or method of production.

Intact antibodies, also known as immunoglobulins, are typically tetrameric glycosylated proteins composed of two light (L) chains of approximately 25 kDa each and two heavy (H) chains of approximately 50 kDa each. Two types of light chain, designated as the $\lambda$ chain and the $\kappa$ chain, are found in antibodies. Depending on the amino acid sequence of the constant domain of heavy chains, immunoglobulins can be assigned to five major classes: A, D, E, G, and M, and several of these may be further divided into subclasses (isotypes), e.g., $IgG_1$, $IgG_2$, $IgG_3$, $IgG_4$, $IgA_1$, and $IgA_2$.

The subunit structures and three-dimensional configurations of different classes of immunoglobulins are well known in the art. For a review of antibody structure, see Harlow et al., supra. Briefly, each light chain is composed of an N-terminal variable domain ($V_L$) and a constant domain ($C_L$). Each heavy chain is composed of an N-terminal variable domain ($V_H$), three or four constant domains ($C_H$), and a hinge region. The $C_H$ domain most proximal to $V_H$ is designated as $C_H1$. The $V_H$ and $V_L$ domains consist of four regions of relatively conserved sequence called framework regions (FR1, FR2, FR3, and FR4), which form a scaffold for three regions of hypervariable sequence called complementarity determining regions (CDRs). The CDRs contain most of the residues responsible for specific interactions with the antigen. The three CDRs are referred to as CDR1, CDR2, and CDR3. CDR constituents on the heavy chain are referred to as H1, H2, and H3, while CDR constituents on the light chain are referred to as L1, L2, and L3, accordingly. CDR3 and, particularly H3, are the greatest source of molecular diversity within the antigen-binding domain. H3, for example, can be as short as two amino acid residues or greater than 26. In particular embodiments, a heavy chain CDR3 (H3) comprises between about 4 amino acids (see, for example, Ab No. 2) and 22 amino acids (see, for example, Ab Nos. 20 and 34).

The Fab fragment (Fragment antigen-binding) consists of the $V_H$-$C_H1$ and $V_L$-$C_L$ domains covalently linked by a disulfide bond between the constant regions. To overcome the tendency of non-covalently linked $V_H$ and $V_L$ domains in the Fv to dissociate when co-expressed in a host cell, a so-called single chain (sc) Fv fragment (scFv) can be constructed. In a scFv, a flexible and adequately long polypeptide links either the C-terminus of the $V_H$ to the N-terminus of the $V_L$ or the C-terminus of the $V_L$ to the N-terminus of the $V_H$. Most commonly, a 15-residue $(Gly_4Ser)_3$ peptide is used as a linker, but other linkers are also known in the art.

Antibody diversity is a result of combinatorial assembly of multiple germline genes encoding variable regions and a variety of somatic events. The somatic events include recombination of variable gene segments with diversity (D) and joining (J) gene segments to make a complete $V_H$ region and the recombination of variable and joining gene segments to make a complete $V_L$ region. The recombination process itself is imprecise, resulting in the loss or addition of amino acids at the V(D)J junctions. These mechanisms of diversity occur in the developing B cell prior to antigen exposure. After antigenic stimulation, the expressed antibody genes in B cells undergo somatic mutation.

Based on the estimated number of germline gene segments, the random recombination of these segments, and random $V_H$-$V_L$ pairing, up to $1.6\times10^7$ different antibodies could be produced (Fundamental Immunology, 3rd ed., ed. Paul, Raven Press, New York, N.Y., 1993). When other processes that contribute to antibody diversity (such as somatic mutation) are taken into account, it is thought that upwards of $1\times10^{10}$ different antibodies could be potentially generated (Immunoglobulin Genes, 2nd ed., eds. Jonio et al., Academic Press, San Diego, Calif., 1995). Because of the many processes involved in antibody diversity, it is highly unlikely that independently generated antibodies will have identical or even substantially similar amino acid sequences in the CDRs.

The disclosure provides novel CDRs derived from human immunoglobulin gene libraries. The structure for carrying a CDR will generally be an antibody heavy or light chain or a portion thereof, in which the CDR is located at a location corresponding to the CDR of naturally occurring $V_H$ and $V_L$. The structures and locations of immunoglobulin variable domains may be determined, for example, as described in Kabat et al., Sequences of Proteins of Immunological Interest, No. 91-3242, National Institutes of Health Publications, Bethesda, Md., 1991.

The amino acid sequences of anti-P2X4 antibodies 1-48, 208, and 287 to 321, including their $V_H$ and $V_L$ domains are set forth in the Figures and described herein.

Anti-P2X4 antibodies may optionally comprise antibody constant regions or parts thereof. For example, a $V_L$ domain may have attached, at its C terminus, antibody light chain constant domains including human $C\kappa$ or $C\lambda$ chains. Similarly, a specific antigen-binding domain based on a $V_H$ domain may have attached all or part of an immunoglobulin heavy chain derived from any antibody isotope, e.g., IgG, IgA, IgE, and IgM and any of the isotope subclasses, which include but are not limited to, $IgG_1$ and $IgG_4$. The DNA and amino acid sequences for the C-terminal fragment of are well known in the art (see, e.g., Kabat et al., Sequences of Proteins of Immunological Interest, No. 91-3242, National Institutes of Health Publications, Bethesda, Md., 1991).

Certain embodiments comprise a $V_H$ and/or $V_L$ domain of an Fv fragment from a P2X4 antibody.

In certain embodiments, the $V_H$ and/or $V_L$ domains may be germlined, i.e., the framework regions (FRs) of these domains are mutated using conventional molecular biology techniques to match those produced by the germline cells. In other embodiments, the framework sequences remain diverged from the consensus germline sequences.

In certain embodiments, the antibodies specifically bind an epitope within the extracellular domain of human P2X4. In certain embodiments, the antibodies specifically bind an epitope within the extracellular domain of human or mouse P2X4, with an affinity of more than $10^6M^{-1}$, more than $10^7M^{-1}$, or more than $10^8M^{-1}$.

It is contemplated that antibodies of the invention may also bind with other proteins, including, for example, recombinant proteins comprising all or a portion of the P2X4 extracellular domain.

One of ordinary skill in the art will recognize that the antibodies of this invention may be used to detect, measure, and inhibit proteins that differ somewhat from P2X4. The antibodies are expected to retain the specificity of binding so long as the target protein comprises a sequence which is at least about 60%, 70%, 80%, 90%, 95%, or more identical to any sequence of at least 100, 80, 60, 40, or 20 of contiguous amino acids of P2X4 (NCBI Ref. No. Q99571). The percent identity is determined by standard alignment algorithms such as, for example, Basic Local Alignment Tool (BLAST) described in Altshul et al. (1990) J. Mol. Biol., 215: 403-410, the algorithm of Needleman et al. (1970) J. Mol. Biol., 48: 444-453, or the algorithm of Meyers et al. (1988) Comput. Appl. Biosci., 4: 11-17.

In addition to the sequence homology analyses, epitope mapping (see, e.g., Epitope Mapping Protocols, ed. Morris, Humana Press, 1996) and secondary and tertiary structure analyses can be carried out to identify specific 3D structures assumed by the disclosed antibodies and their complexes with antigens. An example of such a 3D structure is provided for Antibody No. 11. Such methods include, but are not limited to, X-ray crystallography (Engstom (1974) Biochem. Exp. Biol., 11:7-13) and computer modeling of virtual representations of the presently disclosed antibodies (Fletterick et al. (1986) Computer Graphics and Molecular Modeling, in Current Communications in Molecular Biology, Cold Spring Harbor Laboratory, Cold Spring Harbor, N.Y.).

**Nucleic Acids, Cloning and Expression Systems**

The present disclosure provides the amino acid sequence of the disclosed antibodies. Once provided with this information, one of skill in the art could readily obtain nucleic acid molecules encoding the disclosed antibodies. The nucleic acids may comprise DNA or RNA and may be wholly or partially synthetic or recombinant. Reference to a nucleotide sequence encompasses a DNA molecule with the specified sequence, and encompasses a RNA molecule with the specified sequence in which U is substituted for T, unless context requires otherwise.

The nucleic acids molecules of the invention comprise a coding sequence for a CDR, a $V_H$ domain, and/or a $V_L$ domain disclosed herein.

The present disclosure also provides constructs in the form of plasmids, vectors, phagemids, transcription or expression cassettes which comprise at least one nucleic acid molecule encoding a CDR, a $V_H$ domain, and/or a $V_L$ domain disclosed herein.

The disclosure further provides a host cell which comprises one or more constructs as above.

Also provided are nucleic acids encoding any CDR (H1, H2, H3, L1, L2, or L3), $V_H$ or $V_L$ domain, as well as methods of making of the encoded products. The method comprises expressing the encoded product from the encoding nucleic acid. Expression may be achieved by culturing under appropriate conditions recombinant host cells containing the nucleic acid. Following production by expression a $V_H$ or $V_L$ domain, or specific binding member may be isolated and/or purified using any suitable technique, then used as appropriate.

Antigen-binding fragments, $V_H$ and/or $V_L$ domains and encoding nucleic acid molecules and vectors may be isolated and/or purified from their natural environment, in substantially pure or homogeneous form, or, in the case of nucleic acid, free or substantially free of nucleic acid or genes of origin other than the sequence encoding a polypeptide with the required function.

Systems for cloning and expression of a polypeptide in a variety of different host cells are well known in the art. For cells suitable for producing antibodies, see Gene Expression Systems, Academic Press, eds. Fernandez et al., 1999. Briefly, suitable host cells include bacteria, plant cells, mammalian cells, and yeast and baculovirus systems. Mammalian cell lines available in the art for expression of a heterologous polypeptide include Chinese hamster ovary cells, HeLa cells, baby hamster kidney cells, NS0 mouse myeloma cells, and many others. A common bacterial host is *E. coli.* Any protein expression system compatible with the invention may be used to produce the disclosed antibodies. Suitable expression systems include transgenic animals described in Gene Expression Systems, Academic Press, eds. Fernandez et al., 1999.

Suitable vectors can be chosen or constructed, so that they contain appropriate regulatory sequences, including promoter sequences, terminator sequences, polyadenylation sequences, enhancer sequences, marker genes and other sequences as appropriate. Vectors may be plasmids or viral, e.g., phage, or phagemid, as appropriate. For further details see, for example, Sambrook et al., Molecular Cloning: A Laboratory Manual, 2nd ed., Cold Spring Harbor Laboratory Press, 1989. Many known techniques and protocols for manipulation of nucleic acid, for example, in preparation of nucleic acid constructs, mutagenesis, sequencing, introduction of DNA into cells and gene expression, and analysis of proteins, are described in detail in Current Protocols in Molecular Biology, 2nd Edition, eds. Ausubel et al., John Wiley & Sons, 1992.

A further aspect of the disclosure provides a host cell comprising a nucleic acid as disclosed here. A still further aspect provides a method comprising introducing such nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g., vaccinia or, for insect cells, baculovirus. For bacterial cells, suitable techniques may include calcium chloride transformation, electroporation and transfection using bacteriophage. The introduction of the nucleic acid into the cells may be followed by causing or allowing expression from the nucleic acid, e.g., by culturing host cells under conditions for expression of the gene.

**Methods of Use**

The disclosed anti-P2X4 antibodies are capable of modulating the electrophysiological activity of P2X4. In particular, antibodies provided herein may be used to inhibit or potentiate P2X4 channel conductance. Such antibodies can be used to treat P2X4-associated medical disorders in mammals, especially, in humans. In particular, antibodies that inhibit P2X4 activity are useful for the treatment of neuropathic pain. Antibodies that potentiate P2X4 activity are useful in other therapeutic methods, including but not limited to microglia-mediated diseases and disorders and macrophage-mediated diseases and disorders.

As demonstrated in the Examples, binding of P2X4 with an anti-P2X4 antibody modulates P2X4 biological activity by potentiating or reducing passage of ions through the P2X4 channel.

The antibodies or antibody compositions of the present invention are administered in therapeutically effective amounts. Generally, a therapeutically effective amount may vary with the subject's age, condition, and sex, as well as the severity of the medical condition of the subject. The appropriate dose is chosen based on clinical indications by a treating physician.

The antibodies may be given as a bolus dose, to maximize the circulating levels of antibodies for the greatest length of time after the dose. Continuous infusion may also be used after the bolus dose.

**Pharmaceutical Compositions and Methods of Administration**

The invention provides pharmaceutical compositions comprising anti-P2X4 antibodies. Such compositions are likely suitable for pharmaceutical use and administration to patients. The compositions typically comprise one or more antibodies of the present invention and a pharmaceutically acceptable excipient. The phrase "pharmaceutically acceptable excipient" includes any and all solvents, dispersion media, coatings, antibacterial agents and antifungal agents, isotonic agents, and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions. The pharmaceutical compositions may also be included in a container, pack, or dispenser together with instructions for administration.

A pharmaceutical composition of the invention is formulated to be compatible with its intended route of administration. Methods to accomplish the administration are known to those of ordinary skill in the art. The administration may, for example, be intravenous, intraperitoneal, intramuscular, intracavity, subcutaneous or transdermal. In one embodiment, neuropathic pain is treated by intrathecal administration. It may also be possible to obtain compositions which may be topically or orally administered, or which may be capable of transmission across mucous membranes.

Solutions or suspensions used for intradermal or subcutaneous application typically include one or more of the following components: a sterile diluent such as water for injection, saline solution, fixed oils, polyethylene glycols, glycerin, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; and agents for the adjustment of tonicity such as sodium chloride or dextrose. The pH can be adjusted with acids or bases, such as hydrochloric acid or sodium hydroxide. Such preparations may be enclosed in ampoules, disposable syringes or multiple dose vials made of glass or plastic.

Pharmaceutical compositions suitable for injection include sterile aqueous solutions or dispersions and sterile powders for the extemporaneous preparation of sterile injectable solutions or dispersion. For intravenous administration, suitable carriers include physiological saline, bacteriostatic water, Cremophor EL (BASF, Parsippany, N.J.) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars; polyalcohols such as mannitol, sorbitol, and sodium chloride in the composition. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and/or by the use of surfactants. Prolonged absorption of the injectable compositions can be brought about by including in the composition an agent which delays absorption, for example, aluminum monostearate, and gelatin.

Oral compositions generally include an inert diluent or an edible carrier. They can be enclosed in gelatin capsules or compressed into tablets. For oral administration, the antibodies can be combined with excipients and used in the form of tablets, troches, or capsules. Pharmaceutically compatible binding agents, and/or adjuvant materials can be included as part of the composition. The tablets, pills, capsules, troches, and the like can contain any of the following ingredients, or compounds of a similar nature; a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known in the art, and include, for example, detergents, bile salts, and fusidic acid derivatives. Transmucosal administration may be accomplished, for example, through the use of lozenges, nasal sprays, inhalers, or suppositories. For example, in case of antibodies that comprise the Fc portion, compositions may be capable of transmission across mucous membranes in intestine, mouth, or lungs (e.g., via the FcRn receptor-mediated pathway as described in U.S. Pat. No. 6,030,613). For transdermal administration, the active compounds may be formulated into ointments, salves, gels, or creams as generally known in the art. For administration by inhalation, the antibodies may be delivered in the form of an aerosol spray from pressured container or dispenser, which contains a suitable propellant, e.g., a gas such as carbon dioxide, or a nebulizer.

In certain embodiments, the presently disclosed antibodies are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions containing the presently disclosed antibodies can also be used as pharmaceutically acceptable carriers. These can be prepared according to methods known to those skilled in the art, for example, as described in U.S. Pat. No. 4,522,811.

It may be advantageous to formulate oral or parenteral compositions in a dosage unit form for ease of administration and uniformity of dosage. The term "dosage unit form" as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of the composition of the invention can be determined by standard pharmaceutical procedures in cell cultures or experimental animals, e.g., for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Compositions that exhibit large therapeutic indices are preferred.

The data obtained from electrophysiological experiments and animal studies can be used in formulating a range of dosage for use in humans. A dose may be formulated in animal models to achieve a circulating plasma concentration range that includes the $IC_{50}$ (i.e., the concentration of the antibody which achieves a half-maximal inhibition of symptoms). Circulating levels in plasma may be measured, for example, by high performance liquid chromatography. The effects of any particular dosage can be monitored by a suitable bioassay. The dosage lies preferably within a range of circulating concentrations with little or no toxicity. The dosage may vary depending upon the dosage form employed and the route of administration utilized.

The following examples are put forth so as to provide those of ordinary skill in the art with a complete disclosure and description of how to make and use the anti-P2X4 antibodies in assay, screening, and therapeutic methods of the invention.

## EXAMPLES

### Example 1: Production and isolation of recombinant P2X4 proteins

Human P2X purinoceptor 4 (Q99571), a natural variant of human P2X purinoceptor 4 with an S to G mutation at position 242 (Corresponds to variant rs25644) and murine P2X purinoceptor 4 (Q9JJX6) proteins were designed with a C-terminal AVI tag (Avidity LLC) and a C-terminal Histidine tag. The constructs were cloned into pFASTBAC1 vectors (Life Technologies). Bacmids were generated in DH10Bac (Life Technologies) *E. coli* cells. Bacmids were subsequently transfected into Sf9 insect cells (*Spodoptera frugiperda* Sf9 cells from Life Technologies, cat no 11496-015) for production of recombinant baculovirus particles, which in turn were used to infect Sf9 cells for protein expression.

Expression parameters were assessed by monitoring expression level, protein quality and the homogeneity of the receptor using a modified Fluorescence-detection size-exclusion chromatography (FSEC) method described by Backmark et al., (Protein Sci. 22 (2013) 1124-1132). This method is similar to the basic FSEC concept as described by Kawate and Gouaux (Structure 14 (2006) 673-681), but applied a fluorescent probe that specifically interacts with the Histidine tag on the protein. Cells were typically innoculated at a density of 1.0x10e6/mL in SF900II medium. Cells were infected with a multiplicity of infection of 2 at a cell density of 2 x 10E6 cells/ml. Protein expression was performed at 27°C and cells were harvested 72 hours post infection. Expression parameters were selected to enhance the quantity of trimer and homogeneity of protein present as trimers. As assayed by fluorescent size exclusion chromatography, the protein preparation contains 50-75% trimer. Although the total amount of receptor increased with longer post infection times, FSEC analysis indicated that protein quality declined when the expression time was increased past 72 hours.

Human P2X4 receptor and mouse P2X4 were purified as follows. Membranes were prepared from SF9 cells. Membrane proteins were extracted from the membranes by detergent solubilization, using combinations of detergents, salts, buffers and additives, including n-Dodecyl-beta-D-Maltoside CAS 69227-93-6 (0-2% (w/v)), n-Dodecyl thio-Maltoside CAS 148565-58-6 (0-1% (w/v)), (3-[(3-Cholamidopropyl)-Dimethylammonio]-1-Propane Sulfonate/N,N-Dimethyl-3-Sulfo-N-[3-[[3α,5β,7α,12α)-3,7,12-Trihydroxy-24-Oxocholan-24-yl]Amino]propyl]-1-Propanaminium Hydroxide abbreviated to CHAPS CAS 75621-03-3 (0-0.6 % (w/v)), and Cholesteryl Hemisuccinate CAS 102601-49-0 (0-0.12% (w/v)). Without being bound to theory, higher concentrations of the indicated substances as well as alternative detergents support extraction of the protein from the membranes. The proteins underwent standard affinity and size exclusion chromatography purification. The purified protein was formulated in a buffer which contained 50 mM Tris-HCl pH 8.0, 600 mM NaCl, 10% (v/v) glycerol, 0.025 (w/v) % n-Dodecyl-beta-D-Maltoside CAS 69227-93-6, 0.0125% (w/v), n-Dodecyl thio-Maltoside CAS 148565-58-6, 0.0075% (w/v) (3-[(3-Cholamidopropyl)-Dimethylammonio]-1-Propane Sulfonate/N,N-Dimethyl-3-Sulfo-N-[3-[[3α,5β,7α,12α)-3,7,12-Trihydroxy-24-Oxocholan-24-yl]Amino]propyl]-1-Propanaminium Hydroxide abbreviated to CHAPS CAS 75621-03-3, and 0.0015% (w/v) Cholesteryl Hemisuccinate CAS 102601-49-0.

The purified protein was formulated under alternative conditions, including phosphate buffers and HEPES buffers 2 -[4-(2-hydroxyethyl)piperazin-1-yl]ethanesulfonic acid buffers CAS 7365-45-9. The pH of the various buffers has ranged from 7.0-8.0. Salt (NaCl) has been varied between 120-600 mM. Glycerol can be excluded from the protein formulation. Various detergents have been used in protein formulation, such as lauryl maltose neopentyl glycol 2,2-didecylpropane-1,3-bis-β-D-maltopyranoside, decyl maltose neopentyl glycol 2,2-dioctylpropane-1,3-bis-β-D-maltopyranoside, octyl maltose neopentyl glycol 2,2-dihexylpropane-1,3-bis-β-D-maltopyranoside, CYMAL-5 5-Cyclohexyl-1-pentyl-β-D-maltoside CAS 250692-65-0, n-Tetradecylphosphocholine 77733-28-9, n-Decyl-β-D-Maltopyranoside CAS 82494-09-5, n-octyl-β-D-glucoside CAS 29836-26-8 and n-nonyl-β-D-glucoside CAS 69984-73-2. Formulations in other detergents are also possible. The concentration of Cholesteryl Hemisuccinate CAS 102601-49-0 can be varied and excluded from the protein formulation as well.

### Example 2: Purification of Trimeric P2X4 complexes

*In vivo,* P2X receptors form funtional trimeric ion channels. The solubilised and purified P2X4 proteins are typically present in a range of oligomeric states, including monomers, dimers, trimers, and hexamers (i.e., dimers of trimers). This range of oligomeric states is described for example, by references (Backmark et al., Protein Sci. 22 (2013) 1124-1132; Kawate et al., Structure 14 (2006) 673-681; Kawate et al., Nature 460 (2009) 592-598; Nakazawa et al., European Journal of Pharmacology 518 (2005) 107-110; Nicke et al., Mol. Pharmacol. 63 (2003) 243-252). To obtain a stable predominantly trimeric arrangement, solubilization conditions were adjusted.

Combinations of detergents, additives, buffers and pH were varied. Optimal conditions were selected to increase the FSEC signature of the trimer while reducing larger order oligomeric arrangments and aggregates. Such undesirable forms were eluted in the void volume of the size-exclusion columns applied. Conditions tested included KPO4-HCl pH 7.4, 600 mM NaCl and 2% (w/v) n-dodecyl-beta-maltopyranoside CAS 69227-93-6. Optimal solubilization was obtained in buffers containing combinations of the detergents including n-Dodecyl-beta-D-Maltoside CAS 69227-93-6, n-Dodecyl thio-Maltoside CAS 148565-58-6, (3-[(3-Cholamidopropyl)-Dimethylammonio]-1-Propane Sulfonate/N,N-Dimethyl-3-Sulfo-N-[3-[[3α,5β,7α,12α)-3,7,12-Trihydroxy-24-Oxocholan-24-yl]Amino]propyl]-1-Propanaminium Hydroxide abbreviated to CHAPS CAS 75621-03-3, and the additive Cholesteryl Hemisuccinate CAS 102601-49-0. The purified protein was formulated in a buffer which contained 50 mM Tris-HCl pH 8.0, 600 mM NaCl, 10% (v/v) glycerol, 0.025 (w/v) % n-Dodecyl-beta-D-Maltoside CAS 69227-93-6, 0.0125% (w/v), n-Dodecyl thio-Maltoside CAS 148565-58-6, 0.0075% (w/v) (3-[(3-Cholamidopropyl)-Dimethylammonio]-1-Propane Sulfonate/N,N-Dimethyl-3-Sulfo-N-[3-[[3α,5β,7α,12α)-3,7,12-Trihydroxy-24-Oxocholan-24-yl]Amino]propyl]-1-Propanaminium Hydroxide abbreviated to CHAPS CAS 75621-03-3, and 0.0015% (w/v) Cholesteryl Hemisuccinate CAS 102601-49-0.

### Example 3: Anti-P2X4 specific antibodies were isolated using phage display selection.

Naive human single chain Fv (scFv) phage display libraries were cloned into a phagemid vector based on the filamentous phage M13 were used for selections (Lloyd (2009) Protein Eng Des Sel 22, 159-168; Vaughan et al., Nature biotechnology 14, 309-314, 1996). Anti-P2X4 specific antibodies were isolated from the phage display libraries using a series of selection cycles on recombinant human P2X4 (hu P2X4), essentially as previously described by Vaughan et al (Vaughan et al., supra). In brief, human P2X4 in PBS (Dulbecco's PBS, pH7.4) was immobilised onto wells of a MaxiSorp® microtitre plate (Nunc) overnight at 4°C. Wells were washed with PBS then blocked for 1 hour with PBS-Marvel dried skimmed milk (3% w/v). Purified phage in PBS-Marvel (3% w/v) were added to the wells and allowed to bind coated antigen for 1 hour at room temperature. Unbound phage was removed by a series of wash cycles using PBS. Bound phage particles were eluted with trypsin for 30 minutes at 37°C, infected into *E.coli* TG1 bacteria and rescued for the next round of selection. Alternatively, anti-P2X4 antibodies were isolated as described above except deselection of the purified phage library against the C-terminal peptide huP2X4$_{370-388}$ (Alomone Labs #APR-002) or phenyl hydrophobic interaction chromatography (HIC) beads was performed prior to selection with the antigen.

**Example 4: Generation of rat anti-murine P2X4 antibodies using hybridoma technology.**

***Immunisations***

Purified recombinant murine P2X4 protein and murine P2X4 transfected HEK 293F cells were used to immunise Sprague Dawley rats in three groups. For group 1, rats were immunised with murine P2X4 protein; for group 2, rats were immunised with murine P2X4 transfected HEK 293F cells; and for group 3, rats were immunised by alternating murine P2X4 protein and murine P2X4 transfected HEK 293F cells.

A twenty eight day immunization protocol was used with a priming immunization on day 0, followed by four subsequent booster immunizations on days 7, 15, 22 and 24. For group 1, equal volumes of complete Freund's adjuvant and murine P2X4 protein (total protein: 100 μg) were emulsified together, and delivered to the rats subcutaneously at two sites (200 μL per site). For the subsequent three booster injections, the same amount of protein was used, emulsified in Freund's incomplete adjuvant. For group 2, murine P2X4 transfected HEK 293F cells were resuspended at 5E7 cells per mL in PBS and emulsified with equal volumes of complete Freund's adjuvant. As above, the cells were injected into rats at two sites (200 μL per site). For the subsequent three booster injections, the same number of cells was used, emulsified in Freund's incomplete adjuvant. For group 3, the priming immunization was with murine P2X4 protein as per group 1 above, followed by three booster immunizations with murine P2X4 transfected HEK 293F cells, murine P2X4 protein, and murine P2X4 transfected HEK 293F cells.

The final boosts were given intraperitoneally on day 24, group 1 and group 3 rats received murine P2X4 protein (400 μL at 50 μg/mL in Tris buffer), and group 2 rats received murine transfected HEK 293F cells (400 μL at 5E7/mL).

Tail vein bleeds were obtained from the rats before immunisation, on day 13 after the first immunization, and on day 20 after second immunisation. The IgG titres to anti-murine P2X4 were determined by a cell-based DELFIA (dissociation-enhanced lanthanide fluorescence immunoassay) assay.

***Assessment of rat immune response to murine P2X4 using a cell-based DELFIA***

The IgG titres to murine P2X4 in sera were determined by a cell based DELFIA using both mP2X4 transfected HEK 293F and parental HEK cells. In order to reduce anti-HEK 293F cell specific antibodies in sera, before being assayed the serum samples from rats immunised with either cells alone or the alternating protein and cells strategy were incubated with non-transfected HEK 293F cells. The sera from rats immunised with protein were assayed without this pre-adsorption step.

Murine P2X4 transfected HEK 293F and parental HEK cells were plated in culture media onto black collagen coated 96 well microtitre plates at a density of 30,000 cells per well. After overnight incubation at 37°C in a 5% $CO_2$ incubator, the culture supernatant was removed and the cells were fixed with 3.7% formaldehyde solution at 50 μL per well. All subsequent incubations were carried out at room temperature. After 5 minutes fixation, the formaldehyde solution was discarded and replaced with 200 μL of 3% marvel/PBS blocking buffer. After one hour, the blocking buffer was removed and the serum samples added in a 3-fold dilution series (50 μL per well starting from a 1:200 dilution). After incubating for one hour, the wells were washed gently three times with PBS supplemented with 0.05% (v/v) Tween 20. A biotinylated polyclonal goat anti-rat IgG Fc gamma specific secondary antibody (diluted 1:500 in marvel/PBS) was added then at 50 μL per well. Following a further one hour incubation and three gentle washes as above, Eu-N1-labeled streptavidin (Perkin Elmer) was added to the wells (diluted to 100 ng/mL in marvel/PBS, 50 μL per well). After 30 minutes incubation time, the wells were gently washed five times and DELFIA enhancement solution was added. The reaction was allowed to develop for 10 minutes, and then the plate was then read using a PerkinElmer EnVision 2103 multilabel plate reader. The TRF (time-resolved fluorescence) signal in each well was measured (excitation 340 nm, emission 615 nm).

The serum titration curves for murine P2X4 transfected HEK 293F cells and parental HEK 293F cells were plotted and the respective area under the curves (AUC) calculated. For rats immunized with murine P2X4 transfected HEK 293F cells, specific mP2X4 IgG titres were derived by subtracting the AUC values from parental HEK cells from the AUC values for the murine P2X4 transfected cells.

***Monoclonal rat IgG isolation***

Four days after the final boost, lymph nodes were aseptically harvested and cells were isolated by mechanical disruption and counted. These cells were mixed with SP2/0 myeloma cells and fused using an electrofusion apparatus. The resultant fusions were mixed with a methylcellulose-based semi-solid media and plated out into OmniTray plates.

The semi-solid media comprised CloneMatrix and DMEM supplemented with 20% FCS, 10% BM Condimed H1, 1mM sodium pyruvate and OPI media supplement, 2% hypoxanthine azaserine and FITC conjugated goat anti-rat IgG. The cells in semi-solid media were cultured for 13 days at 37°C in a 5% $CO_2$ incubator. During this incubation period, clonal colonies are formed from a single progenitor hybridoma cell. These colonies secrete IgG that is trapped in the vicinity of the colony by the FITC conjugated anti-IgG present in the semi-solid media. The resultant immune complex formation can be observed around the cell as a fluorescent 'halo' when visualised by ClonePix FL colony picker (Molecular Devices). These haloed colonies are then picked into 96 well microtitre plates.

After 3-5 days in culture, the supernatants of the picked colonies were harvested and screened for murine P2X4 specificity by comparing binding to murine P2X4 transfected HEK 293F cells and parental HEK 293F cells by a cell-based fluorometric microvolume assay technology (FMAT) assay.

***DNA sequencing of rat IgG***

Messenger RNA (mRNA) was extracted from cells using magnetic oligo (dT) particles and converted into cDNA. PCR amplification was performed using poly-C and constant region VH/VL primers.

***Rat IgG purifications***

Prior to purification, the hybridomas were tested by ELISA using a goat anti-rat IgG2a coated microtitre plate to determine which clones secreted Rat IgG2a, as this isotype is purified using a different purification matrix to rat IgG1, IgG2b and IgG2c isotypes.

Cells were propagated in 24 well plates and overgrown in serum free HL-1 medium supplemented with Hyper-Zero and glutamine. After 10 days, the supernatants were transferred to 96 well masterblocks and rat IgG1, IgG2b and IgG2c isotypes were purified on 20μL Phytips containing ProPlus resin (Phynexus). Rat IgG2a antibodies were purified on custom packed Phytips containing CaptureSelect IgG-Fc multiple species resin (Lift Technologies) using Perkin Elmer Minitrack. The captured rat IgGs were eluted with 75 μL of 100 mM HEPES, 140 mM NaCl pH 3.0 then neutralised with an equal volume of 200 mM HEPES pH 8.0. The purified IgGs were quantified using an absorbance reading at 280 nm in UV-Star 384 well plate.

***Reformatting of rat IgGs to human IgG1***

Rat hybridoma IgG clones were molecularly reformatted to generate chimeric constructs expressing rat VH and VL domains and human IgG1 constant domains essentially as described by Persic et al., 1997 (Gene 187, 9-18) with the following modifications. An OriP fragment was included in the expression vectors to facilitate use with CHO-transient cells and to allow episomal replication. The VH domain was cloned into a vector (pEU1.4) containing the human heavy chain constant domains and regulatory elements to express whole IgG1 heavy chain in mammalian cells. This constant region contained the triple mutations (TM) L234F/L235E/P331S resulting in an effector null human IgG1 (Oganesyan et al.,(2008) Acta Crystallogr D Biol Crystallogr. 64, 700-704). Similarly, the VL domain was cloned into a vector (pEU4.4) for the expression of the human light chain (lambda) constant domains and regulatory elements to express whole IgG light chain in mammalian cells. To obtain IgGs, the heavy and light chain IgG expressing vectors were transfected into CHO-transient mammalian cells. IgGs were expressed and secreted into the medium. Harvests were filtered prior to purification, then IgG was purified using Protein A chromatography. Culture supernatants were loaded on a column of appropriate size of Ceramic Protein A (Pall 20078-036) and washed with 50 mM Tris-HCl pH 8.0, 250 mM NaCl. Bound IgG was eluted from the column using 0.1 M Sodium Citrate (pH 3.0) and neutralised by the addition of Tris-HCl (pH 9.0). The eluted material was buffer exchanged into PBS using Nap10 columns (GE Lifesciences 17-0854-02) and the concentration of IgG was determined spectrophotometrically using an extinction coefficient based on the amino acid sequence of the IgG (Pace et al., (1995) Protein Sci. 4, 2411-23). The purified IgG were analysed for purity using SDS-PAGE.

**Example 5: Identification of human P2X4 binding antibodies from phage display selections.**

ScFv antibodies identified from the phage display method described in Example 3 were expressed in bacteria and screened as unpurified bacterial periplasmic extracts (which contain scFv), prepared in: 0.2M HEPES buffer pH7.4, 0.5 mM EDTA and 0.5 M sucrose. Alternatively, the heavy and light chain variable regions were amplified by PCR and cloned into a vector for expression as human IgG1 antibodies in HEK293F cells.

For screening of bacterial scFv samples, 5μl of bacterial extract was added to a 384 well assay plate (Corning 3655). Assay buffer was prepared as follows: 1X Hanks Balanced Salt Solution (HBSS) (Sigma H8264), 0.1% (v/v) BSA (PAA K05-013), 20mM HEPES (Gibco 15630) and 1U/ml Apyrase (Sigma A6535) and 5μl added to the assay plate with the bacterial scFv extract. Anti-myc detection reagent (Serotec MCA2200) and anti-mouse DyLight649 (Jackson Immuno Research Labs 115-495-071) were diluted in assay buffer to 15.6nM and 24nM respectively in the same solution and 5μl added to the assay plate with the scFv sample. HEK293F cells expressing human P2X4 (huP2X4) (Q99571, ENSP00000336607) were diluted to $2.6e^5$cells/ml in assay buffer and 15μl added to the assay plate. In parallel scFv samples were also tested for binding to HEK293F cells that did not express huP2X4.

For screening of the HEK293F expressed IgG samples, 2.5μl of cell culture supernatant was added to the 384 well assay plate (Corning 3655). Assay buffer was prepared as described above and 7.5μl was added to the assay plate with the IgG sample. Anti-human AlexaFluor 647 (Life Technologies A21445) was diluted in assay buffer to 6nM and 10μl added to the assay plate with the IgG sample. HEK293F cells expressing huP2X4 (Q99571, ENSP00000336607) were diluted to $4e^5$cells/ml in assay buffer and 10μl added to the assay plate. In parallel IgG samples were also tested for binding to HEK293F cells that did not express huP2X4. Assay plates set up to screen both types of samples were sealed with a Topseal plate sealer (Perkin Elmer 6005250) and incubated at room temperature for at least 4hours before reading on the Fluorescence Microvolume Assay Technology (FMAT), a fluorescence based platform that detects fluorescence localized to bead or cells settled at the bottom of a microwell (Dietz et al., Cytometry 23:177-186 (1996), Miraglia et al., J. Biomol. Screening 4:193-204 (1999)). Data was analysed using the FMAT analysis software and events were gated based on fluorescence 0-10,000 FL1 counts, colour typically 0.15 to 0.40 and size 10-60. A minimum count of 20 events was set as a threshold before data was reported for each well. ScFv showing binding to the HEK293F huP2X4 cells, but not to the control HEK293F cells were selected for further testing if the FL1 count was above 1000 on the huP2X4 cells, IgG samples showing a specific huP2X4 binding signal of greater than 200 FL1 counts were identified as hits and characterised further.

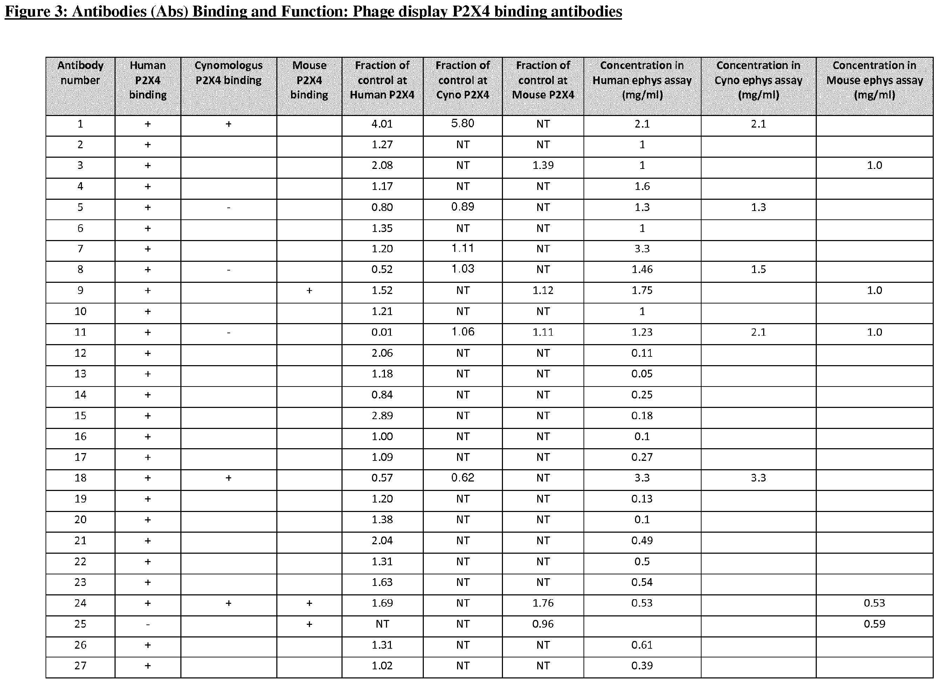

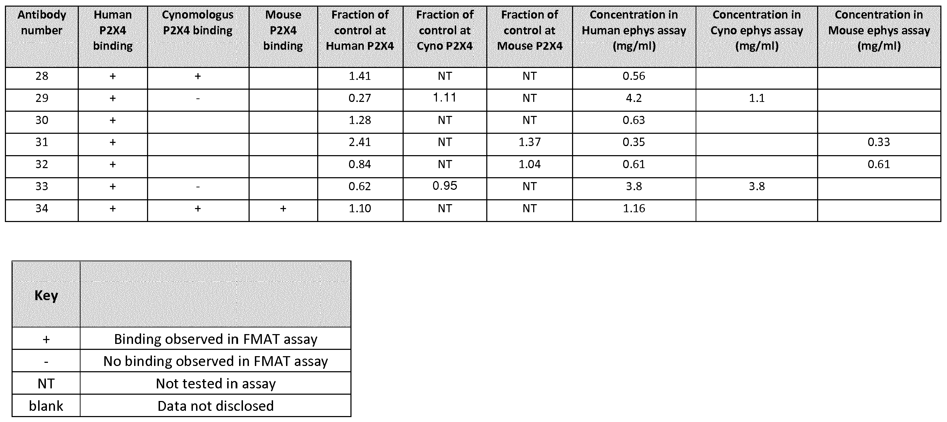

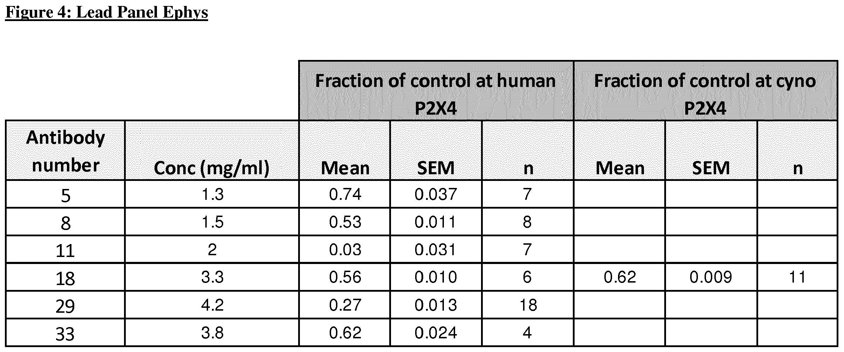

ScFv or IgG samples which showed a specific binding signal to HEK293F huP2X4 cells as unpurified samples were subjected to DNA sequencing (Vaughan et al. supra, Nature Biotechnology 14: 309-314), (Osbourn 1996;Immunotechnology. 2, 181-196). Unique scFvs were expressed in bacteria and purified by affinity chromatography (as described by Bannister et al (2006) Biotechnology and bioengineering, 94. 931-937). Those scFv that confirmed binding to human P2X4 were generated as full IgGs and expressed and purified as described in Example 4. Purified IgG antibodies were tested for functional activity in the electrophysiology assay and for binding to cells expressing mouse and cynomologus P2X4 using the same method described for the human P2X4 cells described above except a titration of purified IgG sample was used. Results of electrophysiology assays are provided at Figures 3 and 4.

**Example 6: Identification of hybridoma IgGs that bind specifically to murine P2X4**

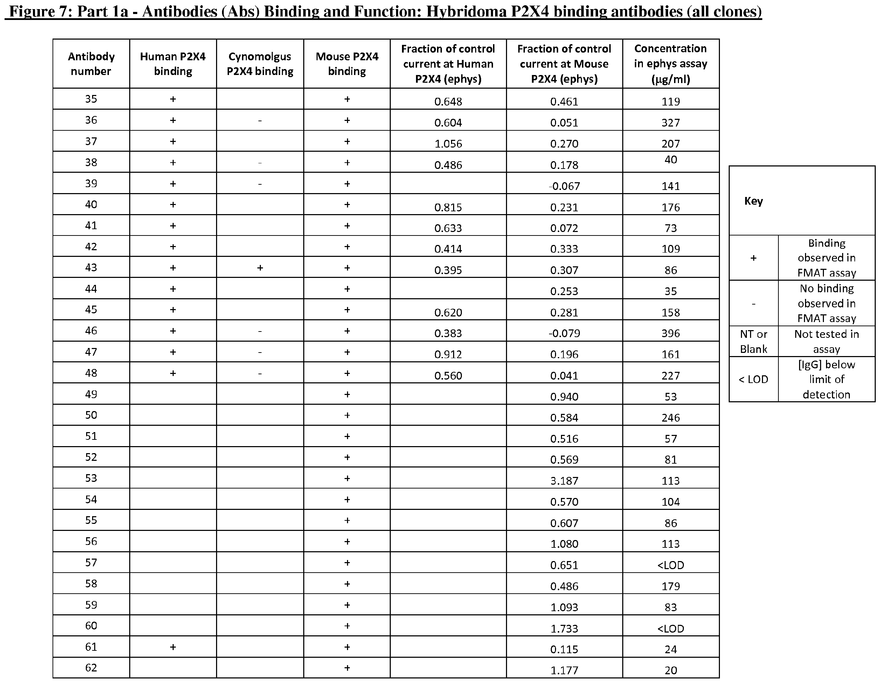

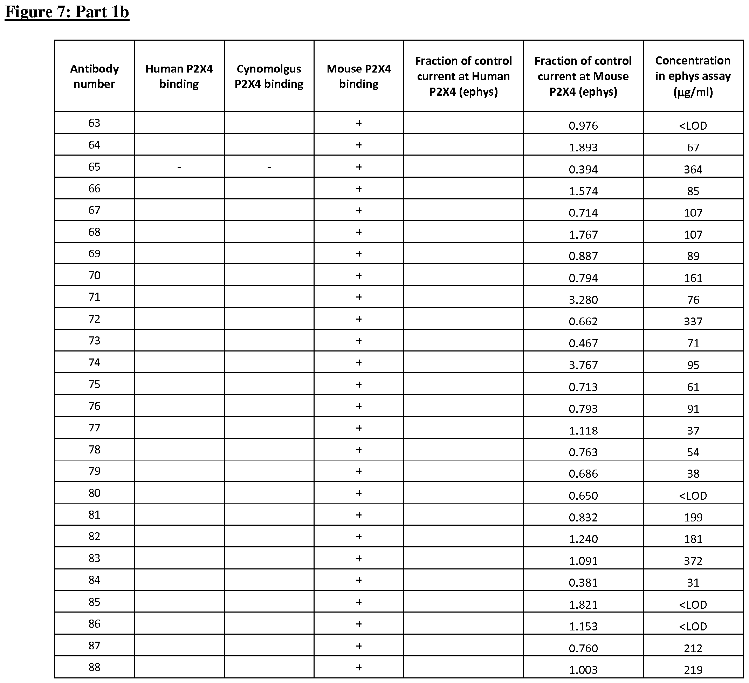

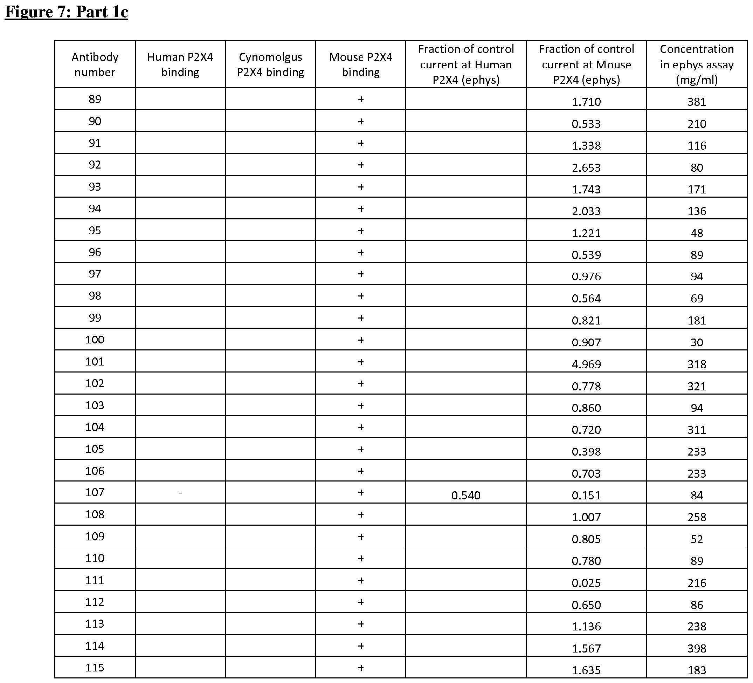

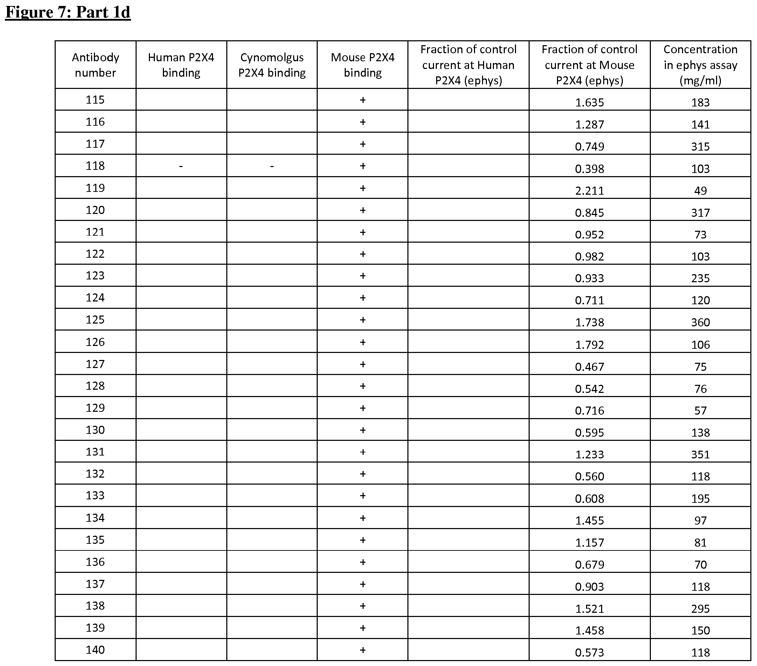

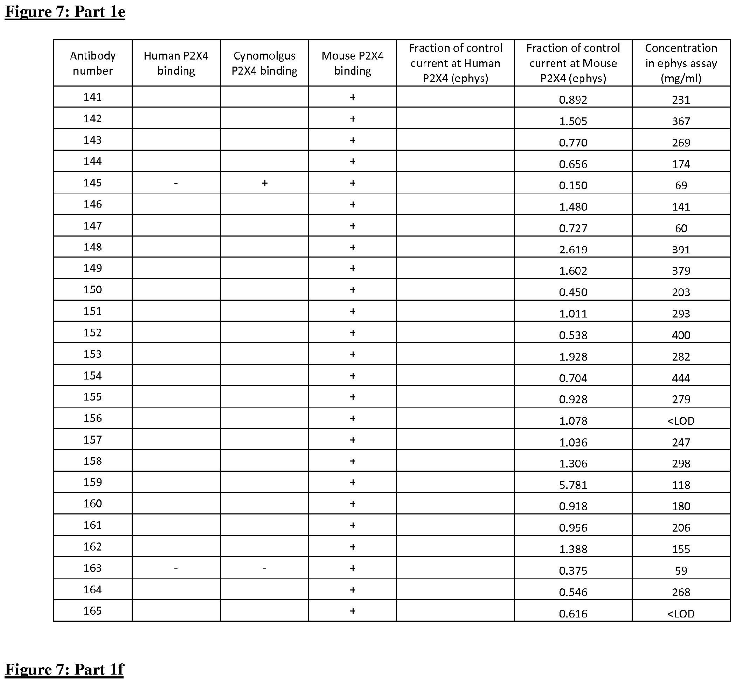

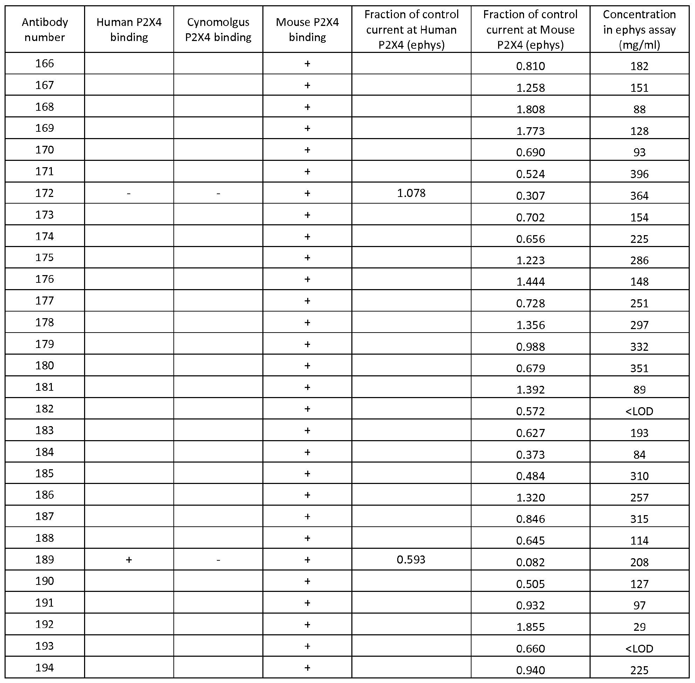

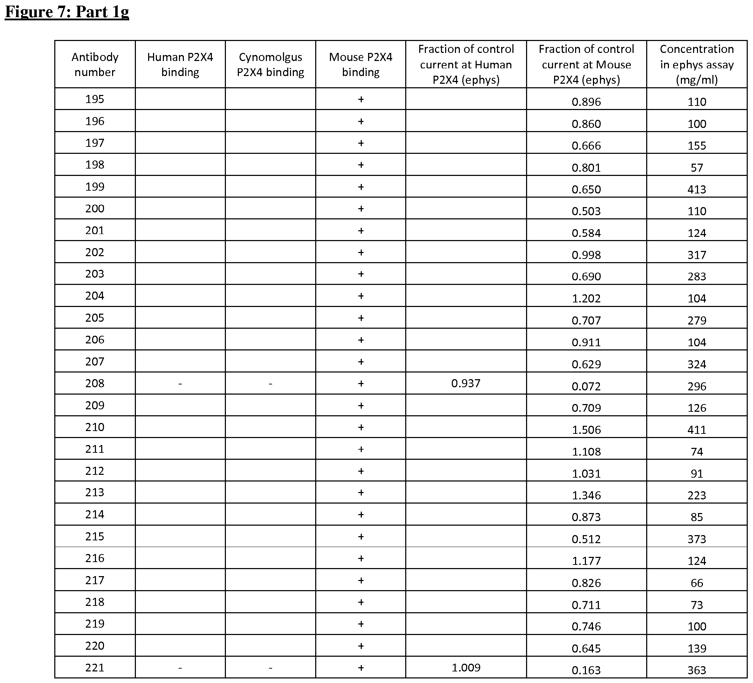

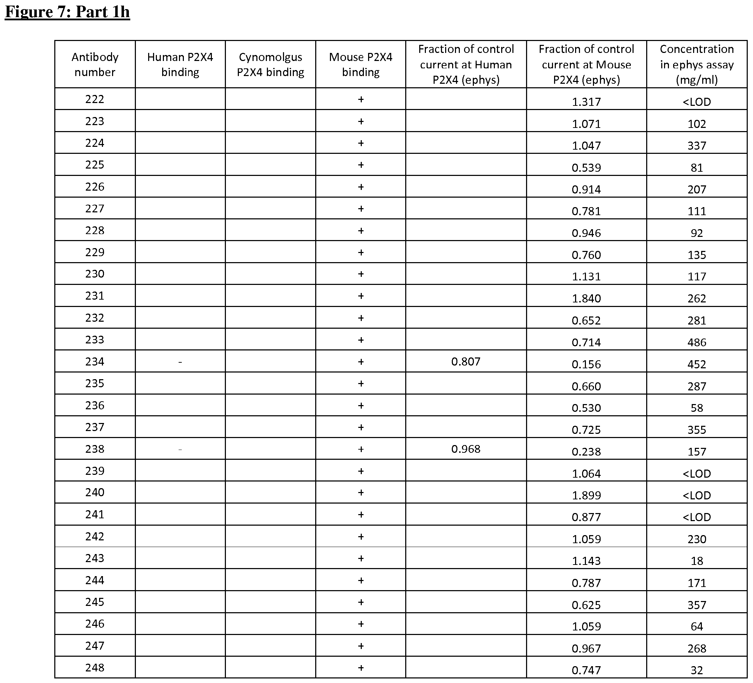

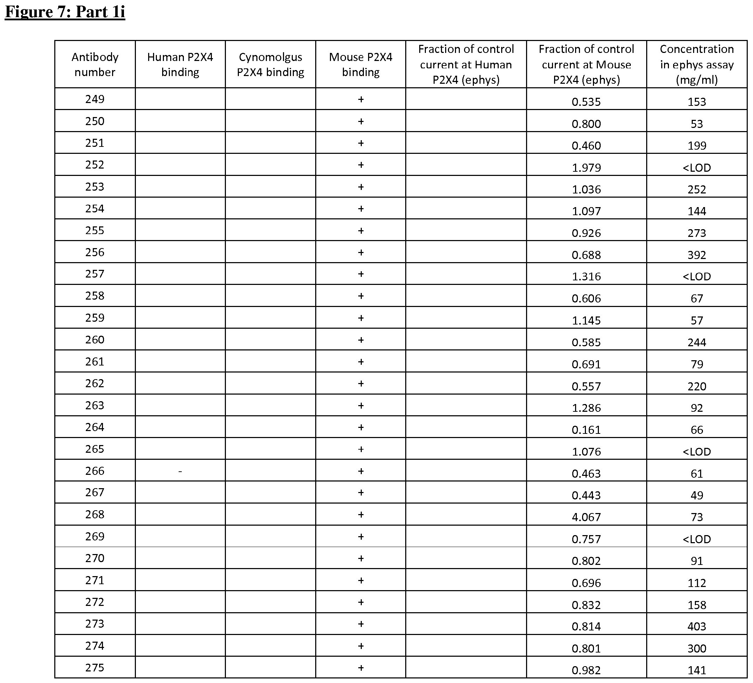

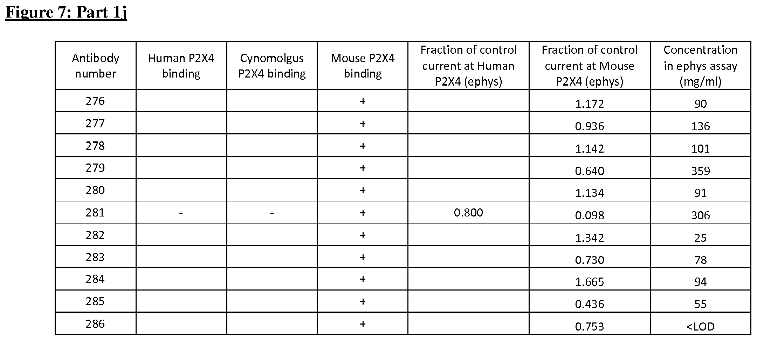

Supernatants generated from the immunisations were screened to identify IgGs with specific binding to mP2X4. Briefly supernatants were diluted 10 fold into assay buffer (HBSS, 0.1% (v/v) BSA, 20mM HEPES and 1U/ml Apyrase) and 5μl added to the assay plate. Anti-rat detection antibody labeled with Alexa Fluor 647 (Jackson Immuno Research labs) was diluted to 6nM and 10μl added to the assay plate. HEK293F cells expressing mP2X4 were diluted to $2.6e^5$/ml and 15μl added to the assay plate. IgG samples were also tested for non-specific binding in parallel by testing the samples for binding to HEK293F cells. IgGs demonstrating specific binding to mP2X4 and no binding to HEK293F cells were identified as hits and selected for antibody purification and analysis by electrophysiology. Results of the electrophysiology screen are provided in Figure 7 together with the binding results for these samples against human and cyno P2X4 expressing cell lines using the same assay described previously

**Example 7: Generation of human P2X4 variants and expression by transient transfection in HEK293F cells**

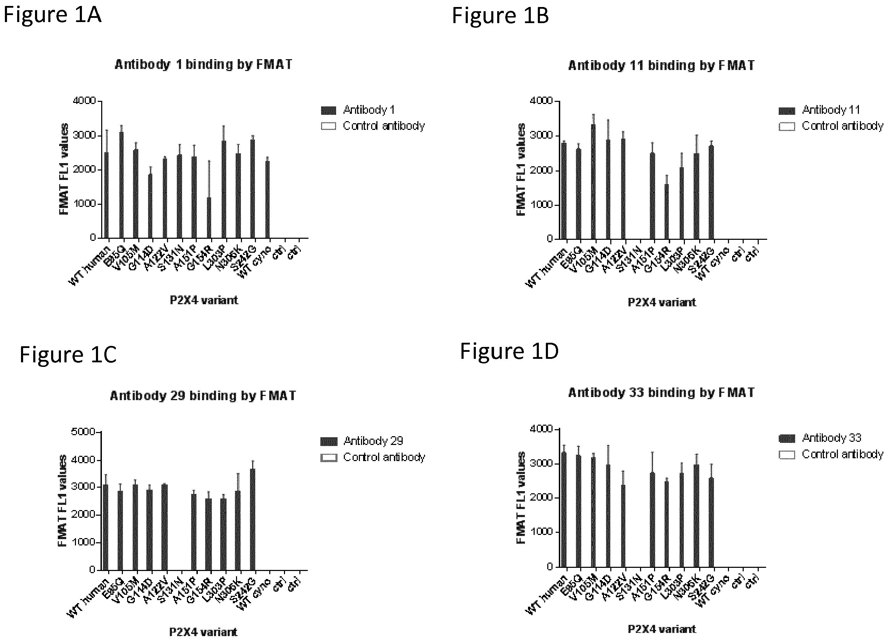

To determine the epitope to which the P2X4 functional antibodies bind the following mutations were generated in human P2X4; E95Q, V105M, G114D, A122V, S131N, A151P, G154R, L303P, N306K. DNA vectors containing huP2X4 sequences with these changes were generated using standard molecular biology techniques. DNA vectors were transfected into HEK293F cells using 293-fectin (Life Technologies 12347019) following the manufacturers guidelines. Cells expressing the huP2X4 variants were incubated with Antibody Nos. 1, 11, 29, and 33 together with the anti-human AlexaFluor 647 (Life Technologies A21445) detection reagent. Binding was measured using the FMAT plate reader. Variant S131N was shown to be important for the binding of Antibody Nos. 11, 29, and 33. Figures 1A-1D show the results of FMAT assays characterizing binding of P2X4 antibodies to HEK293F cells expressing variants of human P2X4.

**Example 8: Electrophysiological characterization of monoclonal antibodies to P2X4.**

***Methods for phage display derived mAbs:- Figures 3* & *4***

HEK 293F cells stably expressing human P2X4, mouse P2X4 or cynomolgus P2X4 were harvested at 50% confluency using accutase. Cells were then resuspended in 10 ml Freestyle 293F media supplemented with HEPES (10 mM) + apyrase (1U/ml, ATPase/ADPase activity = 1) at a density of 2-3e$^6$ cells/ml. P2X4 function was assayed using the automated electrophysiology platform QPatch 16X (Sophion) in population patch configuration. Composition of QPatch extracellular buffer (QEB) was (in mM) NaCl (140), KCl (2), $MgCl_2$ (1) $CaCl_2$ (2), HEPES (10). Final composition of compound plate extracellular buffer (CPEB1) was NaCl (137.6), KCl (2.2), $MgCl_2$ (0.66), $CaCl_2$ (1.3), HEPES (6.6), $KH_2PO_4$ (0.49), $NaH_2PO_4$ (2.66). pH of extracellular buffers was adjusted to 7.4 with NaOH (1 M), osmolarity was adjusted to 300 mOsm with sucrose and the solutions were 0.2 μm filtered. Compound plate extracellular buffer was supplemented with 0.1% bovine serum albumin. The QPatch intracellular buffer contained (in mM) CsF (140), NaCl (10), EGTA (1), HEPES (10). pH of the intracellular buffer was adjusted to 7.3 with CsOH (1 M) and the solution was 0.2 μm filtered. IgGs were titrated to pH 7.4 with NaOH (1 M).

After obtaining whole cell configuration, cells were voltage clamped at -50 mV with 70% series resistance compensation employed. The ligand agonist adenosine 5'-triphosphate disodium salt (ATP, 3 μM) in CPEB1 was applied for 3 seconds every 5 minutes for 20 minutes resulting in 4 control agonist responses. Each agonist response was washed off with CPEB1 + apyrase (1U/ml). 4 additional agonist responses were then measured every 5 minutes in the continued presence of the test IgG or an isotype control IgG (NIP 228). Exemplar traces showing the effect of inhibitory IgGs 5 mins after IgG application can be seen in Figure 8A whereas an example of a potentiating IgG can be seen in Figure 8D. Electrophysiology data presented in Figure 3 and Figure 4 were leak subtracted by subtracting the current in the absence of ligand and the magnitude of the P2X4 response measured as the peak inward current in the presence of ligand. Peak inward current in the presence of IgG+ATP after 5 minutes IgG incubation was expressed as a fraction of the 4$^{th}$ control ATP response. Data were subsequently normalised to a time and concentration matched isotype control antibody response using the equation $I_{norm} = I_{IgG}*(1/I_{isotype})$ where $I_{IgG}$ = fraction of control current for the test IgG and $I_{isotype}$ = fraction of control current for the isotype control IgG. Six IgGs were found to significantly inhibit human P2X4 currents; Antibody Nos. 5, 8, 11, 18, 29, and 33 (Figures 3, 4, 8A). Inhibition of P2X4 currents was rapid, occurring at the first time point following IgG addition, whereas the isotype control IgG NIP 228 had no significant effect. IgGs were subsequently tested for function against mouse and cynomolgus P2X4 (Figures 3, 4) and data reported as a mean of n = 3-4 experiments.

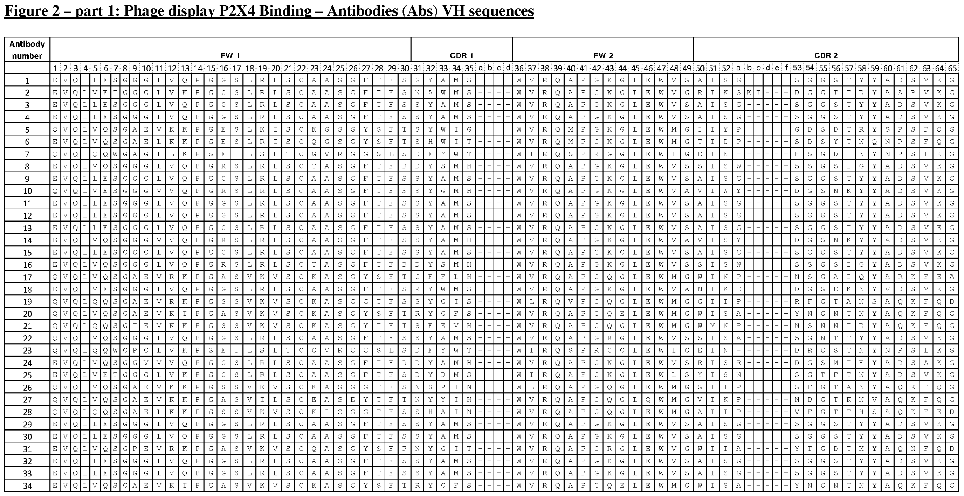

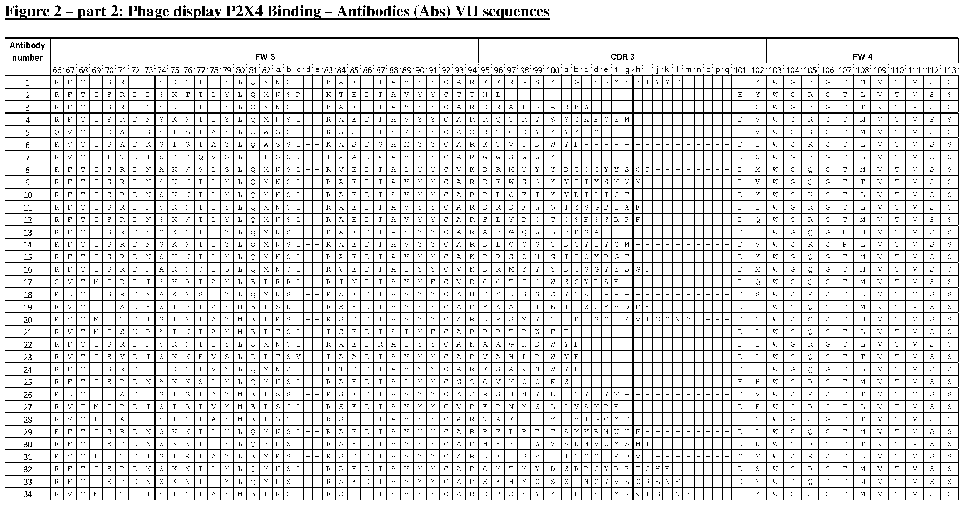

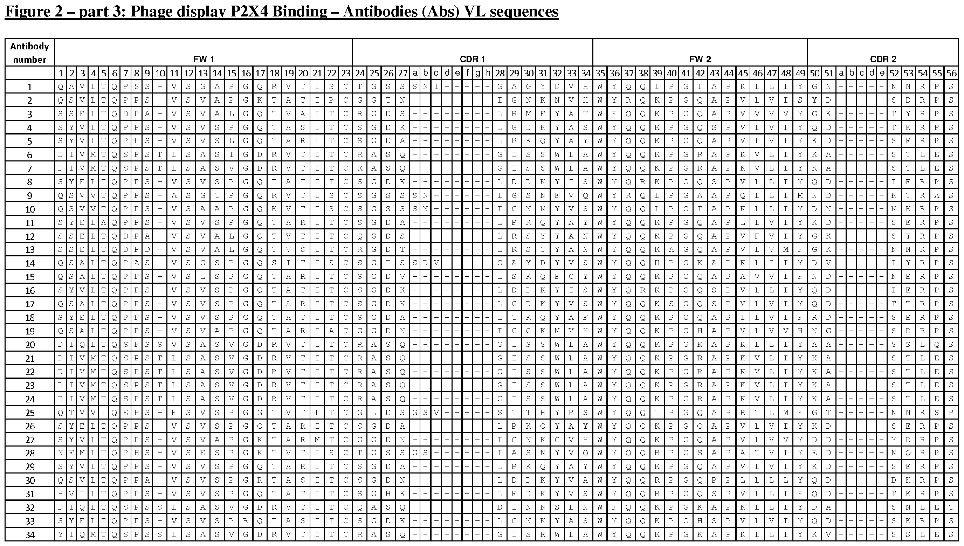

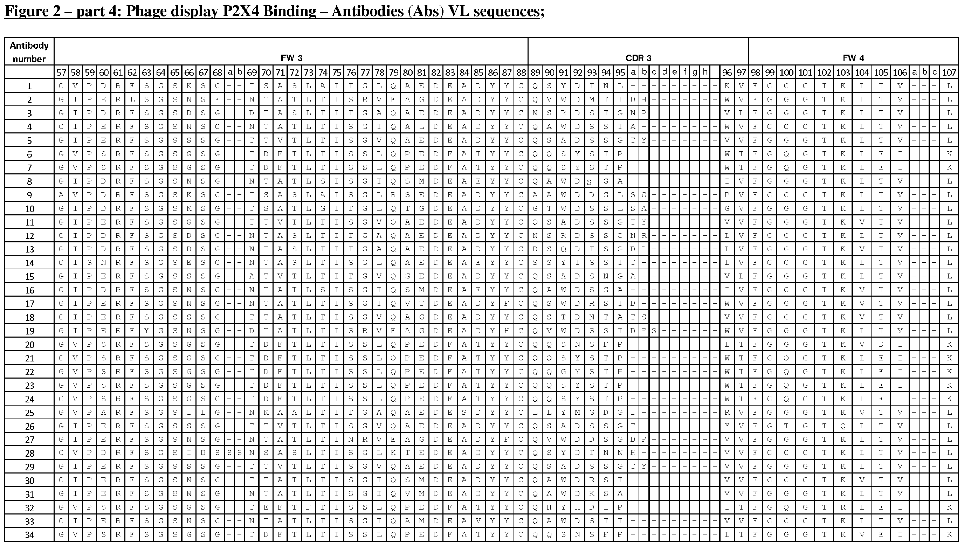

Sequences for phage display antibodies are provided in Figure 2. Results of cross reactivity for phage display antibodies between human, cynomolgus monkey, and mouse are provided in Figure 3. Figure 9 provides a structural analysis of the epitope/paratope interface. Figure 10 provides the sequence of the predicted P2X4 epitope.

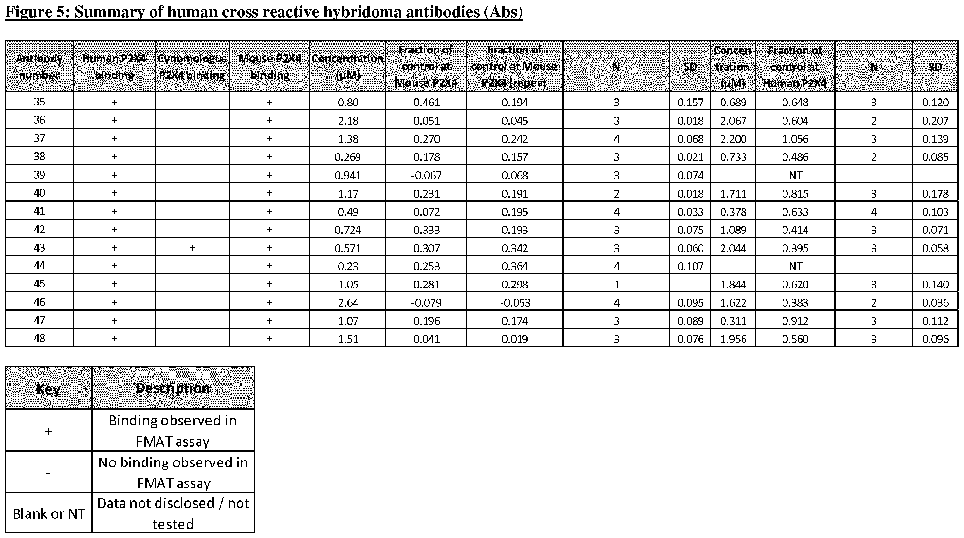

***Methods for hybridoma derived mAbs.- Figures 5, 7 & 8***

HEK 293F cells stably expressing either mouse P2X4 (Uniprot # Q9JJX6) or human P2X4 (Uniprot # Q99571) were harvested at 50% confluency using accutase. Cells were then resuspended in 10 ml Freestyle 293F media supplemented with HEPES (10 mM) + apyrase (1U/ml, ATPase/ADPase activity = 1) at a density of 2-3e$^6$ cells/ml. P2X4 function was assayed using the automated electrophysiology platform QPatch 16X (Sophion) in population patch configuration. Composition of QPatch extracellular buffer (QEB) was (in mM) NaCl (140), KCl (2), $MgCl_2$ (1) $CaCl_2$ (2), HEPES (10). Final composition of compound plate extracellular buffer (CPEB2) was NaCl (115.5), KCl (1.3), $MgCl_2$ (0.66), $CaCl_2$ (1.32), HEPES (56.1). pH of extracellular buffers was adjusted to 7.4 with NaOH (1 M) and the solutions were 0.2 μm filtered. The QPatch intracellular buffer contained (in mM) CsF (140), NaCl (10), EGTA (1), HEPES (10). pH of the intracellular buffer was adjusted to 7.3 with CsOH (1 M) and the solution was 0.2 μm filtered. IgGs were titrated to pH 7.4 with NaOH (1 M). After obtaining whole cell configuration, cells were voltage clamped at -50 mV with 70% series resistance compensation employed. The ligand agonist adenosine 5'-triphosphate disodium salt (ATP) (6 μM for mouse P2X4, 3 μM for human P2X4) in QEB was applied for 3 seconds then washed off with QEB + apyrase (1U/ml). CPEB2 + IgG was then incubated for 3 minutes followed by a second ATP addition. Data were leak subtracted by subtracting the current in the absence of ligand and the magnitude of the P2X4 response measured as the peak inward current in the presence of ligand. The ATP response after IgG addition was expressed as a fraction of the ATP response prior to IgG addition. The hIgG1 NIP 228 TM was used as a control antibody to determine the cutoff for defining functional antibodies. IgGs were initially screened in duplicate (Figure 5, First screen and Figure 7) at mP2X4 and expressed as mean of n = 1-2 experiments. The control antibody NIP 228 TM had a fraction of control current of 1.08 +/- 0.27 (Mean +/- S.D), n = 49. From these data the cutoff for defining functional inhibitory antibodies was set at <0.5 (>~ 2 standard deviations from the mean). Functional antibodies from the first screen were repeated with a larger sample set (n = 3-4) and data reported as mean +/- SD (Figure 5).

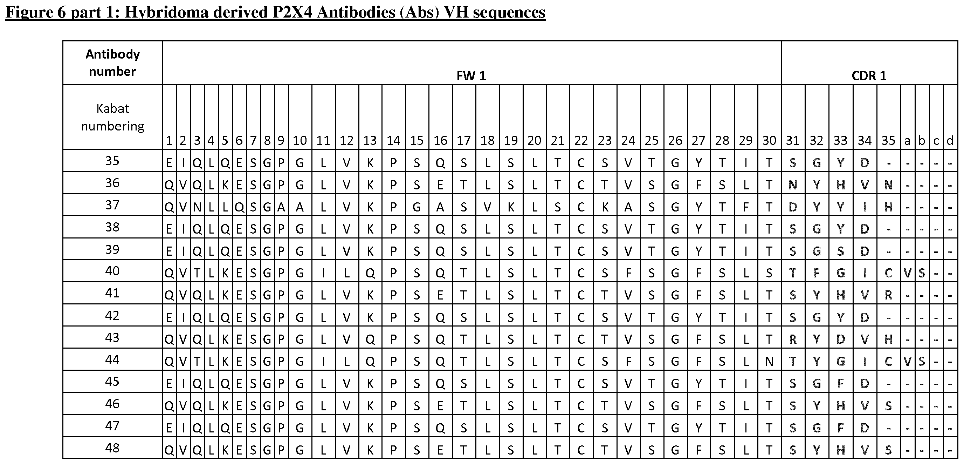

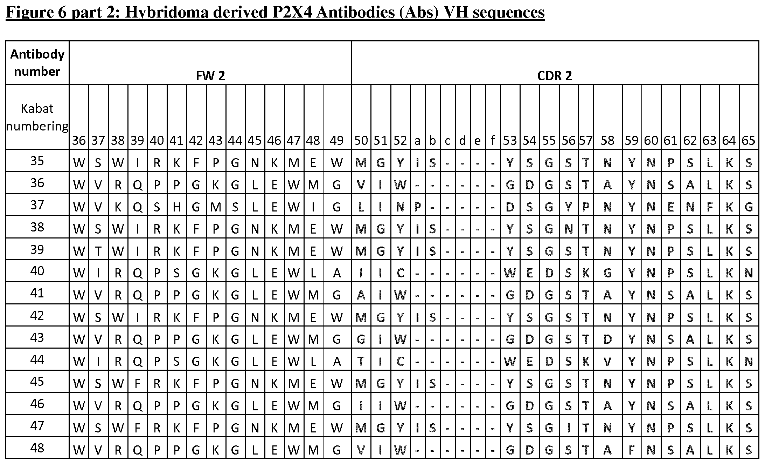

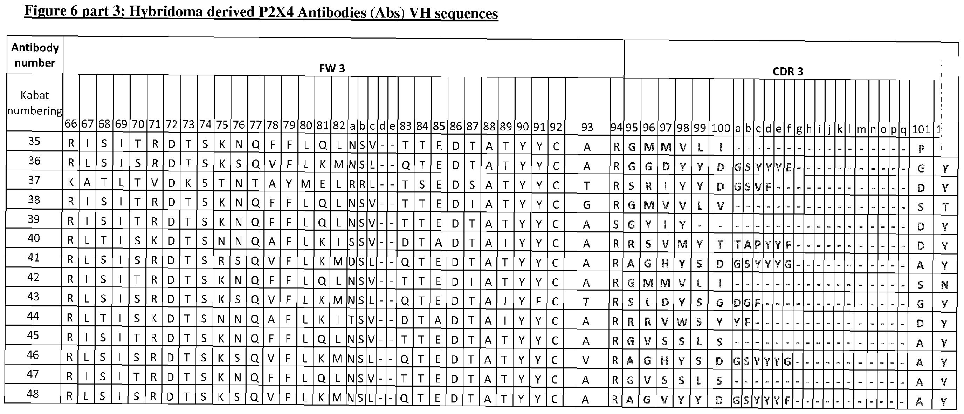

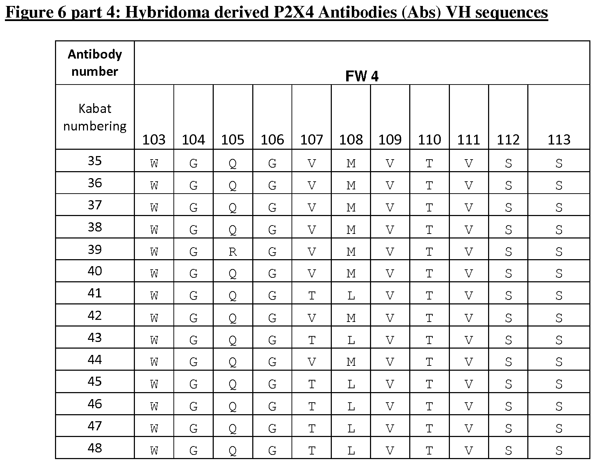

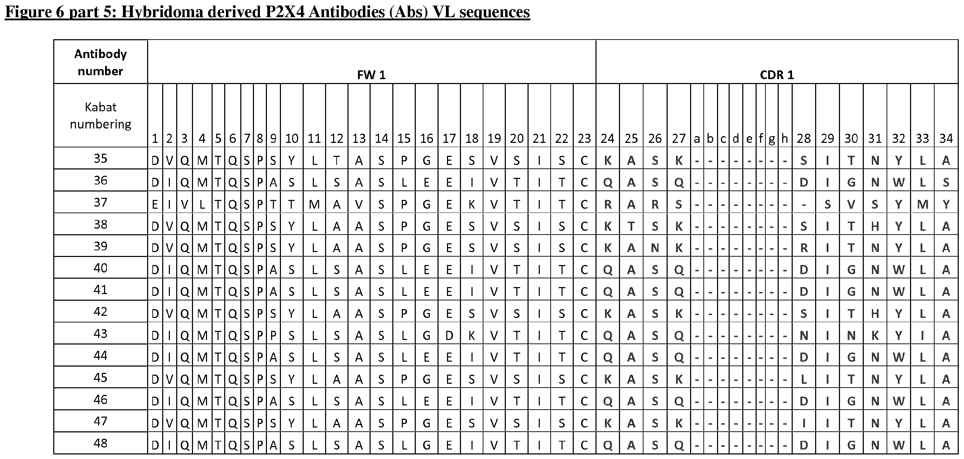

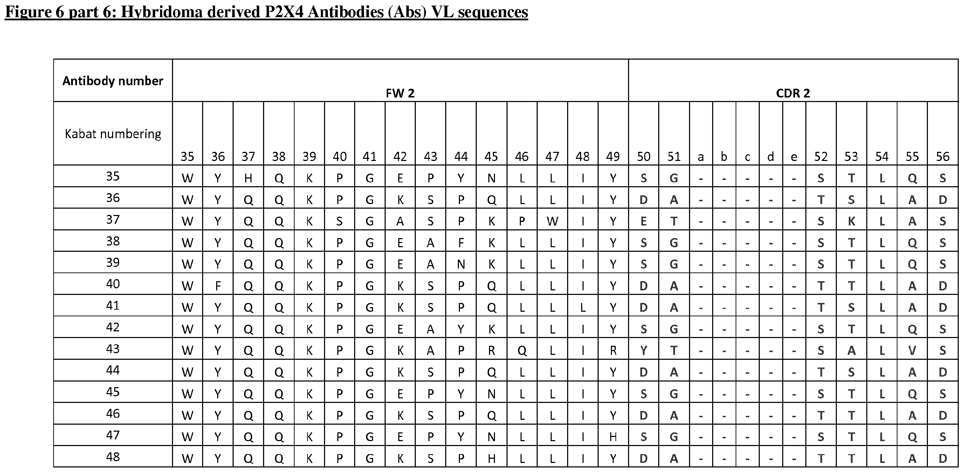

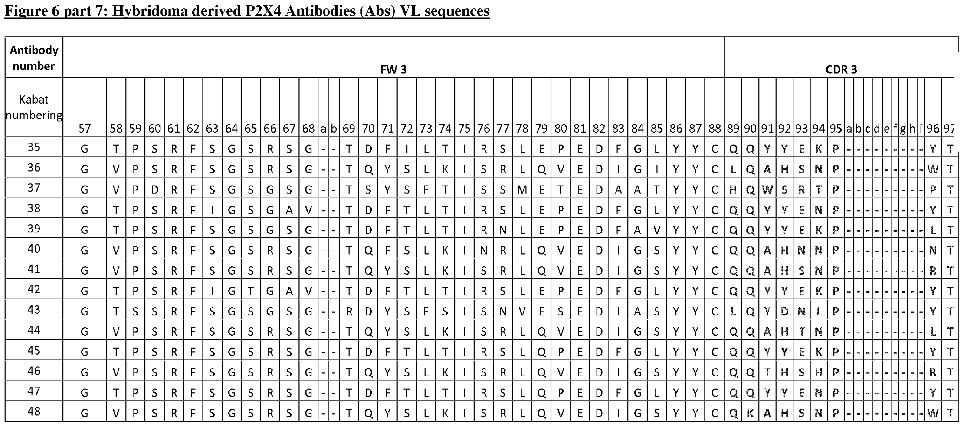

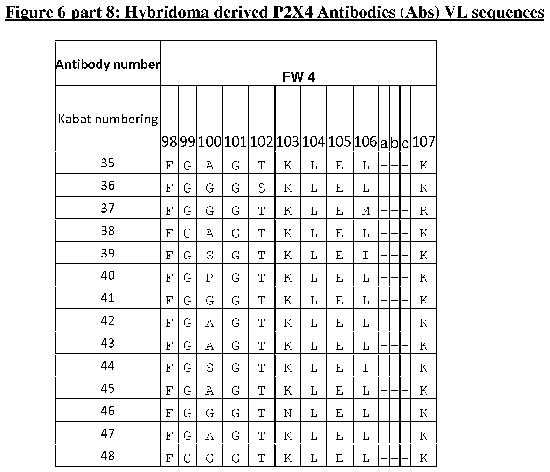

Results of cross reactivity for hybridoma antibodies between human and mouse are provided at Figures 5 and 7. Sequences for hybridoma antibodies are provided at Figures 6 and 13.

**Example 9: *In vivo* testing of monoclonal antibodies to P2X4 in Seltzer model of neuropathic pain**

50 female C57BL/6 mice were used for the studies. All mice underwent insertion of transponders for identification purposes at least 5 days before the start of the study. Mechanical hyperalgesia was determined using an analgysemeter (Randall & Selitto 1957) (Ugo Basile). An increasing force was applied to the dorsal surface of each hind paw in turn until a withdrawal response was observed. The application of force was halted at this point and the weight in grams recorded. Data was expressed as withdrawal threshold in grams for ipsilateral and contralateral paws. Following the establishment of baseline readings mice were divided into 2 groups with approximately equal ipsilateral/contralateral ratios which underwent surgery to partially ligate the sciatic nerve or served as sham operated controls. Operated mice were anaesthetised with isoflurane. Following this approximately 1cm of the left sciatic nerve was exposed by blunt dissection through an incision at the level of the mid thigh. A suture (9/0 Virgin Silk: Ethicon) was then passed through the dorsal third of the nerve and tied tightly. The incision was then closed using glue and the mice were allowed to recover for at least six days prior to commencement of testing. Sham operated mice underwent the same protocol but following exposure of the nerve the mice were sutured and allowed to recover.

Mice were tested for onset of hyperalgesia on days 7 and 10 post surgery. Any mice showing an ipsilateral/contralateral ratio of greater than 80% were classed as non-responders and removed from the study. Following testing on day 10 mice were further sub-divided into groups giving the final treatment groups;

A. Group 1: Sham operated + NIP 228 TM 5μg per mouse intra-thecal (N=10)
B. Group 2: Nerve ligated + NIP 228 TM 5μg per mouse intra-thecal (N=10)
C. Group 3: Nerve ligated + Antibody No. 208 5μg per mouse intra-thecal (N=10)
D. Group 4: Nerve ligated + Antibody No. 38 5μg per mouse intra-thecal (N=10)

Mice were administered NIP 228 TM (Isotype control) or test molecules on day 13 and were retested for changes in mechanical hyperalgesia at 4hrs post dose and also on 1, 2, 4 and 7 days post dose. For dosing mice were anaesthetised with isoflurane. Intra-thecal administration was carried out manually into the L4-L6 area of the spinal cordNIP 228 TM and all test compounds were supplied as 1.02mg/ml = solutions = 1μg per μl = 5μg per mouse.
Ipsilateral and contralateral readings were taken for each animal at each test time and were entered into EXCEL for calculation of ipsilateral/contralateral ratios. Summary data was transferred into PRISM for graphical and statistical analysis. Results were analysed using 2-way ANOVA. Pairwise comparisons where appropriate were made using Tukey's test.

Analysis of the results showed that partial ligation of the sciatic nerve caused a mechanical hyperalgesia which manifested as a significant reduction in the ipsilateral/contralateral ratio on day 7 and 10 when compared to sham operated controls. Following treatment with NIP228, operated mice did not show any change in the level of mechanical hyperalgesia from pre-dose levels indicating a lack of effect of the isotype control on mechanical hyperalgesia. The administration of Antibody No. 208 produced a significant reversal which was significant for up to 4 days post dose after which the response returned to baseline levels. Similar effects were seen with Antibody No. 38 (Fig13).

**Example 10: Generation of mouse anti-human P2X4 antibodies by hybridoma technology**

**Methods for mouse anti-human P2X4 antibody generation were carried out in the same way as described in the previous section of rat anti-murine P2X4 antibody generation, other than the following differences:**

***Immunisations***

Human P2X4 (hP2X4) transfected HEK 293F and XS63 cells were used to immunise CD1 mice in three groups. In group 1, mice were immunised with hP2X4 transfected HEK 293F cells, group 2 mice were immunised with hP2X4 transfected XS63 cells, and group 3 mice were immunised by alternating hP2X4 transfected XS63 cells and hP2X4 transfected HEK 293F cells.

hP2X4 transfected cells were re-suspended at 1E8/mL and emulsified with equal volumes of complete Freund's adjuvant, and injected into mice at two sites, 100 μL per site. For the subsequent 3 injections, the same number of cells was emulsified in Freund's incomplete adjuvant and injections were performed as above. The last boost was carried out on day 24, injecting 200 μL of transfected cells at 1E8/mL intraperitoneally.

***Assessment of mouse immune response to hP2X4 using a cell-based DELFIA***

The serum IgG titres to hP2X4 were determined by a cell-based time-resolved fluorescence assays (DELFIA) using parental HEK 293F cells and hP2X4 transfected HEK 293F cells.

***Monoclonal mouse IgG isolation***

Lymphoid cells isolated from spleens and lymph nodes were fused with SP2/0 myeloma cells using an electrofusion method. The fusions were plated out into semi-solid selection media containing FITC conjugated goat anti-mouse IgG.

***Cell binding assay for mouse IgGs***

Supernatants were initially screened for IgGs that specifically bound to hP2X4 using both the hP2X4 expressing HEK 293F and XS63 cells, and parental HEK 293F cells. The IgGs that showed specific binding to hP2X4, and no binding to parental HEK293F cells, were selected for further specificity testing on mouse P2X4 (mP2X4) HEK 293F cells. IgGs which specifically bound to hP2X4 or to both hP2X4 and mP2X4 were selected for antibody purification and functional analysis by electrophysiology.

***DNA sequencing and purification of mouse IgGs***

Messenger RNA (mRNA) was extracted from hybridoma cells using magnetic oligo (dT) particles and reverse transcribed into cDNA. Polymerase chain reaction (PCR) amplification was performed using poly-C and constant region VH or VL primers specific to all mouse IgG subclasses.

Mouse IgGs of all subclasses (IgG1, IgG2a, IgG2b and IgG3) were purified from overgrown cell culture supernatants on ProPlus resin (Phynexus).

***Functional screening by electrophysiology***

HEK 293F cells stably expressing human P2X4 (Uniprot # Q99571) were harvested at 50% confluency using accutase. Cells were then resuspended in 10 ml Freestyle 293F media supplemented with HEPES (10 mM) + apyrase (1U/ml, ATPase/ADPase activity = 1) at a density of 2-3$e^6$ cells/ml. P2X4 function was assayed using the automated electrophysiology platform QPatch 16X (Sophion) in population patch configuration. Composition of QPatch extracellular buffer (QEB) was (in mM) NaCl (140), KCl (2), $MgCl_2$ (1) $CaCl_2$ (2), HEPES (10).

Final composition of compound plate extracellular buffer (CPEB2) was NaCl (115.5), KCl (1.3), $MgCl_2$ (0.66), $CaCl_2$ (1.32), HEPES (56.1). pH of extracellular buffers was adjusted to 7.4 with NaOH (1 M) and the solutions were 0.2 $\mu$m filtered. The QPatch intracellular buffer contained (in mM) CsF (140), NaCl (10), EGTA (1), HEPES (10). pH of the intracellular buffer was adjusted to 7.3 with CsOH (1 M) and the solution was 0.2 $\mu$m filtered. IgGs were titrated to pH 7.4 with NaOH (1 M). After obtaining whole cell configuration, cells were voltage clamped at - 50 mV with 70% series resistance compensation employed. The ligand agonist adenosine 5'-triphosphate disodium salt (ATP, 3 $\mu$M) in QEB was applied for 3 seconds then washed off with QEB + apyrase (1U/ml). CPEB2 + IgG was then incubated for 3 minutes followed by a second ATP addition. Data were leak subtracted by subtracting the current in the absence of ligand and the magnitude of the P2X4 response measured as the peak inward current in the presence of ligand. The ATP response after IgG addition was expressed as a fraction of the ATP response prior to IgG addition. The hIgG1 NIP 228 TM was used as a control antibody to determine the cutoff for defining functional antibodies. Results are provided at Figure 23. Antibody sequences are provided in Figure 13.

**Example 11: Affinity maturation of Antibody 11**

Antibody No. 11 was optimised for affinity via two approaches either; targeted or random mutagenesis followed by affinity-based phage display selections. In the targeted approach, large scFv-phage libraries derived from the lead clone were created by oligonucleotide-directed mutagenesis of the variable heavy (VH) complementarity determining regions 3 (CDR3) and light (VL) chain CDR3 using standard molecular biology techniques as described (Clackson, T. and Lowman, H.B. Phage Display - A Practical Approach, 2004. Oxford University Press*).* The libraries were subjected to affinity-based phage display selections in order to select variants with higher affinity for human P2X4. The selections were performed essentially as described previously in Example 3 with the exception of lowering the concentration of immobilised human P2X4 over four rounds of selection (10$\mu$g/ml-1.25$\mu$g/ml). Antibodies with improved affinity were identified in a competition assay based on Antibody 11 binding to huP2X4 expressing cells (described in Example 12). To generate further affinity improvement, CDR mutations from improved antibodies were recombined into new scFvs using standard molecular biology techniques.

Antibody 11 was also optimised using a random mutagenesis approach to identify key residues within the entire variable domain that may improve binding to human P2X4. Such a technique is described by Gram *et al.* [Gram et al., 1992, Proc. Natl. Acad. Sci., USA, 89:3576-3580], who used error-prone PCR. In some embodiments one or two amino acid substitutions are made within an entire variable domain or set of CDRs. The generated library was subjected to affinity-based selections as described for the targeted selections outlined above.

Exemplary antibodies from this selection method are disclosed herein as Antibodies 287 to 315, and an alignment of their sequences is shown in Figure 12.

**Example 12: Identification of higher affinity antibodies against human P2X4**

Phage display selection outputs described in example 11, were screened for activity in a competition assay based on Antibody 11 binding to huP2X4 expressing cells. Briefly Antibody 11 IgG was labelled with DyLight ® 650 using a Lightning-Link® Rapid DyLight® 650 conjugation kit following the manufacturer's instructions (Innova Biosciences Ltd). Bacterially expressed scFv were collected into 0.2M HEPES buffer pH7.4, 0.5 mM EDTA and 0.5 M sucrose as peri plasmic extracts and added to the assay plate (Corning ® 3655) together with assay buffer (HBSS, 0.1% BSA, 1U/ml apyrase, either with or without 20mM HEPES). Antibody 11-Dylight® 650 was added to each well except the wells used to define the background binding, to a final concentration of 2nM. HEK293F huP2X4 cells were added to each well at a final density of approximately 2000 cells per well. Plates were covered and incubated at room temperature for 2 to 3 hours before reading on a mirrorball® plate reader (TTP Labtech, Ltd) and determining the total FL3 fluorescence per well (Median (mean intensity) fluorescence multiplied by the number of objects). Individual events were gated on size and fluorescence and a minimum object number of greater than 25 was used to determine wells with sufficient events to report a FL3 total value. % specific binding was calculated for each well using the following equation, maximal FL3 total values were defined from wells that did not receive any scFv but did receive peri plasmic sample buffer:

$$\%\, specific\ binding = \frac{sample\ FL3\ total\ - background\ FL3\ total}{maximal\ FL3\ total - background\ FL3\ total} X\ 100$$

Samples where the binding signal was lower than 85% specific binding were selected for sequencing and sequence unique hits were generated as purified scFv.

To confirm the inhibition of these scFv antibodies, purified scFv antibodies were diluted in assay buffer described above to generate a dilution series and the diluted samples were added to the assay plate before the addition of Antibody 11- DyLight® 650 to a final concentration of 2nM, followed by approximately 2000 HEK293F huP2X4 cells per well. Plates were incubated at room temperature for 2 to 3 hours before being read on the mirrorball® plate reader. Data was analysed as described above and scFv clones showing inhibition were generated as full IgG antibodies.

**Example 13: Identification of antibodies with improved potency against human P2X4 using the human P2X4 1321N1 cell line FLIPR® assay**

Antibodies identified in the Antibody 11 competition assay described in example 12 were generated as purified IgG and titrated to generate a dilution series. These antibodies were diluted in assay buffer containing HBSS and 0.1% BSA and pre-incubated with 1321N1 cells expressing huP2X4 for 30mins where the cells had previously been loaded with Fluo-4 NW calcium dye (Molecular Probes™, Life Technologies) following the manufacturer's instructions. P2X4 was activated by the addition of 1μM ATP diluted in assay buffer and the resulting rise in intracellular calcium was detected by the calcium dye and measured by an increase in fluorescence using the FLIPR® Tetra plate reader (Molecular Devices, LLC). Data was calculated to determine the maximum fluorescence observed over the background fluorescence for the duration of the assay. These data were then analysed to determine % maximal response over the buffer response alone seen in wells where ATP was omitted, using the following equation:

$$\%\, maximal\ response = \frac{sample\ response - buffer\ response}{total\ response - buffer\ response} X\ 100$$

Data was analysed in Prism (GraphPad Software, Inc) to determine $IC_{50}$ values using the following equation:

```
Y = Bottom + (Top-Bottom)/(1+10^((LogIC50-X)*HillSlope))
```

To enable ranking of antibodies the top and bottom of the curves were constrained to 100 and 0 respectively. Geometric means of the $IC_{50}$ values for the antibodies tested are listed in Figure 15 and an example of the $IC_{50}$ curves for two antibodies are shown in Figure 19 together with an isotype control antibody

**Example 14:** Identification of antibodies with improved potency against human P2X4 using the HEK 293F huP2X4 cell line on the automated electrophysiology platform Qpatch 16X.

HEK 293F cells stably expressing human P2X4, were harvested at 50% confluency using accutase. Cells were then resuspended in 10 ml Freestyle 293F media supplemented with HEPES (10 mM) + apyrase (1U/ml, ATPase/ADPase activity = 1) at a density of 2-3e$^6$ cells/ml. P2X4 function was assayed using the automated electrophysiology platform QPatch 16X (Sophion) in population patch configuration. Composition of QPatch extracellular buffer (QEB) was (in mM) NaCl (140), KCl (2), $MgCl_2$ (1) $CaCl_2$ (2), HEPES (10). pH of extracellular buffers was adjusted to 7.4 with NaOH (1 M), osmolarity was adjusted to 300 mOsm with sucrose and the solutions were 0.2 μm filtered. The QPatch intracellular buffer (QIB) contained (in mM) CsF (140), NaCl (10), EGTA (1), HEPES (10). pH of the intracellular buffer was adjusted to 7.3 with CsOH (1 M) and the solution was 0.2 μm filtered. IgGs were titrated to pH 7.4 with NaOH (1 M).

For determination of the potency of optimized variants of Antibody 11, IgGs were serially diluted in QEB + 0.1% bovine serum albumin and tested for function on Qpatch 16X in population patch configuration. Extracellular buffer was QEB, intracellular buffer was QIB and ATP wash buffer was QEB + apyrase (1U/ml). In this assay, ATP (3 μM) was applied every 10 mins for 3 s with a total of 5 applications per experiment. The first two ATP additions (ATP 1 & ATP 2) were preceded by preincubation for 5 mins with QEB buffer + 0.1 % BSA whereas the following three ATP additions were preceded by 5 mins incubation with ascending doses of IgG. Log and half log doses of IgG were interleaved in post analysis to generate 6 point dose response curves (dose range 100 - 0.3 nM). Data were leak subtracted by subtracting the current in the absence of ligand and the magnitude of the P2X4 response measured as the peak inward current in the presence of ligand. The peak inward current in response to ATP was expressed as fraction of control current (ATP2) and labeled as $I/I_{basal}$. Data were fit in Prism using a log (inhibitor) vs. response - Variable slope (four parameters) equation. Y=Bottom + (Top-Bottom)/(1+10^((LogIC50-X)*HillSlope)). The top of the IgG dose response curves was defined by the response to 0.3 nM NIP 228 and constrained to this value. The bottom of the curve was constrained such that it was greater than zero. See Figure 14-15.

**Example 15** Potency determination of hybridoma derived antibodies at mouse and human P2X4. HEK 293F cells expressing P2X4 were handled as in example 8. Potency of hybridoma derived IgGs was assayed on Qpatch 16X in population patch configuration. For determination of IgG potency, IgGs were serially diluted in PBS + 0.1% bovine serum albumin and tested for function on Qpatch 16X in population patch configuration. IgGs were then diluted 1:3 in QEB + 0.1% BSA resulting in a final buffer composition of NaCl (137.6), KCl (2.2), $MgCl_2$ (0.66), $CaCl_2$ (1.3), HEPES (6.6), $KH_2PO_4$ (0.49), $NaH_2PO_4$ (2.66), BSA (0.1%) equivalent to CPEB1. Extracellular buffer was QEB, intracellular buffer was QIB and ATP wash buffer was QEB + apyrase (1U/ml). In this assay, ATP (3 μM) was applied every 10 mins for 3 s with a total of 5 applications per experiment. The first two ATP additions (ATP 1 & ATP 2) were preceded by preincubation for 5 mins with CPEB1 + 0.1 % BSA whereas the following three ATP additions were preceded by 5 mins incubation with ascending doses of IgG. Log and half log doses were interleaved in post analysis to generate 6 point dose response curves. Data were leak subtracted by subtracting the current in the absence of ligand and the magnitude of the P2X4 response measured as the peak inward current in the presence of ligand. The peak inward current in response to ATP was expressed as fraction of control current (ATP2). Data were fit in Prism using a log (inhibitor) vs. response - Variable slope (four parameters) equation. Y=Bottom + (Top-Bottom)/(1+10^((LogIC50-X)*HillSlope)). The top of the IgG dose response curves was constrained to 1 whereas the bottom of the curve was constrained such that it was greater than zero. See Figure 16.

**Example 16 Efficacy of mouse reactive antibodies at native mouse microglial P2X4**

Culture of mouse microglia:

Primary mouse microglia were cultured from C57 neonatal pups, P2. Brains were removed from the skulls of mice and kept in media (DMEM + 10% FCS + pen/strep). They were then rolled across filter paper to remove the sticky vasculature and meninges before placing in 20 ml fresh media and triturating to give a single cell suspension. Cells were then filter sterilised through a 40 μm cell strainer then centrifuged at 1200rpm for 5 min. Cells were then resuspended in 40ml media per flask at 4 brains per T175 flask and cultured for 1 week. After this, the media was supplemented with GM-CSF (5 ng/ml) and the cells cultured for a further week. Microglia were removed by shaking overnight in an orbital shaker incubator (no $CO_2$) with HEPES supplemented in the media (20 mM). Purified microglia were centrifuged at 1200 rpm for 5 mins and resuspended in 20 ml DMEM + 10% FCS + pen/strep growth media. Cells were counted and seeded in ultra low bind T75 cell culture flasks (Corning) at 7e6 cells/flask. Microglia were then maintained in culture for 1-7 days before being used for Qpatch 16X electrophysiology assays or FLIPR calcium imaging assays.

Cell handling Qpatch:

1 x T75 flask was washed twice with dPBS and cells were harvested using accutase treatment for 5-10 mins.

Cells were then resuspended in 293F Freestyle media + 20 mM HEPES + 1U/ml accutase (10 ml) and spun down at 800 rpm for 5 mins. Cells were then resuspended in 3 ml 293F Freestyle media + 20 mM HEPES + 1U/ml accutase and 1 ml of cell suspension used per experiment.

Qpatch 16X was used in population patch configuration and cells voltage clamped at -70 mV. Cells were perfused with either a control antibody or test antibody for 5 minutes before ATP (30 μM) was applied. Current in the absence of ATP was subtracted from all data. Inward current in response to ATP was measured (see Figure 15). External buffer was QEB and internal buffer was QIB. See Figure 17 & 18.

FLIPR:

Microglia were plated in Cell Coat Poly-D-Lysine coated 384 well plates (black, uclear) with 30 μl per well and cultured in a humidified incubator at 37 °C for 48 hours.

Media was removed and replaced with 20 ul per well of HBSS buffer + 20 mM HEPES + 0.1% BSA, supplemented with Screen Quest™ Fluo-8 No Wash Calcium Assay Kit (AAT Bioquest, Inc.) as per the manufacturers instructions. Cells were then incubated at 37°C for 30 mins then returned to room temperature for 15 mins before assaying on FLIPR (Molecular devices). Ivermectin (12 μM) was made up in a further 384 well compound plate (Compound plate 1). IgGs were made up in PBS + 0.1 % BSA (compound plate 2). ATP (30 uM) was made up in HBSS + 20 mM HEPES + 0.1 % BSA in a separate 384 well compound plate (Compound plate 3 ). Fluo-8 was excited at a wavelength of 470-495 nm and the emitted light measured at a wavelength of 515-575 nm. Camera gain was adjusted to give 1000 counts at rest with an exposure of 0.4 s. 10 ul of solution from compound plate 1 was added to the cells and the fluorescence measured. After 5 mins incubation, 10 ul of solution from compound plate 2 was added. 15 min later, ATP (5 uM final) was added and the peak end fluorescence measured between 200-300 sec post ATP addition. Fluorescence counts were normalised to the ATP response in the absence of antibody (minus background fluorescence) and plotted as % of ATP response (See figure 21). 10 point dose response curves for each IgG were constructed from duplicate wells and the data fit in Prism using a log (inhibitor) vs. response - Variable slope (four parameters) equation. Y=Bottom + (Top-Bottom)/(1+10^((LogIC50-X)*HillSlope)). See Figure 20.

**Example 17: Functional effect of P2X4 antibodies on human monocyte derived macrophages.**

Cell culture

Human monocytes were isolated from the mononuclear fraction of peripheral blood by centrigugation on a Ficoll-Paque gradient. Cells were then purified by incubating in a T175 cell culture flask in cell culture media in the absence of serum for 1 hour. Non-adherent cells were removed and the remaining cells grown in RPMI Glutamax I media supplemented with 10% FCS (HI/GI) + 1% P/S + 100 ng/ml M-CSF for 7 days. Cells were fed on day 2-3 by adding an additional 10 ml of media. Macrophage were harvested by accutase treatment for 10 mins followed by cell scraping and replated in ultra-low bind T75 flasks at 6e6 cells per flask. Cells were then cultured for a further 1-10 days before being used for electrophysiological recording.

On the day of experiment, cells were harvested with accutase and resuspended in 3 ml CHO ACF media + 20 mM HEPES. 1ml of cell suspension was used per experiment on Qpatch 16X in population patch configuration. Qpatch 16X assay parameters were as described for example 16. Nippon antagonist refers to 1H-naphtho[1,2-b][1,4]diazepine-2,4(3H,5H)-dione (described in Patents WO-2010/093061 and EP2397480A1 See Figures 21 & 22.

**Claims**

1. An antibody or antigen binding fragment thereof that specifically binds a human P2X4 polypeptide and modulates channel activity, wherein the antibody or fragment thereof comprises a VH comprising:

   a. a heavy chain variable region CDR1 comprising the sequence: SFAMS;
   b. a heavy chain variable region CDR2 comprising the sequence: AISGSG GSTYYADSVKG;
   c. a heavy chain variable region CDR3 comprising the sequence: QFDYWSTYSGPTAFDL;

   in combination with a VL comprising:

   a. a light chain variable region CDR1 comprising the sequence SGDALPRQYAY
   b. a light chain variable region CDR2 comprising the sequence KDSERPS
   c. a light chain variable region CDR3 comprising the sequence QSADSSGTYVV.

2. The antibody or antigen binding fragment thereof according to claim 1, comprising a VH of sequence:

   EVQLLESGGGLVQPGGSLRLSCAASGFTFSSFAMSWVRQAPGKGLEWVSAISGSGGSTYYADSVKGRFTISRDNSKNTLYLQMNSLRAEDTAVYYCSRQFDYWSTYSGPTAFDLWGRGTLVTVSS;

   and a VL of sequence

   SYELAQPPSVSVSPGQTARITCSGDALPRQYAYWYQQKPGQAPLLVIYKDSERPSGIPERFSGSGSGTTVTLTISGVQAEDEADYYCQSADSSGTYVVFGGGTKVTVL.

3. The antibody of any one of claims 1-2, wherein the antigen binding fragment thereof is a single chain antibody, a single chain variable fragment (scFv), a Fab fragment, or a F(ab')2 fragment.

4. A polynucleotide encoding the antibody or antigen binding fragment thereof of any one of claims 1-3.

5. A vector comprising the polynucleotide of claim 4.

6. A host cell comprising the vector of claim 5.

7. A pharmaceutical composition comprising one or more antibodies or antigen binding fragments thereof according to any one of claims 1 to 3 and a pharmaceutically acceptable excipient.

8. An antibody or antigen binding fragment thereof according to any one of claims 1 to 3 for use in the treatment of neuropathic pain in a mammal.

**Patentansprüche**

1. Antikörper oder antigenbindendes Fragment davon, der bzw. das ein menschliches P2X4-Polypeptid spezifisch bindet und Kanalaktivität moduliert, wobei der Antikörper bzw. das Fragment davon eine VH, die:

   a. eine Schwere-Kette-variable-Region-CDRl umfassend die Sequenz: SFAMS;
   b. eine Schwere-Kette-variable-Region-CDR2 umfassend die Sequenz: AISGSGGSTYYADSVKG;
   c. eine Schwere-Kette-variable-Region-CDR3 umfassend die Sequenz: QFDYWSTYSGPTAFDL;

   umfasst, in Kombination mit einer VL, die:

a. eine Leichte-Kette-variable-Region-CDRl umfassend die Sequenz SGDALPRQYAY
b. eine Leichte-Kette-variable-Region-CDR2 umfassend die Sequenz KDSERPS
c. eine Leichte-Kette-variable-Region-CDR3 umfassend die Sequenz QSADSSGTYVV

umfasst, umfasst.

**2.** Antikörper oder antigenbindendes Fragment davon nach Anspruch 1, umfassend eine VH der Sequenz:

EVQLLESGGGLVQPGGSLRLSCAASGFTFSSFAMSWVRQAPGKGLEWVSAISGSGGSTY
YADSVKGRFTISRDNSKNTLYLQMNSLRAEDTAVYYCSRQFDYWSTYSGPTAFDLWGR
GTLVTVSS;

und eine VL der Sequenz

SYELAQPPSVSVSPGQTARITCSGDALPRQYAYWYQQKPGQAPLLVIYKDSERPSGIPER
FSGSGSGTTVTLTISGVQAEDEADYYCQSADSSGTYVVFGGGTKVTVL.

**3.** Antikörper gemäß einem der Ansprüche 1-2, wobei es sich bei dem antigenbindenden Fragment davon um einen Einzelkette-Antikörper, ein Einzelkettevariables-Fragment (Single Chain Variable Fragment, scFv), ein Fab-Fragment oder ein F(ab')2-Fragment handelt.

**4.** Polynukleotid, codierend den Antikörper bzw. das antigenbindende Fragment davon gemäß einem der Ansprüche 1-3.

**5.** Vektor, umfassend das Polynukleotid gemäß Anspruch 4.

**6.** Wirtszelle, umfassend den Vektor gemäß Anspruch 5.

**7.** Pharmazeutische Zusammensetzung, umfassend einen oder mehrere Antikörper oder antigenbindende Fragmente davon nach einem der Ansprüche 1 bis 3 und einen pharmazeutisch annehmbaren Exzipienten.

**8.** Antikörper oder antigenbindendes Fragment davon nach einem der Ansprüche 1 bis 3 zur Verwendung bei der Behandlung neuropathischer Schmerzen bei einem Säuger.

## Revendications

**1.** Anticorps ou fragment de celui-ci de liaison à l'antigène qui se lie spécifiquement à un polypeptide P2X4 humain et module une activité de canal, l'anticorps ou le fragment de celui-ci comprenant une VH comprenant :

a. une région variable de chaîne lourde CDR1 comprenant la séquence : SFAMS ;
b. une région variable de chaîne lourde CDR2 comprenant la séquence : AISGSGGSTYYADSVKG ;
c. une région variable de chaîne lourde CDR3
comprenant la séquence : QFDYWSTYSGPTAFDL ;

en combinaison avec une VL comprenant :

a. une région variable de chaîne légère CDR1 comprenant la séquence SGDALPRQYAY
b. une région variable de chaîne légère CDR2 comprenant la séquence KDSERPS
c. une région variable de chaîne légère CDR3 comprenant la séquence QSADSSGTYVV.

**2.** Anticorps ou fragment de celui-ci de liaison à l'antigène, selon la revendication 1, comprenant une VH de séquence :

EVQLLESGGGLVQPGGSLRLSCAASGFTFSSFAMSWVRQAPGKGLEWVSAISGSG GSTYYADSVKGRFTISRDNSKNTLYLQMNSLRAEDTAVYYCSRQFDYWSTYSGPT AFDLWGRGTLVTVSS ;

et une VL de séquence :

SYELAQPPSVSVSPGQTARITCSGDALPRQYAYWYQQKPGQAPLLVIYKDSERPS GIPERFSGSGSGTTVTLTISGVQAEDEADYYCQSADSSGTYVVFGGGTKVTVL.

**3.** Anticorps de l'une quelconque des revendications 1-2, dans lequel le fragment de celui-ci de liaison à l'antigène est un anticorps en simple chaîne, un fragment variable en simple chaîne (scFv), un fragment Fab ou un fragment F(ab')2.

**4.** Polynucléotide codant pour l'anticorps ou le fragment de celui-ci de liaison à l'antigène de l'une quelconque des revendications 1-3.

**5.** Vecteur comprenant le polynucléotide de la revendication 4.

**6.** Cellule hôte comprenant le vecteur de la revendication 5.

**7.** Composition pharmaceutique comprenant un ou plusieurs anticorps ou fragment(s) de celui-ci/ceux-ci de liaison à l'antigène, selon l'une quelconque des revendications 1 à 3, et un excipient acceptable d'un point de vue pharmaceutique.

**8.** Anticorps ou fragment de celui-ci de liaison à l'antigène, selon l'une quelconque des revendications 1 à 3, destiné à être utilisé dans le traitement d'une douleur neuropathique chez un mammifère.

Figure 1A

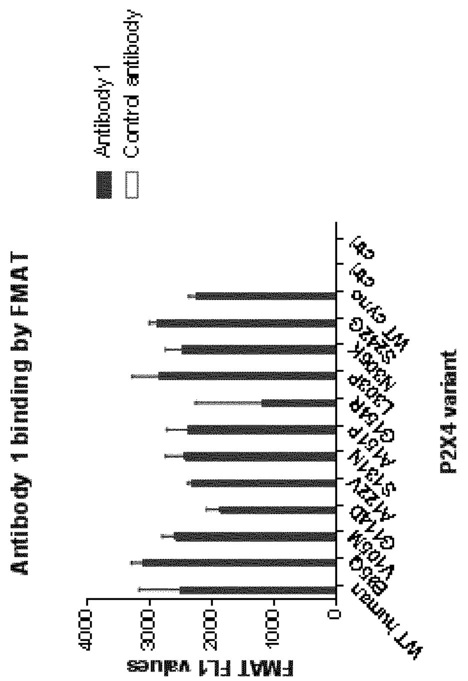


Figure 1B

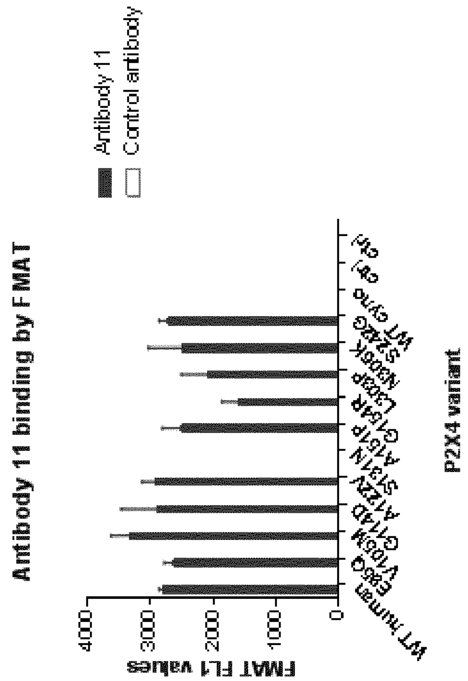


Figure 1C

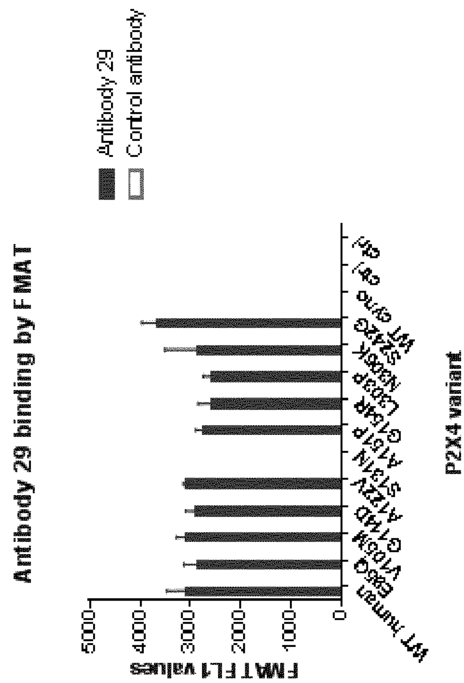


Figure 1D

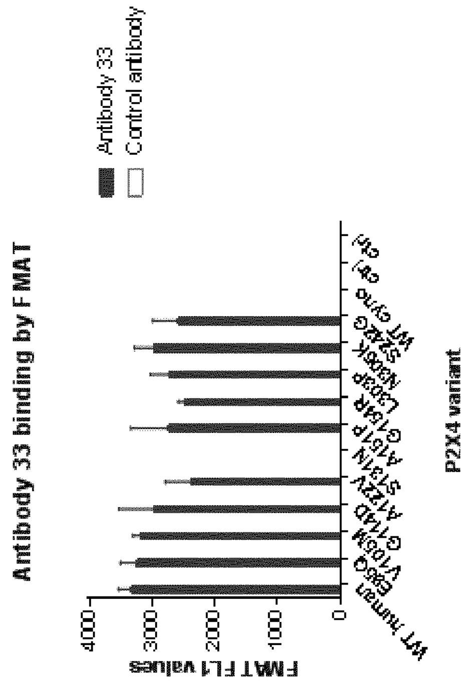

## Figure 2 – part 1: Phage display P2X4 Binding – Antibodies (Abs) VH sequences

| Antibody number | FW 1 | | | | | | | | | | | | | | | | | | | | | | | | | | | | | | CDR 1 | | | | | | | | |
|---|---|---|---|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | a | b | c | d |
| 1 | E | V | Q | L | L | E | S | G | G | G | L | V | Q | P | G | G | S | L | R | L | S | C | A | A | S | G | F | T | F | S | G | Y | A | M | S | - | - | - | - |
| 2 | E | V | Q | L | V | E | T | G | G | G | L | V | K | P | G | G | S | L | R | L | S | C | A | A | S | G | F | T | F | S | N | A | W | M | S | - | - | - | - |
| 3 | E | V | Q | L | L | E | S | G | G | G | L | V | Q | P | G | G | S | L | R | L | S | C | A | A | S | G | F | T | F | S | S | Y | A | M | S | - | - | - | - |
| 4 | E | V | Q | L | L | E | S | G | G | G | L | V | Q | P | G | G | S | L | R | L | S | C | A | A | S | G | F | T | F | S | S | Y | A | M | S | - | - | - | - |
| 5 | Q | V | Q | L | V | Q | S | G | A | E | V | K | K | P | G | E | S | L | K | I | S | C | K | G | S | G | Y | S | F | T | S | Y | W | I | G | - | - | - | - |
| 6 | E | V | Q | L | V | Q | S | G | A | E | L | K | K | P | G | E | S | L | R | I | S | C | Q | G | S | G | Y | S | F | T | S | H | W | I | T | - | - | - | - |
| 7 | Q | V | Q | L | Q | Q | W | G | A | G | L | L | K | P | S | E | T | L | S | L | T | C | G | V | R | G | G | S | L | S | D | F | Y | W | T | - | - | - | - |
| 8 | E | V | Q | L | V | Q | S | G | G | G | L | V | Q | P | G | R | S | L | R | L | S | C | T | A | S | G | F | T | F | D | D | Y | S | M | H | - | - | - | - |
| 9 | E | V | Q | L | L | E | S | G | G | G | L | V | Q | P | G | G | S | L | R | L | S | C | A | A | S | G | F | T | F | S | S | Y | A | M | S | - | - | - | - |
| 10 | Q | V | Q | L | V | E | S | G | G | G | V | V | Q | P | G | R | S | L | R | L | S | C | A | A | S | G | F | T | F | S | S | Y | G | M | H | - | - | - | - |
| 11 | E | V | Q | L | L | E | S | G | G | G | L | V | Q | P | G | G | S | L | R | L | S | C | A | A | S | G | F | T | F | S | S | Y | A | M | S | - | - | - | - |
| 12 | E | V | Q | L | L | E | S | G | G | G | L | V | Q | P | G | G | S | L | R | L | S | C | A | A | S | G | F | T | F | S | S | Y | A | M | S | - | - | - | - |
| 13 | E | V | Q | L | L | E | S | G | G | G | L | V | Q | P | G | G | S | L | R | L | S | C | A | A | S | G | F | T | F | S | S | Y | A | M | S | - | - | - | - |
| 14 | E | V | Q | L | V | Q | S | G | G | G | V | V | Q | P | G | R | S | L | R | L | S | C | A | A | S | G | F | T | F | S | S | Y | A | M | H | - | - | - | - |
| 15 | E | V | Q | L | L | E | S | G | G | G | L | V | Q | P | G | G | S | L | R | L | S | C | A | A | S | G | F | T | F | S | S | Y | A | M | S | - | - | - | - |
| 16 | E | V | Q | L | V | Q | S | G | G | G | L | V | Q | P | G | R | S | L | R | L | S | C | T | A | S | G | F | T | F | D | D | Y | S | M | H | - | - | - | - |
| 17 | Q | V | Q | L | V | Q | S | G | A | E | V | R | K | P | G | A | S | V | K | V | S | C | K | A | S | G | Y | S | F | T | G | F | F | L | H | - | - | - | - |
| 18 | E | V | Q | L | V | E | S | G | G | G | L | V | Q | P | G | G | S | L | R | L | S | C | A | A | S | G | F | T | F | S | R | Y | W | M | S | - | - | - | - |
| 19 | Q | V | Q | L | Q | Q | S | G | A | E | V | R | K | P | G | S | S | V | K | V | S | C | K | A | S | G | G | T | F | S | S | Y | G | I | S | - | - | - | - |
| 20 | Q | V | Q | L | V | Q | S | G | A | E | V | K | T | P | G | A | S | V | K | V | S | C | K | A | S | G | Y | S | F | T | R | Y | G | F | S | - | - | - | - |
| 21 | Q | V | Q | L | Q | Q | S | G | T | E | V | K | K | P | G | S | S | V | K | V | S | C | K | A | S | G | Y | T | F | T | S | F | E | V | H | - | - | - | - |
| 22 | Q | V | Q | L | V | Q | S | G | G | G | L | V | Q | P | G | G | S | L | R | L | S | C | A | A | S | G | F | T | F | S | S | Y | A | M | S | - | - | - | - |
| 23 | Q | V | Q | L | Q | Q | W | G | P | G | L | V | K | P | S | E | T | L | S | L | T | C | G | V | R | G | G | S | L | S | D | F | Y | W | T | - | - | - | - |
| 24 | E | V | Q | L | V | Q | S | G | G | V | V | V | Q | P | G | G | S | L | R | L | S | C | A | A | S | G | F | T | F | D | D | Y | A | M | H | - | - | - | - |
| 25 | E | V | Q | L | V | E | T | G | G | G | L | V | K | P | G | G | S | L | R | L | S | C | A | A | S | G | F | T | F | S | D | Y | D | M | S | - | - | - | - |
| 26 | Q | V | Q | L | V | Q | S | G | A | E | V | K | K | P | G | S | S | V | K | V | S | C | K | A | S | G | G | T | F | S | N | S | P | I | N | - | - | - | - |
| 27 | Q | V | Q | L | V | Q | S | G | A | E | V | K | K | P | G | A | S | V | I | L | S | C | E | A | S | E | Y | T | F | T | N | Y | Y | I | H | - | - | - | - |
| 28 | Q | V | Q | L | Q | Q | S | G | A | E | L | K | K | P | G | S | S | V | K | V | S | C | K | I | S | G | G | T | F | S | S | H | A | I | N | - | - | - | - |
| 29 | E | V | Q | L | L | E | S | G | G | G | L | V | Q | P | G | G | S | L | R | L | S | C | A | A | S | G | F | T | F | S | S | Y | A | M | S | - | - | - | - |
| 30 | E | V | Q | L | L | E | S | G | G | G | L | V | Q | P | G | G | S | L | R | L | S | C | A | A | S | G | F | T | F | S | S | Y | A | M | S | - | - | - | - |
| 31 | E | V | Q | L | V | Q | S | G | P | E | V | R | K | P | G | A | S | V | K | V | S | C | Q | A | S | G | Y | S | F | P | N | Y | G | I | T | - | - | - | - |
| 32 | E | V | Q | L | L | E | S | G | G | G | L | V | Q | P | G | G | S | L | R | L | S | C | A | A | S | G | F | T | F | S | S | Y | A | M | S | - | - | - | - |
| 33 | E | V | Q | L | L | E | S | G | G | G | L | V | Q | P | G | G | S | L | R | L | S | C | A | A | S | G | F | T | F | S | S | Y | A | M | S | - | - | - | - |
| 34 | E | V | Q | L | V | Q | S | G | A | E | V | K | T | P | G | A | S | V | K | V | S | C | K | A | S | G | Y | S | F | T | R | Y | G | F | S | - | - | - | - |

| Antibody number | FW 2 | | | | | | | | | | | | | | CDR 2 | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|
| | 36 | 37 | 38 | 39 | 40 | 41 | 42 | 43 | 44 | 45 | 46 | 47 | 48 | 49 | 50 | 51 | 52 | a | b | c | d | e | f | 53 | 54 | 55 | 56 | 57 | 58 | 59 | 60 | 61 | 62 | 63 | 64 | 65 |
| 1 | W | V | R | Q | A | P | G | K | G | L | E | W | V | S | A | I | S | G | - | - | - | - | - | S | G | G | S | T | Y | Y | A | D | S | V | K | G |
| 2 | W | V | R | Q | A | P | G | K | G | L | E | W | V | G | R | I | K | S | K | T | - | - | - | D | G | G | T | T | D | Y | A | A | P | V | K | G |
| 3 | W | V | R | Q | A | P | G | K | G | L | E | W | V | S | A | I | S | G | - | - | - | - | - | S | G | G | S | T | Y | Y | A | D | S | V | K | G |
| 4 | W | V | R | Q | A | P | G | K | G | L | E | W | V | S | A | I | S | G | - | - | - | - | - | S | G | G | S | T | Y | Y | A | D | S | V | K | G |
| 5 | W | V | R | Q | M | P | G | K | G | L | E | W | M | G | I | I | Y | P | - | - | - | - | - | G | D | S | D | T | R | Y | S | P | S | F | Q | G |
| 6 | W | V | R | Q | M | P | G | K | G | L | E | W | M | G | T | I | D | P | - | - | - | - | - | S | D | S | Y | T | N | Q | N | P | S | F | Q | G |
| 7 | W | I | R | Q | S | P | R | G | G | L | E | W | I | G | E | I | N | - | - | - | - | - | - | H | S | G | D | I | N | Y | N | P | S | L | K | S |
| 8 | W | V | R | Q | A | P | G | K | G | L | E | W | V | S | S | I | S | W | - | - | - | - | - | S | S | G | S | I | G | Y | A | D | S | V | K | G |
| 9 | W | V | R | Q | A | P | G | K | G | L | E | W | V | S | A | I | S | G | - | - | - | - | - | S | G | G | S | T | Y | Y | A | D | S | V | K | G |
| 10 | W | V | R | Q | A | P | G | K | G | L | E | W | V | A | V | I | W | Y | - | - | - | - | - | D | G | S | N | K | Y | Y | A | D | S | V | K | G |
| 11 | W | V | R | Q | A | P | G | K | G | L | E | W | V | S | A | I | S | G | - | - | - | - | - | S | G | G | S | T | Y | Y | A | D | S | V | K | G |
| 12 | W | V | R | Q | A | P | G | K | G | L | E | W | V | S | A | I | S | G | - | - | - | - | - | S | G | G | S | T | Y | Y | A | D | S | V | K | G |
| 13 | W | V | R | Q | A | P | G | K | G | L | E | W | V | S | A | I | S | G | - | - | - | - | - | S | G | G | S | T | Y | Y | A | D | S | V | E | G |
| 14 | W | V | R | Q | A | P | G | K | G | L | E | W | V | A | V | I | S | Y | - | - | - | - | - | D | G | S | N | K | Y | Y | A | D | S | V | K | G |
| 15 | W | V | R | Q | A | P | G | K | G | L | E | W | V | S | A | I | S | G | - | - | - | - | - | S | G | G | S | T | Y | Y | A | D | S | V | K | G |
| 16 | W | V | R | Q | A | P | G | K | G | L | E | W | V | S | S | I | S | W | - | - | - | - | - | S | S | G | S | I | G | Y | A | D | S | V | K | G |
| 17 | W | V | R | Q | A | P | G | Q | G | L | E | W | M | G | W | I | N | P | - | - | - | - | - | N | S | G | A | I | Q | Y | A | R | K | F | E | A |
| 18 | W | V | R | Q | A | P | G | K | G | L | E | W | V | A | N | I | K | E | - | - | - | - | - | D | G | S | E | K | N | Y | V | D | S | V | K | G |
| 19 | W | L | R | Q | V | P | G | Q | G | L | E | W | M | G | G | I | I | P | - | - | - | - | - | R | F | G | T | A | N | S | A | Q | K | F | Q | D |
| 20 | W | V | R | Q | A | P | G | Q | E | L | E | W | M | G | W | I | S | A | - | - | - | - | - | Y | N | G | N | T | N | Y | A | Q | K | F | Q | G |
| 21 | W | V | R | Q | A | P | G | Q | G | L | E | W | M | G | W | M | N | P | - | - | - | - | - | N | S | N | N | T | D | Y | A | Q | K | F | Q | G |
| 22 | W | V | R | Q | A | P | G | R | G | L | E | W | V | S | S | I | S | A | - | - | - | - | - | S | G | N | T | T | Y | Y | A | D | S | V | K | G |
| 23 | W | I | R | Q | S | P | R | G | G | L | E | W | I | G | E | I | N | - | - | - | - | - | - | D | R | G | S | T | N | Y | N | P | S | L | K | S |
| 24 | W | V | R | Q | A | P | G | K | G | L | E | W | V | S | R | I | S | R | - | - | - | - | - | D | G | S | M | T | E | Y | A | D | S | A | K | G |
| 25 | W | I | R | Q | A | P | G | K | G | L | E | W | L | S | Y | I | S | N | - | - | - | - | - | S | G | T | F | T | N | Y | A | D | S | V | K | G |
| 26 | W | L | R | Q | A | P | G | Q | G | L | E | W | M | G | S | I | I | P | - | - | - | - | - | S | F | G | T | A | N | Y | A | Q | K | F | Q | G |
| 27 | W | V | R | Q | A | P | G | Q | G | L | Q | W | M | G | V | I | K | P | - | - | - | - | - | N | D | G | T | K | N | V | A | Q | K | F | Q | G |
| 28 | W | V | R | Q | A | P | G | Q | G | L | E | W | M | G | A | I | I | P | - | - | - | - | - | V | F | G | T | T | H | S | A | Q | K | F | E | D |
| 29 | W | V | R | Q | A | P | G | K | G | L | E | W | V | S | A | I | S | G | - | - | - | - | - | S | G | G | S | T | Y | Y | A | D | S | V | K | G |
| 30 | W | V | R | Q | A | P | G | K | G | L | E | W | V | S | A | I | S | G | - | - | - | - | - | S | G | G | S | T | Y | Y | A | D | S | V | K | G |
| 31 | W | V | R | Q | A | P | G | R | G | L | E | W | V | G | W | I | I | A | - | - | - | - | - | Y | T | G | D | T | K | Y | A | Q | N | F | Q | D |
| 32 | W | V | R | Q | A | P | G | K | G | L | E | W | V | S | A | I | S | G | - | - | - | - | - | S | G | G | S | T | Y | Y | A | D | S | V | K | G |
| 33 | W | V | R | Q | A | P | G | K | G | L | E | W | V | S | A | I | S | G | - | - | - | - | - | S | G | G | S | T | Y | Y | A | D | S | V | K | G |
| 34 | W | V | R | Q | A | P | G | Q | E | L | E | W | M | G | W | I | S | A | - | - | - | - | - | Y | N | G | N | T | N | Y | A | Q | K | F | Q | G |

**Figure 2 – part 2: Phage display P2X4 Binding – Antibodies (Abs) VH sequences**

| Antibody number | FW 3 | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|
| | 66 | 67 | 68 | 69 | 70 | 71 | 72 | 73 | 74 | 75 | 76 | 77 | 78 | 79 | 80 | 81 | 82 | a | b | c | d | e | 83 | 84 | 85 | 86 | 87 | 88 | 89 | 90 | 91 | 92 | 93 | 94 |
| 1 | R | F | T | I | S | R | D | N | S | K | N | T | L | Y | L | Q | M | N | S | L | - | - | R | A | E | D | T | A | V | Y | Y | C | A | R |
| 2 | R | F | T | I | S | R | D | D | S | K | T | T | L | Y | L | Q | M | N | S | P | - | - | K | T | E | D | T | A | V | Y | Y | C | T | T |
| 3 | R | F | T | I | S | R | D | N | S | K | N | T | L | Y | L | Q | M | N | S | L | - | - | R | A | E | D | T | A | V | Y | Y | C | A | R |
| 4 | R | F | T | I | S | R | D | N | S | K | N | T | L | Y | L | Q | M | N | S | L | - | - | R | A | E | D | T | A | V | Y | Y | C | A | R |
| 5 | Q | V | T | I | S | A | D | K | S | I | S | T | A | Y | L | Q | W | S | S | L | - | - | K | A | S | D | T | A | M | Y | Y | C | A | S |
| 6 | R | V | T | I | S | A | D | K | S | I | S | T | A | Y | L | Q | W | S | S | L | - | - | K | A | S | D | S | A | M | Y | Y | C | A | R |
| 7 | R | V | T | I | L | V | D | T | S | K | K | Q | V | S | L | K | L | S | S | V | - | - | T | A | A | D | A | A | V | Y | Y | C | A | R |
| 8 | R | F | T | I | S | R | D | N | A | K | N | S | L | S | L | Q | M | N | S | L | - | - | R | V | E | D | T | A | L | Y | Y | C | V | K |
| 9 | R | F | T | I | S | R | D | N | S | K | N | T | L | Y | L | Q | M | N | S | L | - | - | R | A | E | D | T | A | V | Y | Y | C | A | R |
| 10 | R | F | T | I | S | R | D | N | S | K | N | T | L | Y | L | Q | M | N | S | L | - | - | R | A | E | D | T | A | V | Y | Y | C | A | R |
| 11 | R | F | T | I | S | R | D | N | S | K | N | T | L | Y | L | Q | M | N | S | L | - | - | R | A | E | D | T | A | V | Y | Y | C | A | R |
| 12 | R | F | T | I | S | R | D | N | S | K | N | T | L | Y | L | Q | M | N | S | L | - | - | R | A | E | D | T | A | V | Y | Y | C | A | R |
| 13 | R | F | T | I | S | R | D | N | S | K | N | T | L | Y | L | Q | M | N | S | L | - | - | R | A | E | D | T | A | V | Y | Y | C | A | R |
| 14 | R | F | T | I | S | R | D | N | S | K | N | T | L | Y | L | Q | M | N | S | L | - | - | R | A | E | D | T | A | V | Y | Y | C | A | R |
| 15 | R | F | T | I | S | R | D | N | S | K | N | T | L | Y | L | Q | M | N | S | L | - | - | R | A | E | D | T | A | V | Y | Y | C | A | K |
| 16 | R | F | T | I | S | R | D | N | A | K | N | S | L | S | L | Q | M | N | S | L | - | - | R | V | E | D | T | A | L | Y | Y | C | V | K |
| 17 | G | V | T | M | T | R | D | T | S | V | R | T | A | Y | L | E | L | R | R | L | - | - | R | I | N | D | T | A | V | Y | F | C | V | R |
| 18 | R | L | T | I | S | R | D | N | A | K | N | S | L | Y | L | Q | M | N | S | L | - | - | R | A | E | D | T | A | V | Y | Y | C | A | N |
| 19 | R | V | T | I | T | A | D | E | S | T | P | T | A | Y | M | E | L | S | N | L | - | - | R | S | E | D | T | A | V | Y | Y | C | A | R |
| 20 | R | V | T | M | T | T | D | T | S | T | N | T | A | Y | M | E | L | R | S | L | - | - | R | S | D | D | T | A | V | Y | Y | C | A | R |
| 21 | R | V | T | M | T | S | N | P | A | I | N | T | A | Y | M | E | L | T | S | L | - | - | T | S | E | D | T | A | I | Y | F | C | A | R |
| 22 | R | F | T | I | S | R | D | N | S | K | N | T | L | Y | L | Q | M | N | S | L | - | - | R | A | E | D | R | A | L | Y | Y | C | A | K |
| 23 | R | V | T | I | S | V | D | T | S | K | N | E | V | S | L | R | L | T | S | V | - | - | T | A | A | D | T | A | V | Y | Y | C | A | R |
| 24 | R | F | T | I | S | R | D | N | T | K | N | T | V | Y | L | Q | M | N | S | L | - | - | T | T | D | D | T | A | V | Y | Y | C | A | R |
| 25 | R | F | T | I | S | R | D | N | A | K | K | S | L | Y | L | Q | M | N | S | L | - | - | R | A | E | D | T | A | L | Y | Y | C | G | G |
| 26 | R | L | T | I | T | A | D | E | S | T | S | T | A | Y | M | E | L | S | S | L | - | - | R | S | E | D | T | A | V | Y | Y | C | A | G |
| 27 | R | V | T | M | T | R | D | T | S | T | R | T | V | Y | M | E | L | S | G | L | - | - | R | S | E | D | T | A | V | Y | Y | C | V | R |
| 28 | R | V | T | L | T | A | D | E | S | T | N | T | A | Y | M | E | L | S | S | L | - | - | R | S | D | D | T | A | V | Y | Y | C | A | R |
| 29 | R | F | T | I | S | R | D | N | S | K | N | T | L | Y | L | Q | M | N | S | L | - | - | R | A | E | D | T | A | V | Y | Y | C | A | R |
| 30 | R | F | T | I | S | R | D | N | S | K | N | T | L | Y | L | Q | M | N | S | L | - | - | R | A | E | D | T | A | V | Y | Y | C | A | R |
| 31 | R | V | T | L | T | T | D | T | S | T | R | T | A | Y | L | E | M | R | S | L | - | - | R | S | D | D | T | A | V | Y | Y | C | A | R |
| 32 | R | F | T | I | S | R | D | N | S | K | N | T | L | Y | L | Q | M | N | S | L | - | - | R | A | E | D | T | A | V | Y | Y | C | A | R |
| 33 | R | F | T | I | S | R | D | N | S | K | N | T | L | Y | L | Q | M | N | S | L | - | - | R | A | E | D | T | A | V | Y | Y | C | A | R |
| 34 | R | V | T | M | T | T | D | T | S | T | N | T | A | Y | M | E | L | R | S | L | - | - | R | S | D | D | T | A | V | Y | Y | C | A | R |

| Antibody number | CDR 3 | | | | | | | | | | | | | | | | | | | | | | | | |
|---|---|---|---|---|---|---|---|---|---|---|---|---|---|---|---|---|---|---|---|---|---|---|---|---|---|
| | 95 | 96 | 97 | 98 | 99 | 100 | a | b | c | d | e | f | g | h | i | j | k | l | m | n | o | p | q | 101 | 102 |
| 1 | E | E | R | G | S | Y | F | G | F | S | G | Y | Y | Y | T | Y | Y | F | - | - | - | - | - | D | Y |
| 2 | N | L | - | - | - | - | - | - | - | - | - | - | - | - | - | - | - | - | - | - | - | - | - | E | Y |
| 3 | D | R | A | L | G | A | R | R | W | F | - | - | - | - | - | - | - | - | - | - | - | - | - | D | S |
| 4 | R | Q | T | R | Y | S | S | G | A | F | G | Y | M | - | - | - | - | - | - | - | - | - | - | D | V |
| 5 | R | T | G | D | Y | Y | Y | Y | G | M | - | - | - | - | - | - | - | - | - | - | - | - | - | D | V |
| 6 | K | T | V | T | D | W | Y | F | - | - | - | - | - | - | - | - | - | - | - | - | - | - | - | D | L |
| 7 | G | G | S | G | W | Y | L | - | - | - | - | - | - | - | - | - | - | - | - | - | - | - | - | D | S |
| 8 | D | R | M | Y | Y | Y | D | T | G | G | Y | Y | S | G | F | - | - | - | - | - | - | - | - | D | M |
| 9 | D | F | W | S | G | Y | Y | T | T | Y | S | N | V | M | - | - | - | - | - | - | - | - | - | D | V |
| 10 | D | L | G | E | T | Y | Y | D | I | L | T | G | F | - | - | - | - | - | - | - | - | - | - | D | Y |
| 11 | D | R | D | F | W | S | T | Y | S | G | P | T | A | F | - | - | - | - | - | - | - | - | - | D | L |
| 12 | S | L | Y | D | G | T | G | S | F | S | S | R | P | F | - | - | - | - | - | - | - | - | - | D | Q |
| 13 | A | P | G | Q | W | L | V | R | G | A | F | - | - | - | - | - | - | - | - | - | - | - | - | D | I |
| 14 | D | L | G | G | S | Y | D | Y | Y | Y | Y | G | M | - | - | - | - | - | - | - | - | - | - | D | V |
| 15 | D | R | S | C | N | G | I | T | C | Y | R | G | F | - | - | - | - | - | - | - | - | - | - | D | Y |
| 16 | D | R | M | Y | Y | Y | D | T | G | G | Y | Y | S | G | F | - | - | - | - | - | - | - | - | D | M |
| 17 | G | G | T | T | G | W | S | G | Y | D | A | F | - | - | - | - | - | - | - | - | - | - | - | D | Q |
| 18 | Y | Y | D | S | S | G | Y | Y | A | L | - | - | - | - | - | - | - | - | - | - | - | - | - | D | S |
| 19 | E | K | A | I | I | E | T | T | S | G | E | A | D | P | F | - | - | - | - | - | - | - | - | D | I |
| 20 | D | P | S | M | Y | Y | F | D | L | S | G | Y | R | V | T | G | G | N | Y | F | - | - | - | D | Y |
| 21 | R | R | T | D | W | F | F | - | - | - | - | - | - | - | - | - | - | - | - | - | - | - | - | D | L |
| 22 | A | A | G | K | D | W | Y | F | - | - | - | - | - | - | - | - | - | - | - | - | - | - | - | D | L |
| 23 | V | A | H | L | D | W | Y | F | - | - | - | - | - | - | - | - | - | - | - | - | - | - | - | D | L |
| 24 | E | S | A | V | N | W | Y | F | - | - | - | - | - | - | - | - | - | - | - | - | - | - | - | D | L |
| 25 | G | V | Y | G | G | K | S | - | - | - | - | - | - | - | - | - | - | - | - | - | - | - | - | E | H |
| 26 | R | S | H | N | Y | E | L | Y | Y | Y | Y | M | - | - | - | - | - | - | - | - | - | - | - | D | V |
| 27 | E | P | N | Y | S | L | L | V | A | Y | P | F | - | - | - | - | - | - | - | - | - | - | - | D | F |
| 28 | V | A | E | K | V | V | V | V | T | G | Q | Y | F | - | - | - | - | - | - | - | - | - | - | D | S |
| 29 | P | E | L | P | E | T | A | M | V | R | N | W | H | F | - | - | - | - | - | - | - | - | - | D | L |
| 30 | H | F | Y | T | W | V | A | D | N | V | G | Y | S | H | I | - | - | - | - | - | - | - | - | D | D |
| 31 | D | F | I | S | V | I | T | Y | G | G | L | P | D | V | F | - | - | - | - | - | - | - | - | G | M |
| 32 | G | Y | T | Y | Y | D | S | R | R | G | Y | R | P | T | G | H | F | - | - | - | - | - | - | D | S |
| 33 | S | F | H | Y | C | S | S | T | N | C | Y | V | E | G | R | E | N | F | - | - | - | - | - | D | Y |
| 34 | D | P | S | M | Y | Y | F | D | L | S | G | Y | R | V | T | G | G | N | Y | F | - | - | - | D | Y |

| Antibody number | FW 4 | | | | | | | | | | |
|---|---|---|---|---|---|---|---|---|---|---|---|
| | 103 | 104 | 105 | 106 | 107 | 108 | 109 | 110 | 111 | 112 | 113 |
| 1 | W | G | R | G | T | M | V | T | V | S | S |
| 2 | W | G | R | G | T | L | V | T | V | S | S |
| 3 | W | G | R | G | T | T | V | T | V | S | S |
| 4 | W | G | R | G | T | M | V | T | V | S | S |
| 5 | W | G | K | G | T | M | V | T | V | S | S |
| 6 | W | G | R | G | T | L | V | T | V | S | S |
| 7 | W | G | P | G | T | L | V | T | V | S | S |
| 8 | W | G | Q | G | T | M | V | T | V | S | S |
| 9 | W | G | Q | G | T | T | V | T | V | S | S |
| 10 | W | G | K | G | T | L | V | T | V | S | S |
| 11 | W | G | R | G | T | L | V | T | V | S | S |
| 12 | W | G | R | G | T | M | V | T | V | S | S |
| 13 | W | G | Q | G | P | M | V | T | V | S | S |
| 14 | W | G | R | G | P | L | V | T | V | S | S |
| 15 | W | G | Q | G | T | M | V | T | V | S | S |
| 16 | W | G | Q | G | T | M | V | T | V | S | S |
| 17 | W | G | Q | G | T | M | V | T | V | S | S |
| 18 | W | G | R | G | T | L | V | T | V | S | S |
| 19 | W | G | Q | G | T | M | V | T | V | S | S |
| 20 | W | G | Q | G | T | M | V | T | V | S | S |
| 21 | W | G | Q | G | T | L | V | T | V | S | S |
| 22 | W | G | R | G | T | L | V | T | V | S | S |
| 23 | W | G | Q | G | T | T | V | T | V | S | S |
| 24 | W | G | Q | G | T | L | V | T | V | S | S |
| 25 | W | G | R | G | T | M | V | T | V | S | S |
| 26 | W | G | R | G | T | T | V | T | V | S | S |
| 27 | W | G | R | G | T | L | V | T | V | S | S |
| 28 | W | G | Q | G | T | T | V | T | V | S | S |
| 29 | W | G | Q | G | T | M | V | T | V | S | S |
| 30 | W | G | R | G | T | T | V | T | V | S | S |
| 31 | W | G | R | G | T | L | V | T | V | S | S |
| 32 | W | G | R | G | T | M | V | T | V | S | S |
| 33 | W | G | Q | G | T | M | V | T | V | S | S |
| 34 | W | G | Q | G | T | M | V | T | V | S | S |

## Figure 2 – part 3: Phage display P2X4 Binding – Antibodies (Abs) VL sequences

| Antibody number | FW 1 | | | | | | | | | | | | | | | | | | | | | | | CDR 1 | | | | | | | | | | | | |
|---|---|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | a | b | c | d | e | f | g | h | 28 |
| 1 | Q | A | V | L | T | Q | P | S | S | – | V | S | G | A | P | G | Q | R | V | T | I | S | C | T | G | S | S | S | N | I | – | – | – | – | – | G |
| 2 | Q | S | V | L | T | Q | P | P | S | – | V | S | V | A | P | G | K | T | A | T | I | P | C | G | G | T | N | – | – | – | – | – | – | – | – | I |
| 3 | S | S | E | L | T | Q | D | P | A | – | V | S | V | A | L | G | Q | T | V | A | I | T | C | R | G | D | S | – | – | – | – | – | – | – | – | L |
| 4 | S | Y | V | L | T | Q | P | P | S | – | V | S | V | S | P | G | Q | T | A | S | I | T | C | S | G | D | K | – | – | – | – | – | – | – | – | L |
| 5 | S | Y | V | L | T | Q | P | P | S | – | V | S | V | S | L | G | Q | T | A | R | I | T | C | S | G | D | A | – | – | – | – | – | – | – | – | L |
| 6 | D | I | V | M | T | Q | S | P | S | T | L | S | A | S | I | G | D | R | V | T | I | T | C | R | A | S | Q | – | – | – | – | – | – | – | – | G |
| 7 | D | I | V | M | T | Q | S | P | S | T | L | S | A | S | V | G | D | R | V | T | I | T | C | R | A | S | Q | – | – | – | – | – | – | – | – | G |
| 8 | S | Y | E | L | T | Q | P | P | S | – | V | S | V | S | P | G | Q | T | A | T | I | T | C | S | G | D | K | – | – | – | – | – | – | – | – | L |
| 9 | Q | S | V | V | T | Q | P | P | S | – | A | S | G | T | P | G | Q | R | V | T | I | S | C | S | G | S | S | S | N | – | – | – | – | – | – | I |
| 10 | Q | S | V | V | T | Q | P | P | S | – | V | S | A | A | P | G | Q | K | V | T | I | S | C | S | G | S | S | S | N | – | – | – | – | – | – | I |
| 11 | S | Y | E | L | A | Q | P | P | S | – | V | S | V | S | P | G | Q | T | A | R | I | T | C | S | G | D | A | – | – | – | – | – | – | – | – | L |
| 12 | S | S | E | L | T | Q | D | P | A | – | V | S | V | A | L | G | Q | T | V | T | I | T | C | Q | G | D | S | – | – | – | – | – | – | – | – | L |
| 13 | S | S | E | L | T | Q | D | P | D | – | V | S | V | A | L | G | Q | T | V | S | I | T | C | R | G | D | T | – | – | – | – | – | – | – | – | L |
| 14 | Q | S | A | L | T | Q | P | A | S | – | V | S | G | S | P | G | Q | S | I | T | I | S | C | S | G | T | S | S | D | V | – | – | – | – | – | G |
| 15 | Q | S | A | L | T | Q | P | P | S | – | V | S | L | S | P | G | Q | T | A | R | I | T | C | S | G | D | V | – | – | – | – | – | – | – | – | L |
| 16 | S | Y | V | L | T | Q | P | P | S | – | V | S | V | S | P | G | Q | T | A | T | I | T | C | S | G | D | K | – | – | – | – | – | – | – | – | L |
| 17 | Q | S | A | L | T | Q | P | P | S | – | V | S | V | S | P | G | Q | T | A | R | I | T | C | S | G | D | K | – | – | – | – | – | – | – | – | L |
| 18 | S | Y | E | L | T | Q | P | P | S | – | V | S | V | S | P | G | Q | T | A | T | I | T | C | S | G | D | A | – | – | – | – | – | – | – | – | L |
| 19 | Q | S | A | L | T | Q | P | P | S | – | V | S | V | A | P | G | Q | T | A | R | I | A | C | G | G | D | N | – | – | – | – | – | – | – | – | I |
| 20 | D | I | Q | L | T | Q | S | P | S | S | V | S | A | S | V | G | D | R | V | T | I | T | C | R | A | S | Q | – | – | – | – | – | – | – | – | G |
| 21 | D | I | V | M | T | Q | S | P | S | T | L | S | A | S | V | G | D | R | V | T | I | T | C | R | A | S | Q | – | – | – | – | – | – | – | – | G |
| 22 | D | I | V | M | T | Q | S | P | S | T | L | S | A | S | V | G | D | R | V | T | I | T | C | R | A | S | Q | – | – | – | – | – | – | – | – | G |
| 23 | D | I | V | M | T | Q | S | P | S | T | L | S | A | S | V | G | D | R | V | T | I | T | C | R | A | S | Q | – | – | – | – | – | – | – | – | G |
| 24 | D | I | V | M | T | Q | S | P | S | T | L | S | A | S | V | G | D | R | V | T | I | T | C | R | A | S | Q | – | – | – | – | – | – | – | – | G |
| 25 | Q | T | V | V | I | Q | E | P | S | – | F | S | V | S | P | G | G | T | V | T | L | T | C | G | L | D | S | G | S | V | – | – | – | – | – | S |
| 26 | S | Y | E | L | T | Q | P | P | S | – | V | S | V | S | P | G | Q | T | A | R | I | T | C | S | G | D | A | – | – | – | – | – | – | – | – | L |
| 27 | S | Y | V | L | T | Q | P | P | S | – | V | S | V | A | P | G | K | T | A | R | M | T | C | G | G | D | N | – | – | – | – | – | – | – | – | I |
| 28 | N | F | M | L | T | Q | P | H | S | – | V | S | E | S | P | G | K | T | V | T | I | S | C | T | G | S | S | G | S | – | – | – | – | – | – | I |
| 29 | S | Y | V | L | T | Q | P | P | S | – | V | S | V | S | P | G | Q | T | A | R | I | T | C | S | G | D | A | – | – | – | – | – | – | – | – | L |
| 30 | Q | S | V | L | T | Q | P | P | A | – | V | S | V | S | P | G | R | T | A | S | I | T | C | S | G | D | N | – | – | – | – | – | – | – | – | L |
| 31 | H | V | I | L | T | Q | P | P | S | – | V | S | V | S | P | G | Q | T | A | T | I | T | C | S | G | H | K | – | – | – | – | – | – | – | – | L |
| 32 | D | I | Q | L | T | Q | S | P | S | S | L | S | A | S | V | G | D | R | V | T | I | T | C | Q | A | S | Q | – | – | – | – | – | – | – | – | D |
| 33 | S | Y | E | L | T | Q | P | P | S | – | V | S | V | S | P | R | Q | T | A | S | I | T | C | S | G | D | K | – | – | – | – | – | – | – | – | L |
| 34 | Y | I | Q | M | T | Q | S | P | S | S | L | S | A | S | V | G | D | R | V | T | I | T | C | R | A | S | Q | – | – | – | – | – | – | – | – | G |

| Antibody number | CDR 1 | | | | | | FW 2 | | | | | | | | | | | | | | | CDR 2 | | | | | | | | | | | |
|---|---|---|---|---|---|---|---|---|---|---|---|---|---|---|---|---|---|---|---|---|---|---|---|---|---|---|---|---|---|---|---|---|---|
| | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | a | b | c | d | e | 52 | 53 | 54 | 55 | 56 |
| 1 | A | G | Y | D | V | H | W | Y | Q | Q | L | P | G | T | A | P | K | L | L | I | Y | G | N | – | – | – | – | – | N | N | R | P | S |
| 2 | G | N | K | N | V | H | W | Y | R | Q | K | P | G | Q | A | P | V | L | V | I | S | Y | D | – | – | – | – | – | S | D | R | P | S |
| 3 | R | M | F | Y | A | T | W | F | Q | Q | K | P | G | Q | A | P | V | V | V | V | Y | G | K | – | – | – | – | – | T | Y | R | P | S |
| 4 | G | D | K | Y | A | S | W | Y | Q | Q | K | P | G | Q | S | P | V | L | V | I | Y | Q | D | – | – | – | – | – | T | K | R | P | S |
| 5 | P | K | Q | Y | A | Y | W | Y | Q | Q | K | P | G | Q | A | P | V | L | V | I | Y | K | D | – | – | – | – | – | S | E | R | P | S |
| 6 | I | S | S | W | L | A | W | Y | Q | Q | K | P | G | R | A | P | K | V | L | I | Y | K | A | – | – | – | – | – | S | T | L | E | S |
| 7 | I | S | S | W | L | A | W | Y | Q | Q | K | P | G | R | A | P | K | V | L | I | Y | K | A | – | – | – | – | – | S | T | L | E | S |
| 8 | D | D | K | Y | I | S | W | Y | Q | R | K | P | G | Q | S | P | V | L | L | I | Y | Q | D | – | – | – | – | – | I | E | R | P | S |
| 9 | G | S | N | F | V | Q | W | Y | R | Q | L | P | G | A | A | P | Q | L | L | I | M | N | D | – | – | – | – | – | K | T | R | A | S |
| 10 | G | N | N | Y | V | S | W | Y | Q | Q | L | P | G | T | A | P | K | L | L | I | Y | D | N | – | – | – | – | – | N | K | R | P | S |
| 11 | P | R | Q | Y | A | Y | W | Y | Q | Q | K | P | G | Q | A | P | L | L | V | I | Y | K | D | – | – | – | – | – | S | E | R | P | S |
| 12 | R | S | Y | Y | A | N | W | Y | Q | Q | K | P | G | Q | A | P | V | F | V | I | Y | G | K | – | – | – | – | – | S | Y | R | P | S |
| 13 | R | S | Y | Y | A | N | W | Y | Q | Q | K | A | G | Q | A | P | V | L | V | M | F | G | K | – | – | – | – | – | N | N | R | P | S |
| 14 | A | Y | D | Y | V | S | W | Y | Q | Q | H | P | G | K | A | P | K | L | I | I | Y | D | V | – | – | – | – | – | I | Y | R | P | S |
| 15 | S | K | Q | F | G | Y | W | Y | Q | Q | K | P | G | Q | A | P | A | V | V | I | F | N | D | – | – | – | – | – | N | E | R | P | S |
| 16 | D | D | K | Y | I | S | W | Y | Q | R | K | P | G | Q | S | P | V | L | L | I | Y | Q | D | – | – | – | – | – | I | E | R | P | S |
| 17 | G | D | K | Y | V | S | W | Y | Q | Q | K | S | G | Q | S | P | V | L | V | I | Y | Q | D | – | – | – | – | – | T | T | R | P | S |
| 18 | T | K | Q | Y | A | F | W | Y | Q | Q | K | P | G | Q | A | P | I | L | V | I | F | R | D | – | – | – | – | – | S | E | R | P | S |
| 19 | G | G | K | M | V | H | W | Y | Q | Q | K | P | G | H | A | P | V | L | V | V | H | N | G | – | – | – | – | – | S | D | R | P | S |
| 20 | I | S | S | W | L | A | W | Y | Q | Q | K | P | G | K | A | P | K | L | L | I | Y | A | A | – | – | – | – | – | S | S | L | Q | S |
| 21 | I | S | S | W | L | A | W | Y | Q | Q | K | P | G | R | A | P | K | V | L | I | Y | K | A | – | – | – | – | – | S | T | L | E | S |
| 22 | I | S | S | W | L | A | W | Y | Q | Q | K | P | G | R | A | P | K | V | L | I | Y | K | A | – | – | – | – | – | S | T | L | E | S |
| 23 | I | S | S | W | L | A | W | Y | Q | Q | K | P | G | R | A | P | K | V | L | I | Y | K | A | – | – | – | – | – | S | T | L | E | S |
| 24 | I | S | S | W | L | A | W | Y | Q | Q | K | P | G | R | A | P | K | V | L | I | Y | K | A | – | – | – | – | – | S | T | L | E | S |
| 25 | T | T | H | Y | P | S | W | Y | Q | Q | T | P | G | Q | A | P | R | T | L | M | F | G | T | – | – | – | – | – | N | N | R | S | P |
| 26 | P | K | Q | Y | A | Y | W | Y | Q | Q | K | P | G | Q | A | P | V | L | V | I | Y | K | D | – | – | – | – | – | S | E | R | P | S |
| 27 | G | N | K | G | V | H | W | Y | Q | Q | K | P | G | Q | A | P | V | L | V | V | Y | D | D | – | – | – | – | – | Y | D | R | P | S |
| 28 | A | S | N | Y | V | Q | W | Y | Q | Q | R | P | G | S | A | P | A | T | V | I | Y | E | D | – | – | – | – | – | N | Q | R | P | S |
| 29 | P | K | Q | Y | A | Y | W | Y | Q | Q | K | P | G | Q | A | P | V | L | V | I | Y | K | D | – | – | – | – | – | S | E | R | P | S |
| 30 | D | D | K | Y | V | A | W | Y | Q | Q | R | P | G | Q | P | P | L | L | L | L | Y | Q | D | – | – | – | – | – | D | K | R | P | S |
| 31 | E | D | K | Y | V | S | W | Y | Q | Q | R | P | G | Q | S | P | V | L | L | I | F | Q | D | – | – | – | – | – | T | K | R | P | S |
| 32 | I | N | N | S | L | N | W | F | Q | Q | K | P | G | K | A | P | K | L | L | I | Y | D | A | – | – | – | – | – | S | N | L | E | T |
| 33 | G | N | K | Y | A | S | W | Y | Q | Q | K | P | G | H | S | P | V | L | V | I | Y | Q | D | – | – | – | – | – | S | K | R | P | S |
| 34 | I | S | R | W | L | A | W | Y | Q | Q | K | P | G | K | A | P | K | L | L | I | Y | K | V | – | – | – | – | – | S | S | L | E | S |

**Figure 2 – part 4: Phage display P2X4 Binding – Antibodies (Abs) VL sequences;**

| Antibody number | FW 3 | | | | | | | | | | | | | | | | | | | | | | | | | | | | | | | | | CDR 3 | | | | | | | | | | | | | | | | | | FW 4 | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | 57 | 58 | 59 | 60 | 61 | 62 | 63 | 64 | 65 | 66 | 67 | 68 | a | b | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | a | b | c | d | e | f | g | h | i | 96 | 97 | 98 | 99 | 100 | 101 | 102 | 103 | 104 | 105 | 106 | a | b | c | 107 |
| 1 | G | V | P | D | R | F | S | G | S | K | S | G | - | - | T | S | A | S | L | A | I | T | G | L | Q | A | E | D | E | A | D | Y | Y | C | Q | S | Y | D | T | N | L | - | - | - | - | - | - | - | - | - | K | V | F | G | G | G | T | K | L | T | V | - | - | - | L |
| 2 | G | I | P | E | R | L | S | G | S | N | S | E | - | - | N | T | A | T | L | T | I | S | R | V | E | A | G | D | E | A | D | Y | Y | C | Q | V | W | D | M | T | T | D | H | - | - | - | - | - | - | - | W | V | F | G | G | G | T | K | L | T | V | - | - | - | L |
| 3 | G | I | P | D | R | F | S | G | S | D | S | G | - | - | D | T | A | S | L | T | I | T | G | A | Q | A | E | D | E | A | D | Y | Y | C | N | S | R | D | S | T | G | N | P | - | - | - | - | - | - | - | V | L | F | G | G | G | T | K | L | T | V | - | - | - | L |
| 4 | G | I | P | E | R | F | S | G | S | N | S | G | - | - | N | T | A | T | L | T | I | S | G | T | Q | A | L | D | E | A | D | Y | Y | C | Q | A | W | D | S | S | T | A | - | - | - | - | - | - | - | - | W | V | F | G | G | G | T | K | L | T | V | - | - | - | L |
| 5 | G | I | P | E | R | F | S | G | S | S | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | L | T | V | - | - | - | L |
| 6 | G | V | P | S | R | F | S | G | S | G | S | G | - | - | T | D | F | T | L | T | I | S | S | L | Q | P | E | D | F | A | T | Y | Y | C | Q | Q | S | Y | S | T | P | - | - | - | - | - | - | - | - | - | W | T | F | G | Q | G | T | K | L | E | I | - | - | - | K |
| 7 | G | V | P | S | R | F | S | G | S | G | S | G | - | - | T | D | F | T | L | T | I | S | S | L | Q | P | E | D | F | A | T | Y | Y | C | Q | Q | S | Y | S | T | P | - | - | - | - | - | - | - | - | - | W | T | F | G | Q | G | T | K | L | E | I | - | - | - | K |
| 8 | G | I | P | D | R | F | S | G | S | N | S | G | - | - | N | T | A | T | L | S | I | S | G | T | Q | S | M | D | E | A | E | Y | Y | C | Q | A | W | D | S | G | A | - | - | - | - | - | - | - | - | - | I | V | F | G | G | G | T | K | L | T | V | - | - | - | L |
| 9 | A | V | P | D | R | F | S | G | S | K | S | G | - | - | T | S | A | S | L | A | I | S | G | L | R | S | E | D | E | A | D | Y | Y | C | A | A | W | D | D | G | L | S | G | - | - | - | - | - | - | - | P | V | F | G | G | G | T | K | L | T | V | - | - | - | L |
| 10 | G | I | P | D | R | F | S | G | S | K | S | G | - | - | T | S | A | T | L | G | I | T | G | L | Q | T | G | D | E | A | D | Y | Y | C | G | T | W | D | S | S | L | S | A | - | - | - | - | - | - | - | G | V | F | G | G | G | T | K | L | T | V | - | - | - | L |
| 11 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 12 | G | I | P | D | R | F | S | G | S | D | S | G | - | - | N | T | A | S | L | T | I | T | G | A | Q | A | E | D | E | A | D | Y | Y | C | N | S | R | D | S | S | G | N | R | - | - | - | - | - | - | - | L | V | F | G | G | G | T | K | L | T | V | - | - | - | L |
| 13 | G | I | P | D | R | F | S | G | S | D | S | G | - | - | N | T | A | S | L | T | I | T | G | A | Q | A | E | D | E | A | D | Y | Y | C | D | S | Q | D | T | S | G | D | L | - | - | - | - | - | - | - | L | V | F | G | G | G | T | K | V | T | V | - | - | - | L |
| 14 | G | I | S | N | R | F | S | G | S | E | S | G | - | - | N | T | A | S | L | T | I | S | G | L | Q | A | E | D | E | A | E | Y | Y | C | S | S | Y | I | S | S | T | T | - | - | - | - | - | - | - | - | L | V | F | G | G | G | T | K | L | T | V | - | - | - | L |
| 15 | G | I | P | E | R | F | S | G | S | S | S | G | - | - | A | T | V | T | L | T | I | T | G | V | Q | G | E | D | E | A | D | Y | Y | C | Q | S | A | D | S | N | G | A | - | - | - | - | - | - | - | - | V | L | F | G | G | G | T | K | L | T | V | - | - | - | L |
| 16 | G | I | P | D | R | F | S | G | S | N | S | G | - | - | N | T | A | T | L | S | I | S | G | T | Q | S | M | D | E | A | E | Y | Y | C | Q | A | W | D | S | G | A | - | - | - | - | - | - | - | - | - | I | V | F | G | G | G | T | K | V | T | V | - | - | - | L |
| 17 | G | I | P | E | R | F | S | G | S | N | S | G | - | - | N | T | A | T | L | T | I | S | G | T | Q | V | T | D | E | A | D | Y | F | C | Q | S | W | D | R | S | T | D | - | - | - | - | - | - | - | - | W | V | F | G | G | G | T | K | V | T | V | - | - | - | L |
| 18 | G | I | P | E | R | F | S | G | S | S | S | G | - | - | T | T | A | T | L | T | I | S | G | V | Q | A | G | D | E | A | D | Y | Y | C | Q | S | T | D | N | T | A | T | S | - | - | - | - | - | - | - | V | V | F | G | G | G | T | K | V | T | V | - | - | - | L |
| 19 | G | I | P | E | R | F | Y | G | S | N | S | G | - | - | D | T | A | T | L | T | I | S | R | V | E | A | G | D | E | A | D | Y | H | C | Q | V | W | D | S | S | I | D | P | S | - | - | - | - | - | - | W | V | F | G | G | G | T | K | L | T | V | - | - | - | L |
| 20 | G | V | P | S | R | F | S | G | S | G | S | G | - | - | T | D | F | T | L | T | I | S | S | L | Q | P | E | D | F | A | T | Y | Y | C | Q | Q | S | N | S | F | P | - | - | - | - | - | - | - | - | - | L | T | F | G | G | G | T | K | V | D | I | - | - | - | K |
| 21 | G | V | P | S | R | F | S | G | S | G | S | G | - | - | T | D | F | T | L | T | I | S | S | L | Q | P | E | D | F | A | T | Y | Y | C | Q | Q | S | Y | S | T | P | - | - | - | - | - | - | - | - | - | W | T | F | G | Q | G | T | K | L | E | I | - | - | - | K |
| 22 | G | V | P | S | R | F | S | G | S | G | S | G | - | - | T | D | F | T | L | T | I | S | S | L | Q | P | E | D | F | A | T | Y | Y | C | Q | Q | G | Y | S | T | P | - | - | - | - | - | - | - | - | - | W | T | F | G | Q | G | T | K | L | E | I | - | - | - | K |
| 23 | G | V | P | S | R | F | S | G | S | G | S | G | - | - | T | D | F | T | L | T | I | S | S | L | Q | P | E | D | F | A | T | Y | Y | C | Q | Q | S | Y | S | T | P | - | - | - | - | - | - | - | - | - | W | T | F | G | Q | G | T | K | L | E | I | - | - | - | K |
| 24 | G | V | P | S | R | F | S | G | S | G | S | G | - | - | T | D | F | T | L | T | I | S | S | L | Q | P | E | D | F | A | T | Y | Y | C | Q | Q | S | Y | S | T | P | - | - | - | - | - | - | - | - | - | W | T | F | G | Q | G | T | K | L | E | I | - | - | - | K |
| 25 | G | V | P | A | R | F | S | G | S | I | L | G | - | - | N | K | A | A | L | T | I | T | G | A | Q | A | E | D | E | S | D | Y | Y | C | L | L | Y | M | G | D | G | I | - | - | - | - | - | - | - | - | R | V | F | G | G | G | T | K | V | T | V | - | - | - | L |
| 26 | G | I | P | E | R | F | S | G | S | S | S | G | - | - | T | T | V | T | L | T | I | S | G | V | Q | A | E | D | E | A | D | Y | Y | C | Q | S | A | D | S | S | G | T | - | - | - | - | - | - | - | - | Y | V | F | G | T | G | T | Q | L | T | V | - | - | - | L |
| 27 | G | I | P | E | R | F | S | G | S | N | S | G | - | - | N | T | A | T | L | T | I | N | R | V | E | A | G | D | E | A | D | Y | F | C | Q | V | W | D | D | S | G | D | P | - | - | - | - | - | - | - | V | V | F | G | G | G | T | K | L | T | V | - | - | - | L |
| 28 | G | V | P | D | R | F | S | G | S | I | D | S | S | S | N | S | A | S | L | T | I | S | G | L | K | T | E | D | E | A | D | Y | Y | C | Q | S | Y | D | T | N | N | H | - | - | - | - | - | - | - | - | V | V | F | G | G | G | T | K | L | T | V | - | - | - | L |
| 29 | G | I | P | E | R | F | S | G | S | S | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | L | T | V | - | - | - | L |
| 30 | G | I | P | E | R | F | S | G | S | N | S | G | - | - | T | T | A | T | L | T | I | S | G | T | Q | S | M | D | E | A | D | Y | Y | C | Q | A | W | D | R | S | T | - | - | - | - | - | - | - | - | - | V | V | F | G | G | G | T | K | V | T | V | - | - | - | L |
| 31 | G | I | P | E | R | F | S | G | S | N | S | G | - | - | N | T | A | T | L | T | I | S | G | I | Q | V | M | D | E | A | D | Y | Y | C | Q | A | W | D | K | S | A | - | - | - | - | - | - | - | - | - | V | V | F | G | G | G | T | K | L | T | V | - | - | - | L |
| 32 | G | V | P | S | R | F | S | G | S | G | S | G | - | - | T | E | F | T | F | T | I | S | S | L | Q | P | E | D | F | A | T | Y | Y | C | Q | H | Y | H | D | L | P | - | - | - | - | - | - | - | - | - | I | T | F | G | Q | G | T | R | L | E | I | - | - | - | K |
| 33 | G | I | P | E | R | F | S | G | S | N | S | G | - | - | N | T | A | T | L | T | I | S | G | T | Q | A | M | D | E | A | V | Y | Y | C | Q | A | W | D | S | T | I | - | - | - | - | - | - | - | - | - | V | V | F | G | G | G | T | K | L | T | V | - | - | - | L |
| 34 | G | V | P | S | R | F | S | G | S | G | S | G | - | - | T | D | F | T | L | T | I | S | S | L | Q | P | E | D | F | A | T | Y | Y | C | Q | Q | S | N | S | F | P | - | - | - | - | - | - | - | - | - | L | T | F | G | G | G | T | K | V | E | I | - | - | - | K |

**Figure 3: Antibodies (Abs) Binding and Function: Phage display P2X4 binding antibodies**

| Antibody number | Human P2X4 binding | Cynomologus P2X4 binding | Mouse P2X4 binding | Fraction of control at Human P2X4 | Fraction of control at Cyno P2X4 | Fraction of control at Mouse P2X4 | Concentration in Human ephys assay (mg/ml) | Concentration in Cyno ephys assay (mg/ml) | Concentration in Mouse ephys assay (mg/ml) |
|---|---|---|---|---|---|---|---|---|---|
| 1 | + | + | | 4.01 | 5.80 | NT | 2.1 | 2.1 | |
| 2 | + | | | 1.27 | NT | NT | 1 | | |
| 3 | + | | | 2.08 | NT | 1.39 | 1 | | 1.0 |
| 4 | + | | | 1.17 | NT | NT | 1.6 | | |
| 5 | + | - | | 0.80 | 0.89 | NT | 1.3 | 1.3 | |
| 6 | + | | | 1.35 | NT | NT | 1 | | |
| 7 | + | | | 1.20 | 1.11 | NT | 3.3 | | |
| 8 | + | - | | 0.52 | 1.03 | NT | 1.46 | 1.5 | |
| 9 | + | | + | 1.52 | NT | 1.12 | 1.75 | | 1.0 |
| 10 | + | | | 1.21 | NT | NT | 1 | | |
| 11 | + | - | | 0.01 | 1.06 | 1.11 | 1.23 | 2.1 | 1.0 |
| 12 | + | | | 2.06 | NT | NT | 0.11 | | |
| 13 | + | | | 1.18 | NT | NT | 0.05 | | |
| 14 | + | | | 0.84 | NT | NT | 0.25 | | |
| 15 | + | | | 2.89 | NT | NT | 0.18 | | |
| 16 | + | | | 1.00 | NT | NT | 0.1 | | |
| 17 | + | | | 1.09 | NT | NT | 0.27 | | |
| 18 | + | + | | 0.57 | 0.62 | NT | 3.3 | 3.3 | |
| 19 | + | | | 1.20 | NT | NT | 0.13 | | |
| 20 | + | | | 1.38 | NT | NT | 0.1 | | |
| 21 | + | | | 2.04 | NT | NT | 0.49 | | |
| 22 | + | | | 1.31 | NT | NT | 0.5 | | |
| 23 | + | | | 1.63 | NT | NT | 0.54 | | |
| 24 | + | + | + | 1.69 | NT | 1.76 | 0.53 | | 0.53 |
| 25 | - | | + | NT | NT | 0.96 | | | 0.59 |
| 26 | + | | | 1.31 | NT | NT | 0.61 | | |
| 27 | + | | | 1.02 | NT | NT | 0.39 | | |

| Antibody number | Human P2X4 binding | Cynomologus P2X4 binding | Mouse P2X4 binding | Fraction of control at Human P2X4 | Fraction of control at Cyno P2X4 | Fraction of control at Mouse P2X4 | Concentration in Human ephys assay (mg/ml) | Concentration in Cyno ephys assay (mg/ml) | Concentration in Mouse ephys assay (mg/ml) |
|---|---|---|---|---|---|---|---|---|---|
| 28 | + | + | | 1.41 | NT | NT | 0.56 | | |
| 29 | + | - | | 0.27 | 1.11 | NT | 4.2 | 1.1 | |
| 30 | + | | | 1.28 | NT | NT | 0.63 | | |
| 31 | + | | | 2.41 | NT | 1.37 | 0.35 | | 0.33 |
| 32 | + | | | 0.84 | NT | 1.04 | 0.61 | | 0.61 |
| 33 | + | - | | 0.62 | 0.95 | NT | 3.8 | 3.8 | |
| 34 | + | + | + | 1.10 | NT | NT | 1.16 | | |

| Key | |
|---|---|
| + | Binding observed in FMAT assay |
| - | No binding observed in FMAT assay |
| NT | Not tested in assay |
| blank | Data not disclosed |

**Figure 4: Lead Panel Ephys**

| | | Fraction of control at human P2X4 | | | Fraction of control at cyno P2X4 | | |
|---|---|---|---|---|---|---|---|
| Antibody number | Conc (mg/ml) | Mean | SEM | n | Mean | SEM | n |
| 5 | 1.3 | 0.74 | 0.037 | 7 | | | |
| 8 | 1.5 | 0.53 | 0.011 | 8 | | | |
| 11 | 2 | 0.03 | 0.031 | 7 | | | |
| 18 | 3.3 | 0.56 | 0.010 | 6 | 0.62 | 0.009 | 11 |
| 29 | 4.2 | 0.27 | 0.013 | 18 | | | |
| 33 | 3.8 | 0.62 | 0.024 | 4 | | | |

**Figure 5: Summary of human cross reactive hybridoma antibodies (Abs)**

| Antibody number | Human P2X4 binding | Cynomologus P2X4 binding | Mouse P2X4 binding | Concentration (µM) | Fraction of control at Mouse P2X4 | Fraction of control at Mouse P2X4 (repeat | N | SD | Concen tration (µM) | Fraction of control at Human P2X4 | N | SD |
|---|---|---|---|---|---|---|---|---|---|---|---|---|
| 35 | + | | + | 0.80 | 0.461 | 0.194 | 3 | 0.157 | 0.689 | 0.648 | 3 | 0.120 |
| 36 | + | | + | 2.18 | 0.051 | 0.045 | 3 | 0.018 | 2.067 | 0.604 | 2 | 0.207 |
| 37 | + | | + | 1.38 | 0.270 | 0.242 | 4 | 0.068 | 2.200 | 1.056 | 3 | 0.139 |
| 38 | + | | + | 0.269 | 0.178 | 0.157 | 3 | 0.021 | 0.733 | 0.486 | 2 | 0.085 |
| 39 | + | | + | 0.941 | -0.067 | 0.068 | 3 | 0.074 | | NT | | |
| 40 | + | | + | 1.17 | 0.231 | 0.191 | 2 | 0.018 | 1.711 | 0.815 | 3 | 0.178 |
| 41 | + | | + | 0.49 | 0.072 | 0.195 | 4 | 0.033 | 0.378 | 0.633 | 4 | 0.103 |
| 42 | + | | + | 0.724 | 0.333 | 0.193 | 3 | 0.075 | 1.089 | 0.414 | 3 | 0.071 |
| 43 | + | + | + | 0.571 | 0.307 | 0.342 | 3 | 0.060 | 2.044 | 0.395 | 3 | 0.058 |
| 44 | + | | + | 0.23 | 0.253 | 0.364 | 4 | 0.107 | | NT | | |
| 45 | + | | + | 1.05 | 0.281 | 0.298 | 1 | | 1.844 | 0.620 | 3 | 0.140 |
| 46 | + | | + | 2.64 | -0.079 | -0.053 | 4 | 0.095 | 1.622 | 0.383 | 2 | 0.036 |
| 47 | + | | + | 1.07 | 0.196 | 0.174 | 3 | 0.089 | 0.311 | 0.912 | 3 | 0.112 |
| 48 | + | | + | 1.51 | 0.041 | 0.019 | 3 | 0.076 | 1.956 | 0.560 | 3 | 0.096 |

| Key | Description |
|---|---|
| + | Binding observed in FMAT assay |
| - | No binding observed in FMAT assay |
| Blank or NT | Data not disclosed / not tested |

**Figure 6 part 1: Hybridoma derived P2X4 Antibodies (Abs) VH sequences**

| Antibody number | FW 1 | | | | | | | | | | | | | | | | | | | | | | | | | | | | | CDR 1 | | | | | | | | |
|---|---|---|---|---|---|---|---|---|---|---|---|---|---|---|---|---|---|---|---|---|---|---|---|---|---|---|---|---|---|---|---|---|---|---|---|---|---|---|
| Kabat numbering | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | a | b | c | d |
| 35 | E | I | Q | L | Q | E | S | G | P | G | L | V | K | P | S | Q | S | L | S | L | T | C | S | V | T | G | Y | T | I | T | S | G | Y | D | - | - | - | - | - |
| 36 | Q | V | Q | L | K | E | S | G | P | G | L | V | K | P | S | E | T | L | S | L | T | C | T | V | S | G | F | S | L | T | N | Y | H | V | N | - | - | - | - |
| 37 | Q | V | N | L | L | Q | S | G | A | A | L | V | K | P | G | A | S | V | K | L | S | C | K | A | S | G | Y | T | F | T | D | Y | Y | I | H | - | - | - | - |
| 38 | E | I | Q | L | Q | E | S | G | P | G | L | V | K | P | S | Q | S | L | S | L | T | C | S | V | T | G | Y | T | I | T | S | G | Y | D | - | - | - | - | - |
| 39 | E | I | Q | L | Q | E | S | G | P | G | L | V | K | P | S | Q | S | L | S | L | T | C | S | V | T | G | Y | T | I | T | S | G | S | D | - | - | - | - | - |
| 40 | Q | V | T | L | K | E | S | G | P | G | I | L | Q | P | S | Q | T | L | S | L | T | C | S | F | S | G | F | S | L | S | T | F | G | I | C | V | S | - | - |
| 41 | Q | V | Q | L | K | E | S | G | P | G | L | V | K | P | S | E | T | L | S | L | T | C | T | V | S | G | F | S | L | T | S | Y | H | V | R | - | - | - | - |
| 42 | E | I | Q | L | Q | E | S | G | P | G | L | V | K | P | S | Q | S | L | S | L | T | C | S | V | T | G | Y | T | I | T | S | G | Y | D | - | - | - | - | - |
| 43 | Q | V | Q | L | K | E | S | G | P | G | L | V | Q | P | S | Q | T | L | S | L | T | C | T | V | S | G | F | S | L | T | R | Y | D | V | H | - | - | - | - |
| 44 | Q | V | T | L | K | E | S | G | P | G | I | L | Q | P | S | Q | T | L | S | L | T | C | S | F | S | G | F | S | L | N | T | Y | G | I | C | V | S | - | - |
| 45 | E | I | Q | L | Q | E | S | G | P | G | L | V | K | P | S | Q | S | L | S | L | T | C | S | V | T | G | Y | T | I | T | S | G | F | D | - | - | - | - | - |
| 46 | Q | V | Q | L | K | E | S | G | P | G | L | V | K | P | S | E | T | L | S | L | T | C | T | V | S | G | F | S | L | T | S | Y | H | V | S | - | - | - | - |
| 47 | E | I | Q | L | Q | E | S | G | P | G | L | V | K | P | S | Q | S | L | S | L | T | C | S | V | T | G | Y | T | I | T | S | G | F | D | - | - | - | - | - |
| 48 | Q | V | Q | L | K | E | S | G | P | G | L | V | K | P | S | E | T | L | S | L | T | C | T | V | S | G | F | S | L | T | S | Y | H | V | S | - | - | - | - |

**Figure 6 part 2: Hybridoma derived P2X4 Antibodies (Abs) VH sequences**

| Antibody number | FW 2 | | | | | | | | | | | | | | CDR 2 | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|
| Kabat numbering | 36 | 37 | 38 | 39 | 40 | 41 | 42 | 43 | 44 | 45 | 46 | 47 | 48 | 49 | 50 | 51 | 52 | a | b | c | d | e | f | 53 | 54 | 55 | 56 | 57 | 58 | 59 | 60 | 61 | 62 | 63 | 64 | 65 |
| 35 | W | S | W | I | R | K | F | P | G | N | K | M | E | W | M | G | Y | I | S | - | - | - | - | Y | S | G | S | T | N | Y | N | P | S | L | K | S |
| 36 | W | V | R | Q | P | P | G | K | G | L | E | W | M | G | V | I | W | - | - | - | - | - | - | G | D | G | S | T | A | Y | N | S | A | L | K | S |
| 37 | W | V | K | Q | S | H | G | M | S | L | E | W | I | G | L | I | N | P | - | - | - | - | - | D | S | G | Y | P | N | Y | N | E | N | F | K | G |
| 38 | W | S | W | I | R | K | F | P | G | N | K | M | E | W | M | G | Y | I | S | - | - | - | - | Y | S | G | N | T | N | Y | N | P | S | L | K | S |
| 39 | W | T | W | I | R | K | F | P | G | N | K | M | E | W | M | G | Y | I | S | - | - | - | - | Y | S | G | S | T | N | Y | N | P | S | L | K | S |
| 40 | W | I | R | Q | P | S | G | K | G | L | E | W | L | A | I | I | C | - | - | - | - | - | - | W | E | D | S | K | G | Y | N | P | S | L | K | N |
| 41 | W | V | R | Q | P | P | G | K | G | L | E | W | M | G | A | I | W | - | - | - | - | - | - | G | D | G | S | T | A | Y | N | S | A | L | K | S |
| 42 | W | S | W | I | R | K | F | P | G | N | K | M | E | W | M | G | Y | I | S | - | - | - | - | Y | S | G | S | T | N | Y | N | P | S | L | K | S |
| 43 | W | V | R | Q | P | P | G | K | G | L | E | W | M | G | G | I | W | - | - | - | - | - | - | G | D | G | S | T | D | Y | N | S | A | L | K | S |
| 44 | W | I | R | Q | P | S | G | K | G | L | E | W | L | A | T | I | C | - | - | - | - | - | - | W | E | D | S | K | V | Y | N | P | S | L | K | N |
| 45 | W | S | W | F | R | K | F | P | G | N | K | M | E | W | M | G | Y | I | S | - | - | - | - | Y | S | G | S | T | N | Y | N | P | S | L | K | S |
| 46 | W | V | R | Q | P | P | G | K | G | L | E | W | M | G | I | I | W | - | - | - | - | - | - | G | D | G | S | T | A | Y | N | S | A | L | K | S |
| 47 | W | S | W | F | R | K | F | P | G | N | K | M | E | W | M | G | Y | I | S | - | - | - | - | Y | S | G | I | T | N | Y | N | P | S | L | K | S |
| 48 | W | V | R | Q | P | P | G | K | G | L | E | W | M | G | V | I | W | - | - | - | - | - | - | G | D | G | S | T | A | F | N | S | A | L | K | S |

**Figure 6 part 3: Hybridoma derived P2X4 Antibodies (Abs) VH sequences**

| Antibody number | FW 3 | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| Kabat numbering | 66 | 67 | 68 | 69 | 70 | 71 | 72 | 73 | 74 | 75 | 76 | 77 | 78 | 79 | 80 | 81 | 82 | a | b | c | d | e | 83 | 84 | 85 | 86 | 87 | 88 | 89 | 90 | 91 | 92 |
| 35 | R | I | S | I | T | R | D | T | S | K | N | Q | F | F | L | Q | L | N | S | V | - | - | T | T | E | D | T | A | T | Y | Y | C |
| 36 | R | L | S | I | S | R | D | T | S | K | S | Q | V | F | L | K | M | N | S | L | - | - | Q | T | E | D | T | A | T | Y | Y | C |
| 37 | K | A | T | L | T | V | D | K | S | T | N | T | A | Y | M | E | L | R | R | L | - | - | T | S | E | D | S | A | T | Y | Y | C |
| 38 | R | I | S | I | T | R | D | T | S | K | N | Q | F | F | L | Q | L | N | S | V | - | - | T | T | E | D | I | A | T | Y | Y | C |
| 39 | R | I | S | I | T | R | D | T | S | K | N | Q | F | F | L | Q | L | N | S | V | - | - | T | T | E | D | T | A | T | Y | Y | C |
| 40 | R | L | T | I | S | K | D | T | S | N | N | Q | A | F | L | K | I | S | S | V | - | - | D | T | A | D | T | A | I | Y | Y | C |
| 41 | R | L | S | I | S | R | D | T | S | R | S | Q | V | F | L | K | M | D | S | L | - | - | Q | T | E | D | T | A | T | Y | Y | C |
| 42 | R | I | S | I | T | R | D | T | S | K | N | Q | F | F | L | Q | L | N | S | V | - | - | T | T | E | D | I | A | T | Y | Y | C |
| 43 | R | L | S | I | S | R | D | T | S | K | S | Q | V | F | L | K | M | N | S | L | - | - | Q | T | E | D | T | A | I | Y | F | C |
| 44 | R | L | T | I | S | K | D | T | S | N | N | Q | A | F | L | K | I | T | S | V | - | - | D | T | A | D | T | A | I | Y | Y | C |
| 45 | R | I | S | I | T | R | D | T | S | K | N | Q | F | F | L | Q | L | N | S | V | - | - | T | T | E | D | T | A | T | Y | Y | C |
| 46 | R | L | S | I | S | R | D | T | S | K | S | Q | V | F | L | K | M | N | S | L | - | - | Q | T | E | D | T | A | T | Y | Y | C |
| 47 | R | I | S | I | T | R | D | T | S | K | N | Q | F | F | L | Q | L | N | S | V | - | - | T | T | E | D | T | A | T | Y | Y | C |
| 48 | R | L | S | I | S | R | D | T | S | K | S | Q | V | F | L | K | M | N | S | L | - | - | Q | T | E | D | T | A | T | Y | Y | C |

| Antibody number | FW 3 | CDR 3 | | | | | | | | | | | | | | | | | | | | | | | | | |
|---|---|---|---|---|---|---|---|---|---|---|---|---|---|---|---|---|---|---|---|---|---|---|---|---|---|---|---|
| Kabat numbering | 93 | 94 | 95 | 96 | 97 | 98 | 99 | 100 | a | b | c | d | e | f | g | h | i | j | k | l | m | n | o | p | q | 101 | 1 |
| 35 | A | R | G | M | M | V | L | I | - | - | - | - | - | - | - | - | - | - | - | - | - | - | - | - | - | P | |
| 36 | A | R | G | G | D | Y | Y | D | G | S | Y | Y | Y | E | - | - | - | - | - | - | - | - | - | - | - | G | Y |
| 37 | T | R | S | R | I | Y | Y | D | G | S | V | F | - | - | - | - | - | - | - | - | - | - | - | - | - | D | Y |
| 38 | G | R | G | M | V | V | L | V | - | - | - | - | - | - | - | - | - | - | - | - | - | - | - | - | - | S | T |
| 39 | A | S | G | Y | I | Y | - | - | - | - | - | - | - | - | - | - | - | - | - | - | - | - | - | - | - | D | Y |
| 40 | A | R | R | S | V | M | Y | T | T | A | P | Y | Y | F | - | - | - | - | - | - | - | - | - | - | - | D | Y |
| 41 | A | R | A | G | H | Y | S | D | G | S | Y | Y | Y | G | - | - | - | - | - | - | - | - | - | - | - | A | Y |
| 42 | A | R | G | M | M | V | L | I | - | - | - | - | - | - | - | - | - | - | - | - | - | - | - | - | - | S | N |
| 43 | T | R | S | L | D | Y | S | G | D | G | F | - | - | - | - | - | - | - | - | - | - | - | - | - | - | G | Y |
| 44 | A | R | R | R | V | W | S | Y | Y | F | - | - | - | - | - | - | - | - | - | - | - | - | - | - | - | D | Y |
| 45 | A | R | G | V | S | S | L | S | - | - | - | - | - | - | - | - | - | - | - | - | - | - | - | - | - | A | Y |
| 46 | V | R | A | G | H | Y | S | D | G | S | Y | Y | Y | G | - | - | - | - | - | - | - | - | - | - | - | A | Y |
| 47 | A | R | G | V | S | S | L | S | - | - | - | - | - | - | - | - | - | - | - | - | - | - | - | - | - | A | Y |
| 48 | A | R | A | G | V | Y | Y | D | G | S | Y | Y | Y | F | - | - | - | - | - | - | - | - | - | - | - | A | Y |

**Figure 6 part 4: Hybridoma derived P2X4 Antibodies (Abs) VH sequences**

| Antibody number | FW 4 | | | | | | | | | | |
|---|---|---|---|---|---|---|---|---|---|---|---|
| Kabat numbering | 103 | 104 | 105 | 106 | 107 | 108 | 109 | 110 | 111 | 112 | 113 |
| 35 | W | G | Q | G | V | M | V | T | V | S | S |
| 36 | W | G | Q | G | V | M | V | T | V | S | S |
| 37 | W | G | Q | G | V | M | V | T | V | S | S |
| 38 | W | G | Q | G | V | M | V | T | V | S | S |
| 39 | W | G | R | G | V | M | V | T | V | S | S |
| 40 | W | G | Q | G | V | M | V | T | V | S | S |
| 41 | W | G | Q | G | T | L | V | T | V | S | S |
| 42 | W | G | Q | G | V | M | V | T | V | S | S |
| 43 | W | G | Q | G | T | L | V | T | V | S | S |
| 44 | W | G | Q | G | V | M | V | T | V | S | S |
| 45 | W | G | Q | G | T | L | V | T | V | S | S |
| 46 | W | G | Q | G | T | L | V | T | V | S | S |
| 47 | W | G | Q | G | T | L | V | T | V | S | S |
| 48 | W | G | Q | G | T | L | V | T | V | S | S |

## Figure 6 part 5: Hybridoma derived P2X4 Antibodies (Abs) VL sequences

| Antibody number | FW 1 | | | | | | | | | | | | | | | | | | | | | | CDR 1 | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|
| Kabat numbering | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | a | b | c | d | e | f | g | h | 28 | 29 | 30 | 31 | 32 | 33 | 34 |
| 35 | D | V | Q | M | T | Q | S | P | S | Y | L | T | A | S | P | G | E | S | V | S | I | S | C | K | A | S | K | - | - | - | - | - | - | - | - | S | I | T | N | Y | L | A |
| 36 | D | I | Q | M | T | Q | S | P | A | S | L | S | A | S | L | E | E | I | V | T | I | T | C | Q | A | S | Q | - | - | - | - | - | - | - | - | D | I | G | N | W | L | S |
| 37 | E | I | V | L | T | Q | S | P | T | T | M | A | V | S | P | G | E | K | V | T | I | T | C | R | A | R | S | - | - | - | - | - | - | - | - | - | S | V | S | Y | M | Y |
| 38 | D | V | Q | M | T | Q | S | P | S | Y | L | A | A | S | P | G | E | S | V | S | I | S | C | K | T | S | K | - | - | - | - | - | - | - | - | S | I | T | H | Y | L | A |
| 39 | D | V | Q | M | T | Q | S | P | S | Y | L | A | A | S | P | G | E | S | V | S | I | S | C | K | A | N | K | - | - | - | - | - | - | - | - | R | I | T | N | Y | L | A |
| 40 | D | I | Q | M | T | Q | S | P | A | S | L | S | A | S | L | E | E | I | V | T | I | T | C | Q | A | S | Q | - | - | - | - | - | - | - | - | D | I | G | N | W | L | A |
| 41 | D | I | Q | M | T | Q | S | P | A | S | L | S | A | S | L | E | E | I | V | T | I | T | C | Q | A | S | Q | - | - | - | - | - | - | - | - | D | I | G | N | W | L | A |
| 42 | D | V | Q | M | T | Q | S | P | S | Y | L | A | A | S | P | G | E | S | V | S | I | S | C | K | A | S | K | - | - | - | - | - | - | - | - | S | I | T | H | Y | L | A |
| 43 | D | I | Q | M | T | Q | S | P | P | S | L | S | A | S | L | G | D | K | V | T | I | T | C | Q | A | S | Q | - | - | - | - | - | - | - | - | N | I | N | K | Y | I | A |
| 44 | D | I | Q | M | T | Q | S | P | A | S | L | S | A | S | L | E | E | I | V | T | I | T | C | Q | A | S | Q | - | - | - | - | - | - | - | - | D | I | G | N | W | L | A |
| 45 | D | V | Q | M | T | Q | S | P | S | Y | L | A | A | S | P | G | E | S | V | S | I | S | C | K | A | S | K | - | - | - | - | - | - | - | - | L | I | T | N | Y | L | A |
| 46 | D | I | Q | M | T | Q | S | P | A | S | L | S | A | S | L | E | E | I | V | T | I | T | C | Q | A | S | Q | - | - | - | - | - | - | - | - | D | I | G | N | W | L | A |
| 47 | D | V | Q | M | T | Q | S | P | S | Y | L | A | A | S | P | G | E | S | V | S | I | S | C | K | A | S | K | - | - | - | - | - | - | - | - | I | I | T | N | Y | L | A |
| 48 | D | I | Q | M | T | Q | S | P | A | S | L | S | A | S | L | G | E | I | V | T | I | T | C | Q | A | S | Q | - | - | - | - | - | - | - | - | D | I | G | N | W | L | A |

**Figure 6 part 6: Hybridoma derived P2X4 Antibodies (Abs) VL sequences**

| Antibody number | FW 2 | | | | | | | | | | | | | | | CDR 2 | | | | | | | | | | | | |
|---|---|---|---|---|---|---|---|---|---|---|---|---|---|---|---|---|---|---|---|---|---|---|---|---|---|---|---|---|
| Kabat numbering | 35 | 36 | 37 | 38 | 39 | 40 | 41 | 42 | 43 | 44 | 45 | 46 | 47 | 48 | 49 | 50 | 51 | a | b | c | d | e | 52 | 53 | 54 | 55 | 56 |
| 35 | W | Y | H | Q | K | P | G | E | P | Y | N | L | L | I | Y | S | G | - | - | - | - | - | S | T | L | Q | S |
| 36 | W | Y | Q | Q | K | P | G | K | S | P | Q | L | L | I | Y | D | A | - | - | - | - | - | T | S | L | A | D |
| 37 | W | Y | Q | Q | K | S | G | A | S | P | K | P | W | I | Y | E | T | - | - | - | - | - | S | K | L | A | S |
| 38 | W | Y | Q | Q | K | P | G | E | A | F | K | L | L | I | Y | S | G | - | - | - | - | - | S | T | L | Q | S |
| 39 | W | Y | Q | Q | K | P | G | E | A | N | K | L | L | I | Y | S | G | - | - | - | - | - | S | T | L | Q | S |
| 40 | W | F | Q | Q | K | P | G | K | S | P | Q | L | L | I | Y | D | A | - | - | - | - | - | T | T | L | A | D |
| 41 | W | Y | Q | Q | K | P | G | K | S | P | Q | L | L | L | Y | D | A | - | - | - | - | - | T | S | L | A | D |
| 42 | W | Y | Q | Q | K | P | G | E | A | Y | K | L | L | I | Y | S | G | - | - | - | - | - | S | T | L | Q | S |
| 43 | W | Y | Q | Q | K | P | G | K | A | P | R | Q | L | I | R | Y | T | - | - | - | - | - | S | A | L | V | S |
| 44 | W | Y | Q | Q | K | P | G | K | S | P | Q | L | L | I | Y | D | A | - | - | - | - | - | T | S | L | A | D |
| 45 | W | Y | Q | Q | K | P | G | E | P | Y | N | L | L | I | Y | S | G | - | - | - | - | - | S | T | L | Q | S |
| 46 | W | Y | Q | Q | K | P | G | K | S | P | Q | L | L | I | Y | D | A | - | - | - | - | - | T | T | L | A | D |
| 47 | W | Y | Q | Q | K | P | G | E | P | Y | N | L | L | I | H | S | G | - | - | - | - | - | S | T | L | Q | S |
| 48 | W | Y | Q | Q | K | P | G | K | S | P | H | L | L | I | Y | D | A | - | - | - | - | - | T | T | L | A | D |

**Figure 6 part 7: Hybridoma derived P2X4 Antibodies (Abs) VL sequences**

| Antibody number | FW 3 | | | | | | | | | | | | | | | | | | | | | | | | | | | | | | | | | CDR 3 | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| Kabat numbering | 57 | 58 | 59 | 60 | 61 | 62 | 63 | 64 | 65 | 66 | 67 | 68 | a | b | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | a | b | c | d | e | f | g | h | i | 96 | 97 |
| 35 | G | T | P | S | R | F | S | G | S | R | S | G | - | - | T | D | F | I | L | T | I | R | S | L | E | P | E | D | F | G | L | Y | Y | C | Q | Q | Y | Y | E | K | P | - | - | - | - | - | - | - | - | - | Y | T |
| 36 | G | V | P | S | R | F | S | G | S | R | S | G | - | - | T | Q | Y | S | L | K | I | S | R | L | Q | V | E | D | I | G | I | Y | Y | C | L | Q | A | H | S | N | P | - | - | - | - | - | - | - | - | - | W | T |
| 37 | G | V | P | D | R | F | S | G | S | G | S | G | - | - | T | S | Y | S | F | T | I | S | S | M | E | T | E | D | A | A | T | Y | Y | C | H | Q | W | S | R | T | P | - | - | - | - | - | - | - | - | - | P | T |
| 38 | G | T | P | S | R | F | I | G | S | G | A | V | - | - | T | D | F | T | L | T | I | R | S | L | E | P | E | D | F | G | L | Y | Y | C | Q | Q | Y | Y | E | N | P | - | - | - | - | - | - | - | - | - | Y | T |
| 39 | G | T | P | S | R | F | S | G | S | G | S | G | - | - | T | D | F | T | L | T | I | R | N | L | E | P | E | D | F | A | V | Y | Y | C | Q | Q | Y | Y | E | K | P | - | - | - | - | - | - | - | - | - | L | T |
| 40 | G | V | P | S | R | F | S | G | S | R | S | G | - | - | T | Q | F | S | L | K | I | N | R | L | Q | V | E | D | I | G | S | Y | Y | C | Q | Q | A | H | N | N | P | - | - | - | - | - | - | - | - | - | N | T |
| 41 | G | V | P | S | R | F | S | G | S | R | S | G | - | - | T | Q | Y | S | L | K | I | S | R | L | Q | V | E | D | I | G | S | Y | Y | C | Q | Q | A | H | S | N | P | - | - | - | - | - | - | - | - | - | R | T |
| 42 | G | T | P | S | R | F | I | G | T | G | A | V | - | - | T | D | F | T | L | T | I | R | S | L | E | P | E | D | F | G | L | Y | Y | C | Q | Q | Y | Y | E | K | P | - | - | - | - | - | - | - | - | - | Y | T |
| 43 | G | T | S | S | R | F | S | G | S | G | S | G | - | - | R | D | Y | S | F | S | I | S | N | V | E | S | E | D | I | A | S | Y | Y | C | L | Q | Y | D | N | L | P | - | - | - | - | - | - | - | - | - | Y | T |
| 44 | G | V | P | S | R | F | S | G | S | R | S | G | - | - | T | Q | Y | S | L | K | I | S | R | L | Q | V | E | D | I | G | S | Y | Y | C | Q | Q | A | H | T | N | P | - | - | - | - | - | - | - | - | - | L | T |
| 45 | G | T | P | S | R | F | S | G | S | R | S | G | - | - | T | D | F | T | L | T | I | R | S | L | Q | P | E | D | F | G | L | Y | Y | C | Q | Q | Y | Y | E | K | P | - | - | - | - | - | - | - | - | - | Y | T |
| 46 | G | V | P | S | R | F | S | G | S | R | S | G | - | - | T | Q | Y | S | L | K | I | S | R | L | Q | V | E | D | I | G | S | Y | Y | C | Q | Q | T | H | S | H | P | - | - | - | - | - | - | - | - | - | R | T |
| 47 | G | T | P | S | R | F | S | G | S | R | S | G | - | - | T | D | F | T | L | T | I | R | S | L | Q | P | E | D | F | G | L | Y | Y | C | Q | Q | Y | Y | E | N | P | - | - | - | - | - | - | - | - | - | Y | T |
| 48 | G | V | P | S | R | F | S | G | S | R | S | G | - | - | T | Q | Y | S | L | K | I | S | R | L | Q | V | E | D | I | G | S | Y | Y | C | Q | K | A | H | S | N | P | - | - | - | - | - | - | - | - | - | W | T |

## Figure 6 part 8: Hybridoma derived P2X4 Antibodies (Abs) VL sequences

| Antibody number | FW 4 | | | | | | | | | | | | |
|---|---|---|---|---|---|---|---|---|---|---|---|---|---|
| Kabat numbering | 98 | 99 | 100 | 101 | 102 | 103 | 104 | 105 | 106 | a | b | c | 107 |
| 35 | F | G | A | G | T | K | L | E | L | - | - | - | K |
| 36 | F | G | G | G | S | K | L | E | L | - | - | - | K |
| 37 | F | G | G | G | T | K | L | E | M | - | - | - | R |
| 38 | F | G | A | G | T | K | L | E | L | - | - | - | K |
| 39 | F | G | S | G | T | K | L | E | I | - | - | - | K |
| 40 | F | G | P | G | T | K | L | E | L | - | - | - | K |
| 41 | F | G | G | G | T | K | L | E | L | - | - | - | K |
| 42 | F | G | A | G | T | K | L | E | L | - | - | - | K |
| 43 | F | G | A | G | T | K | L | E | L | - | - | - | K |
| 44 | F | G | S | G | T | K | L | E | I | - | - | - | K |
| 45 | F | G | A | G | T | K | L | E | L | - | - | - | K |
| 46 | F | G | G | G | T | N | L | E | L | - | - | - | K |
| 47 | F | G | A | G | T | K | L | E | L | - | - | - | K |
| 48 | F | G | G | G | T | K | L | E | L | - | - | - | K |

## Figure 7: Part 1a - Antibodies (Abs) Binding and Function: Hybridoma P2X4 binding antibodies (all clones)

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (µg/ml) |
|---|---|---|---|---|---|---|
| 35 | + | | + | 0.648 | 0.461 | 119 |
| 36 | + | - | + | 0.604 | 0.051 | 327 |
| 37 | + | | + | 1.056 | 0.270 | 207 |
| 38 | + | - | + | 0.486 | 0.178 | 40 |
| 39 | + | - | + | | -0.067 | 141 |
| 40 | + | | + | 0.815 | 0.231 | 176 |
| 41 | + | | + | 0.633 | 0.072 | 73 |
| 42 | + | | + | 0.414 | 0.333 | 109 |
| 43 | + | + | + | 0.395 | 0.307 | 86 |
| 44 | + | | + | | 0.253 | 35 |
| 45 | + | | + | 0.620 | 0.281 | 158 |
| 46 | + | - | + | 0.383 | -0.079 | 396 |
| 47 | + | - | + | 0.912 | 0.196 | 161 |
| 48 | + | - | + | 0.560 | 0.041 | 227 |
| 49 | | | + | | 0.940 | 53 |
| 50 | | | + | | 0.584 | 246 |
| 51 | | | + | | 0.516 | 57 |
| 52 | | | + | | 0.569 | 81 |
| 53 | | | + | | 3.187 | 113 |
| 54 | | | + | | 0.570 | 104 |
| 55 | | | + | | 0.607 | 86 |
| 56 | | | + | | 1.080 | 113 |
| 57 | | | + | | 0.651 | <LOD |
| 58 | | | + | | 0.486 | 179 |
| 59 | | | + | | 1.093 | 83 |
| 60 | | | + | | 1.733 | <LOD |
| 61 | + | | + | | 0.115 | 24 |
| 62 | | | + | | 1.177 | 20 |

| Key | |
|---|---|
| + | Binding observed in FMAT assay |
| - | No binding observed in FMAT assay |
| NT or Blank | Not tested in assay |
| < LOD | [IgG] below limit of detection |

**Figure 7: Part 1b**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (µg/ml) |
|---|---|---|---|---|---|---|
| 63 | | | + | | 0.976 | <LOD |
| 64 | | | + | | 1.893 | 67 |
| 65 | - | - | + | | 0.394 | 364 |
| 66 | | | + | | 1.574 | 85 |
| 67 | | | + | | 0.714 | 107 |
| 68 | | | + | | 1.767 | 107 |
| 69 | | | + | | 0.887 | 89 |
| 70 | | | + | | 0.794 | 161 |
| 71 | | | + | | 3.280 | 76 |
| 72 | | | + | | 0.662 | 337 |
| 73 | | | + | | 0.467 | 71 |
| 74 | | | + | | 3.767 | 95 |
| 75 | | | + | | 0.713 | 61 |
| 76 | | | + | | 0.793 | 91 |
| 77 | | | + | | 1.118 | 37 |
| 78 | | | + | | 0.763 | 54 |
| 79 | | | + | | 0.686 | 38 |
| 80 | | | + | | 0.650 | <LOD |
| 81 | | | + | | 0.832 | 199 |
| 82 | | | + | | 1.240 | 181 |
| 83 | | | + | | 1.091 | 372 |
| 84 | | | + | | 0.381 | 31 |
| 85 | | | + | | 1.821 | <LOD |
| 86 | | | + | | 1.153 | <LOD |
| 87 | | | + | | 0.760 | 212 |
| 88 | | | + | | 1.003 | 219 |

**Figure 7: Part 1c**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 89 | | | + | | 1.710 | 381 |
| 90 | | | + | | 0.533 | 210 |
| 91 | | | + | | 1.338 | 116 |
| 92 | | | + | | 2.653 | 80 |
| 93 | | | + | | 1.743 | 171 |
| 94 | | | + | | 2.033 | 136 |
| 95 | | | + | | 1.221 | 48 |
| 96 | | | + | | 0.539 | 89 |
| 97 | | | + | | 0.976 | 94 |
| 98 | | | + | | 0.564 | 69 |
| 99 | | | + | | 0.821 | 181 |
| 100 | | | + | | 0.907 | 30 |
| 101 | | | + | | 4.969 | 318 |
| 102 | | | + | | 0.778 | 321 |
| 103 | | | + | | 0.860 | 94 |
| 104 | | | + | | 0.720 | 311 |
| 105 | | | + | | 0.398 | 233 |
| 106 | | | + | | 0.703 | 233 |
| 107 | - | | + | 0.540 | 0.151 | 84 |
| 108 | | | + | | 1.007 | 258 |
| 109 | | | + | | 0.805 | 52 |
| 110 | | | + | | 0.780 | 89 |
| 111 | | | + | | 0.025 | 216 |
| 112 | | | + | | 0.650 | 86 |
| 113 | | | + | | 1.136 | 238 |
| 114 | | | + | | 1.567 | 398 |
| 115 | | | + | | 1.635 | 183 |

**Figure 7: Part 1d**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 115 | | | + | | 1.635 | 183 |
| 116 | | | + | | 1.287 | 141 |
| 117 | | | + | | 0.749 | 315 |
| 118 | - | - | + | | 0.398 | 103 |
| 119 | | | + | | 2.211 | 49 |
| 120 | | | + | | 0.845 | 317 |
| 121 | | | + | | 0.952 | 73 |
| 122 | | | + | | 0.982 | 103 |
| 123 | | | + | | 0.933 | 235 |
| 124 | | | + | | 0.711 | 120 |
| 125 | | | + | | 1.738 | 360 |
| 126 | | | + | | 1.792 | 106 |
| 127 | | | + | | 0.467 | 75 |
| 128 | | | + | | 0.542 | 76 |
| 129 | | | + | | 0.716 | 57 |
| 130 | | | + | | 0.595 | 138 |
| 131 | | | + | | 1.233 | 351 |
| 132 | | | + | | 0.560 | 118 |
| 133 | | | + | | 0.608 | 195 |
| 134 | | | + | | 1.455 | 97 |
| 135 | | | + | | 1.157 | 81 |
| 136 | | | + | | 0.679 | 70 |
| 137 | | | + | | 0.903 | 118 |
| 138 | | | + | | 1.521 | 295 |
| 139 | | | + | | 1.458 | 150 |
| 140 | | | + | | 0.573 | 118 |

**Figure 7: Part 1e**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 141 | | | + | | 0.892 | 231 |
| 142 | | | + | | 1.505 | 367 |
| 143 | | | + | | 0.770 | 269 |
| 144 | | | + | | 0.656 | 174 |
| 145 | - | + | + | | 0.150 | 69 |
| 146 | | | + | | 1.480 | 141 |
| 147 | | | + | | 0.727 | 60 |
| 148 | | | + | | 2.619 | 391 |
| 149 | | | + | | 1.602 | 379 |
| 150 | | | + | | 0.450 | 203 |
| 151 | | | + | | 1.011 | 293 |
| 152 | | | + | | 0.538 | 400 |
| 153 | | | + | | 1.928 | 282 |
| 154 | | | + | | 0.704 | 444 |
| 155 | | | + | | 0.928 | 279 |
| 156 | | | + | | 1.078 | <LOD |
| 157 | | | + | | 1.036 | 247 |
| 158 | | | + | | 1.306 | 298 |
| 159 | | | + | | 5.781 | 118 |
| 160 | | | + | | 0.918 | 180 |
| 161 | | | + | | 0.956 | 206 |
| 162 | | | + | | 1.388 | 155 |
| 163 | - | - | + | | 0.375 | 59 |
| 164 | | | + | | 0.546 | 268 |
| 165 | | | + | | 0.616 | <LOD |

**Figure 7: Part 1f**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 166 | | | + | | 0.810 | 182 |
| 167 | | | + | | 1.258 | 151 |
| 168 | | | + | | 1.808 | 88 |
| 169 | | | + | | 1.773 | 128 |
| 170 | | | + | | 0.690 | 93 |
| 171 | | | + | | 0.524 | 396 |
| 172 | - | - | + | 1.078 | 0.307 | 364 |
| 173 | | | + | | 0.702 | 154 |
| 174 | | | + | | 0.656 | 225 |
| 175 | | | + | | 1.223 | 286 |
| 176 | | | + | | 1.444 | 148 |
| 177 | | | + | | 0.728 | 251 |
| 178 | | | + | | 1.356 | 297 |
| 179 | | | + | | 0.988 | 332 |
| 180 | | | + | | 0.679 | 351 |
| 181 | | | + | | 1.392 | 89 |
| 182 | | | + | | 0.572 | <LOD |
| 183 | | | + | | 0.627 | 193 |
| 184 | | | + | | 0.373 | 84 |
| 185 | | | + | | 0.484 | 310 |
| 186 | | | + | | 1.320 | 257 |
| 187 | | | + | | 0.846 | 315 |
| 188 | | | + | | 0.645 | 114 |
| 189 | + | - | + | 0.593 | 0.082 | 208 |
| 190 | | | + | | 0.505 | 127 |
| 191 | | | + | | 0.932 | 97 |
| 192 | | | + | | 1.855 | 29 |
| 193 | | | + | | 0.660 | <LOD |
| 194 | | | + | | 0.940 | 225 |

**Figure 7: Part 1g**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 195 | | | + | | 0.896 | 110 |
| 196 | | | + | | 0.860 | 100 |
| 197 | | | + | | 0.666 | 155 |
| 198 | | | + | | 0.801 | 57 |
| 199 | | | + | | 0.650 | 413 |
| 200 | | | + | | 0.503 | 110 |
| 201 | | | + | | 0.584 | 124 |
| 202 | | | + | | 0.998 | 317 |
| 203 | | | + | | 0.690 | 283 |
| 204 | | | + | | 1.202 | 104 |
| 205 | | | + | | 0.707 | 279 |
| 206 | | | + | | 0.911 | 104 |
| 207 | | | + | | 0.629 | 324 |
| 208 | - | - | + | 0.937 | 0.072 | 296 |
| 209 | | | + | | 0.709 | 126 |
| 210 | | | + | | 1.506 | 411 |
| 211 | | | + | | 1.108 | 74 |
| 212 | | | + | | 1.031 | 91 |
| 213 | | | + | | 1.346 | 223 |
| 214 | | | + | | 0.873 | 85 |
| 215 | | | + | | 0.512 | 373 |
| 216 | | | + | | 1.177 | 124 |
| 217 | | | + | | 0.826 | 66 |
| 218 | | | + | | 0.711 | 73 |
| 219 | | | + | | 0.746 | 100 |
| 220 | | | + | | 0.645 | 139 |
| 221 | - | - | + | 1.009 | 0.163 | 363 |

**Figure 7: Part 1h**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 222 | | | + | | 1.317 | <LOD |
| 223 | | | + | | 1.071 | 102 |
| 224 | | | + | | 1.047 | 337 |
| 225 | | | + | | 0.539 | 81 |
| 226 | | | + | | 0.914 | 207 |
| 227 | | | + | | 0.781 | 111 |
| 228 | | | + | | 0.946 | 92 |
| 229 | | | + | | 0.760 | 135 |
| 230 | | | + | | 1.131 | 117 |
| 231 | | | + | | 1.840 | 262 |
| 232 | | | + | | 0.652 | 281 |
| 233 | | | + | | 0.714 | 486 |
| 234 | - | | + | 0.807 | 0.156 | 452 |
| 235 | | | + | | 0.660 | 287 |
| 236 | | | + | | 0.530 | 58 |
| 237 | | | + | | 0.725 | 355 |
| 238 | - | | + | 0.968 | 0.238 | 157 |
| 239 | | | + | | 1.064 | <LOD |
| 240 | | | + | | 1.899 | <LOD |
| 241 | | | + | | 0.877 | <LOD |
| 242 | | | + | | 1.059 | 230 |
| 243 | | | + | | 1.143 | 18 |
| 244 | | | + | | 0.787 | 171 |
| 245 | | | + | | 0.625 | 357 |
| 246 | | | + | | 1.059 | 64 |
| 247 | | | + | | 0.967 | 268 |
| 248 | | | + | | 0.747 | 32 |

**Figure 7: Part 1i**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 249 | | | + | | 0.535 | 153 |
| 250 | | | + | | 0.800 | 53 |
| 251 | | | + | | 0.460 | 199 |
| 252 | | | + | | 1.979 | <LOD |
| 253 | | | + | | 1.036 | 252 |
| 254 | | | + | | 1.097 | 144 |
| 255 | | | + | | 0.926 | 273 |
| 256 | | | + | | 0.688 | 392 |
| 257 | | | + | | 1.316 | <LOD |
| 258 | | | + | | 0.606 | 67 |
| 259 | | | + | | 1.145 | 57 |
| 260 | | | + | | 0.585 | 244 |
| 261 | | | + | | 0.691 | 79 |
| 262 | | | + | | 0.557 | 220 |
| 263 | | | + | | 1.286 | 92 |
| 264 | | | + | | 0.161 | 66 |
| 265 | | | + | | 1.076 | <LOD |
| 266 | - | | + | | 0.463 | 61 |
| 267 | | | + | | 0.443 | 49 |
| 268 | | | + | | 4.067 | 73 |
| 269 | | | + | | 0.757 | <LOD |
| 270 | | | + | | 0.802 | 91 |
| 271 | | | + | | 0.696 | 112 |
| 272 | | | + | | 0.832 | 158 |
| 273 | | | + | | 0.814 | 403 |
| 274 | | | + | | 0.801 | 300 |
| 275 | | | + | | 0.982 | 141 |

**Figure 7: Part 1j**

| Antibody number | Human P2X4 binding | Cynomolgus P2X4 binding | Mouse P2X4 binding | Fraction of control current at Human P2X4 (ephys) | Fraction of control current at Mouse P2X4 (ephys) | Concentration in ephys assay (mg/ml) |
|---|---|---|---|---|---|---|
| 276 | | | + | | 1.172 | 90 |
| 277 | | | + | | 0.936 | 136 |
| 278 | | | + | | 1.142 | 101 |
| 279 | | | + | | 0.640 | 359 |
| 280 | | | + | | 1.134 | 91 |
| 281 | - | - | + | 0.800 | 0.098 | 306 |
| 282 | | | + | | 1.342 | 25 |
| 283 | | | + | | 0.730 | 78 |
| 284 | | | + | | 1.665 | 94 |
| 285 | | | + | | 0.436 | 55 |
| 286 | | | + | | 0.753 | <LOD |

Figure 8A

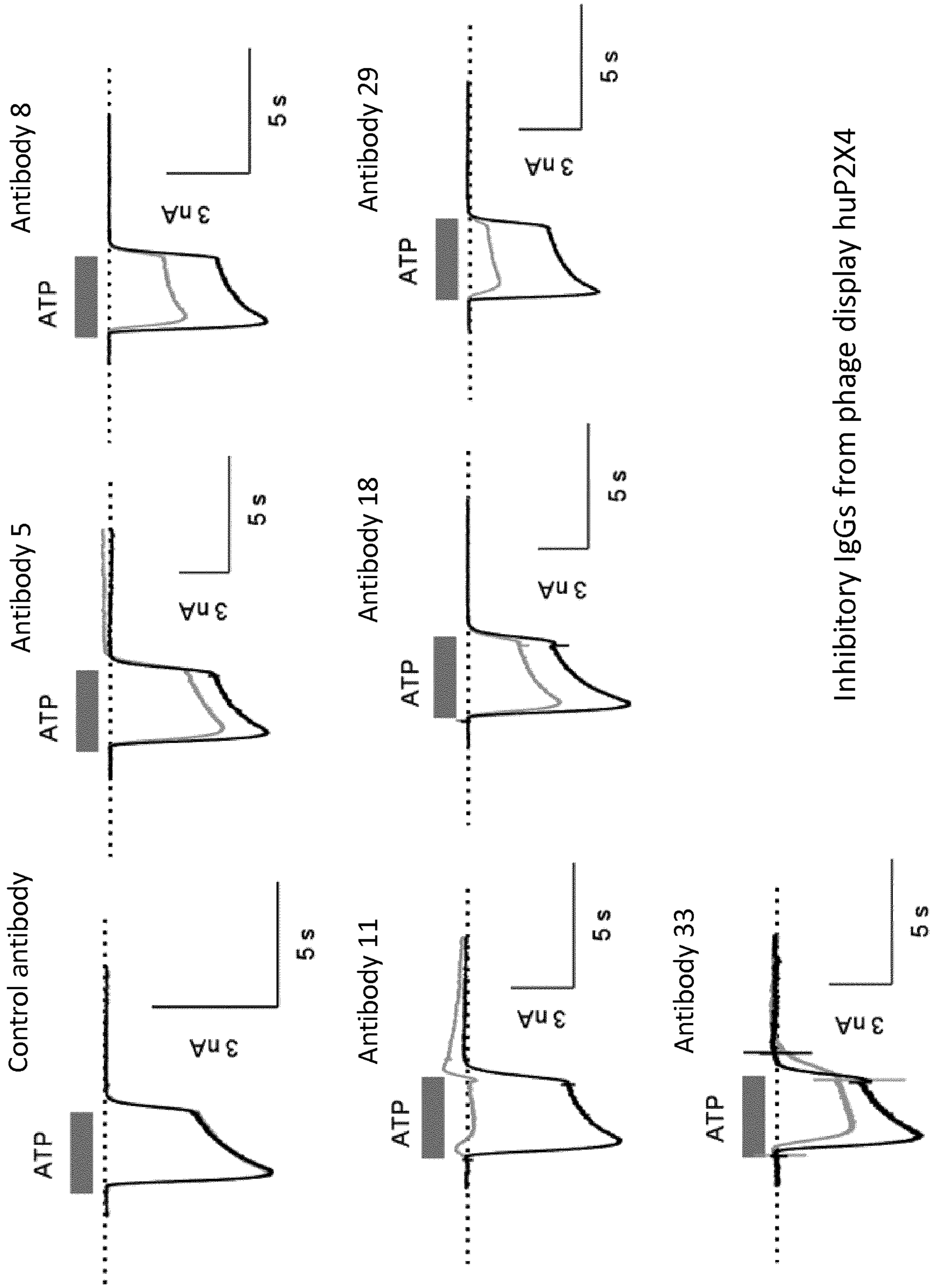


Inhibitory IgGs from phage display huP2X4

Figure 8B

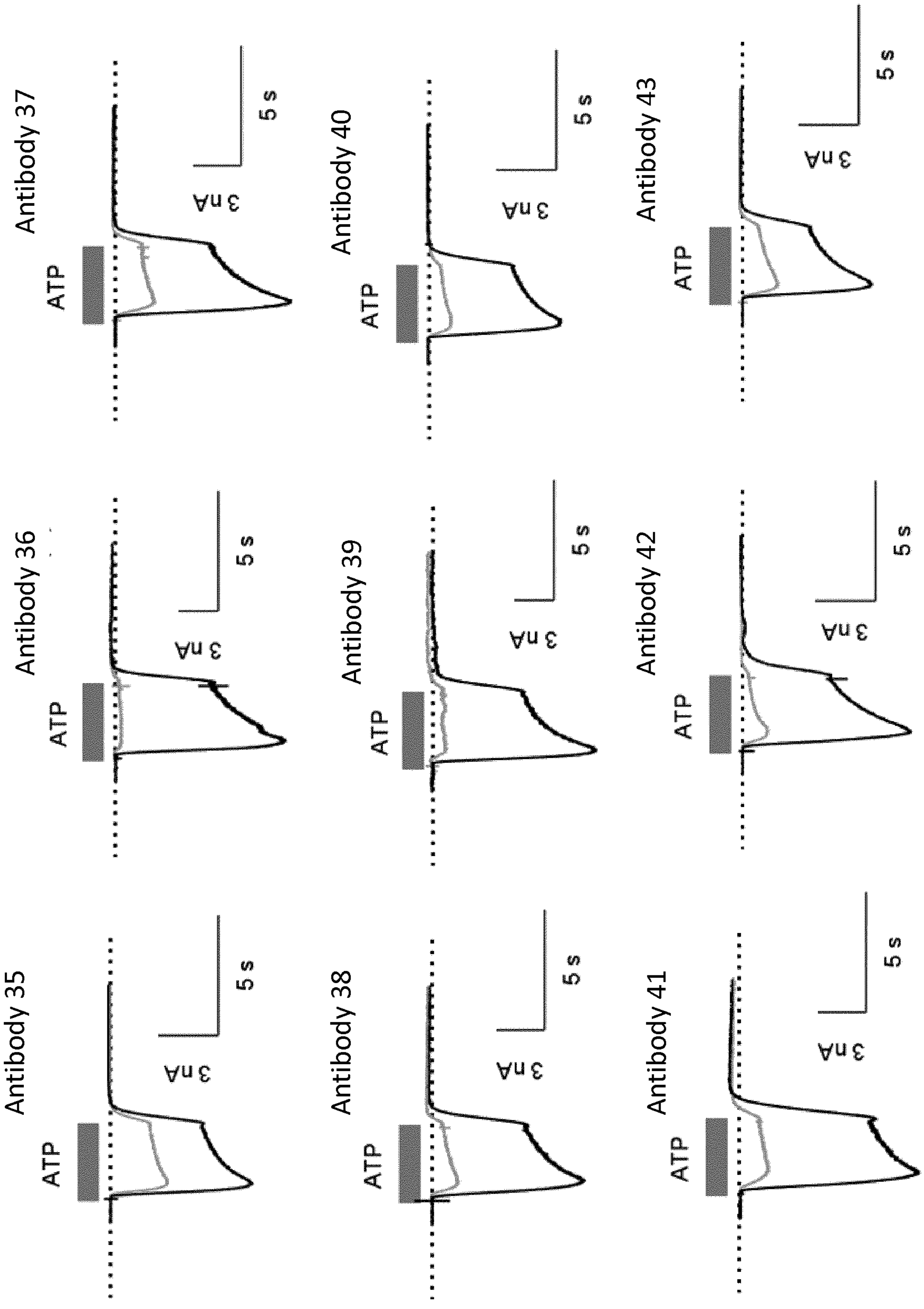


Inhibitory IgGs from hybridoma - activity against mP2X4

Figure 8C

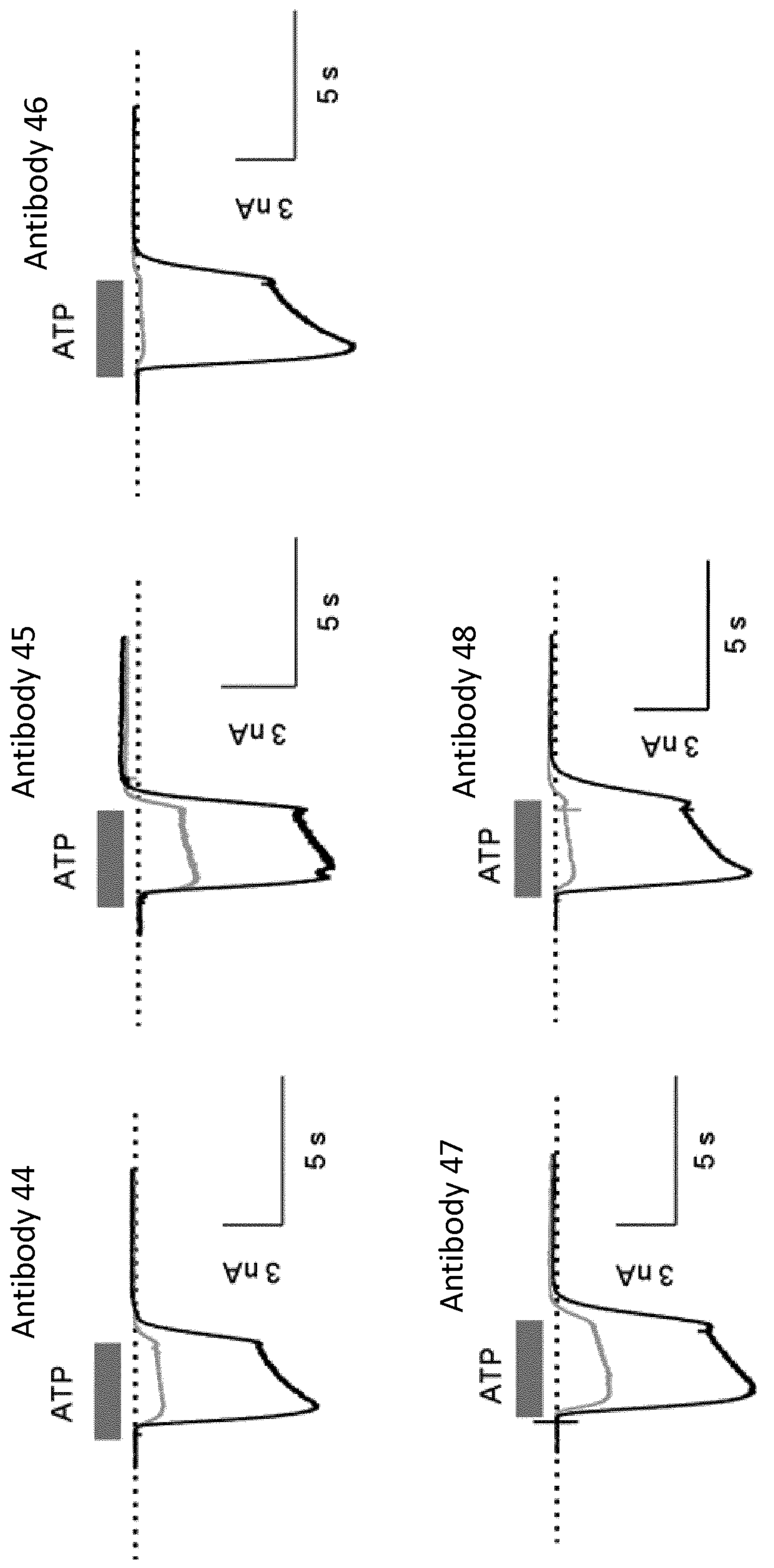


Inhibitory IgGs from hybridoma - activity against mP2X4

**Figure 8D**

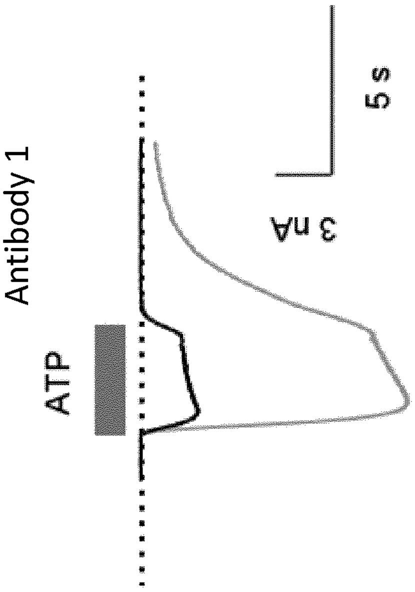


Example of a potentiating IgG - activity against huP2X4

Figure 8E

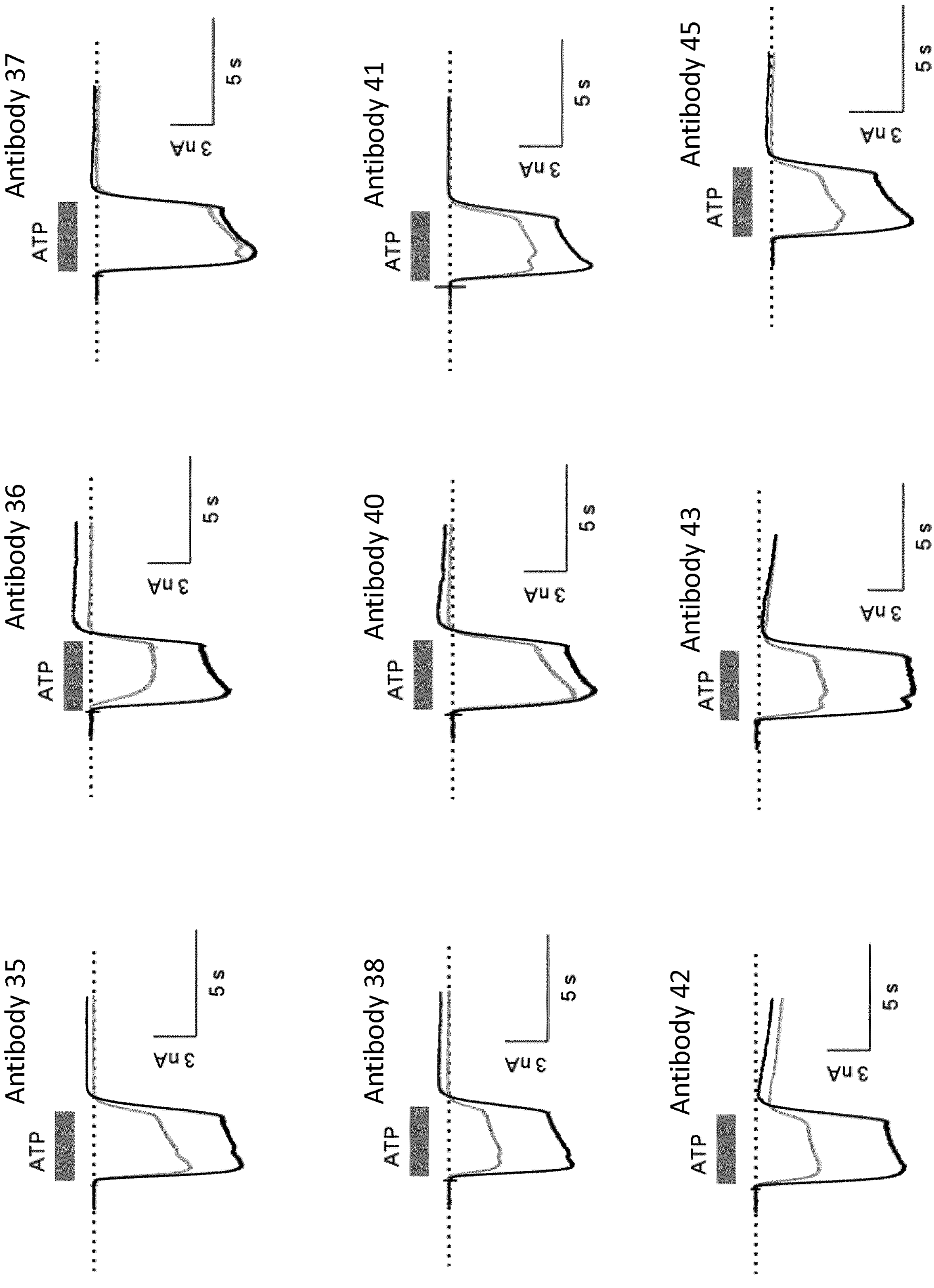


Inhibitory IgGs from hybridoma - activity against huP2X4

Figure 8F

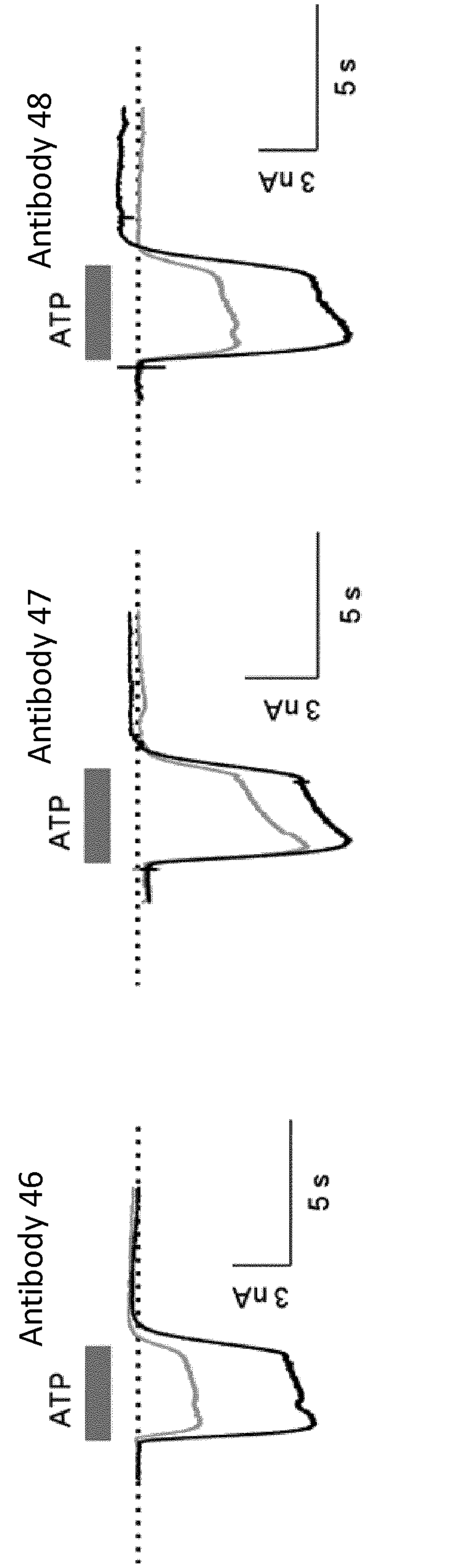


Inhibitory IgGs from hybridoma - activity against huP2X4

**Figure 9**

Bipartite epitope-paratope interface – Antibody No. 11 VHCDRs-P2X4 protomer 1 head (major interface); Antibody No. 11 VLCDRs-P2X4 protomer 2 right flipper (minor interface).

Three Antibody No. 11 molecules can potentially bind the same P2X4 trimer molecule.

The two Fab arms of Antibody No. 11 are not likely to engage the same P2X4 trimer molecule.

The ab is predicted to bind a P2X heteromer across the epitope formed by two P2X4 subunits.

Top (extracellular) view of predicted apo P2X4 trimer-Antibody 11 complex structure

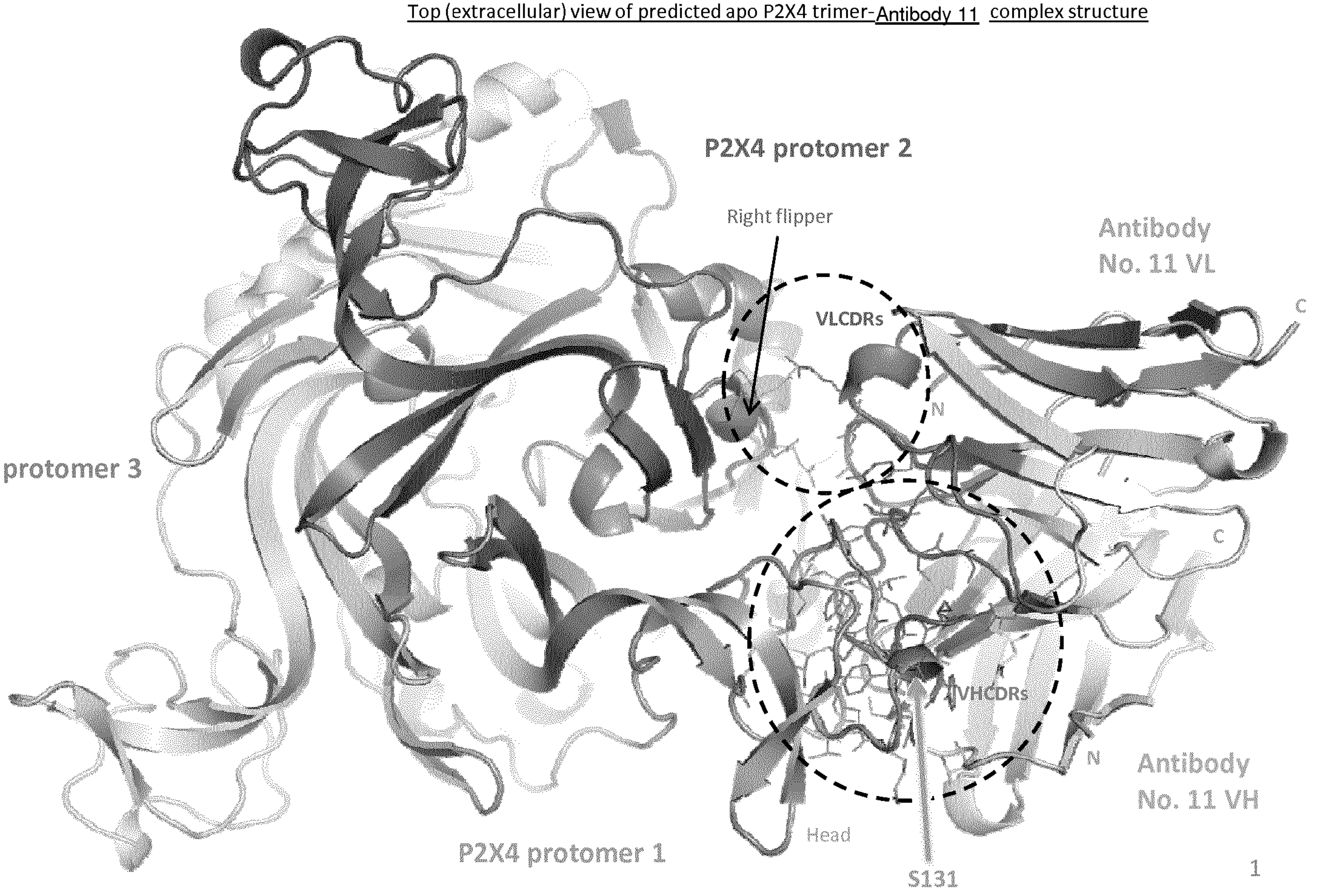

**Figure 10**

Predicted epitope is only moderately identical in sequence between human and mouse P2X4.
All epitope differences in orthologs are restricted to the head region.

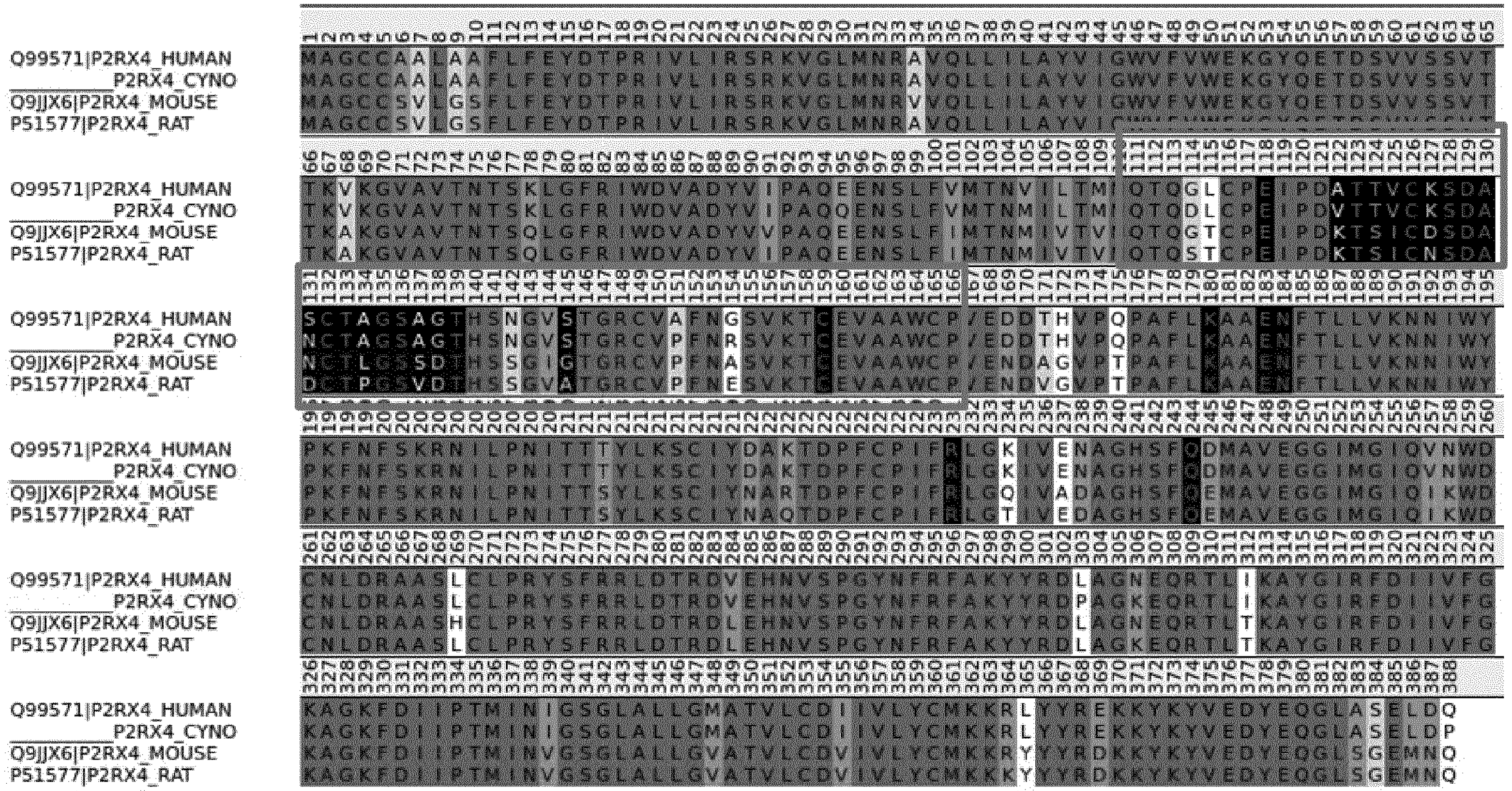


Predicted epitope

Head

Comparison of predicted epitope sequence in orthologs against human P2X4

| Species | % identity | % similarity |
|---|---|---|
| cyno | 92.3 | 92.3 |
| mouse | 65.4 | 76.9 |
| rat | 65.4 | 80.8 |

3

Figure 11

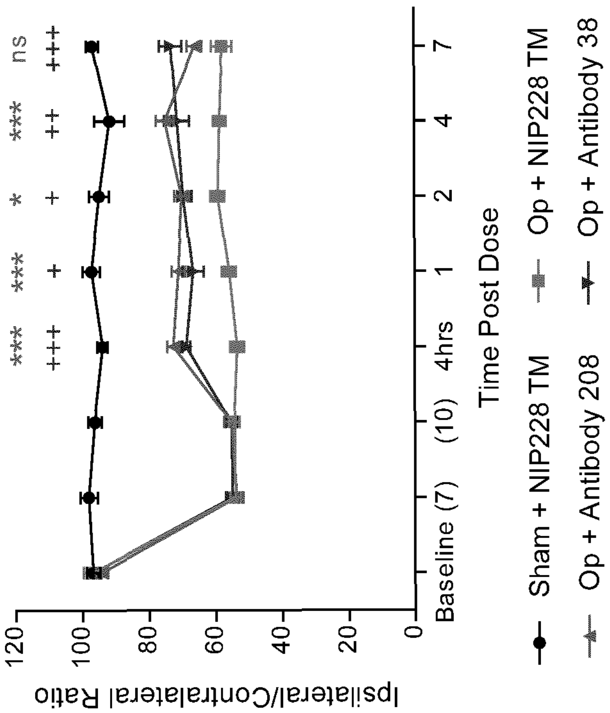
Ipsilateral/Contralateral Ratio
0
20
40
60
80
100
120
Baseline (7) (10) 4hrs 1 2 4 7
Time Post Dose
*** *** * *** ns
+++ + + ++ +++
Sham + NIP228 TM
Op + NIP228 TM
Op + Antibody 208
Op + Antibody 38

# Figure 12: Part 1: Phage display derived P2X4 binding – antibodies (Abs) VH sequences

| Antibody number | FV 1 | | | | | | | | | | | | | | | | | | | | | | | | | | | | | | CDR 1 | | | | | | | | | FV 2 | | | | | | | | | | | | | | CDR 2 | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | a | b | c | d | 36 | 37 | 38 | 39 | 40 | 41 | 42 | 43 | 44 | 45 | 46 | 47 | 48 | 49 | 50 | 51 | 52 | a | b | c | d | e | f | 53 | 54 | 55 | 56 | 57 | 58 | 59 | 60 | 61 | 62 | 63 | 64 | 65 |
| 11 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 287 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 288 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 289 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 290 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 291 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 292 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 293 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 294 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 295 | E | V | Q | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 296 | E | V | Q | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 297 | E | V | R | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 298 | E | V | Q | L | M | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | G | S | T | Y | Y | A | D | S | A | K | G |
| 299 | E | V | Q | L | L | E | S | G | G | G | L | V | Q | P | G | G | P | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 300 | E | V | Q | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | S | S | T | Y | Y | A | D | S | V | K | G |
| 302 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 303 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 304 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 305 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | S | S | T | Y | Y | A | D | S | V | K | G |
| 306 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 307 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 308 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 309 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 310 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 311 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 312 | E | V | Q | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 313 | E | V | Q | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | G | S | T | Y | Y | A | D | S | V | K | G |
| 314 | E | V | Q | L | L | E | S | G | G | G | L | V | Q | P | G | G | S | L | R | L | S | C | A | A | S | G | F | T | F | S | S | Y | A | M | S | - | - | - | - | W | V | R | Q | A | P | G | K | G | L | E | W | V | S | A | I | S | G | - | - | - | - | - | S | G | S | S | T | Y | Y | A | D | S | V | K | G |
| 315 | E | V | Q | L | L | E | S | G | G | G | L | V | Q | P | G | G | S | L | R | L | S | C | A | A | S | G | F | T | F | S | S | F | A | M | S | - | - | - | - | W | V | R | Q | A | P | G | K | G | L | E | W | V | S | A | I | S | G | - | - | - | - | - | S | G | S | S | T | Y | Y | A | D | S | V | K | G |

# Figure 12: Part 2: Phage display derived P2X4 binding – antibodies (Abs) VH sequences

| Antibody number | FV 3 | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|
| | 66 | 67 | 68 | 69 | 70 | 71 | 72 | 73 | 74 | 75 | 76 | 77 | 78 | 79 | 80 | 81 | 82 | a | b | c | d | e | 83 | 84 | 85 | 86 | 87 | 88 | 89 | 90 | 91 | 92 | 93 | 94 |
| 11 | R | F | T | I | S | R | D | N | S | K | N | T | L | Y | L | Q | M | N | S | L | - | - | R | A | E | D | T | A | V | Y | Y | C | A | R |
| 287 | R | F | T | I | S | R | D | N | S | K | N | T | L | Y | L | Q | M | N | S | L | - | - | R | A | E | D | T | A | V | Y | Y | C | A | R |
| 288 | R | F | T | I | S | R | D | N | S | K | N | T | L | Y | L | Q | M | N | S | L | - | - | R | A | E | D | T | A | V | Y | Y | C | A | R |
| 289 | R | F | T | I | S | R | D | N | S | K | N | T | L | Y | L | Q | M | N | S | L | - | - | R | A | E | D | T | A | V | Y | Y | C | A | R |
| 290 | R | F | T | I | S | R | D | N | S | K | N | T | L | Y | L | Q | M | N | S | L | - | - | R | A | E | D | T | A | V | Y | Y | C | S | R |
| 291 | R | F | T | I | S | R | D | N | S | K | N | T | L | Y | L | Q | M | N | S | L | - | - | R | A | E | D | T | A | V | Y | Y | C | A | R |
| 292 | R | F | T | I | S | R | D | N | S | K | N | T | L | Y | L | Q | M | N | S | L | - | - | R | A | E | D | T | A | V | Y | Y | C | A | R |
| 293 | R | F | T | I | S | R | D | N | S | K | N | T | L | Y | L | Q | M | N | S | L | - | - | R | A | E | D | T | A | V | Y | Y | C | A | R |
| 294 | R | F | T | I | S | R | D | N | S | K | N | T | L | Y | L | Q | M | N | S | L | - | - | R | A | E | D | T | A | V | Y | Y | C | A | R |
| 295 | R | F | T | I | S | R | D | N | S | K | N | T | L | Y | L | Q | M | N | S | L | - | - | R | A | E | D | T | A | V | Y | Y | C | A | R |
| 296 | R | L | T | I | S | R | D | N | S | K | N | T | L | Y | L | Q | M | N | S | L | - | - | R | A | E | D | T | A | V | Y | Y | C | A | R |
| 297 | R | F | T | I | S | R | D | N | S | K | N | T | L | Y | L | Q | M | N | S | L | - | - | R | A | E | D | T | A | V | Y | Y | C | A | R |
| 298 | R | F | T | I | S | R | D | N | S | K | N | T | L | Y | L | Q | M | N | S | L | - | - | R | A | E | D | T | A | V | Y | Y | C | A | R |
| 299 | R | F | T | I | S | R | D | N | S | K | N | T | L | Y | L | Q | M | N | S | L | - | - | R | A | E | D | T | A | A | Y | Y | C | A | R |
| 300 | R | F | A | I | S | R | D | N | S | K | N | T | L | Y | L | Q | M | N | S | L | - | - | R | A | E | D | T | A | V | Y | Y | C | A | R |
| 302 | R | F | A | I | S | R | D | N | S | K | N | T | L | Y | L | Q | M | N | S | L | - | - | R | A | E | D | T | A | V | Y | Y | C | S | R |
| 303 | R | F | A | I | S | R | D | N | S | K | N | T | L | Y | L | Q | M | N | S | L | - | - | R | A | E | D | T | A | V | Y | Y | C | S | R |
| 304 | R | F | A | I | S | R | D | N | S | K | N | T | L | Y | L | Q | M | N | S | L | - | - | R | A | E | D | T | A | V | Y | Y | C | S | R |
| 305 | R | F | T | I | S | R | D | N | S | K | N | T | L | Y | L | Q | M | N | S | L | - | - | R | A | E | D | T | A | V | Y | Y | C | A | R |
| 306 | R | F | A | I | S | R | D | N | S | K | N | T | L | Y | L | Q | M | N | S | L | - | - | R | A | E | D | T | A | V | Y | Y | C | A | R |
| 307 | R | F | T | I | S | R | D | N | S | K | N | T | L | Y | L | Q | M | N | S | L | - | - | R | A | E | D | T | A | V | Y | Y | C | A | R |
| 308 | R | F | A | I | S | R | D | N | S | K | N | T | L | Y | L | Q | M | N | S | L | - | - | R | A | E | D | T | A | V | Y | Y | C | S | R |
| 309 | R | F | T | I | S | R | D | N | S | K | N | T | L | Y | L | Q | M | N | S | L | - | - | R | A | E | D | T | A | V | Y | Y | C | S | R |
| 310 | R | F | A | I | S | R | D | N | S | K | N | T | L | Y | L | Q | M | N | S | L | - | - | R | A | E | D | T | A | V | Y | Y | C | A | R |
| 311 | R | F | T | I | S | R | D | N | S | K | N | T | L | Y | L | Q | M | N | S | L | - | - | R | A | E | D | T | A | V | Y | Y | C | S | R |
| 312 | R | F | T | I | S | R | D | N | S | K | N | T | L | Y | L | Q | M | N | S | L | - | - | R | A | E | D | T | A | V | Y | Y | C | S | R |
| 313 | R | F | T | I | S | R | D | N | S | K | N | T | L | Y | L | Q | M | N | S | L | - | - | R | A | E | D | T | A | V | Y | Y | C | S | R |
| 314 | R | F | A | I | S | R | D | N | S | K | N | T | L | Y | L | Q | M | N | S | L | - | - | R | A | E | D | T | A | V | Y | Y | C | S | R |
| 315 | R | F | A | I | S | R | D | N | S | K | N | T | L | Y | L | Q | M | N | S | L | - | - | R | A | E | D | T | A | V | Y | Y | C | A | R |

| Antibody number | CDR 3 | | | | | | | | | | | | | | | | | | | | | | | | |
|---|---|---|---|---|---|---|---|---|---|---|---|---|---|---|---|---|---|---|---|---|---|---|---|---|---|
| | 95 | 96 | 97 | 98 | 99 | 100 | a | b | c | d | e | f | g | h | i | j | k | l | m | n | o | p | q | 101 | 102 |
| 11 | D | R | D | F | W | S | T | Y | S | G | P | T | A | F | - | - | - | - | - | - | - | - | - | D | L |
| 287 | H | W | D | W | W | S | T | Y | S | G | P | T | A | F | - | - | - | - | - | - | - | - | - | D | L |
| 288 | D | M | D | Y | W | S | T | Y | S | G | P | T | A | F | - | - | - | - | - | - | - | - | - | D | L |
| 289 | D | H | D | Y | W | S | T | Y | S | G | P | T | A | F | - | - | - | - | - | - | - | - | - | D | L |
| 290 | Q | F | D | Y | W | S | T | Y | S | G | P | T | A | F | - | - | - | - | - | - | - | - | - | D | L |
| 291 | D | R | D | F | W | S | N | R | P | S | P | T | A | F | - | - | - | - | - | - | - | - | - | D | L |
| 292 | D | R | D | F | W | S | G | A | N | R | M | T | A | F | - | - | - | - | - | - | - | - | - | D | L |
| 293 | D | R | D | F | W | S | G | S | S | K | A | T | A | F | - | - | - | - | - | - | - | - | - | D | L |
| 294 | D | R | D | F | W | S | P | G | T | G | L | T | A | F | - | - | - | - | - | - | - | - | - | D | L |
| 295 | D | R | D | F | W | S | T | Y | S | G | P | T | A | F | - | - | - | - | - | - | - | - | - | D | L |
| 296 | D | R | D | F | W | S | T | Y | S | G | P | T | A | F | - | - | - | - | - | - | - | - | - | D | L |
| 297 | D | R | D | F | W | S | T | Y | S | G | P | T | A | F | - | - | - | - | - | - | - | - | - | D | L |
| 298 | D | R | D | F | W | S | T | Y | S | G | P | T | A | F | - | - | - | - | - | - | - | - | - | D | L |
| 299 | D | W | D | F | W | S | T | Y | S | G | P | T | A | F | - | - | - | - | - | - | - | - | - | D | L |
| 300 | D | R | D | F | W | S | T | Y | S | G | P | T | A | F | - | - | - | - | - | - | - | - | - | D | L |
| 302 | Q | F | D | Y | W | S | T | Y | S | G | P | T | A | F | - | - | - | - | - | - | - | - | - | D | L |
| 303 | Q | F | D | Y | W | S | T | Y | S | G | P | T | A | F | - | - | - | - | - | - | - | - | - | D | L |
| 304 | Q | F | D | Y | W | S | T | Y | S | G | P | T | A | F | - | - | - | - | - | - | - | - | - | D | L |
| 305 | D | R | D | F | W | S | T | Y | S | G | P | T | A | F | - | - | - | - | - | - | - | - | - | D | L |
| 306 | D | R | D | F | W | S | T | Y | S | G | P | T | A | F | - | - | - | - | - | - | - | - | - | D | L |
| 307 | D | R | D | F | W | S | T | Y | S | G | P | T | A | F | - | - | - | - | - | - | - | - | - | D | L |
| 308 | Q | F | D | Y | W | S | T | Y | S | G | P | T | A | F | - | - | - | - | - | - | - | - | - | D | L |
| 309 | Q | F | D | Y | W | S | T | Y | S | G | P | T | A | F | - | - | - | - | - | - | - | - | - | D | L |
| 310 | D | R | D | F | W | S | T | Y | S | G | P | T | A | F | - | - | - | - | - | - | - | - | - | D | L |
| 311 | Q | F | D | Y | W | S | T | Y | S | G | P | T | A | F | - | - | - | - | - | - | - | - | - | D | L |
| 312 | Q | F | D | Y | W | S | T | Y | S | G | P | T | A | F | - | - | - | - | - | - | - | - | - | D | L |
| 313 | Q | F | D | Y | W | S | T | Y | S | G | P | T | A | F | - | - | - | - | - | - | - | - | - | D | L |
| 314 | Q | F | D | Y | W | S | T | Y | S | G | P | T | A | F | - | - | - | - | - | - | - | - | - | D | L |
| 315 | D | R | D | F | W | S | T | Y | S | G | P | T | A | F | - | - | - | - | - | - | - | - | - | D | L |

| Antibody number | FV 4 | | | | | | | | | | |
|---|---|---|---|---|---|---|---|---|---|---|---|
| | 103 | 104 | 105 | 106 | 107 | 108 | 109 | 110 | 111 | 112 | 113 |
| 11 | W | G | R | G | T | L | V | T | V | S | S |
| 287 | W | G | R | G | T | L | V | T | V | S | S |
| 288 | W | G | R | G | T | L | V | T | V | S | S |
| 289 | W | G | R | G | T | L | V | T | V | S | S |
| 290 | W | G | R | G | T | L | V | T | V | S | S |
| 291 | W | G | R | G | T | L | V | T | V | S | S |
| 292 | W | G | R | G | T | L | V | T | V | S | S |
| 293 | W | G | R | G | T | L | V | T | V | S | S |
| 294 | W | G | R | G | T | L | V | T | V | S | S |
| 295 | W | G | R | G | T | L | V | T | V | S | S |
| 296 | W | G | R | G | T | L | V | T | V | S | S |
| 297 | W | G | R | G | T | L | V | T | V | S | S |
| 298 | W | G | R | G | T | L | V | T | V | S | S |
| 299 | W | G | R | G | T | L | V | T | V | S | S |
| 300 | W | G | R | G | T | L | V | T | V | S | S |
| 302 | W | G | R | G | T | L | V | T | V | S | S |
| 303 | W | G | R | G | T | L | V | T | V | S | S |
| 304 | W | G | R | G | T | L | V | T | V | S | S |
| 305 | W | G | R | G | T | L | V | T | V | S | S |
| 306 | W | G | R | G | T | L | V | T | V | S | S |
| 307 | W | G | R | G | T | L | V | T | V | S | S |
| 308 | W | G | R | G | T | L | V | T | V | S | S |
| 309 | W | G | R | G | T | L | V | T | V | S | S |
| 310 | W | G | R | G | T | L | V | T | V | S | S |
| 311 | W | G | R | G | T | L | V | T | V | S | S |
| 312 | W | G | R | G | T | L | V | T | V | S | S |
| 313 | W | G | R | G | T | L | V | T | V | S | S |
| 314 | W | G | R | G | T | L | V | T | V | S | S |
| 315 | W | G | R | G | T | L | V | T | V | S | S |

**Figure 12: Part 3: Phage display derived P2X4 binding – antibodies (Abs) VL sequences**

| Antibody number | FW 1 | | | | | | | | | | | | | | | | | | | | | | | CDR 1 | | | | | | | | | | | | | | | | | | | FW 2 | | | | | | | | | | | | | | | CDR 2 | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | a | b | c | d | e | f | g | h | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | a | b | c | d | e | 52 | 53 | 54 | 55 | 56 |
| 11 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 287 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 288 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 289 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 290 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 291 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 292 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 293 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 294 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 295 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 296 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 297 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 298 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | P | V | I | Y | K | D | - | - | - | - | - | S | E | R | P | S |
| 299 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 300 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 302 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | F | R | P | S |
| 303 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 304 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 305 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 306 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 307 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 308 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | F | R | P | S |
| 309 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 310 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 311 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | F | R | P | S |
| 312 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 313 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | F | R | P | S |
| 314 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | E | R | P | S |
| 315 | S | Y | E | L | A | Q | P | P | S | - | V | S | V | S | P | G | Q | T | A | R | I | T | C | S | G | D | A | - | - | - | - | - | - | - | - | L | P | R | Q | Y | A | Y | W | Y | Q | Q | K | P | G | Q | A | P | L | L | V | I | Y | K | D | - | - | - | - | - | S | F | R | P | S |

# Figure 12: Part 4: Phage display derived P2X4 binding – antibodies (Abs) VL sequences

| Antibody number | FW 3 | | | | | | | | | | | | | | | | | | | | | | | | | | | | | | | | | | CDR 3 | | | | | | | | | | | | | | | | | | FW 4 | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | 57 | 58 | 59 | 60 | 61 | 62 | 63 | 64 | 65 | 66 | 67 | 68 | a | b | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | a | b | c | d | e | f | g | h | i | 96 | 97 | 98 | 99 | 100 | 101 | 102 | 103 | 104 | 105 | 106 | a | b | c | 107 |
| 11 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 287 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 288 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 289 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 290 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 291 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 292 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 293 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 294 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 295 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 296 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 297 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 298 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 299 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 300 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 302 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 303 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 304 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 305 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 306 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 307 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 308 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 309 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 310 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 311 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 312 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 313 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | V | V | F | G | G | G | T | K | V | T | V | - | - | - | L |
| 314 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |
| 315 | G | I | P | E | R | F | S | G | S | G | S | G | - | - | T | T | V | T | L | T | I | S | G | V | Q | A | E | D | E | A | D | Y | Y | C | Q | S | A | D | S | S | G | T | Y | - | - | - | - | - | - | - | A | V | F | G | G | G | T | K | V | T | V | - | - | - | L |

# FIGURE 13 Part 1: Hybridoma derived P2X4 binding – antibodies (Abs) VH sequences

| Antibody number | FV 1 | | | | | | | | | | | | | | | | | | | | | | | | | | | | | | CDR 1 | | | | |
|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 |
| 316 | Q | V | Q | L | K | E | S | G | P | G | L | V | K | P | S | E | T | L | S | L | T | C | T | V | S | G | F | S | L | T | N | Y | H | V | N |
| 317 | Q | V | Q | L | K | E | S | G | P | G | L | V | K | P | S | E | T | L | S | L | T | C | T | V | S | G | F | S | L | T | N | Y | H | V | N |
| 318 | Q | V | Q | L | K | E | S | G | P | G | L | V | K | P | S | E | T | L | S | L | T | C | T | V | S | G | F | S | L | T | N | Y | H | V | N |
| 319 | Q | V | Q | L | K | E | S | G | P | G | L | V | A | P | S | Q | S | L | S | I | T | C | T | V | S | G | F | S | L | T | T | Y | G | I | H |
| 320 | Q | V | Q | L | Q | Q | S | G | A | E | L | A | K | P | G | A | S | V | R | M | S | C | K | T | S | G | Y | T | F | S | T | Y | W | M | H |
| 321 | Q | V | Q | L | K | Q | S | G | P | G | L | V | Q | P | S | Q | S | L | S | I | T | C | T | V | S | G | F | S | L | T | T | Y | G | V | H |
| 208 | Q | V | Q | L | K | E | S | G | P | G | L | V | K | P | S | E | T | L | S | L | T | C | T | V | S | G | F | S | L | T | N | Y | P | I | S |

| Antibody number | CDR 1 | | | | FV 2 | | | | | | | | | | | | | | CDR 2 | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|
| | a | b | c | d | 36 | 37 | 38 | 39 | 40 | 41 | 42 | 43 | 44 | 45 | 46 | 47 | 48 | 49 | 50 | 51 | 52 | a | b | c | d | e | f | 53 | 54 | 55 | 56 | 57 | 58 | 59 | 60 | 61 | 62 | 63 | 64 | 65 |
| 316 | - | - | - | - | W | V | R | Q | P | P | G | K | G | L | E | W | M | G | V | I | W | - | - | - | - | - | - | G | D | G | S | T | A | Y | N | S | A | L | K | S |
| 317 | - | - | - | - | W | V | R | Q | P | P | G | K | G | L | E | W | M | G | V | I | W | - | - | - | - | - | - | G | D | G | S | T | A | Y | N | S | A | L | K | S |
| 318 | - | - | - | - | W | V | R | Q | P | P | G | K | G | L | E | W | M | G | V | I | W | - | - | - | - | - | - | G | D | G | S | T | A | Y | N | S | A | L | K | S |
| 319 | - | - | - | - | W | V | R | Q | P | P | G | K | G | L | E | W | L | G | V | I | W | - | - | - | - | - | - | T | R | G | S | T | N | Y | N | S | A | L | M | S |
| 320 | - | - | - | - | W | V | K | Q | R | P | G | Q | G | L | E | W | I | G | Y | I | N | P | - | - | - | - | - | P | T | G | Y | T | E | Y | N | Q | K | F | K | D |
| 321 | - | - | - | - | W | V | R | Q | S | P | G | K | G | L | E | W | L | G | V | I | W | - | - | - | - | - | - | S | G | G | S | T | D | Y | N | A | A | F | I | S |
| 208 | - | - | - | - | W | V | R | Q | P | P | G | K | G | L | E | W | M | G | V | I | W | - | - | - | - | - | - | G | D | G | S | T | S | Y | N | L | A | L | K | S |

# FIGURE 13 Part 2: Hybridoma derived P2X4 binding – antibodies (Abs) VH sequences

| Antibody number | FV 3 | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|
| | 66 | 67 | 68 | 69 | 70 | 71 | 72 | 73 | 74 | 75 | 76 | 77 | 78 | 79 | 80 | 81 | 82 | a | b | c | d | e | 83 | 84 | 85 | 86 | 87 | 88 | 89 | 90 | 91 | 92 | 93 | 94 |
| 316 | R | L | S | I | S | R | D | T | S | K | S | Q | V | F | L | K | M | N | S | L | - | - | Q | T | E | D | T | A | T | Y | Y | C | A | R |
| 317 | R | L | S | I | S | R | D | T | S | K | S | Q | V | F | L | K | M | N | S | L | - | - | Q | T | E | D | T | A | T | Y | Y | C | A | R |
| 318 | R | L | S | I | S | R | D | T | S | K | S | Q | V | F | L | K | M | N | S | L | - | - | Q | T | E | D | T | A | T | Y | Y | C | A | R |
| 319 | R | L | S | I | S | K | D | N | S | K | S | Q | V | F | L | K | M | N | S | L | - | - | Q | T | D | D | T | A | M | Y | F | C | A | R |
| 320 | K | A | T | L | T | A | D | K | S | S | S | T | A | Y | M | Q | L | I | S | L | - | - | T | S | E | D | S | A | V | Y | Y | C | V | H |
| 321 | R | L | S | I | T | K | D | N | S | K | S | Q | V | F | F | K | M | N | S | L | - | - | Q | A | N | D | T | A | I | Y | Y | C | A | R |
| 208 | R | L | S | I | S | R | D | T | S | K | S | Q | V | L | L | K | M | N | S | L | - | - | E | T | E | D | T | A | T | Y | Y | C | A | R |

| Antibody number | CDR 3 | | | | | | | | | | | | | | | | | | | | | | | | | FV 4 | | | | | | | | | | |
|---|---|---|---|---|---|---|---|---|---|---|---|---|---|---|---|---|---|---|---|---|---|---|---|---|---|---|---|---|---|---|---|---|---|---|---|---|
| | 95 | 96 | 97 | 98 | 99 | 100 | a | b | c | d | e | f | g | h | i | j | k | l | m | n | o | p | q | 101 | 102 | 103 | 104 | 105 | 106 | 107 | 108 | 109 | 110 | 111 | 112 | 113 |
| 316 | G | G | D | Y | Y | D | G | S | Y | Y | Y | E | - | - | - | - | - | - | - | - | - | - | - | G | Y | W | G | Q | G | V | M | V | T | V | S | S |
| 317 | G | G | D | Y | Y | D | G | S | Y | Y | Y | E | - | - | - | - | - | - | - | - | - | - | - | G | Y | W | G | Q | G | V | M | V | T | V | S | S |
| 318 | G | G | D | Y | Y | D | G | S | Y | Y | Y | E | - | - | - | - | - | - | - | - | - | - | - | G | Y | W | G | Q | G | V | M | V | T | V | S | S |
| 319 | D | G | Y | Y | G | Y | Y | Y | A | L | - | - | - | - | - | - | - | - | - | - | - | - | - | D | Y | W | G | Q | G | T | S | V | T | V | S | S |
| 320 | E | G | G | M | I | T | T | D | F | H | A | L | - | - | - | - | - | - | - | - | - | - | - | D | Y | W | G | Q | G | T | S | V | T | V | S | S |
| 321 | D | G | Y | Y | A | L | Y | Y | A | M | - | - | - | - | - | - | - | - | - | - | - | - | - | D | Y | W | G | Q | G | T | S | V | T | V | S | S |
| 208 | V | G | V | Y | Y | G | L | L | - | - | - | - | - | - | - | - | - | - | - | - | - | - | - | G | Y | W | G | Q | G | V | M | V | T | V | S | S |

# FIGURE 13 Part 3: Hybridoma derived P2X4 binding – antibodies (Abs) VL sequences

| Antibody number | FW 1 | | | | | | | | | | | | | | | | | | | | | | | CDR 1 | | | | | | | | | | | |
|---|---|---|---|---|---|---|---|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | a | b | c | d | e | f | g | h |
| 316 | D | I | Q | M | T | Q | S | P | A | S | L | S | A | S | L | E | E | I | V | T | I | T | C | Q | A | S | Q | - | - | - | - | - | - | - | - |
| 317 | D | I | Q | M | T | Q | S | P | A | S | L | S | A | S | L | E | E | I | V | T | I | T | C | Q | A | S | Q | - | - | - | - | - | - | - | - |
| 318 | D | I | Q | M | T | Q | S | P | A | S | L | S | A | S | L | E | E | I | V | T | I | T | C | Q | A | S | Q | - | - | - | - | - | - | - | - |
| 319 | Q | I | V | L | K | Q | S | P | A | I | M | S | A | S | P | G | E | K | V | T | M | T | C | S | A | S | S | - | - | - | - | - | - | - | - |
| 320 | D | I | V | M | S | Q | S | P | S | S | L | A | V | S | A | G | E | K | V | T | M | S | C | K | S | S | Q | S | L | L | N | S | R | - | - |
| 321 | D | I | V | M | T | Q | S | H | K | F | M | S | T | S | V | G | D | R | V | S | I | T | C | K | A | S | Q | - | - | - | - | - | - | - | - |
| 208 | N | T | V | M | T | Q | S | P | T | S | M | F | R | S | V | G | D | R | V | T | M | N | C | K | A | S | Q | - | - | - | - | - | - | - | - |

| Antibody number | CDR 1 | | | | | | | FW 2 | | | | | | | | | | | | | | | CDR 2 | | | | | | | | | | | | |
|---|---|---|---|---|---|---|---|---|---|---|---|---|---|---|---|---|---|---|---|---|---|---|---|---|---|---|---|---|---|---|---|---|---|---|---|
| | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | a | b | c | d | e | 52 | 53 | 54 | 55 | 56 |
| 316 | D | I | G | N | W | L | S | W | Y | Q | Q | K | P | G | K | S | P | Q | L | L | I | Y | G | A | - | - | - | - | - | T | S | L | A | D |
| 317 | D | I | G | N | W | L | A | W | Y | Q | Q | K | P | G | K | S | P | Q | L | L | I | Y | D | A | - | - | - | - | - | T | S | L | A | D |
| 318 | D | I | G | N | W | L | A | W | Y | Q | Q | K | P | G | K | S | P | Q | L | L | I | Y | D | A | - | - | - | - | - | T | S | L | A | D |
| 319 | - | S | V | S | Y | M | Y | W | Y | Q | Q | K | P | G | S | S | P | R | L | L | I | Y | D | T | - | - | - | - | - | S | N | L | A | S |
| 320 | T | R | K | N | Y | L | A | W | Y | Q | Q | K | P | G | Q | S | P | K | L | L | I | Y | W | A | - | - | - | - | - | S | T | R | E | S |
| 321 | D | V | S | T | A | V | A | W | Y | Q | Q | K | P | G | Q | S | P | K | L | L | I | Y | S | A | - | - | - | - | - | S | Y | R | Y | T |
| 208 | N | V | G | T | N | V | D | W | Y | Q | Q | K | T | G | Q | S | P | K | L | L | I | Y | G | A | - | - | - | - | - | S | N | R | Y | T |

# FIGURE 13 Part 4: Hybridoma derived P2X4 binding – antibodies (Abs) VL sequences

| Antibody number | FW 3 | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|
| | 57 | 58 | 59 | 60 | 61 | 62 | 63 | 64 | 65 | 66 | 67 | 68 | a | b | 69 | 70 | 71 | 72 | 73 | 74 | 75 | 76 | 77 | 78 | 79 | 80 | 81 | 82 | 83 | 84 | 85 | 86 | 87 | 88 |
| 316 | G | V | P | S | R | F | S | G | S | R | S | G | - | - | T | Q | F | S | L | K | I | S | R | L | Q | V | E | D | I | G | I | Y | Y | C |
| 317 | G | V | P | S | R | F | S | G | S | R | S | G | - | - | T | Q | Y | S | L | K | I | S | R | L | Q | V | E | D | I | G | S | Y | Y | C |
| 318 | G | V | P | S | R | F | S | G | S | R | S | G | - | - | T | Q | Y | S | L | K | I | S | R | L | Q | V | E | D | I | G | I | Y | Y | C |
| 319 | G | V | P | V | R | F | S | Y | S | G | S | G | - | - | T | S | Y | S | L | T | I | S | R | M | E | A | E | D | A | A | T | Y | Y | C |
| 320 | G | V | P | D | R | F | T | G | S | G | S | G | - | - | T | D | F | T | L | T | I | S | S | V | Q | A | E | D | L | A | V | Y | Y | C |
| 321 | G | V | P | D | R | F | T | G | S | G | S | G | - | - | T | D | F | T | F | T | I | S | S | V | Q | A | E | D | L | A | V | Y | Y | C |
| 208 | G | V | P | D | R | F | T | G | S | G | S | G | - | - | T | D | F | T | L | T | I | S | N | M | Q | A | E | D | L | A | V | Y | Y | C |

| Antibody number | CDR 3 | | | | | | | | | | | | | | | | | | FW 4 | | | | | | | | | | | | |
|---|---|---|---|---|---|---|---|---|---|---|---|---|---|---|---|---|---|---|---|---|---|---|---|---|---|---|---|---|---|---|---|
| | 89 | 90 | 91 | 92 | 93 | 94 | 95 | a | b | c | d | e | f | g | h | i | 96 | 97 | 98 | 99 | 100 | 101 | 102 | 103 | 104 | 105 | 106 | a | b | c | 107 |
| 316 | L | Q | A | Y | S | A | P | - | - | - | - | - | - | - | - | - | W | T | F | G | G | G | T | K | L | E | L | - | - | - | K |
| 317 | H | Q | A | H | S | N | P | - | - | - | - | - | - | - | - | - | R | T | F | G | G | G | T | K | L | E | L | - | - | - | K |
| 318 | L | Q | A | Y | S | A | P | - | - | - | - | - | - | - | - | - | W | T | F | G | G | G | T | K | L | E | L | - | - | - | K |
| 319 | Q | Q | W | S | S | Y | P | P | - | - | - | - | - | - | - | - | M | T | F | G | G | G | T | K | L | E | I | - | - | - | K |
| 320 | K | Q | S | Y | N | L | - | - | - | - | - | - | - | - | - | - | Y | T | F | G | G | G | T | K | L | E | I | - | - | - | K |
| 321 | Q | Q | H | Y | S | T | P | - | - | - | - | - | - | - | - | - | W | T | F | G | G | G | T | K | L | E | I | - | - | - | K |
| 208 | L | Q | Y | N | Y | N | P | - | - | - | - | - | - | - | - | - | Y | T | F | G | A | G | T | K | L | E | L | - | - | - | K |

Figure 14: Activity of antibodies in a huP2X4 electrophysiology assay

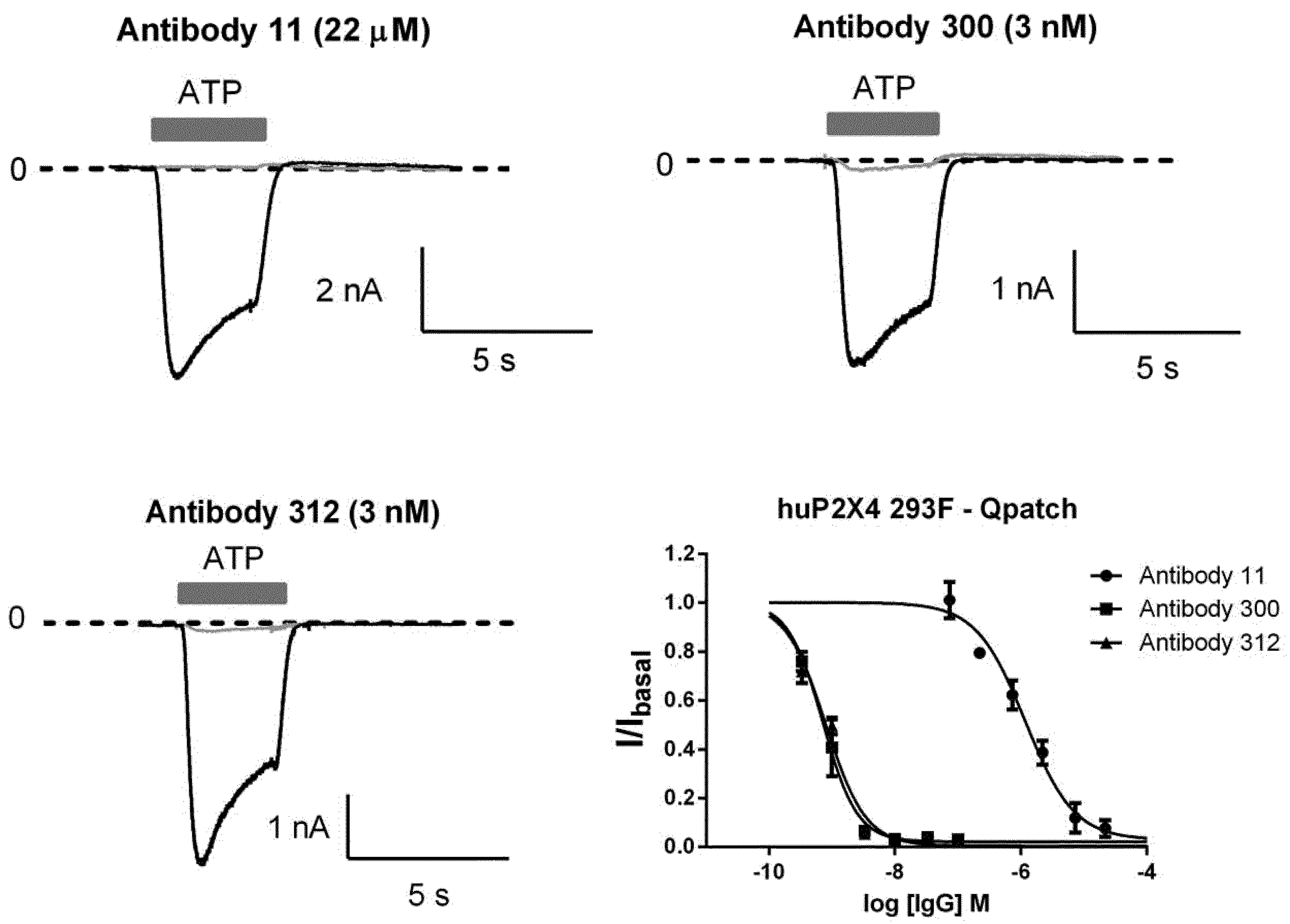

**Figure 15: Potency of Antibody 11 optimized antibodies at huP2X4**

| Antibody number | Geometric mean $IC_{50}$ (Molar) huP2X4 FLIPR assay | $IC_{50}$ (Molar) huP2X4 Ephys (HEK293f) |
|---|---|---|
| 287 | 3.9E-08 | 3.5E-09 |
| 288 | | 4.7E-08 |
| 289 | | 4.5E-08 |
| 290 | 4.6E-07 | 2.6E-09 |
| 291 | 1.1E-07 | 4.9E-09 |
| 292 | 1.8E-07 | 7.4E-09 |
| 293 | 7.7E-08 | 4.9E-09 |
| 294 | 1.6E-07 | 1.5E-08 |
| 295 | 2.4E-07 | 1.2E-09 |
| 296 | 4.5E-08 | 1.3E-09 |
| 297 | 5.3E-08 | 1.8E-09 |
| 298 | 2.9E-08 | 1.1E-09 |
| 299 | | 2.2E-09 |
| 300 | 7.9E-09 | 7.1E-10 |
| 302 | 8.0E-10 | 5.6E-10 |
| 303 | 1.9E-07 | 2.4E-09 |
| 304 | 9.5E-08 | 2.4E-09 |
| 305 | | >1E-07 |
| 306 | | >1E-07 |
| 307 | | >1E-07 |
| 308 | 7.0E-10 | 6.1E-10 |
| 309 | 7.3E-08 | 1.2E-09 |
| 310 | | 6.2E-09 |
| 311 | 4.0E-10 | 5.0E-10 |
| 312 | 5.8E-10 | 5.3E-10 |
| 313 | 3.3E-10 | 5.4E-10 |
| 314 | 3.5E-08 | 1.1E-09 |
| 315 | 3.0E-10 | 6.9E-10 |

blank = data not disclosed

**Figure 16: Potency of hybridoma derived antibodies at mouse and human P2X4**

| Antibody Number | $IC_{50}$ (Molar) huP2X4 Ephys (HEK293f) | n | $IC_{50}$ (Molar) moP2X4 Ephys (HEK293f) | n |
|---|---|---|---|---|
| 38 | 3.26E-07 | 3 | 4.89E-09 | 4 |
| 43 | NT | | 8.13E-09 | 4 |
| 46 | 1.79E-07 | 4 | 2.03E-08 | 8 |
| 208 | > 30E-06 | 4 | 2.57E-08 | 16 |

**NT indicates not tested**

**Figure 17: Effect of P2X4 antibodies on mouse microglial P2X4 currents**

| | huIgG1 | | | | rat IgG1 | | | |
|---|---|---|---|---|---|---|---|---|
| Antibody Number | Concentration (mg/ml) | Current (pA) | n | Significant difference from Control | Concentration (mg/ml) | Current (pA) | n | Significant difference from Control |
| Control IgG | 0.33 | -446.2 | 11 | - | 0.4 | -622.5 | 10 | - |
| 38 | 0.33 | -99.29 | 4 | N | 0.11 | -244.6 | 5 | N |
| 43 | 0.33 | -332.3 | 3 | N | - | NT | - | - |
| 46 | 0.33 | 9.06 | 3 | Y | 0.25 | 40.96 | 7 | Y |
| 208 | 0.33 | 12.12 | 4 | Y | 0.4 | 35.09 | 3 | Y |

Figure 18: Exemplar effect of antibody 208 on native P2X4 currents in primary mouse microglia

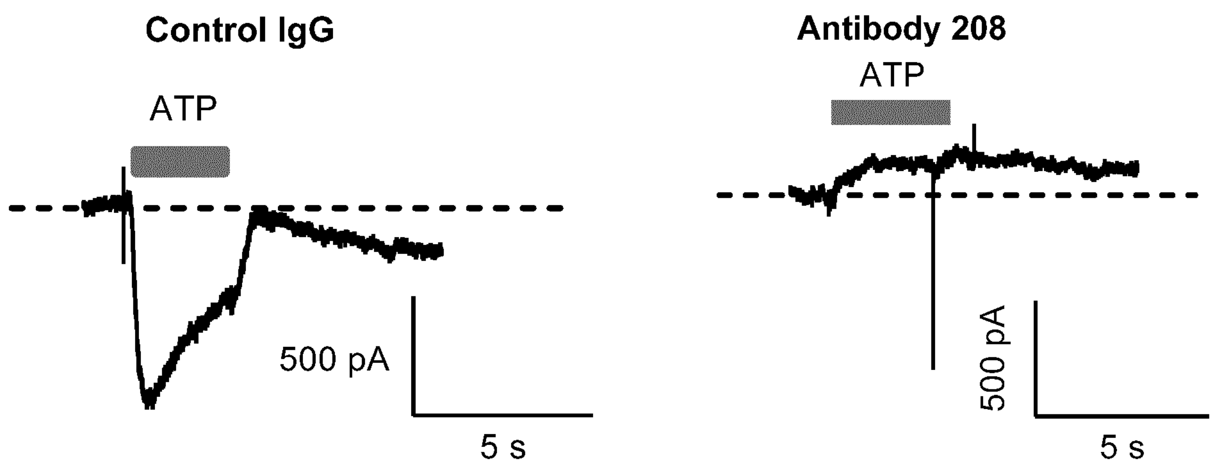

**Figure 19. Example of activity of two anti-P2X4 antibodies in a calcium flux assay.**

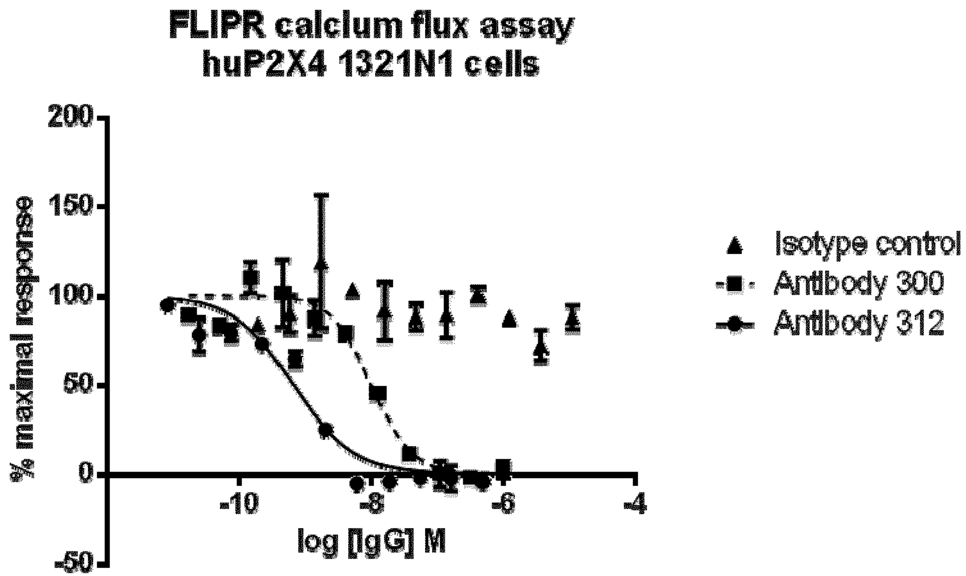

**Figure 20: Effect of mouse reactive P2X4 antibodies on native mouse microglial P2X4 assayed on FLIPR**

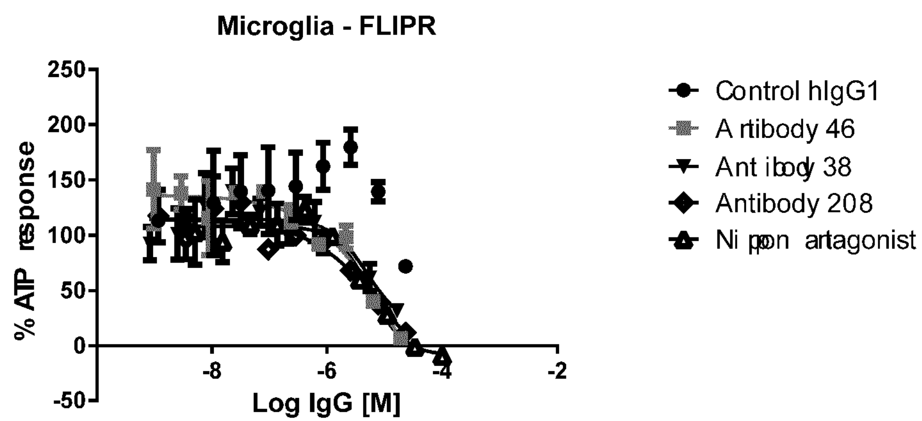


| Antibody Number | $IC_{50}$ moP2X4 FLIPR (microglia) | n |
|---|---|---|
| 38 | 5.0E-06 | 2 |
| 46 | 1.6E-05 | 2 |
| 208 | 9.3E-06 | 2 |
| Nippon antagonist | 5.6E-06 | 2 |

Figure 21: Exemplary electrophysiology current traces from human monocyte derived macrophages

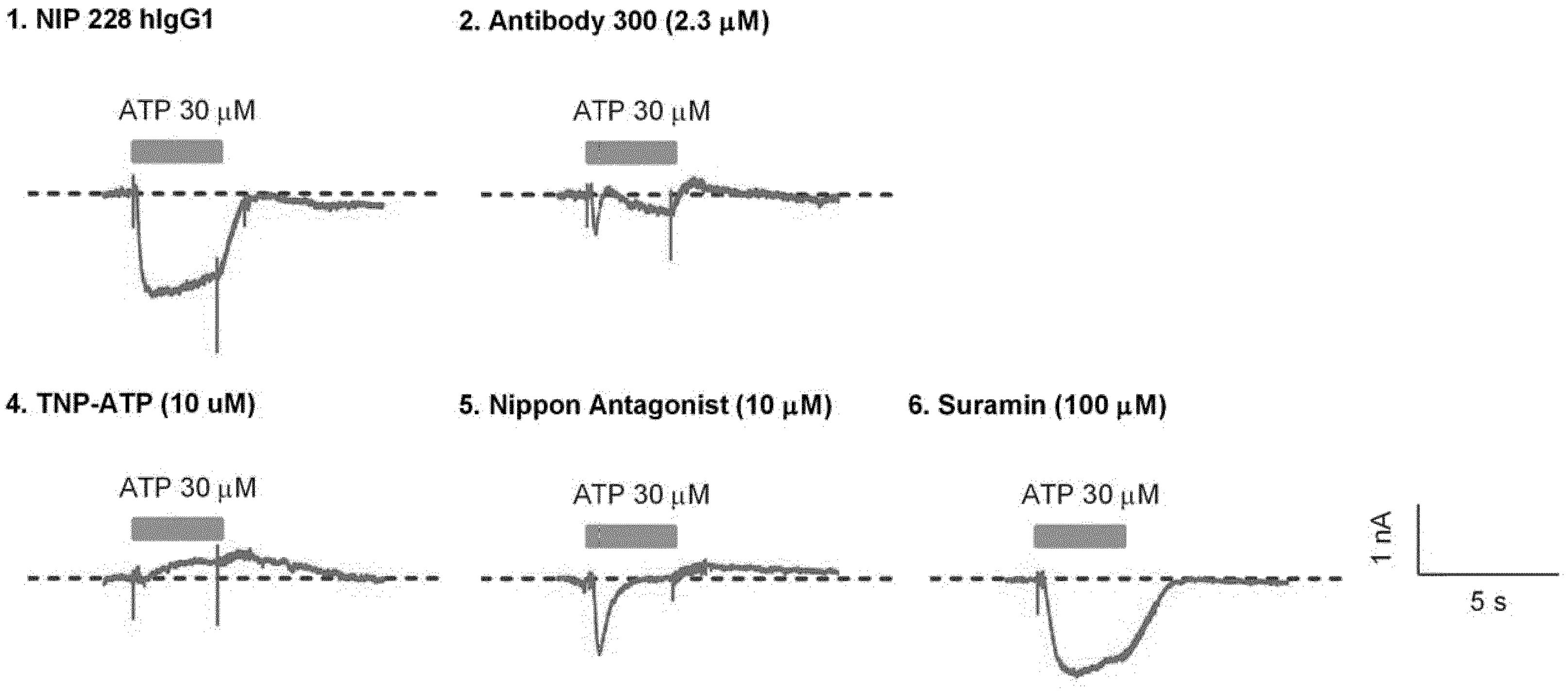

**Figure 22: Summary of the effect of P2X4 antibodies on human monocyte derived macrophage, ATP induced currents.**

| | | Donor 1 | | | Donor 2 | | |
|---|---|---|---|---|---|---|---|
| Nickname | Antibody number | Mean Current (A) | SD | n | Mean Current (A) | SD | n |
| NIP 228 | | -1.4E-09 | 1.3E-09 | 8 | -7.0E-10 | 1.3E-09 | 7 |
| | 300 | -2.5E-11 | 2.4E-10 | 5 | 1.4E-12 | 3.2E-10 | 7 |
| TNP ATP | | 3.6E-10 | 3.1E-10 | 7 | | | |
| Nippon Antagonist | | | | | 1.3E-10 | 7.4E-11 | 6 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0248395 A
- US 2005074819 A
- US 20080287467 A
- US 5565332 A
- US 4816567 A
- US 6030613 A
- US 4522811 A
- WO 2010093061 A
- EP 2397480 A1
- WO 61987929 A


### Non-patent literature cited in the description


- **YOUNG et al.** *JBC,* 2008, vol. 283, 26241-51
- **GUM et al.** *Purinergic Signal,* 2012, vol. 8, 41-56
- **VALENTE et al.** *Biochim Biophys Acta,* 2011, vol. 1808, 2859-66
- **SINGLETON et al.** Dictionary of Microbiology and Molecular Biology. 1994
- The Cambridge Dictionary of Science and Technology. 1988
- The Glossary of Genetics. Springer Verlag, 1991
- **HALE ; MARHAM.** *The Harper Collins Dictionary of Biology,* 1991
- **YOUNG et al.** *J. Biol. Chem.,* 2008, vol. 283, 26241-26251
- **ANTONIO et al.** *Br. J. Pharmacol.,* 2011, vol. 163, 1069-1077
- **VALENTE et al.** *Biochim. Biophys. Acta,* 2011, vol. 1808, 2859-2866
- **BACKMARK et al.** *Protein Sci,* 2013, vol. 22, 1124-1132
- **KAWATE ; GOUAUX.** *Structure,* 2006, vol. 14, 673-681
- **KOHLER ; MILSTEIN.** *Nature,* 1975, vol. 256, 495-499
- **CLACKSON, T. ; LOWMAN, H.B.** Phage Display - A Practical Approach. Oxford University Press, 2004
- **THOMPSON, J. ET.** *J Mol Biol.,* 1996, vol. 256 (1), 77-88
- **OSBOURN, J.K. et al.** *Immunotechnology,* 1996, vol. 2 (3), 181-96
- Antibodies: A Laboratory Manual. Cold Spring Harbor Laboratory, 1988
- Fundamental Immunology. Raven Press, 1993
- Immunoglobulin Genes. Academic Press, 1995
- **KABAT et al.** Sequences of Proteins of Immunological Interest. National Institutes of Health Publications, 1991
- **ALTSHUL et al.** *J. Mol. Biol.,* 1990, vol. 215, 403-410
- **NEEDLEMAN et al.** *J. Mol. Biol.,* 1970, vol. 48, 444-453
- **MEYERS et al.** *Comput. Appl. Biosci.,* 1988, vol. 4, 11-17
- Epitope Mapping Protocols. Humana Press, 1996
- **ENGSTOM.** *Biochem. Exp. Biol.,* 1974, vol. 11, 7-13
- Computer Graphics and Molecular Modeling. **FLETTERICK et al.** Current Communications in Molecular Biology. Cold Spring Harbor Laboratory, 1986
- Gene Expression Systems. Academic Press, 1999
- **SAMBROOK et al.** Molecular Cloning: A Laboratory Manual. Cold Spring Harbor Laboratory Press, 1989
- Current Protocols in Molecular Biology. John Wiley & Sons, 1992
- *CHEMICAL ABSTRACTS,* 69227-93-6
- *CHEMICAL ABSTRACTS,* 148565-58-6
- *CHEMICAL ABSTRACTS,* 75621-03-3
- *CHEMICAL ABSTRACTS,* 102601-49-0
- *CHEMICAL ABSTRACTS,* 7365-45-9
- *CHEMICAL ABSTRACTS,* 250692-65-0
- *CHEMICAL ABSTRACTS,* 82494-09-5
- *CHEMICAL ABSTRACTS,* 29836-26-8
- *CHEMICAL ABSTRACTS,* 69984-73-2
- **KAWATE et al.** *Structure,* 2006, vol. 14, 673-681
- **KAWATE et al.** *Nature,* 2009, vol. 460, 592-598

- **NAKAZAWA et al.** *European Journal of Pharmacology,* 2005, vol. 518, 107-110
- **NICKE et al.** *Mol. Pharmacol.,* 2003, vol. 63, 243-252
- **LLOYD.** *Protein Eng Des Sel,* 2009, vol. 22, 159-168
- **VAUGHAN et al.** *Nature biotechnology,* 1996, vol. 14, 309-314
- **PERSIC et al.** *Gene,* 1997, vol. 187, 9-18
- **OGANESYAN et al.** *Acta Crystallogr D Biol Crystallogr,* 2008, vol. 64, 700-704
- **PACE et al.** *Protein Sci,* 1995, vol. 4, 2411-23
- **DIETZ et al.** *Cytometry,* 1996, vol. 23, 177-186
- **MIRAGLIA et al.** *J. Biomol. Screening,* 1999, vol. 4, 193-204
- **OSBOURN.** *Immunotechnology,* 1996, vol. 2, 181-196
- **BANNISTER et al.** *Biotechnology and bioengineering,* 2006, vol. 94, 931-937
- **GRAM et al.** *Proc. Natl. Acad. Sci., USA,* 1992, vol. 89, 3576-3580